# FASCICULE DE BREVET EUROPEEN

(11) **EP 2 193 136 B1**
(45) Date de publication et mention de la délivrance du brevet: **30.07.2014**
(21) Numéro de dépôt: 08861269.2
(22) Date de dépôt: 01.10.2008
(51) Int. Cl.: C07F 15/02, C07F 19/00, A61K 9/14, A61K 47/08, A61K 49/18, G01N 33/58, A61P 17/00, A61K 8/30

(54) **SOLIDE HYBRIDE ORGANIQUE INORGANIQUE A SURFACE MODIFIEE**
ANORGANISCHER/ORGANISCHER FESTSTOFFHYBRID MIT MODIFIZIERTER OBERFLÄCHE
SOLID INORGANIC/ORGANIC HYBRID WITH MODIFIED SURFACE

(30) Priorité: 01.10.2007 FR 0706875
(43) Date de publication de la demande: 09.06.2010
(73) Titulaire: Centre National de la Recherche Scientifique (CNRS), 75794 Paris Cedex 16 (FR); Université de Versailles Saint-Quentin-en-Yvelines, 78053 Versailles Cédex (FR)
(72) Inventeur: HORCAJADA CORTES, Patricia, F-78190 Trappes (FR); FEREY, Gérard, F-75015 Paris (FR); SERRE, Christian, F-78370 Plaisir (FR); GREF, Ruxandra, F-91270 Verrières-le-Buisson (FR); COUVREUR, Patrick, F-91140 Villebon-sur-Yvette (FR)
(74) Mandataire: Novagraaf Technologies
(86) Numéro de dépôt international: PCT/FR2008/001367
(87) Numéro de publication internationale: WO 2009/077671

(56) Documents cités:
- HORCAJADA, PATRICIA ET AL: "Synthesis and catalytic properties of MIL - 100 ( Fe ), an iron(III) carboxylate with large pores" CHEMICAL COMMUNICATIONS (CAMBRIDGE, UNITED KINGDOM) , (27), 2820-2822 CODEN: CHCOFS; ISSN: 1359-7345, 2007, XP002476454
- SURBLE, SUZY ET AL: "A new isoreticular class of metal-organic-frameworks with the MIL-88 topology" CHEMICAL COMMUNICATIONS (CAMBRIDGE, UNITED KINGDOM) , (3), 284-286 CODEN: CHCOFS; ISSN: 1359-7345, 2006, XP002476456 cité dans la demande
- DATABASE CA [Online] CHEMICAL ABSTRACTS SERVICE, COLUMBUS, OHIO, US; HORCAJADA, PATRICIA ET AL: "MOFS as new materials for drug delivery" XP002476460 extrait de STN Database accession no. 2008:389294 & ABSTRACTS OF PAPERS, 235TH ACS NATIONAL MEETING, NEW ORLEANS, LA, UNITED STATES, APRIL 6-10, 2008 , INOR-828 PUBLISHER: AMERICAN CHEMICAL SOCIETY, WASHINGTON, D. C. CODEN: 69KNN3, 2008,
- HORCAJADA, PATRICIA ET AL: "Metal-organic frameworks as efficient materials for drug delivery" ANGEWANDTE CHEMIE, INTERNATIONAL EDITION , 45(36), 5974-5978 CODEN: ACIEF5; ISSN: 1433-7851, 2006, XP002476583
- DATABASE CA [Online] CHEMICAL ABSTRACTS SERVICE, COLUMBUS, OHIO, US; SHEHU, ERJOLA ET AL: "Design and synthesis of new extended metal organic frameworks ( MOFs )" XP002476732 extrait de STN Database accession no. 2004:983393 & ABSTRACTS, 56TH SOUTHEAST REGIONAL MEETING OF THE AMERICAN CHEMICAL SOCIETY, RESEARCH TRIANGLE PARK, NC, UNITED STATES, NOVEMBER 10-13 , GEN-771 PUBLISHER: AMERICAN CHEMICAL SOCIETY, WASHINGTON, D. C. CODEN: 69FWAQ, 2004,

## Description

### Domaine technique

La présente invention se rapporte à un solide de réseau métal-organique ou « Metal-Organic Framework » (MOF), cristallin poreux, ainsi, notamment, qu'à son procédé de préparation.

Le solide MOF de la présente invention est utilisable par exemple comme agent de contraste et/ou pour le transport de composés pharmaceutiques. Le solide de la présente invention peut également être utilisé pour des applications dans le domaine cosmétique. Il peut également être utilisé pour la vectorisation et/ou le suivi de composés pharmaceutiques dans un organisme. Il peut se présenter, par exemple, sous la forme de cristaux, de poudre, de particules ou de nanoparticules.

Les références entre crochets [X] renvoient à la liste des références à la fin des exemples.

### Etat de la technique

Les réseaux métal-organique ou « Metal-Organic Framework » (MOF) sont des polymères de coordination de charpente hybride inorganique-organique comprenant des ions métalliques et des ligands organiques coordinés aux ions métalliques. Ces matériaux sont organisés en réseau mono-, bi- ou tri-dimensionnels où les clusters métalliques sont reliés entre eux par des ligands espaceurs de façon périodique. Ces matériaux ont une structure cristalline, sont le plus souvent poreux et sont utilisés dans de nombreuses applications industrielles telles que le stockage de gaz, l'adsorption de liquides, la séparation de liquides ou de gaz, la catalyse, etc.

On peut citer par exemple la demande de brevet US 10/039,733 [1] qui décrit un procédé réactionnel faisant intervenir un système de catalyse comprenant un matériau MOF à base de Zinc. Ce même matériau est également utilisé pour le stockage de gaz dans la demande de brevet US 10/061,147 [2].

En outre, les matériaux MOF basés sur des réseaux de même topologie sont qualifiés de « isoréticulaires ». Ces réseaux organisés dans l'espace ont permis d'obtenir une porosité plus homogène. Ainsi, la demande de brevet US 10/137,043 [3] décrit plusieurs matériaux IRMOF (IsoReticular Metal-Organic Framework) à base de zinc utilisés pour le stockage de gaz.

Cependant, les nanoparticules sont difficiles à synthétiser, et notamment les nanoparticules de taille inférieure à 1000 nm, compte tenu de leur nature à s'agréger facilement et compte tenu de la tendance de ces matériaux à s'organiser en réseaux cristallins de grande taille (microns). Ceci entraîne également des problèmes d'inhomogénéité de taille des particules défavorables pour certaines applications.

De plus, la structure de ces matériaux et la topologie des éléments constituants n'ont pas vraiment été étudiées dans l'art antérieur. En outre, les structures ne sont pas toujours contrôlées de façon à obtenir des propriétés spécifiques telles qu'une taille de pores « sur mesure », adaptée aux molécules à adsorber, une structure flexible ou rigide, une surface spécifique et/ou une capacité d'adsorption améliorées, etc- En effet, il est difficile de contrôler l'organisation structurale et la porosité de ces matériaux.

Une des raisons de la difficulté de contrôle de l'organisation structurale est liée aux risques d'interpénétration des réseaux. En effet, au cours de la formation ou polymérisation des matériaux, les réseaux peuvent s'enchevêtrer l'un dans l'autre. L'augmentation du nombre de réseaux interpénétrés conduit à un matériau plus dense avec des pores réduits, d'où une structure inhomogène avec une porosité inadaptée et hétérogène.

Ainsi, des agents « de modelage » ont été utilisés pour l'obtention de structures « contrôlées » comme décrit dans le brevet US 5,648,508 [4]. Les ligands s'organisent autour du métal en encapsulant ces agents « de modelage » dans des cavités ou pores. Cependant, ces agents interagissent fortement avec le matériau MOF, rendant difficile voire impossible leur élimination sans endommager le réseau, ce qui conduit à un solide dont les pores sont déjà occupés par ces agents.

Mais l'utilisation de ces agents ajoute du matériel chimique lors de la synthèse des particules ce qui rend le procédé plus complexe et plus coûteux. En outre, pour des applications médicales, ce modelage semble difficilement adaptable.

Il reste donc de nombreuses améliorations à faire en termes de contrôle de la structure et de la taille de particules afin d'obtenir des matériaux appropriés à chaque application, avec des porosités, des tailles de particules, des capacités de charge homogènes et adaptées.

Par ailleurs, l'utilisation de transporteur et vecteurs de molécules d'intérêt, notamment des molécules à effet thérapeutique ou des marqueurs, est devenu un enjeu important pour le développement de nouvelles méthodes de diagnostique ou de nouveaux médicaments. En effet, les molécules d'intérêt présentent des caractéristiques qui ont une influence sur la pharmacocinétique et la biodistribution de ces molécules et qui ne sont pas toujours favorables ou adaptables vis-à-vis du milieu dans lequel elles sont introduites. Ce sont par exemple des caractéristiques physico-chimiques telles que l'instabilité, la forte tendance à la cristallisation, la faible hydrosolubilité et/ou des caractéristiques biologiques telles que la toxicité, la biodégradabilité, etc.

À titre d'exemple, de nombreux agents anticancéreux ont un index thérapeutique limité par leur activité cytotoxique importante.

L'index thérapeutique peut également être limité par une faible solubilité et une forte tendance à la cristallisation de principes actifs. Ceci peut non seulement conduire à un ralentissement de la dissolution et de l'absorption des principes actifs mais également à un risque d'obstruction vasculaire partielle ou totale par la formation de particules cristallines *in situ* après administration. C'est le cas notamment d'agents anticancéreux alkylants tels que le busulfan qui possèdent des groupements chimiques qui ont une forte tendance à s'auto associer, à travers des interactions hydrophobes ou polaires conduisant à la cristallisation spontanée de ces molécules. Il importe donc d'éviter ce phénomène de cristallisation, par exemple lors de la vectorisation de tels principes actifs.

L'instabilité des principes actifs pose également un problème d'efficacité thérapeutique. En effet, certains principes sont rapidement éliminés par le système immunitaire ou captés par les organes du système réticulo-endothélial (principalement, le foie et la rate). C'est notamment le cas du busulfan, qui est majoritairement capté par le foie dans les 10 à 30 minutes après administration orale ou intraveineuse et qui peut être responsable de l'apparition de la maladie veino-occlusive du foie pour laquelle il n'existe pas de traitement.

Ainsi, divers matériaux, comme par exemple des liposomes ou divers polymères ont été développés pour le transport de composés actifs. Notamment, des vecteurs « furtifs », faiblement reconnus par le système immunitaire et/ou capables d'éviter la capture par ces organes ont été développés afin d'encapsuler et/ou de vectoriser des principes actifs instables et/ou toxiques.

L'article Bone Marrow Transplant. 2002.30 (12), 833-841 [6] décrit par exemple des vecteurs colloïdaux, notamment chargés en busulfan. Malheureusement, le taux d'encapsulation du busulfan est faible, atteignant tout juste 0,5 % en poids du poids total de liposomes. De plus, de tels vecteurs colloïdaux à base de liposomes présentent une faible durée de vie dans le milieu plasmatique due à la dissociation spontanée et à la dégradation métabolique rapide de ces structures lipidiques. Ceci implique une faible efficacité thérapeutique et des volumes importants de dispersions liposomales parfois incompatibles avec les dosages nécessaires au traitement.

Pour pallier ce problème de la stabilité intra-plasmatique, des vecteurs colloïdaux solides à base de polymères non hydrosolubles ont été développés. Ils se présentent sous la forme de nanoparticules polymériques biodégradables et les principes actifs qu'ils peuvent transporter sont libérés progressivement, par diffusion et/ou au fur et à mesure de la dégradation métabolique des nanoparticules. C'est le cas des polymères de la famille des poly(cyanoacrylates d'alkyle), comme décrits dans le brevet US 4,329,332 [7], utilisés pour le transport de produits toxiques et/ou instables.

Cependant, ces nanoparticules présentent un faible taux d'encapsulation. En outre, le taux d'encapsulation dépend de la nature du principe actif à encapsuler dans les nanoparticules de poly(cyanoacrylates d'alkyle). En effet, dans le cas de principes actifs cristallins, faiblement solubles dans l'eau et/ou hydrophobes, ils ont tendance à précipiter et cristalliser dans la phase aqueuse dispersante du procédé de polymérisation *in* situ utilisé pour obtenir ces nanoparticules. Ceci rend l'encapsulation de tels principes actifs difficile avec des taux d'encapsulation au sein de nanoparticules poly(cyanoacrylate d'alkyle) faibles, de l'ordre de 0,1 à 1 % en poids de la masse de polymère engagée.

De plus, de tels vecteurs sont peu adaptés à l'encapsulation de principes actifs très réactifs comme le busulfan par exemple. En effet, dans la mesure où ces vecteurs sont fabriqués par polymérisation in situ en présence du principe actif, celui-ci risque de réagir avec les unités monomériques, empêchant une polymérisation adéquate nécessaire à la réalisation des nanoparticules.

Ainsi, de nombreuses molécules restent difficiles, voire impossibles à encapsuler en raison de leur tendance à la cristallisation, leur faible solubilité dans les solvants et/ou leur instabilité.

En outre, le busulfan pose un véritable défi quant à son encapsulation. Des vecteurs furtifs évitant le foie n'ont pas permis d'atteindre des objectifs d'encapsulation satisfaisants du fait des faibles charges possibles en busulfan. Les charges maximales obtenues avec les liposomes ne dépassent pas les 0,5% en poids.

Plus récemment, l'utilisation de nanoparticules de polymères biodégradables à base de poly(alkyle-cyanoacrylate) a permis d'améliorer le taux d'encapsulation de Busulfan jusqu'à environ 5% en poids, comme décrit dans J. Bouligand, et al., Int. J. Pharm., 2004 [8]. Cependant, lors des administrations répétées des nanoparticules, une forte accumulation de polymères dans l'organisme peut être dramatique, par exemple en chimiothérapie haute dose.

Shehu et al., Abstracts, 56th Southeast Regional Meeting of the American Chemical Society, November 10-13 (2004), GEN-771, décrit des solides MOF cristallins poreux carboxylates de métal, dans lesquels le métal peut être Ni(II), Tb(III) ou Eu(III). Horcajada et al., Angew. Chem. Int. Ed., 45(36), 5974-5978, 2006 décrit des MOFs cristallins poreux isoréticulaires carboxylates de chrome de formule MIII3OX(H2O)2(C6H3(CO2)3) et de structure MIL-100 et MIL-101.

Il existe donc un réel besoin de disposer de composés améliorés capables d'échapper au système immunitaire et/ou à leur capture par certains organes, par exemple le foie, évitant ainsi leur accumulation dans ces organes.

Il existe également un réel besoin de disposer de composés capables de vectoriser des principes actifs vers des cibles spécifiques.

Il existe en outre un réel besoin de disposer de nouveaux composés capables de transporter et des principes actifs, par exemple des principes actifs présentant des difficultés particulières d'encapsulation liées à leur instabilité, leur forte tendance à cristalliser, leur faible solubilité, leur caractère amphiphile, hydrophile, etc. En outre, il existe un besoin de disposer de nouveaux composés avec des capacités de charge suffisantes surtout si l'on envisage des administrations répétées de nanoparticules.

De plus, il existe un réel besoin de disposer de composés permettant de libérer de façon contrôlée des principes actifs.

### Exposé de l'invention

La présente invention a précisément pour but de répondre à ces besoins et inconvénients de l'art antérieur en fournissant un solide MOF cristallin poreux comprenant une succession tridimensionelle de motifs répondant à la formule (I) suivante :

MₘOₖXₗLₚ Formule (I)

dans laquelle :
- M est un ion métallique Fe²⁺ ou Fe³⁺,
- m, k, 1 et p sont des nombres - 0 choisis de façon à respecter la neutralité des charges du motif ; de préférence, m, k, 1 et p sont indépendamment 0 à 4, par exemple m et p sont indépendamment 1, 2 ou 3 et/ou k et l sont indépendamment 0 ou 1 ;
- X est un ligand choisi dans le groupe comprenant OH⁻, Cl⁻, F⁻, I⁻, Br⁻, SO₄²⁻, NO₃⁻, ClO₄⁻, R¹-(COO)ₙ⁻, R¹-(S0₃)ₙ⁻, R¹-(PO₃)ₙ⁻, où R¹ est un hydrogène, un alkyle en C₁ à C₈, linéaire ou ramifié, n = 1 à 6 ;
- L est un ligand espaceur di-, tri-, tétra- ou hexa-carboxylate choisi dans le groupe constitué de:

| | | |
|---|---|---|
| 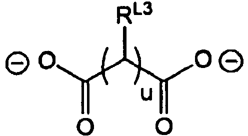 | | 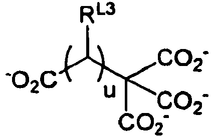 |
| 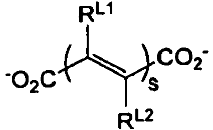 | | 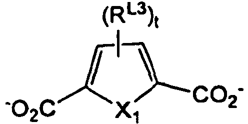 |
| 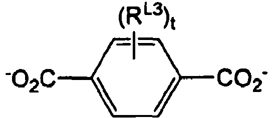 | | 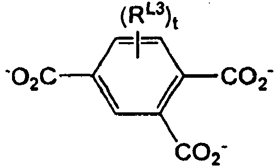 |
| 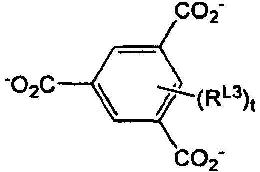 | | 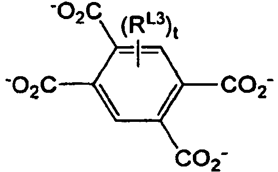 |
| 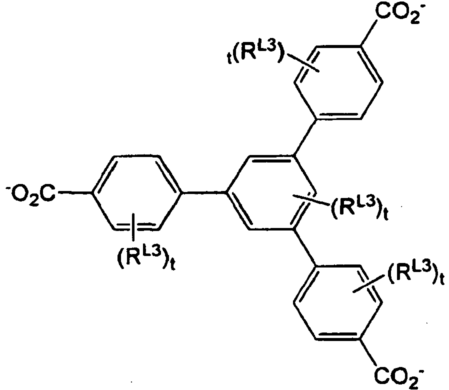 | | 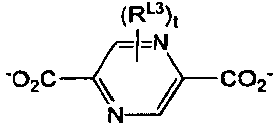 |
| 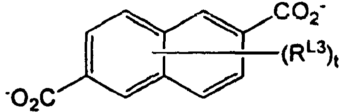 | | 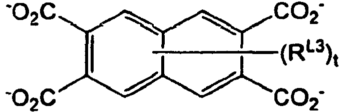 |
| 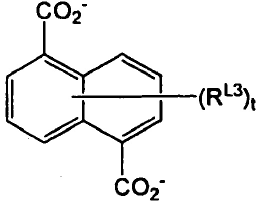 | | 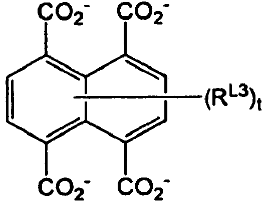 |
| 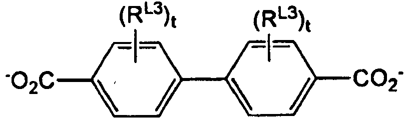 | | |
| 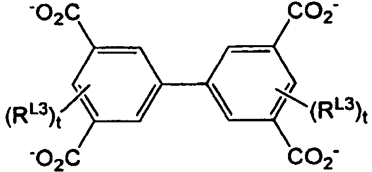 | | |
| 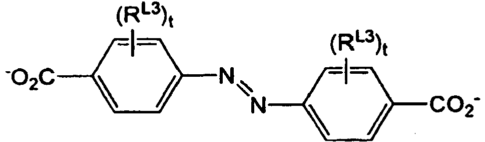 | | |
| 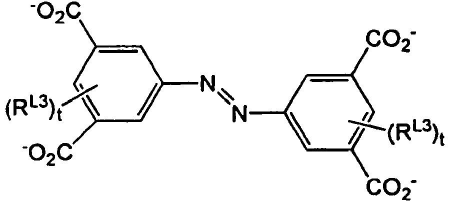 | | |
| 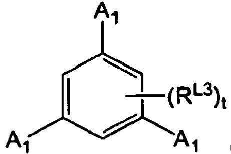 | où A₁, A₂ et A₃ représentent indépendamment | |
| 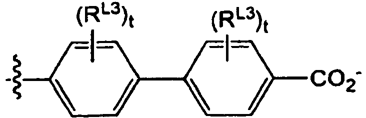 | | |

dans lesquels :
X₁ représente O ou S,
s représente un entier de 1 à 4,
chaque occurrence de t représente indépendamment un entier de 1 à 4,
u représente un entier de 1 à 7,
R^{L1} et R^{L2} représentent indépendamment H, un halogène ou un alkyle en C₁ à C₆, et
chaque occurrence de R^{L3} représente indépendamment H, un halogène, OH, NH₂, NO₂ ou un alkyle en C₁ à C₆;
dans lequel la surface est modifiée en ce qu'elle comprend au moins un agent de surface organique choisi dans le groupe comprenant un oligosaccharide, un polysaccharide, un glycosaminoglycanne, un polymère, un tensioactif, une vitamine, un coenzyme, un anticorps ou fragment d'anticorps, un aminoacide ou un peptide.

Diverses occurrences de M dans les motifs de formule (I) peuvent être identiques ou différentes sont décrites. Ainsi, l'expression « M est un ion métallique choisi dans le groupe comprenant Fe²⁺, Fe³⁺, Zn²⁺, Ti³⁺, Ti⁴⁺, Zr²⁺, Zr⁴⁺ Ca²⁺, Cu²⁺, Gd³⁺, Mg²⁺, Mn²⁺, Mn³⁺, Mn⁴⁺, Si⁴⁺» figurant ci-dessus et dans le présent document est équivalente à l'expression : « chaque occurrence de M représente indépendamment un ion métallique choisi dans le groupe comprenant Fe²⁺, Fe³⁺, Zn²⁺, Ti³⁺, Ti⁴⁺, Zr²⁺, Zr⁴⁺ Ca²⁺, Cu²⁺, Gd³⁺, Mg²⁺, Mn²⁺, Mn³⁺, Mn⁴⁺, Si⁴⁺».

Dans certains modes de réalisation, M représente Fe²⁺, Fe³⁺, Ti³⁺, Ti⁴⁺, Zr²⁺ ou Zr⁴⁺.

On entend par « solide » au sens de la présente invention tout type de matériau cristallin. Ledit solide peut par exemple se présenter sous forme de cristaux, de poudre, de particules de formes variées, par exemple de forme sphérique, lamellaire, etc. Les particules peuvent être sous la forme de nanoparticules.

Par « nanoparticule », on entend une particule de taille inférieure à 1µm. En particulier, les nanoparticules de solide MOF selon l'invention peuvent avoir un diamètre inférieur à 1000 nanomètres, de préférence inférieur à 500 nm, de manière plus préférée inférieur à 250 nm, tout particulièrement inférieur à 100 nm.

Le terme « substitué » désigne par exemple le remplacement d'un radical hydrogène dans une structure donnée par un radical R² tel que défini précédemment. Lorsque plus d'une position peut être substituée, les substituants peuvent être les mêmes ou différents à chaque position.

On entend par « ligand espaceur », un ligand (incluant par exemple les espèces neutres et les ions) coordiné à au moins deux métaux, participant à l'éloignement entre ces métaux et à la formation d'espaces vides ou pores. Le ligand espaceur peut comprendre 1 à 6 groupements fonctionnels A, tels que définis précédemment, qui peuvent être monodentates ou bidentates, c'est à dire comprendre 1 ou 2 points d'attachement au métal. Les points d'attachement au métal sont représentés par le sigle # dans les formules. Lorsque la structure d'une fonction A comporte 2 #, cela signifie que la coordination au métal peut se faire par l'un, l'autre ou les deux points d'attachement.

On entend par « alkyle » au sens de la présente invention, un radical carboné linéaire, ramifié ou cyclique, saturé ou insaturé, éventuellement substitué, comprenant 1 à 12 atomes de carbone, par exemple 1 à 10 atomes de carbone, par exemple 1 à 8 atomes de carbone, par exemple 1 à 6 atomes de carbone.

On entend par « alcène » au sens de la présente invention, un radical alkyle, tel que défini précédemment, présentant au moins une double liaison carbone-carbone.

On entend par « alcyne » au sens de la présente invention, un radical alkyle, tel que défini précédemment, présentant au moins une triple liaison carbone-carbone.

On entend par « aryle » au sens de la présente invention, un système aromatique comprenant au moins un cycle satisfaisant la règle d'aromaticité de Hückel. Ledit aryle est éventuellement substitué et peut comprendre de 6 à 50 atomes de carbone, par exemple 6 à 20 atomes de carbone, par exemple 6 à 10 atomes de carbone.

On entend par « hétéroaryle » au sens de la présente invention, un système comprenant au moins un cycle aromatique de 5 à 50 chaînons parmi lesquels au moins un chaînon du cycle aromatique est un hétéroatome, notamment choisi dans le groupe comprenant le soufre, l'oxygène, l'azote, le bore. Ledit hétéroaryle est éventuellement substitué et peut comprendre de 1 à 50 atomes de carbone, de préférence 1 à 20 atomes de carbone, de préférence 3 à 10 atomes de carbone.

On entend par « cycloalkyle » au sens de la présente invention, un radical carboné cyclique, saturé ou insaturé, éventuellement substitué, qui peut comprendre 3 à 20 atomes de carbone, de préférence 3 à 10 atomes de carbone.

On entend par « haloalkyle » au sens de la présente invention, un radical alkyle tel que défini précédemment, ledit système alkyle comprenant au moins un halogène.

On entend par « hétéroalkyle » au sens de la présente invention, un radical alkyle tel que défini précédemment, ledit système alkyle comprenant au moins un hétéroatome, notamment choisi dans le groupe comprenant le soufre, l'oxygène, l'azote, le bore.

On entend par « hétérocycle » au sens de la présente invention, un radical carboné cyclique comprenant au moins un hétéroatome, saturé ou insaturé, éventuellement substitué, et qui peut comprendre 2 à 20 atomes de carbone, de préférence 5 à 20 atomes de carbone, de préférence 5 à 10 atomes de carbone. L'hétéroatome peut être par exemple choisi dans le groupe comprenant le soufre, l'oxygène, l'azote, le bore.

On entend par « alkoxy », « aryloxy », « hétéroalkoxy » et « hétéroaryloxy » au sens de la présente invention, respectivement un radical alkyle, aryle, hétéroalkyle et hétéroaryle liés à un atome d'oxygène.

On entend par « alkylthio », « arylthio », « hétéroalkylthio » et « hétéroarylthio » au sens de la présente invention, respectivement un radical alkyle, aryle, hétéroalkyle et hétéroaryle liés à un atome de soufre.

On entend par « base de schiff », un groupement fonctionnel qui contient une double liaison C=N de formule générale R^{X1}R^{X2}-C=N-R^{X3}, avec R^{X1}, R^{X2} et R^{X3} sont tels que définis précédemment.

Par « structure tridimensionnelle », on entend une succession ou répétition tridimensionnelle de motifs de formule (I) tel que l'on entend de façon conventionnelle dans le domaine des matériaux MOFs, que l'on caractérise également comme « polymères métallo-organiques ».

Sauf indication contraire, les divers modes de réalisation qui suivent concernant les matériaux MOF s'appliquent autant à l'utilisation qu'au procédé de l'invention précités.

On entend par « agent de surface » selon l'invention une molécule recouvrant en partie ou en totalité la surface du solide permettant de moduler les propriétés de surface du matériau, par exemple :
- de modifier sa biodistribution, par exemple pour éviter sa reconnaissance par le système réticulo endothélial (« furtivité »), et/ou
- de lui conférer des propriétés de bioadhésion intéressantes lors de l'administration par voies orale, oculaire, nasale, et/ou
- de lui permettre un ciblage spécifique de certaines organes/tissus malades, etc.

Selon l'invention, plusieurs agents de surface peuvent être utilisés pour combiner les propriétés précitées.

Selon l'invention un agent de surface combinant au moins deux des propriétés précitées peut être utilisé.

Selon l'invention, l'agent de surface organique peut être choisi par exemple dans le groupe comprenant :
- un oligosaccharide comme les cyclodextrines,
- un polysaccharide comme le chitosan, le dextran, le fucoïdane, l'alginate, la pectine, l'amylose, l'amidon, la cellulose, le xylane,
- un glycosaminoglycanne comme l'acide hyaluronique, l'héparine,
- un polymère comme le polyéthylène glycol (PEG), l'alcool polyvinylique, le polyéthylène imine,
- un tensioactif comme le pluronic, la lécithine.
- les vitamines comme la biotine
- les coenzymes comme l'acide lipoique
- les anticorps ou fragment d'anticorps
- les aminoacides ou peptides.

En outre, le greffage d'un agent de surface à la surface du solide MOF selon l'invention permet de répondre aux besoins liés à la vectorisation du composé vers des cibles biologiques spécifiques et/ou à la furtivité du matériau. Ceci permet de moduler la biodistribution du matériau.

Selon l'invention, l'agent de surface peut être greffé ou déposé à la surface du solide selon l'invention, par exemple adsorbé en surface ou lié par liaison covalente, par liaison hydrogène, par liaison de Van der Waals, par interaction électrostatique. L'agent de surface peut également être incorporé par enchevêtrement lors de la fabrication du solide.

L'agent de surface peut être par exemple un agent de surface phosphaté incorporé pendant ou après la synthèse du solide.

L'agent de surface peut également être une molécule de ciblage, c'est-à-dire une molécule reconnaissant ou étant reconnue spécifiquement par une cible biologique. L'association des solides MOF de l'invention avec une molécule de ciblage permet ainsi de diriger les nanoparticules selon l'invention, et donc de vectoriser les principes actifs, vers cette cible biologique, cellule, tissus, organe.

De préférence, le solide selon l'invention peut comprendre au moins une molécule de ciblage, comme agent de surface organique, qui peut être choisie dans le groupe comprenant : la biotine, l'avidine, l'acide folique, l'acide lipoique, l'acide ascorbique, un anticorps ou fragment d'anticorps, un peptide, une protéine.

Ainsi, l'agent de surface organique peut être une molécule de ciblage choisie dans le groupe comprenant la biotine, le chitosan, l'acide lipoique, un anticorps ou fragment d'anticorps, un peptide.

Par exemple, la présence de la biotine en surface peut être mise à profit afin de coupler des ligands de manière aisée, par exemple par simple incubation. Pour cela, il est possible d'utiliser des protocoles décrits dans les publications S. Balthasar et al., Biomaterials, Volume 26, Issue 15, May 2005, 2723-2732 [17] et R. Gref et al., Biomaterials, Volume 24, Issue 24, November 2003, 4529-4537 [18].

Un autre ligand peut être utilisé à la place de la biotine, par exemple l'acide folique. Ce ligand présente un intérêt certain dans le domaine du cancer ainsi que le montrent les publications [17] et [18] précitées.

Cette méthode de modification de surface présente l'avantage de ne pas perturber le coeur des solide MOF, en particulier lorsqu'ils contiennent du gaz, et de pouvoir se faire pendant ou après la synthèse des solides MOF, donc d'offrir une variété de recouvrements possibles.

Il est également possible d'utiliser un mélange de polymères portant des fonctions susceptibles d'interagir avec la particule (MOF) en tant qu'agent de surface afin de répondre à un cahier de charge précis, par exemple la bioadhésion, la reconnaissance spécifique, etc.

Parmi les agents de surface permettant un ciblage spécifique de certains organes/tissus malades, on peut citer par exemple, les vitamines (biotine, acide folique, acide lipoique, acide ascorbique), les anticorps ou fragment d'anticorps, les peptides, les protéines.

Selon l'invention, l'agent de surface peut être choisi par exemple dans le groupe comprenant un oligosaccharide, un polysaccharide, le chitosan, le dextran, l'acide hyaluronique, l'héparine, le fucoïdane, l'alginate, la pectine, l'amilose, les cyclodextrines, l'amidon, la cellulose, le xylane, un polymère ou un copolymère, par exemple le polyéthylène glycol (PEG), le pluronic, l'alcool polyvinylique, le polyéthylène imine, etc.

Par ailleurs, le solide MOF selon l'invention a l'avantage de pouvoir avoir une structure cristalline contrôlée, avec une topologie et une distribution particulière, qui procure à ce matériau des propriétés spécifiques. Ces propriétés spécifiques se retrouvent dans les différentes formes du solide MOF de la présente invention mentionnées ci-dessus.

Dans la présente, un solide MOF décrit peut comprendre des atomes de métaux di-, tri- ou tétravalents. Les atomes métalliques peuvent avoir une géométrie octaédrique, pentaédrique, tétraédrique, voire être en coordinence supérieure dans la structure du matériau.

Par « coordinence » ou « nombre de coordination », on entend le nombre de liaisons pour laquelle les deux électrons partagés dans la liaison proviennent du même atome. L'atome donneur d'électrons acquiert une charge positive alors que l'atome accepteur d'électrons acquiert une charge négative.

En outre, les atomes métalliques peuvent être isolés ou regroupés en « clusters » métalliques. Le solide MOF selon l'invention peut par exemple être construit à partir de chaînes de polyèdres, de dimères, trimères ou tétramères de polyèdres. Par exemple, le solide MOF selon l'invention peut être construit à partir de chaînes d'octaèdres, de dimères, trimères ou tétramères d'octaèdres. Par exemple, les matériaux MOF carboxylate de fer selon l'invention peuvent être construits à partir de chaines d'octaèdres liés par les sommets ou les arêtes ou de trimères d'octaèdres connectés par un atome d'oxygène central.

On entend par « cluster métallique » au sens de la présente invention un ensemble d'atomes contenant au moins deux métaux liés par des liaisons ionocovalentes, soit directement par des anions, par exemple O, OH, Cl, etc., soit par le ligand organique.

De plus, le solide MOF selon l'invention peut se présenter sous différentes formes ou « phases » compte tenu des divers possibilités d'organisation et de connections des ligands au métal ou au groupement métallique.

On entend par « phase » au sens de la présente invention une composition hybride comprenant au moins un métal et au moins un ligand organique possédant une structure cristalline définie.

L'organisation spatiale cristalline du solide de la présente invention est à la base des caractéristiques et propriétés particulières de ce matériau, et régit notamment la taille des pores, qui a une influence sur la surface spécifique du matériau et sur les caractéristiques d'adsorption, mais également la densité du matériau, celle-ci étant relativement faible, la proportion de métal dans ce matériau, la stabilité du matériau, la rigidité et la flexibilité de sa structure, etc.

Le solide MOF selon l'invention peut être isoréticulaire, c'est à dire comprendre des réseaux de même topologie.

En outre, le solide de la présente invention peut comprendre des motifs qui contiennent soit un seul type d'ion métallique, soit plusieurs types d'ions métalliques.

Par exemple, le solide de la présente invention peut comprendre une succession tridimentionnelle de trois motifs différents. Par exemple également, le solide de la présente invention peut comprendre une succession tridimentionnelle de deux motifs différents.

En outre, la taille des pores peut être ajustée par le choix de ligands espaceurs appropriés.

Le ligand L du motif de formule (I) des solides MOF de la présente invention est un ligand di-, tri-, tétra- ou hexa-carboxylate choisi dans le groupe comprenant :

| | | |
|---|---|---|
| 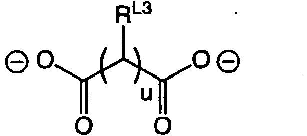 | | 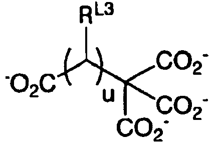 |
| 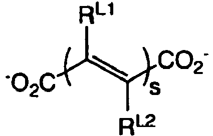 | | 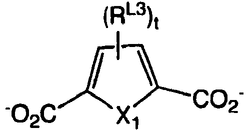 |
| 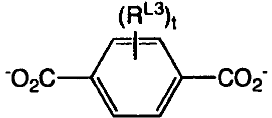 | | 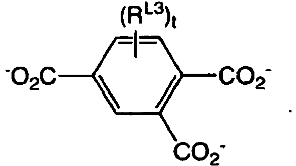 |
| 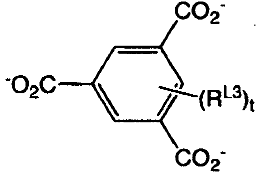 | | 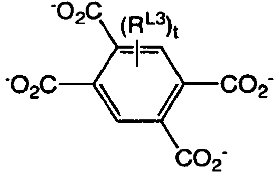 |
| 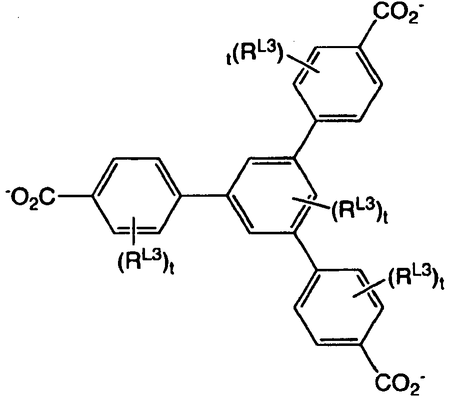 | | 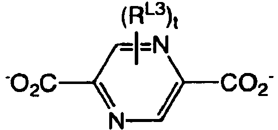 |
| 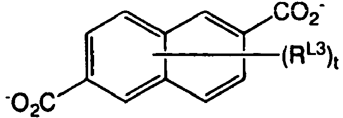 | | 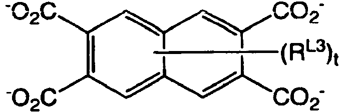 |
| 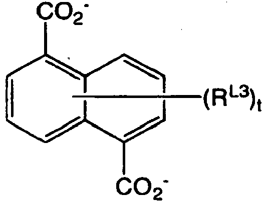 | | 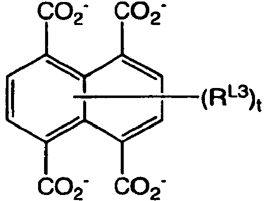 |
| 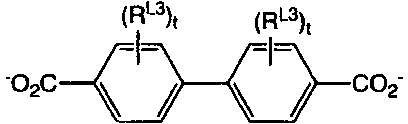 | | |
| 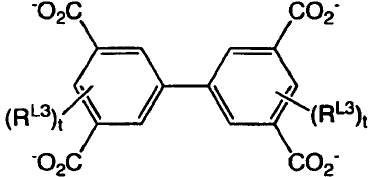 | | |
| 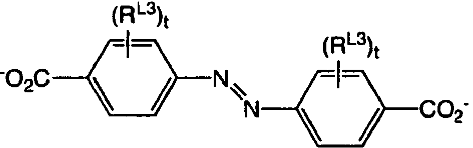 | | |
| 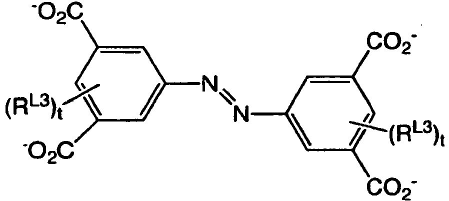 | | |
| 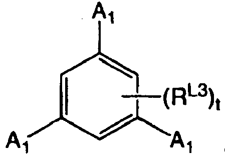 | où A₁, A₂ et A₃ représentent | |
| indépendamment | 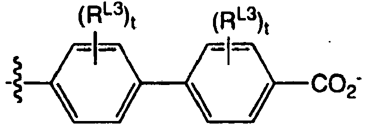 | |

dans lesquels :
X₁ représente O ou S,
s représente un entier de 1 à 4,
chaque occurrence de t représente indépendamment un entier de 1 à 4,
u représente un entier de 1 à 7,
R^{L1} et R^{L2} représentent indépendamment H, un halogène ou un alkyle en C₁ à C₆ (de préférence méthyle ou éthyle), et
chaque occurrence de R^{L3} représente indépendamment H, un halogène (de préférence F, Cl ou Br), OH, NH₂, NO₂ ou un alkyle en C₁ à C₆ (de préférence méthyle ou éthyle).

En particulier, le ligand L du motif de formule (I) de la présente invention peut être un ligand di-, tri- ou tétra- carboxylate choisi dans le groupe comprenant : C₂H₂(CO₂⁻)₂ (fumarate), C₂H₄(CO₂⁻)₂ (succinate), C₃H₆(CO₂⁻)₂ (glutarate), C₄H₄(CO₂⁻)₂ (muconate), C₄H₈(CO₂⁻)₂ (adipate), C₇H₁₄(CO₂⁻)₂ (azelate), C₅H₃S(CO₂⁻)₂ (2,5-thiophènedicarboxylate), C₆H₄(CO₂⁻)₂ (téréphtalate), C₆H₂N₂(CO₂⁻)₂ (2,5-pyrazine dicarboxylate), C₁₀H₆(CO₂⁻)₂ (naphtalène-2,6-dicarboxylate), C₁₂H₈(CO₂⁻)₂ (biphényle-4,4'-dicarboxylate), C₁₂H₈N₂(CO₂⁻)₂ (azobenzènedicarboxylate), C₆H₃(CO₂⁻)₃ (benzène-1,2,4-tricarboxylate), C₆H₃(CO₂⁻)₃ (benzène-1,3,5-tricarboxylate), C₂₄H₁₅(CO₂⁻)₃ (benzène-1,3,5-tribenzoate), C₆H₂(CO₂⁻)₄ (benzène-1,2,4,5-tétracarboxylate, C₁₀H₄(CO₂⁻)₄ (naphtalène-2,3,6,7-tétracarboxylate), C₁₀H₄(CO₂⁻)₄ (naphtalène-1,4,5,8-tétracarboxylate), C₁₂H₆(CO₂⁻)₄ (biphényl-3,5,3',5'-tétracarboxylate), et les analogues modifiés choisis dans le groupe comprenant le 2-aminotéréphtalate, le 2-nitrotéréphtalate, le 2-méthyltéréphtalate, le 2-chlorotéréphtalate, le 2-bromotéréphtalate, le 2,5-dihydroxotéréphtalate, le tétrafluorotéréphtalate, le tétraméthyltéréphtalate, le diméthyl-4,4'-biphénydicarboxylate, le tétraméthyl-4,4'-biphénydicarboxylate, le dicarboxy-4,4'-biphénydicarboxylate, le 2,5-pyrazyne dicarboxylate. Le ligand L du motif de formule (I) de la présente invention peut également représenter le 2,5 diperfluoroterephthalate, azobenzene 4,4'-dicarboxylate, 3,3'-dichloro azobenzene 4,4'-dicarboxylate, 3,3'-dihydroxo azobenzene 4,4'-dicarboxylate, 3,3'-diperfluoro azobenzene 4,4'-dicarboxylate, 3,5,3',5'-azobenzene tetracarboxylate, 2,5-dimethyl terephthalate, perfluorosuccinate, perfluoromuconate, perfluoro glutarate, 3,5,3',5' perfluoro-4,4'-azobenzene dicarboxylate, 3,3'-diperfluoro azobenzene 4,4'-dicarboxylate.

La plupart des ligands listés ci-dessus sont commerciaux. Le lecteur pourra se référer à la partie Exemples pour la préparation des ligands non commerciaux.

Dans un mode de réalisation, le ligand L présente une activité biologique. Les solides hybrides nanoporeux selon l'invention possèdent une partie minérale, le métal (fer), et une partie organique, un ligand avec deux ou plusieurs fonctions complexantes (carboxylate, phosphate, amide, etc). L'incorporation de ligands organiques qui possèdent une activité biologique a l'avantage de permettre une libération contrôlée de molécules actives en fonction de la vitesse de dégradation du matériau (il s'agit des ligands biologiquement actifs précités qui sont libérés lors de la dégradation du matériau MOF). Ainsi, le matériau MOF lui-même est « bioactif », c'est-à-dire qu'il est susceptible de libérer des composants ayant une activité biologique.

En outre, la libération de ces molécules actives qui font partie du réseau MOF peut être combinée avec la libération d'autres principes actifs encapsulés dans les solides MOF selon l'invention. Cet aspect d'encapsulation de principes actifs est décrit *infra* dans le présent document.

Ainsi, la présente invention concerne également des solides MOF comprenant des ligands biologiquement actifs et encapsulant un ou plusieurs principes actifs, avec une activité peut-être complémentaire ou différente, et leur utilisation pour thérapies combinées. La thérapie combinée est mise en oeuvre par relargage (i) du principe actif encapsulé dans les pores du matériau MOF et (ii) des ligands biologiquement actifs incorporés dans le réseau (la charpente) du matériau MOF cristallin.

Il existe de nombreuses molécules organiques biologiquement actives comprenant des fonctions complexantes, susceptibles de former solides hybrides poreux selon la présente invention.

Par exemple, il peut s'agir de l'acide azelaique (HO₂C(CH₂)₇CO₂H, agent dermatologique avec une activité antinéoplasique), du meprobamate (anticonvulsivante, sédatif, relaxant musculaire, anti-anxiété), de l'acide aminosalicylique (antituberculose), du chlodronate, pamidrontate, alendronate et etidronate (antinéoplasique osée, prophylactique d'Osteoporosis), des azobenzènes (activité antimicrobienne, inhibiteurs de la COX), des porphyrines ou des aminoacides (Lys, Arg, Asp, Cys, Glu, Gln, etc.), de l'acide dibenzofuran-4,6-dicarboxylique (inhibiteur de transtryretin), de l'acide dipicolinique (inhibiteur de la dihydrodipicolinate reductase), de l'acide glutamique, de l'acide fumarique, de l'acide succinique, de l'acide subérique, de l'acide adipique, de l'acide nicotinique, du nicotinamide, de purines, pyrimidines....

Citons par exemple, l'activité antimicrobienne ou anti-inflammatoire (NSAIDs, inhibiteurs COX) des azobenzenes. À ce titre, le lecteur pourra se référer aux références suivantes : G. Oros, T. Cserhati, E. Forgacs, Chemosphere 52, 2003, 185 [ref 35], A.M. Badawi, E.M.S. Azzam, S.M.I. Morsy, Bioorg. Med. Chem., 14, 2006, 8661 [ref 36] et W-J. Tsai, Y-J Shiao, S-J Lin, W-F Chiou, L-C Lin, T-H Yang, C-M teng, T-S Wu, L-M Yang, Bioorg. Med. Chem. Letters 16, 2006, 4440 [ref 37].

Ainsi, le ligand L peut être un ligand biologiquement actif choisi dans le groupe comprenant C₇H₁₄(CO₂⁻)₂ (azelate) ; aminosalicylate (groupes carboxylique, amino et hydroxo) ; chlodronate, pamidrontate, alendronate et etidronate (comportant des groupes phosphonate) ; meprobamate (comportant des groupes carbamate) ; des porphyrines comprenant des groupes carboxylates, phosphonates et/ou amino ; des aminoacides (Lys, Arg, Asp, Cys, Glu, Gln, etc.) qui comportent des groupes amino, carboxylate, amide et/ou imine ; des azobenzènes comprenant des groupes carboxylates, phosphonates, et/ou amino ; le dibenzofuran-4,6-dicarboxylate, le dipicolinate (ligand mixte de type pyridine avec des groupes carboxyliques) ; le glutamate, le fumarate, le succinate, le suberate, l'adipate, le nicotinate, nicotinamide, purines, pyrimidines...

Des solides hybrides poreux à base de fer et de ligands azobenzene, ainsi que la démonstration de leur activité antimicrobienne, l'étude de leur dégradation dans des milieux physiologiques et leur activité sur des cellules, sont décrits dans la partie « Exemples ».

L'anion X du motif de formule (I) de la présente invention peut être choisi dans le groupe comprenant OH⁻, Cl⁻, Br⁻, F⁻, R-(COO)ₙ⁻, PF₆⁻, NO₃⁻, SO₄²⁻, ClO₄⁻, avec R et n tels que définis précédemment.

En particulier, l'anion X du motif de formule (I) de la présente invention peut être choisi dans le groupe comprenant OH⁻, Cl⁻, F⁻, CH₃-COO⁻, PF₆⁻, ClO₄⁻, ou alors un ligand carboxylate choisi dans la liste ci-dessus.

Dans un mode de réalisation particulier, l'anion X peut être choisi dans le groupe comprenant OH⁻, Cl⁻, F⁻ et R-(COO)ₙ⁻ où R représente -CH₃, -C₆H₃,-C₆H₄, -C₁₀H₄ ou -C₆(CH₃)₄.

Dans un mode de réalisation, l'anion X peut être sous une forme isotopique adaptée aux techniques d'imagerie telles que la tomograhie par émission de positrons (TEP).

La **tomographie par émission de positrons (TEP)** est une méthode d'imagerie médicale nucléaire qui permet de mesurer en trois dimensions l'activité métabolique d'un organe grâce aux émissions produites par les positrons issus de la désintégration d'un produit radioactif injecté au préalable. La TEP repose sur le principe général de la scintigraphie qui consiste à injecter un traceur dont on connaît le comportement et les propriétés biologiques pour obtenir une image du fonctionnement d'un organe. Ce traceur est marqué par un atome radioactif (carbone, fluor, azote, oxygène...) qui émet des positrons dont l'annihilation produit elle-même deux photons. La détection de la trajectoire de ces photons par le collimateur de la caméra TEP permet de localiser le lieu de leur émission et donc la concentration du traceur en chaque point de l'organe. C'est cette information quantitative que l'on représente sous la forme d'une image faisant apparaitre en couleurs les zones de forte concentration du traceur.

Ainsi la TEP permet de visualiser les activités du métabolisme des cellules : on parle d'imagerie fonctionnelle par opposition aux techniques d'imagerie dite structurelle comme celles basées sur les rayons X (radiologie ou CT-scan) qui se limitent aux images de l'anatomie. Par conséquent, la tomographie par émission de positrons est un outil diagnostic qui permet de déceler certaines pathologies qui se traduisent par une altération de la physiologie normale comme les cancers. La TEP est aussi utilisée en recherche biomédicale, par exemple en imagerie cérébrale où elle permet de révéler les régions actives du cerveau lors de telle ou telle activité cognitive de manière analogue à ce qui se fait avec l'imagerie par résonance magnétique fonctionnelle.

Par exemple, X peut représenter ¹⁸F⁻, qui est un émetteur de positrons et permet donc l'utilisation des solides MOF de l'invention pour des applications impliquant l'imagerie TEP.

Ainsi, dans un mode de réalisation, dans le motif de formule (I), au moins une occurrence du ligand X est ¹⁸F⁻.

Dans un mode de réalisation, le ligand L est un ligand fluoré ; c'est-à-dire comprenant au moins un substituant F. Par exemple, il peut s'agir de d'un ligand tétrafluorotéréphtalate, perfluorosuccinate, perfluoromuconate, perfluoro glutarate, 2,5 diperfluoroterephtalate, 3,6 perfluoro 1,2,4,5 benzenetetracarboxylate, 3,5,3',5' perfluoro-4,4'-azobenzene dicarboxylate, 3,3'-diperfluoro azobenzene 4,4'-dicarboxylate.

Les ligands fluorés précités peuvent être enrichis en isotope ¹⁸F par des techniques classiques de radiosynthèse bien connues de l'homme du métier.

La technique TEP permet d'obtenir des images très détaillées des tissus vivants. Le radio-isotope fluor-18 (¹⁸F) (t1/2 = 110 minutes) est un émetteur de positrons ; les positrons émis sont instantanément annihilés par les électrons de la matière environnante et ce sont les rayons gamma résultants qui sont détectés.

Ainsi, l'invention concerne également l'utilisation de solides MOF selon l'invention comme marqueur utilisable en imagerie médicale, telle que l'imagerie TEP.

Ainsi, il est fourni un procédé pour visualiser des tissus vivants par TEP comprenant l'administration d'un solide MOF selon l'invention à un sujet, et la visualisation des tissus par imagerie TEP. En particulier, le solide MOF contient au moins un ligand fluoré ¹⁸F et/ou ¹⁸F comme contre-ion (i.e., au moins une occurrence de X dans le motif de formule (I) représente ¹⁸F), tel que ceux précités.

Par ailleurs, la présence d'atomes de fluor dans les solides MOF (dans la charpente même des solides MOF (anion X=F), par l'intermédiaire de ligands L fluorés ou de par la présence de molécules fluorées dans les pores ou à la surface des solides MOF de l'invention), permet d'envisager l'utilisation de ces solides MOF pour des applications en imagerie médicale telle que l'échographie.

Ainsi, l'invention concerne également l'utilisation de solides MOF selon l'invention pour la fabrication d'un agent de contraste utilisable en imagerie médicale, notamment en échographie, échosonographie ou imagerie de résonance magnétique.

Le développement d'un agent de contraste pour l'échographie suppose l'introduction dans les tissus à examiner de réflecteurs efficaces des ultrasons. Le réflecteur idéal étant des microbulles gazeuses, il s'agissait d'injecter un gaz dans les veines du patient. Formulé en microbulles de quelques microns de diamètre, l'administration du gaz devient inoffensive. Cependant, une fois dans la circulation, des microbulles d'air, sous l'action combinée de la pression artérielle et de la pression de Laplace, se dissolvent dans le sang en l'espace de quelques secondes.

Jusqu'à présent, l'emploi de composés perfluorés, dont la solubilité dans l'eau est extrêmement faible, a permis de formuler des microbulles injectables ayant une persistance intravasculaire suffisante pour permettre un examen radiologique efficace. Plusieurs agents de contraste viennent d'être mis sur le marché ; notamment à base de C₃F₈, SF₆ ou C₆F₁₄. Ceux-ci permettent, en particulier, de visualiser la bordure endocardiale et de diagnostiquer des anomalies cardiaques structurales ou fonctionnelles. Ils facilitent également la visualisation des vaisseaux et la détection de défauts de perfusion, tumeurs et autres l 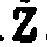 sions.

Les FC combinent une inertie chimique et biologique exceptionnelle avec une forte capacité de dissolution des gaz, une extrême hydrophobie, ainsi qu'une lipophobie prononcée. Leur très faible solubilité dans l'eau permet de stabiliser les microbulles injectables qui servent d'agent de contraste en échographie.

Ainsi, les solides MOFs selon la présente invention, et contenant des molécules perfluorés peuvent servir au diagnostic par échosonographie ou par imagerie de résonance magnétique.

Ainsi, il est fourni un procédé de diagnostique par échographie, échosonographie ou par imagerie de résonance magnétique comprenant l'administration d'un solide MOF selon l'invention à un sujet, et la visualisation des tissus par échographie, échosonographie ou par imagerie de résonance magnétique. En particuler, le solide MOF contient au moins une molécule perfluorée, telle que celles précitées.

De plus, comme discuté *infra,* les caractéristiques structurales particulières des solides MOF de la présente invention, notamment en terme de flexibilité ou de taille des pores, en font des adsorbants de grande capacité de charge, de grande sélectivité et de grande pureté. Ils rendent donc possible l'adsorption de molécules fluorées, comme par exemple les FCs, avec un coût énergétique favorable et un temps de relargage plus élevé.

En outre, la présence d'atomes de fluor dans les solides MOF (dans la charpente même des solides MOF (anion X=F), par l'intermédiaire de ligands L fluorés ou de par la présence de molécules fluorées dans les pores ou à la surface des nanoparticules de l'invention), permet d'envisager l'utilisation de ces solides MOF pour le transport de l'oxygène à des fins médicales (e.g., substituts sanguins).

Dans la présente, on entend par « substitut sanguin » un matériau permettant d'encapsuler de l'oxygène, de le transporter et de le relarguer vers les tissus et organes qui ont besoin d'être oxygénés (par exemple, lors d'une intervention chirurgicale, ou lors d'une hémorragie).

Il existe actuellement des émulsions de fluorocarbones (FC) submicroniques, stables et injectables, stériles et prêtes à l'emploi, qui permettent de délivrer de l'oxygène aux tissus et, par exemple, de réduire le recours à la transfusion sanguine en chirurgie.

La nature formule l'oxygène en un complexe de Fe hydrosoluble, l'hémoglobine, elle-même encapsulée dans le globule rouge.

Les avantages des substituts sanguins par rapport aux transfusions comprennent:
- éviter des contaminations,
- être utilisable pour tout type de groupe sanguin,
- être acceptées par tous les patients (même les témoins de Jehovah),
- pouvoir être facilement transportés et stockés donc très utiles en cas d'urgence.
Des substituts sanguins à base de fluorocarbures, matériaux biologiquement très inertes, sont capables de dissoudre de fortes quantités de gaz pour délivrer l'oxygène aux tissus. Les fluorocarbures étant insolubles dans l'eau, elles sont administrées sous la forme d'une émulsion qui doit a) être stable et 2) rapidement excrétable.

Oxygent® est une des émulsions développées à ce jour dans ce domaine. Il s'agit d'une composition comprenant 60% en poids par volume de perfluorooctyl bromide (C₈F₁₇Br), stabilisée contre la diffusion moléculaire par quelques % de C₁₀F₂₁Br, émulsifiée à l'aide de phospholipides en gouttelettes de l'ordre de 200 nm de diamètre. Ce produit présente des effets secondaires chez les patients, et s'est d'ailleurs vu refusé sa mise sur le marché américain par la FDA en Février 2005 en raison de problèmes de sûreté.

Ainsi, selon l'invention, le ligand L du motif de formule (I) des solides MOF de la présente invention est un ligand di-, tri-, tétra- ou hexa-carboxylate choisi dans le groupe comprenant :

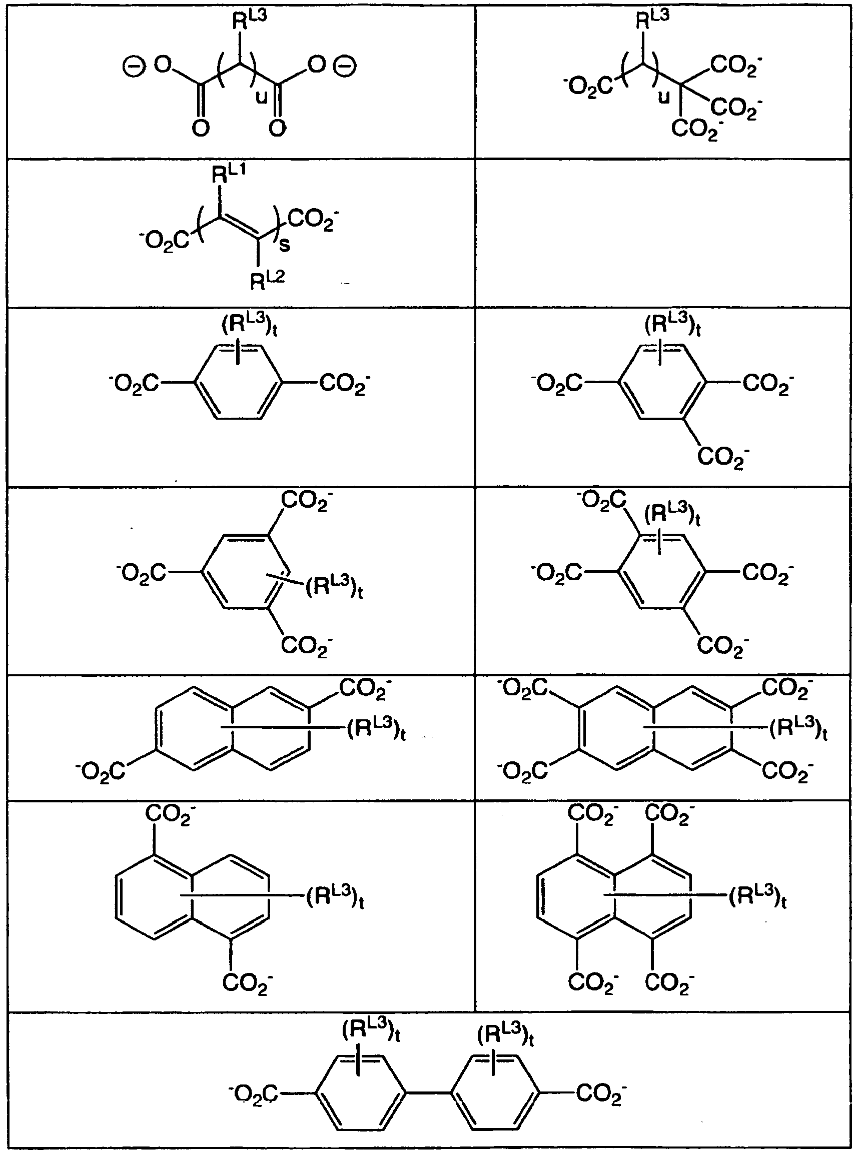

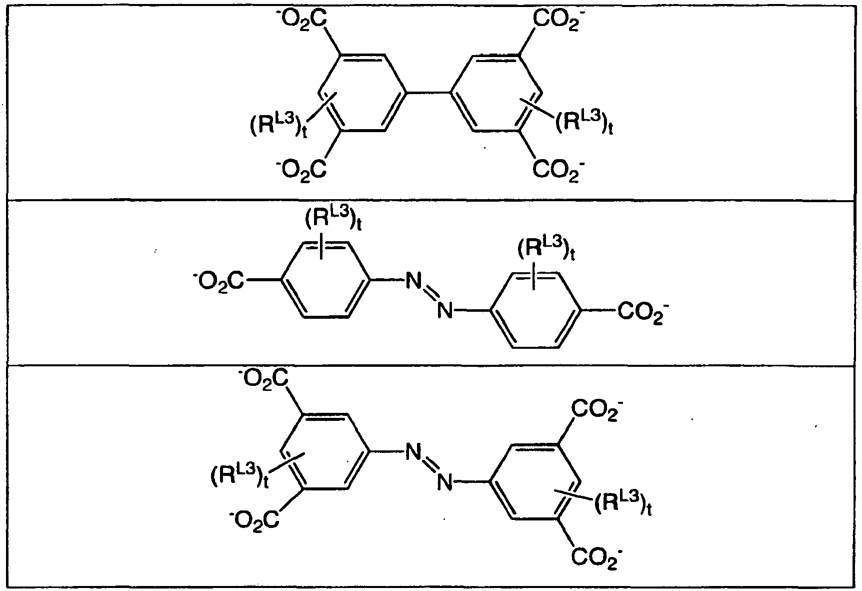

dans lesquels :
s représente un entier de 1 à 4,
chaque occurrence de t représente indépendamment un entier de 1 à 4,
u représente un entier de 1 à 7,
R^{L1} et R^{L2} représentent indépendamment H, un halogène ou un alkyle en C₁ à C₆ (de préférence méthyle ou éthyle), et au moins une occurrence de R^{L1} ou R^{L2} représente F, et
chaque occurrence de R^{L3} représente indépendamment H, un halogène (de préférence F, Cl ou Br), OH, NH₂, NO₂ ou un alkyle en C₁ à C₆ (de préférence méthyle ou éthyle), et au moins une occurrence de R^{L3}représente F.
De préférence, chaque occurrence de R^{L1} et R^{L2} représente F.
De préférence, chaque occurrence de R^{L3}représente F.
Par exemple, L peut représenter HOOC-C8F16 COOH.

Comme décrit généralement *infra,* la surface de ces MOFs peut être modifiée avec un agent de surface tel que le polyéthylène glycol (PEG) afin de leur conférer une furtivité.

La surface des solides MOF peut être également stabilisée par des amphiphiles fluorés afin de contrôler la libération d'oxygène (diffusion retardée de l'oxygène des pores des solides MOF). Le lecteur pourra se référer à la section traitant de la modification des surfaces des solides MOF de la présente invention, et adapter les enseignements précités au greffage de ligands amphiphiles fluorés.

Ainsi, il est fourni un procédé de libération d'oxygène in vivo comprenant l'administration d'un solide MOF selon l'invention à un sujet, ledit solide MOF comprenant dans ses pores ou à sa surface au moins un fluorocarbone ou une molécule fluorée telle que celles précitées, et de l'oxygène encapsulé.

En particulier, le solide MOF selon l'invention, peut comprendre un pourcentage de métal en phase sèche de 5 à 40%, de préférence de 18 à 31%.

Le pourcentage massique (%m) est une unité de mesure utilisée en chimie et en métallurgie pour désigner la composition d'un mélange ou d'un alliage, c'est-à-dire les proportions de chaque composant dans le mélange.

1 %m d'un composant = 1g du composant pour 100 g de mélange ou encore 1 kg dudit composant pour 100 kg de mélange.

Les solides MOF de la présente invention présentent notamment l'avantage d'avoir une stabilité thermique jusqu'à une température de 350°C.

En particulier, le solide MOF de la présente invention présente notamment l'avantage d'avoir une stabilité thermique de 120°C à 350°C.

En particulier, le solide MOF selon l'invention, peut être sous forme particulaire avec un diamètre des particules inférieur à 4 µm, de préférence inférieur à 1000 nanomètres.

En particulier, le solide MOF selon l'invention, peut être sous la forme de nanoparticules. En particulier, le solide MOF selon l'invention peut avoir un diamètre de particule inférieur à 1000 nanomètres, de préférence inférieur à 500 nm, de manière plus préférée inférieur à 250 nm, tout particulièrement inférieur à 100 nm.

En particulier, le solide MOF selon l'invention peut avoir une taille de pores de 0,4 à 6 nm, de préférence de 0,5 à 5,2 nm, et de manière plus préférée de 0,5 à 3,4 nm.

En particulier, le solide MOF selon l'invention peut avoir une surface spécifique (BET) de 5 à 6000 m²/g, de préférence de 5 à 4500 m²/g.

En particulier, le solide MOF selon l'invention peut avoir un volume poreux de 0,05 à 4 cm²/g, de préférence de 0,05 à 2 cm²/g.

Dans le cadre de l'invention, le volume poreux signifie le volume accessible pour les molécules de gaz et/ou de liquide.

Les inventeurs ont mis en évidence que les matériaux MOF comprenant une structure tridimensionnelle de motifs de formule (I) peuvent se présenter sous la forme d'une structure rigide ou flexible.

Le solide MOF de la présente invention peut se présenter sous la forme d'une structure robuste, qui a une charpente rigide et ne se contracte que très peu lorsque les pores se vident, ou sous la forme d'une structure flexible, qui peut se gonfler et se dégonfler faisant varier l'ouverture des pores en fonction de la nature des molécules adsorbées.

Ces molécules adsorbées qui peuvent être, par exemple, des solvants et/ou des gaz.

On entend par « structure rigide », au sens de la présente invention des structures qui se gonflent ou se contractent que très faiblement, c'est à dire avec une amplitude jusqu'à 10%.

Ainsi, un matériau MOF de structure rigide peut se gonfler ou se contracter avec une amplitude de 0 à 10%.

En particulier, le solide MOF selon l'invention, peut avoir une structure rigide qui se gonfle ou se contracte avec une amplitude de 0 à 10%.

Les structures rigides peuvent par exemple être construites à base de chaînes ou de trimères d'octaèdres.

Par exemple, le solide MOF de structure rigide selon l'invention peut avoir un pourcentage de métal en phase sèche de 5 à 40%, par exemple de 18 à 31%.

Par exemple, le solide MOF de structure rigide selon l'invention peut avoir une taille de pores de 0,4 à 6 nm, par exemple de 0,5 à 5,2 nm, par exemple de 0,5 à 3,4 nm.

Par exemple, le solide MOF de structure rigide selon l'invention peut avoir un volume poreux de 0 à 4 cm³/g, par exemple de 0,05 à 2 cm³/g.

On entend par « structure flexible », au sens de la présente invention des structures qui se gonflent ou se contractent avec une grande amplitude, notamment avec une amplitude supérieure à 10%, par exemple supérieure à 50%.

En particulier, un matériau MOF de structure flexible peut se gonfler ou se contracter avec une amplitude de 10% à 300%, de préférence de 50 à 300%.

Les structures flexibles peuvent par exemple être construites à base de chaînes ou de trimères d'octaèdres.

En particulier, le solide MOF selon l'invention, peut avoir une structure flexible qui se gonfle ou se contracte avec une amplitude supérieure à 10%, par exemple de 50 à 300%.

Par exemple, le solide MOF de structure flexible selon l'invention peut avoir un pourcentage de métal en phase sèche de 5 à 40%, par exemple de 18 à 31%.

Par exemple, le solide de structure flexible selon l'invention peut avoir une taille de pores de 0,4 à 6 nm, par exemple de 0,5 à 5,2 nm, par exemple de 0,5 à 1,6 nm.

Par exemple, le solide de structure flexible selon l'invention peut avoir un volume poreux de 0 à 3 cm³/g, par exemple de 0 à 2 cm³/g. Le volume poreux représente le volume accessible équivalent (formes ouvertes) pour les molécules de solvant.

La présente invention peut être mise en oeuvre avec des matériaux MOF de structure rigide ou flexible.

En outre, les inventeurs ont mis en évidence expérimentalement que l'amplitude de la flexibilité dépend de la nature du ligand et du solvant utilisé, comme décrit dans la partie « Exemples » ci-dessous (notamment dans l'Exemple 10).

Différents matériaux MOF ont été élaborés par les inventeurs à l'Institut Lavoisier de Versailles avec des phases variées, nommées « MIL » (pour « Matériau Institut Lavoisier »). L'appellation « MIL » de ces structures est suivie d'un nombre arbitraire n donnée par les inventeurs pour identifier les différentes phases.

Les inventeurs ont également mis en évidence que certains solides selon l'invention peuvent présenter un nombre plus élevé de phases possibles par rapport aux matériaux MOF classiquement rencontrés dans la littérature. Par exemple, différentes phases ont été obtenues pour les solides selon l'invention carboxylates de fer (III), par exemple MIL-53, MIL-69, MIL-88A, MIL-88B, MIL-88Bt, MIL-88C, MIL-88D, MIL-89, MIL-100, MIL-101, MIL-102. Ces différentes phases sont présentées dans la partie « Exemples ».

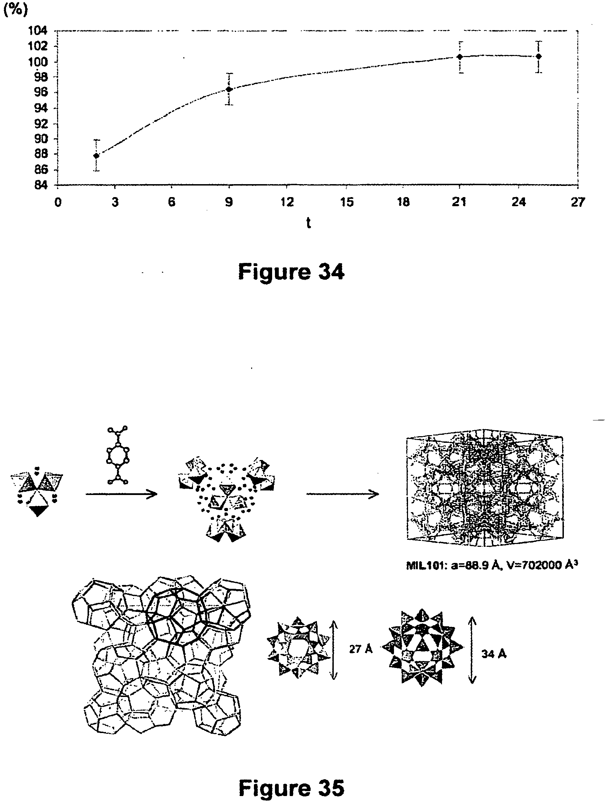

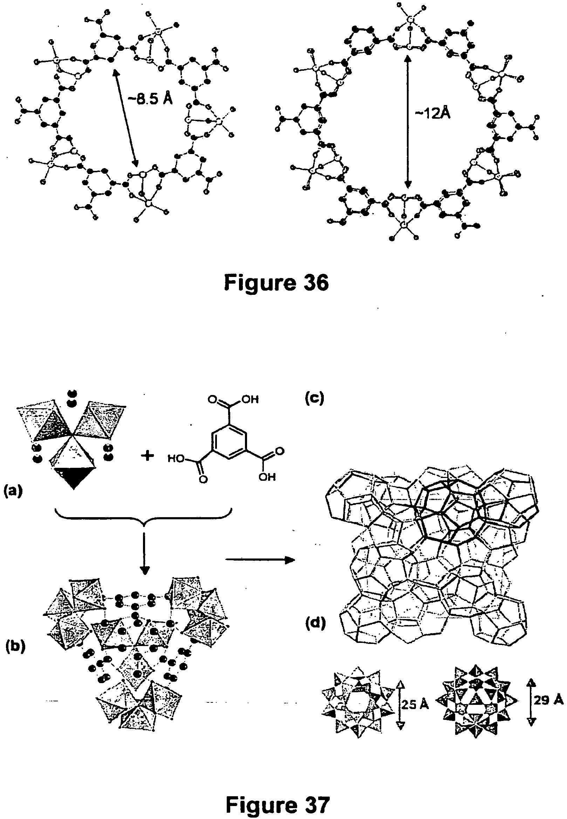

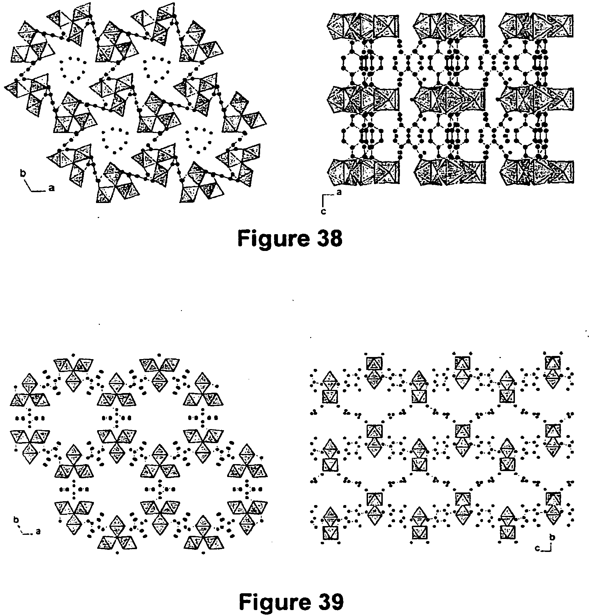

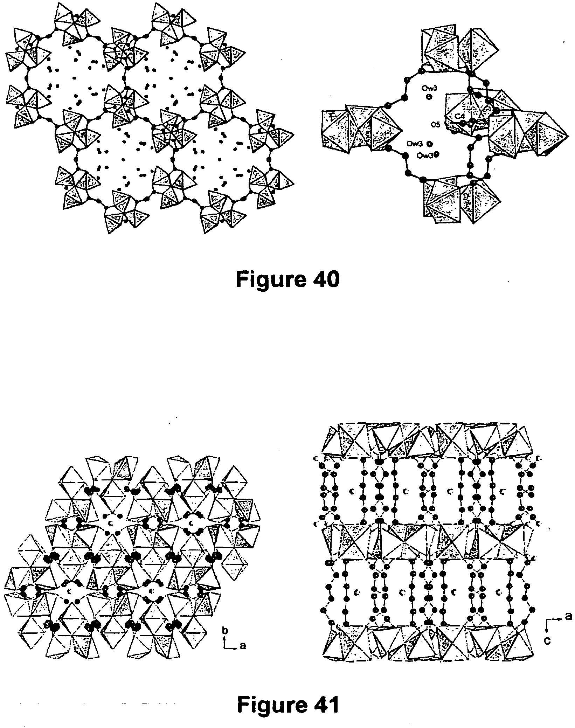

Les caractéristiques cristallographiques de ces structures sont connues, et ont fait l'objet de nombreux rapports. Par ailleurs, les dénominations « MIL » précitées sont des bien connues de l'homme du métier. On citera par exemple :
**MIL-53:** Whitfield, T. R.; Wang, X.; Liu, L.; Jacobson, A. J. Solid State Sci. 2005, 7, 1096.
**MIL-69:** T. Loiseau et al, C. R. Chimie, 8 765 (2005).
**MIL-88A :** (a) Serre et al., « Role of solvent-host interactions that lead to very large swelling of hybrid frameworks », Science, 2007, Vol. 315, 1828-1831 ; (b) Surblé et al., « A new isoreticular class of metal-organic frameworks with the MIL-88 topology », Chem. Comm., 2006, 284-286 ; (c) Mellot-Draznieks et al., « Very large swelling in hybrid frameworks : a combined computational and power diffraction study », J. Am. Chem. Soc., 2005, Vol. 127, 16273-16278. La structure d'un solide MIL-88A hydraté est représentée en figure 40.
**MIL-88B, MIL-88C et MIL-88D :** Pour ces types structuraux, le lecteur pourra se référer aux publications concernant le type MIL-88A ci-dessus, à savoir, (a) Serre et al., « Role of solvent-host interactions that lead to very large swelling of hybrid frameworks », Science, 2007, Vol. 315, 1828-1831 ; (b) Surblé et al., « A new isoreticular class of metal-organic frameworks with the MIL-88 topology », Chem. Comm., 2006, 284-286.
**MIL-89 :** C. Serre, F. Millange, S. Surblé, G. Férey Angew. Chem. Int. Ed. 2004, 43, 6286: A new route to the synthesis of trivalent transition metals porous carboxylates with trimeric SBU. La structure d'un solide MIL-89 est représentée en figure 41.
**MIL-100** : Horcajada et al., « Synthesis and catalytic properties of MIL-100(Fe), an iron(III) carboxylate with large pores », Chem. Comm., 2007, 2820-2822. La structure d'un solide MIL-100 est représentée en figures 35 et 36.
**MIL-101 :** Férey et al., « A chromium terephthalate-based solid with unusally large pore volumes and surface area », Science, 2005, Vol. 309, 2040-2042. La structure d'un solide MIL-101 est représentée en figure 37.
**MIL-102:** S. Surblé, F. Millange, C. Serre, T. Düren, M. Latroche, S. Bourrelly, P.L. Llewellyn and G. Férey « MIL-102: A Chromium Carboxylate Metal Organic Framework with Gas Sorption Analysis » J. Am. Chem. Soc. 128 (2006), 46, 14890*.* La structure d'un solide MIL-102 est représentée en figure 38.
**MIL-88B_4CH3, MIL-88B_CH3, MIL-88B_2CF3, MIL-88B_20H, MIL-88B_NO2, MIL-88B_NH2, MIL-88B_Cl, MIL-88B_Br, MIL-88B_4F:** Pour ce type structural, le lecteur pourra se référer aux publications concernant le type MIL-88 ci-dessus, à savoir, (a) Serre et al., « Role of solvent-host interactions that lead to very large swelling of hybrid frameworks », Science, 2007, Vol. 315, 1828-1831 ; (b) Surblé et al., « A new isoreticular class of metal-organic frameworks with the MIL-88 topology », Chem. Comm., 2006, 284-286 ; (c) Mellot-Draznieks et al., « Very large swelling in hybrid frameworks : a combined computational and power diffraction study », J. Am. Chem. Soc., 2005, Vol. 127, 16273-16278. La structure d'un solide MIL-88B_4CH₃ est représentée en figure 39.

En particulier, le solide MOF selon l'invention, peut avoir un motif de formule choisie dans le groupe comprenant :
- Fe(OH)[C₆H₄(CO₂)₂] de structure flexible, par exemple MIL-53
- Fe₃OX[C₂H₂(CO₂)₂]₃ de structure flexible, par exemple MIL-88A
- Fe₃OX[C₄H₄(CO₂)₂]₃ de structure flexible, par exemple MIL-89
- Fe₃OX[C₆H₄(CO₂)₂]₃ de structure flexible, par exemple MIL-88B
- Fe₃OX[O₂C-C₆(CH₃)₄-CO₂]₃.nH₂O de structure flexible, par exemple MIL-88Bt
- Fe₃OX[C₆H₄(CO₂)₂]₃ de structure rigide, par exemple MIL-101
- Fe₃OX[C₆H₃(CO₂)₃]₃ de structure rigide, par exemple MIL-100
- Fe₃OX[C₁₀H₆(CO₂)₂]₃ de structure flexible, par exemple MIL-88C
- Fe₃OX[C₁₂H₈(CO₂)₂]₃ de structure flexible, par exemple MIL-88D
dans laquelle X est tel que défini précédemment.

Tout particulièrement, le solide MOF selon l'invention, peut avoir un motif de formule choisie dans le groupe comprenant :
- MIL-101 (Fe) ou Fe₃O[C₆H₄-(CO₂₎₂]₃.X.nH₂O (X=F, Cl, OH) de structure rigide
- MIL-101 -Cl (Fe) ou Fe₃O[Cl-C₆H₃-(CO₂₎₂]₃.X.nH₂O (X=F, Cl, OH) de structure rigide
- MIL-101-NH₂ (Fe) ou Fe₃O[NH₂ -C₆H₃-(CO₂₎₂]₃.X.nH₂O (X=F, Cl, OH) de structure rigide
- MIL-101-2CF₃ (Fe) ou Fe₃O[(CF₃)₂ -C₆H₂-(CO₂₎₂]₃.X.nH₂O (X=F, Cl, OH) de structure rigide
- MIL-88B-NO₂ (Fe) ou Fe₃O[C₆H₃NO₂-(CO₂₎₂]₃.X.nH₂O (X=F, Cl, OH) de structure flexible
- MIL-88H-2OH (Fe) ou Fe₃O[C₆H₂ (OH)₂-(CO₂₎₂]₃.X.nH₂O (X=F, Cl, OH) de structure flexible
- MIL-88B-NH₂ (Fe) ou Fe₃O[C₆H₃NH₂-(CO₂₎₂]3.X.nH₂O (X=F, Cl, OH) de structure flexible
- MIL-88B-CH₃ (Fe) ou Fe₃O[C₆H₃CH₃ -(CO₂₎₂]₃.X.nH₂O (X=F, Cl, OH) de structure flexible
- MIL-88B-Cl (Fe) ou Fe₃O[C₆H₃Cl-(CO₂₎₂]₃.X.nH₂O (X=F, Cl, OH) de structure flexible
- MIL-88B-4CH₃ (Fe) ou Fe₃O[C₆ (CH₃)₄-(CO₂₎₂]₃.X.nH₂O (X=F, Cl, OH) de structure flexible
- MIL-88B-4F (Fe) ou Fe₃O[C₆F₄-(CO₂₎₂]₃.X.nH₂O (X=F, Cl, OH) de structure flexible
- MIL-88B-Br (Fe) ou Fe₃O[C₆H₃Br-(CO₂₎₂]₃.X.nH₂O (X=F, Cl, OH) de structure flexible
- MIL-88B-2CF₃ (Fe) ou Fe₃O[(CF₃)₂-C₆H₂-(CO₂)₂]₃.X.nH₂O (X=F, Cl, OH) de structure flexible
- MIL-88D 4CH₃ (Fe) ou Fe₃O[C₁₂H₄(CH₃)₄-(CO₂₎₂]₃.X.nH₂O (X=F, Cl, OH) de structure flexible
- MIL-88D 2CH₃ (Fe) ou Fe₃O[C₁₂H₆(CH₃)₂-(CO₂₎₂]₃.X.nH₂O (X=F, Cl, OH) de structure flexible
- MIL-88E (Pyr) (Fe) ou Fe₃O[C₄H₃N₂-(CO₂₎₂]₃.X.nH₂O (X=F, Cl, OH) de structure flexible
- MIL-88F (Thio) (Fe) ou Fe₃O[C₄H₂S-(CO₂₎₂]₃X.nH₂O (X=F, Cl, OH) de structure flexible
- MIL-53-20H (Fe) ou FeO(OH)[C₆H₂ (OH)₂-(CO₂)₂].X.nH₂O (X=F, Cl, OH) de structure flexible
- MIL-53-NH₂ (Fe) ou FeO(OH)[C₆H₂ -NH₂-(CO₂)₂].X.nH₂O (X=F, Cl, OH) de structure flexible
- MIL-53-Cl (Fe) ou FeO(OH)[C₆H₂ Cl-(CO₂)₂].X.nH₂O (X=F, Cl, OH) de structure flexible
- MIL-53-Br (Fe) ou FeO(OH)[C₆H₂ Br-(CO₂₎₂].X.nH₂O (X=F, Cl, OH) de structure flexible
- MIL-53-2CF₃ (Fe) ou FeO(OH)[C₆H₂ (CF₃)₂-(CO₂₎₂].X.nH₂O (X=F, Cl, OH) de structure flexible
- MIL-53-CH₃ (Fe) ou FeO(OH)[C₆H₃ CH₃-(CO₂)₂].X.nH₂O (X=F, Cl, OH) de structure flexible
- MIL-53-2COOH (Fe) ou FeO(OH)[C₆H₃ -(CO₂)₄].X.nH₂O (X=F, Cl, OH) de structure flexible
- MIL-88G (AzBz) (Fe) ou Fe₃O[C₁₂H₈N₂-(CO₂₎₂]₃.X.nH₂O (X=F, Cl, OH) de structure flexible
- MIL-88G 2Cl (AzBz-2Cl) (Fe) ou Fe₃O[C₁₂H₆N₂Cl₂-(CO₂₎₂]₃.X.nH₂O (X=F, Cl, OH) de structure flexible.

En outre, à partir d'un même ligand acide carboxylique L et des mêmes bases de fer (chaînes ou trimères), les inventeurs ont pu obtenir des matériaux MOF de même formule générale (I) mais de structures différentes. C'est par exemple le cas des solides MIL-88B et MIL-101. En effet, la différence des solides MIL-88B et MIL-101 réside dans le mode de connexions des ligands aux trimères d'octaèdre : dans le solide MIL-101, les ligands L s'assemblent sous forme de tétraèdres rigides, tandis que dans le solide MIL-88B, ils forment des bipyramides trigonales, rendant possible l'écartement entre les trimères.

Ces différents matériaux sont présentés dans la partie « Exemples » ci-dessous. Le mode d'assemblage de ces ligands peut être contrôlé lors de la synthèse par exemple par l'ajustement du pH. Par exemple, le solide MIL-88 est obtenu en milieu moins acide que le solide MIL-101 comme décrit dans la partie « Exemples » ci-dessous.

En particulier, le solide MOF de la présente invention peut avoir une phase choisie dans le groupe comprenant : MIL-53, MIL-88, MIL-100, MIL-101, MIL-102 décrites dans la partie « Exemples ».

En particulier, le solide MOF selon l'invention, peut comprendre au moins un métal possédant des propriétés paramagnétiques ou diamagnétiques. De préférence, le solide MOF selon l'invention peut comprendre un ou plusieurs métaux paramagnétiques, identiques ou différents, qui peuvent être choisi dans le groupe comprenant le fer, le gadolinium, le manganèse, etc. En particulier, le solide MOF selon l'invention peut comprendre un ou plusieurs ions métalliques paramagnétiques, identiques ou différents, qui peuvent être choisi dans le groupe comprenant Fe²⁺, Fe³⁺, Gd³⁺, Mn²⁺, Mn³⁺.

Par ailleurs, les inventeurs ont mis en évidence, par exemple pour les solides MOF carboxylate de fer, des propriétés inattendues en imagerie. Ainsi, le solide MOF selon l'invention peut être utilisé en imagerie. En outre, l'invention concerne également l'utilisation du solide MOF selon l'invention comme agent de contraste.

En effet, les agents de contraste sont caractérisés par leur relaxivité. Plus elle est importante, plus l'effet des agents de contraste est important. La relaxivité correspond à la capacité des agents de contraste à modifier les temps de relaxation des protons de l'eau du milieu suite à l'application d'un champ magnétique. Elle dépend des propriétés paramagnétiques des métaux utilisés mais également de la quantité et de la mobilité des molécules d'eau qui se coordinent au métal dans la première sphère interne, amenant la contribution la plus importante, ainsi que dans la sphère externe. Ces « sphères de coordination » représentent les atomes immédiatement attachés au centre métallique dans le cas de la 1^{ère} sphère ; pour la sphère externe, cela représente les atomes immédiatement situés au-delà de la 1^{ère} sphère.

Dans le cas du solide de l'invention, outre la susceptibilité magnétique du métal, dans cet exemple le fer(III), les caractéristiques structurales du solide de la présente invention permettent à l'eau de se coordiner autour de la 1^{ère} sphère de coordination et de circuler dans les pores ce qui induit un effet sur les temps de relaxation longitudinal T1 et transversal T2 des protons de l'eau. En particulier, la relaxivité r2 du solide est suffisante pour une utilisation *in vivo* lors des expériences d'écho de gradient.

Par exemple, le solide MOF selon l'invention peut avoir une relaxivité transversale r2 d'au moins d'au moins 18 mMs⁻¹, par exemple d'au moins 8,6 mMs⁻¹.

Par ailleurs, les travaux de recherche menés par les inventeurs leur ont permis d'élaborer une méthode de synthèse souple et modulable permettant d'obtenir les solides MOF selon l'invention ayant une organisation structurale isoréticulaire particulière avec de bons rendements. En outre, le procédé permet d'obtenir des nanoparticules de dimensions souhaitées et des tailles de particules et de pores homogènes.

Ainsi, l'invention concerne également un procédé de préparation d'un solide tel que défini dans la présente invention, comprenant au moins une étape réactionnelle (i) consistant à mélanger dans un solvant polaire :
- au moins une solution comprenant au moins un précurseur inorganique métallique se présentant sous la forme de métal M, d'un sel de métal M ou d'un complexe de coordination comprenant l'ion métallique M dans laquelle M est tel que défini précédemment.
- au moins un ligand L' di-, tri-, tétra- ou hexadentate choisi dans le groupe comprenant :

| | | |
|---|---|---|
| 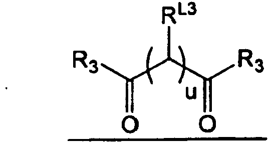 | | 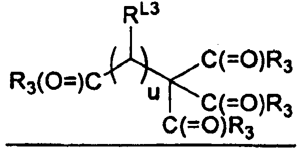 |
| 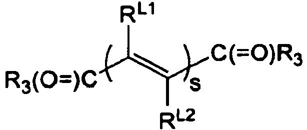 | | 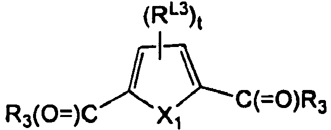 |
| 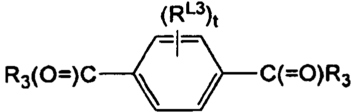 | | 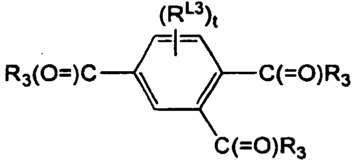 |
| 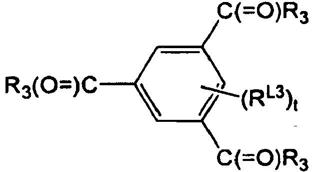 | | 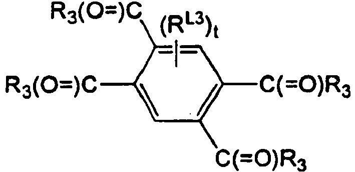 |
| 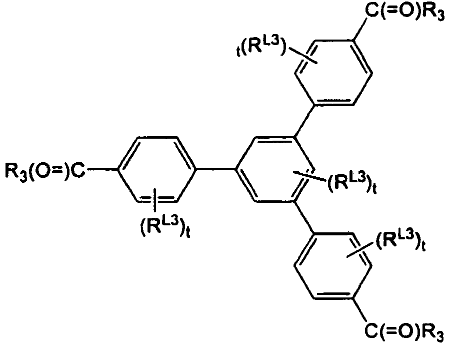 | | 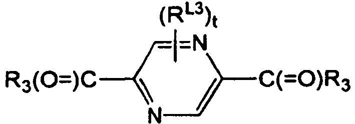 |
| 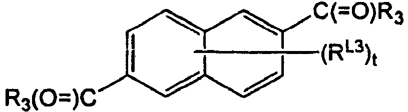 | | 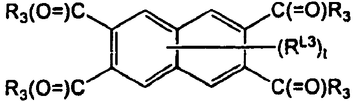 |
| 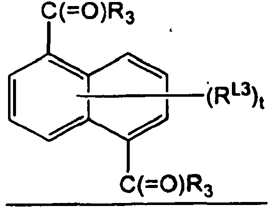 | | 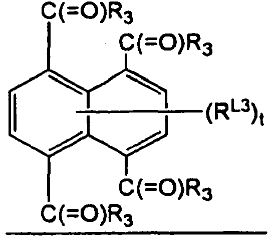 |
| 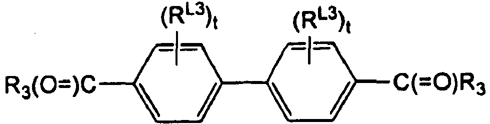 | | |
| 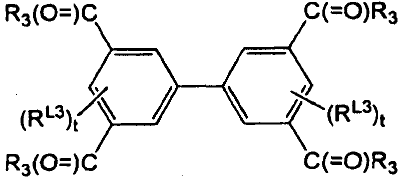 | | |
| 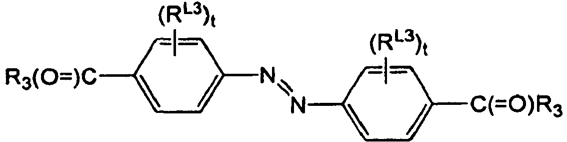 | | |
| 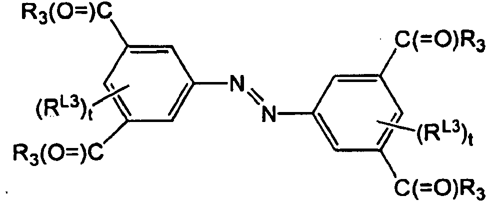 | | |
| 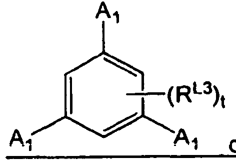 | où A₁, A₂ et A₃ représentent indépendamment | |
| 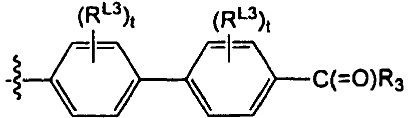 | | |

dans lesquels :
R₃ est choisi dans le groupe comprenant un radical -OH, un radical -OY où Y représente un cation alcalin, un halogène, ou un radical - OR⁴, -O-C(=O)R⁴ ou -NR⁴R⁴', où R⁴ et R⁴' sont des radicaux alkyles en C₁₋₁₂,
X₁ représente O ou S,
s représente un entier de 1 à 4,
chaque occurrence de t représente indépendamment un entier de 1 à 4,
u représente un entier de 1 à 7,
R^{L1} et R^{L2} représentent indépendamment H, un halogène ou un alkyle en C₁ à C₆, et
chaque occurrence de R^{L3} représente indépendamment H, un halogène, OH, NH₂, NO₂ ou un alkyle en C₁ à C₆;

b) une étape (iii) de fixation sur ledit solide d'au moins un agent de surface organique choisi dans le groupe comprenant un oligosaccharide, un polysaccharide, un glycosaminoglycanne, un polymère, un tensioactif, les vitamines, les coenzymes, les anticorps ou fragment d'anticorps, les aminoacides ou peptides ;
de façon à obtenir ledit solide.

Selon l'invention, le procédé de préparation du solide de l'invention comprend une étape (iii) de fixation sur ledit solide d'au moins un agent de surface organique.

Cette étape de fixation (iii) peut être réalisée pendant ou après l'étape réactionnelle (i) ou encore après une étape d'introduction (ii) d'une molécule d'intérêt. Des exemples sont fournis ci-dessous (Exemple 22, Exemple 23, Exemple 24).

Un certain nombre de solides MOF à surface modifiée sont illustrés dans la partie « Exemples ». Il est entendu que ces exemples sont donnés à titre illustratif, et non limitatif. Les méthodes de modification de surface des solides MOF illustrées dans les Exemples sont applicables et/ou adaptables à l'ensemble des solides MOF selon la présente invention (e.g., solides MOF à base de métal M différent de Fe, avec des ligands L différents, et/ou encapsulant ou non au moins un principe actif, un composé d'intérêt cosmétique et/ou un marqueur). Par exemple, ces méthodes peuvent être mises en oeuvre sans difficulté pour la modification des surfaces de l'ensemble des solides MOF décrits dans la présente demande.

Le ligand L' est un ligand di-, tri-, tétra- ou hexadentate choisi dans le groupe comprenant :

| | |
|---|---|
| 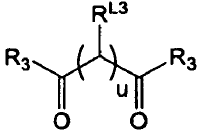 | 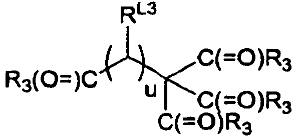 |
| 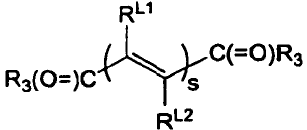 | 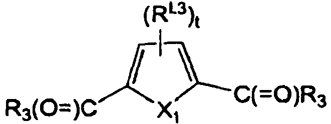 |
| 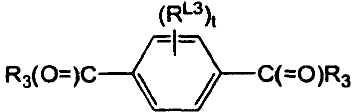 | 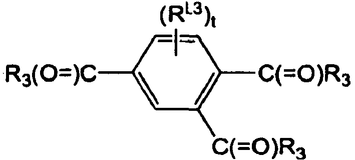 |
| 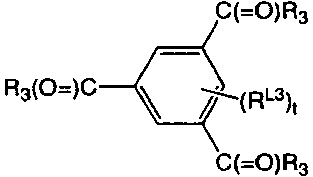 | 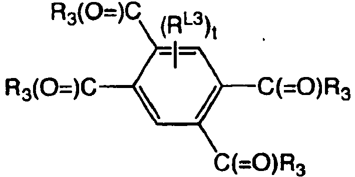 |
| 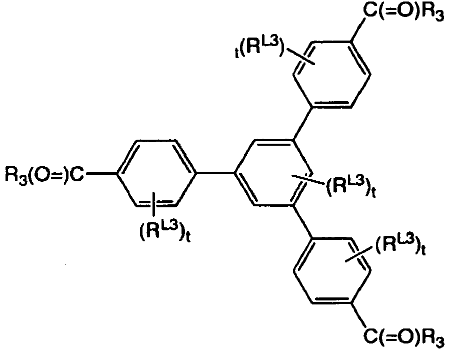 | 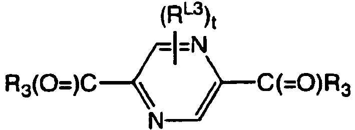 |
| 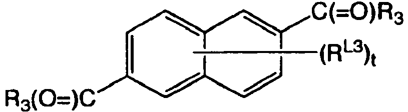 | 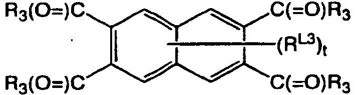 |
| 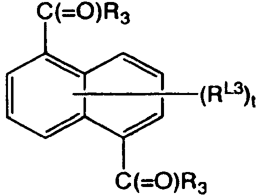 | 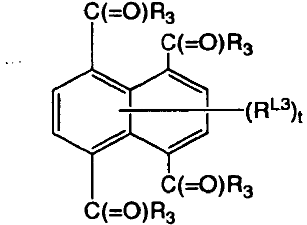 |
| 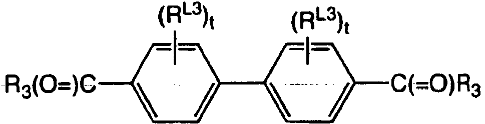 | |
| 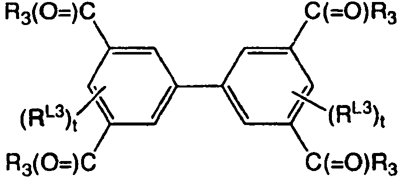 | |
| 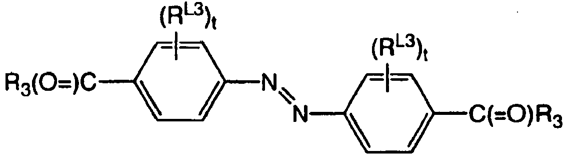 | |
| 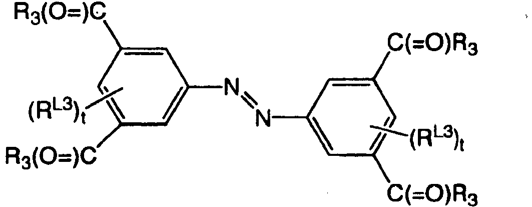 | |
| 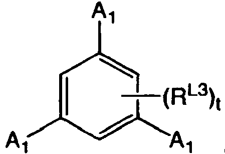 | où A₁, A₂ et A₃ représentent |
| indépendamment | 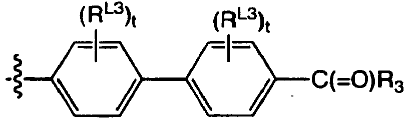 |

dans lesquels :
R³ est choisi dans le groupe comprenant un radical -OH, un radical -OY où Y représente un cation alcalin, un halogène, ou un radical -OR⁴, -O-C(=O)R⁴ ou -NR⁴R⁴', où R⁴ et R⁴' sont des radicaux alkyles en C₁₋₁₂,
X₁ représente O ou S,
s représente un entier de 1 à 4,
chaque occurrence de t représente indépendamment un entier de 1 à 4,
u représente un entier de 1 à 7,
R^{L1} et R^{L2} représentent indépendamment H, un halogène ou un alkyle en C₁ à C₆ (de préférence méthyle ou éthyle), et
chaque occurrence de R^{L3} représente indépendamment H, un halogène (de préférence F, Cl ou Br), OH, NH₂, NO₂ ou un alkyle en C₁ à C₆ (de préférence méthyle ou éthyle).

Dans un mode de réalisation, chaque occurrence de R³ représente un atome d'hydrogène.

Dans un mode de réalisation, chaque occurrence des radicaux R^{L1}, R^{L2} et R^{L3} représente un atome d'hydrogène.

De préférence, dans l'étape réactionnelle (i), le ligand L' utilisé peut être un acide di-, tri- ou tétra- carboxylique choisi dans le groupe comprenant : C₂H₂(CO₂H)₂ (acide fumarique), C₂H₄(CO₂H)₂ (acide succinique), C₃H₆(CO₂H)₂ (acide glutarique), C₄H₄(CO₂H)₂ (acide muconique), C₄H₈(CO₂H)₂ (acide adipique), C₇H₁₄(CO₂H)₂ (acide azelaique), C₅H₃S(CO₂H)₂ (acide 2,5-thiophènedicarboxylique), C₆H₄(CO₂H)₂ (acide téréphtalique), C₆H₂N₂(CO₂H)₂ (acide 2,5-pyrazine dicarboxylique), C₁₀H₆(CO₂H)₂ (acide naphtalène-2,6-dicarboxylique), C₁₂H₆(CO₂H)₂ (acide biphényle-4,4'-dicarboxylique), C₁₂H₈N₂(CO₂H)₂ (acide azobenzènedicarboxylique), C₆H₃(CO₂H)₃ (acide benzène-1,2,4-tricarboxylique), C₆H₃(CO₂H)₃ (acide benzène-1,3,5-tricarboxylate), C₂₄H₁₅(CO₂H)₃ (acide benzène-1,3,5-tribenzoique), C₆H₂(CO₂H)₄ (acide benzène-1,2,4,5-tétracarboxylique, C₁₀H₄(CO₂H)₄ (acide naphtalène-2,3,6,7-tétracarboxylique), C₁₀H₄(CO₂H)₄ (acide naphtalène-1,4,5,8-tétracarboxylique), C₁₂H₆(CO₂H)₄ (acide biphényl-3,5,3',5'-tétracarboxylique), et les analogues modifiés choisis dans le groupe comprenant l'acide 2-aminotéréphtalique, l'acide 2-nitrotéréphtalique, l'acide 2-méthyltéréphtalique, l'acide 2-chlorotéréphtalique, l'acide 2-bromotéréphtalique, l'acide 2,5-dihydroxotéréphtalique, l'acide tétrafluorotéréphtalique, l'acide tétraméthyltéréphtalique, l'acide diméthyl-4,4'-biphénydicarboxylique, l'acide tétraméthyl-4,4'-biphénydicarboxylique, l'acide dicarboxy-4,4'-biphénydicarboxylique, l'acide 2,5-pyrazyne dicarboxylique. Le ligand L' utilisé peut également être choisi dans le groupe comprenant : l'acide 2,5 diperfluoroterephthalique, l'acide azobenzene 4,4'-dicarboxylique, l'acide 3,3'-dichloro azobenzene 4,4'-dicarboxylique, l'acide 3,3'-dihydroxo azobenzene 4,4'-dicarboxylique, l'acide 3,3'-diperfluoro azobenzene 4,4'-dicarboxylique, l'acide 3,5,3',5'-azobenzene tetracarboxylique, l'acide 2,5-dimethyl terephthalique, l'acide perfluoro glutarique.

Il est bien entendu que, dans la mise en oeuvre du procédé, lorsque le ligand L' est de type carboxylate, celui-ci n'est pas nécessairement sous la forme d'un acide carboxylique. Comme indiqué précédemment, celui-ci peut se présenter sous une forme dérivée où une ou plusieurs fonctions carboxyliques est/sont sous la forme -C(=O)-R³ où R³ peut représenter un radical -OY où Y représente un cation alcalin, un halogène, ou un radical -OR⁴, -O-C(=O)R⁴ ou -NR⁴R⁴', où R⁴ et R^{4'} sont indépendamment des radicaux alkyles en C₁₋₁₂.

La synthèse de matériaux MOF peut être de préférence réalisée en présence d'énergie qui peut être apportée par exemple par le chauffage, comme par exemple des conditions hydrothermales ou solvothermales, mais également par micro-ondes, par ultrasons, par broyage, par un procédé faisant intervenir un fluide supercritique, etc. Les protocoles correspondants sont ceux connus de l'homme du métier. Des exemples non limitatifs de protocoles utilisables pour les conditions hydrothermales ou solvothermales sont décrits par exemple dans K. Byrapsa, et al. « Handbook of hydrothermal technology », Noyes Publications, Parkridge, New Jersey USA, William Andrew Publishing, LLC, Norwich NY USA, 2001 [9]. Pour la synthèse par voie micro-ondes, des exemples non limitatif de protocoles utilisables sont décrits par exemple dans G. Tompsett, et al. ChemPhysChem. 2006, 7, 296 [10] ; dans S.-E. Park, et al. Catal. Survey Asia 2004, 8, 91 [11] ; dans C. S. Cundy, Collect. Czech. Chem. Commum. 1998, 63, 1699 [12] ; ou dans S. H. Jhung, et al. Bull. Kor. Chem. Soc. 2005, 26, 880 [13]. Les conditions en présence d'un broyeur à cylindre peuvent par exemple se référer aux publications A. Pichon et al., Cryst. Eng. Comm. 8, 2006, 211-214 [14] ; D. Braga et al., Angew. Chem. Int. Ed. 45, 2006, 142-246 [15] ; D. Braga et al., Dalton Trans. 2006, 1249-1263 [16].

Les conditions hydrothermales ou solvothermales, dont les températures de réactions peuvent varier entre 0 et 220°C, sont généralement effectuées dans des récipients en verre (ou en plastique) lorsque la température est inférieure à la température d'ébullition du solvant. Lorsque la température est supérieure ou lorsque la réaction s'effectue en présence de fluor, des corps en téflon insérés dans des bombes métalliques sont employés [9].

Les solvants utilisés sont généralement polaires. Notamment les solvants suivants peuvent être utilisés : l'eau, les alcools, le diméthylformamide, le diméthylsulfoxide, l'acétonitrile, le tétrahydrofurane, le diéthylformamide, le chloroforme, le cyclohexane, l'acétone, le cyanobenzène, le dichlorométhane, le nitrobenzène, l'éthylèneglycol, le diméthylacétamide ou des mélanges de ces solvants.

Un ou plusieurs co-solvants peuvent également être ajoutés à n'importe quelle étape de la synthèse pour une meilleure solubilisation des composés du mélange. Il peut s'agir notamment d'acides monocarboxyliques, tels que l'acide acétique, l'acide formique, l'acide benzoïque, etc.

Lorsque le co-solvant est un acide monocarboxylique, celui-ci, outre un effet solubilisateur, permet également d'arrêter la croissance cristalline du solide MOF. En effet, la fonction carboxylique se coordonne avec le fer, lequel ne pourra pas se lier à un autre atome de fer faute de la présence d'une seconde fonction -COOH sur la molécule de co-solvant. Ainsi, la croissance du réseau cristallin s'en trouve ralentie, puis arrêtée. L'ajout d'un co-solvant monocarboxylique, tel que l'acide acétique, l'acide formique, l'acide benzoïque, etc., permet ainsi de réduire la taille des particules de solide MOF obtenues. L'utilisation d'un co-solvant monocarboxylique peut donc favoriser l'obtention de nanoparticules (particules de taille < 1 µm).

En général, le contrôle de la taille des nanoparticules peut être effectué avec l'ajout d'une molécule monocarboxylée. Il peut s'agir d'un des co-solvants précités. Il peut également s'agir d'un agent de surface organique monocarboxylé. La notion d'agents de surface organiques, et leurs utilisation dans le cadre de la présente invention, sont décrites en détail infra. Par exemple, un agent de surface organique monocarboxylé, tel que PEG-COOH, peut être ajouté en cours de synthèse. Celui-ci a la double fonction de :
- arrêter la croissance cristalline du réseau MOF (et donc de permettre l'obtention de nanoparticules de plus petit taille)
- modifier la surface des nanoparticules par greffage de groupements PEG (fonction d'agent de surface organique) .

Un ou plusieurs additifs peuvent également être ajoutés au cours de la synthèse afin de moduler le pH du mélange. Ces additifs sont choisis parmi les acides minéraux ou organiques ou les bases minérales ou organiques. En particulier, l'additif peut être choisi dans le groupe comprenant : HF, HCl, HNO₃, H₂SO₄, NaOH, KOH, la lutidine, l'éthylamine, la méthylamine, l'ammoniac, l'urée, l'EDTA, la tripropylamine, pyridine, etc.

De préférence, l'étape réactionnelle (i) peut être réalisée suivant au moins une des conditions réactionnelles suivantes :
- avec une température de réaction de 0°C à 220°C, de préférence de 50 à 150°C ;
- avec une vitesse d'agitation de 0 à 1000 rpm (ou rotation par minute), de préférence de 0 à 500 ;
- avec un temps de réaction de 1 minute à 96 heures, de préférence de 1 minute à 15 heures ;
- avec un pH de 0 à 7, de préférence de 1 à 5 ;
- avec l'addition d'au moins un co-solvant au solvant, au précurseur, au ligand ou au mélange de ceux-ci, ledit co-solvant étant choisi dans le groupe comprenant l'acide acétique, l'acide formique, l'acide benzoïque ;
- en présence d'un solvant est choisi dans le groupe comprenant l'eau, les alcools R^{S}-OH où R^{S} est un radical alkyle en C, à C₆ linéaire ou ramifié, le diméthylformamide, diméthylsulfoxide, l'acétonitrile, le tétrahydrofurane, le diéthylformamide, le chloroforme, le cyclohexane, l'acétone, le cyanobenzène, le dichlorométhane, le nitrobenzène, l'éthylèneglycol, le diméthylacétamide ou des mélanges de ces solvants, miscibles ou non ;
- dans un milieu supercritique, par exemple dans le CO₂ supercritique ;
- sous micro-ondes et/ou sous ultra-sons ;
- dans des conditions d'électrolyse électrochimique ;
- dans des conditions utilisant un broyeur à cylindres ;
- dans un flux gazeux.

La synthèse de matériaux MOF peut être de préférence réalisée dans des conditions expérimentales propices à la formation de nanoparticules. Par exemple, un contrôle des paramètres suivants peut être important pour la réalisation de nanoparticules de solides MOF selon l'invention :
- température de réaction,
- temps de réaction,
- concentrations en ligand L' et en précurseur inorganique métallique et/ou
- ajout d'un ou plusieurs additifs tel que des modificateurs de pH (acides, bases), minéralisateurs, ou agents favorisant l'arrêt de la croissante crystalline (monoacide carboxylique).

Les fourchettes de valeurs préférées de chacun de ces paramètres peuvent varier selon que la synthèse des nanoparticules est réalisée par la voie hydro/solvothermale, par ultrasons ou par micro-ondes. Par exemple, une température de réaction plus élevée sera généralement utilisée pour la voie hydro/solvothermale (environ 20-150°C) que pour la voie par ultra-sons (environ 0°C).

Comme décrit dans l'exemple 6B, les inventeurs ont démontré que les 4 paramètres précités ont un impact non seulement sur l'obtention de nanoparticules (i.e., particules de diamètre inférieur à 1 µm), mais aussi sur l'obtention d'une bonne cristallisation, un rendement satisfaisant (e.g., >25% en poids) et l'absence d'oxydes de fer.

Des conditions optimales ont été déterminées empiriquement par les inventeurs pour chacune des phases de solide MOF préparées. A titre illustratif, et non limitatif, des exemples de conditions opératoires sont détaillées dans la partie « Exemples ». Il est entendu que les conditions opératoires détaillées dans les « Exemples » ne sont en aucun cas limitatives, des nanoparticules de solide MOF selon l'invention pouvant être obtenues dans des gammes de température, temps de réaction et concentrations, et avec des quantités d'additifs, variant autour des conditions opératoires illustrées dans le Exemples, selon la taille de nanoparticules et la polydispersité souhaitée.

En général, selon le procédé de préparation des solides MOF de l'invention sous forme de nanoparticules, la phase **MIL-88A** est obtenue sous forme de nanoparticules en utilisant les paramètres suivants :

### Voie solvothermale

- la température de réaction est de préférence entre 20 et 200°C, plus particulièrement entre 50 et 100°C, tout particulièrement entre 60 et 70°C
- le temps de réaction se situe entre 30 minutes et 72 heures, plus particulièrement entre 30 minutes et 12 heures, tout particulièrement entre 1 et 4 heures
- la concentration en ligand L' et en précurseur inorganique métallique se situe entre 1 et 200 mmol/L, plus particulièrement entre 30 et 100 mmol/L, tout particulièrement entre 60 et 70 mmol/L
- un monoacide carboxylique est ajouté, de préférence l'acide acétique. Il est entendu que d'autres additifs tel que des modificateurs de pH (acides, bases), minéralisateurs peuvent également être ajoutés.

### Voie ultrasons

- la température de réaction est de préférence entre -5°C et 20°C, plus particulièrement entre -5°C et 10°C, tout particulièrement entre -5°C et 5°C
- le temps de réaction se situe entre 15 minutes et 2 heures, plus particulièrement entre 15 minutes et 1 heure, tout particulièrement entre 15 et 45 minutes
- la concentration en ligand L' et en précurseur inorganique métallique se situe entre 10 mol/l et 10⁻² mol/l, plus particulièrement entre 1 et 10⁻² mol/l, tout particulièrement entre 50 et 200 mmol/l
- un monoacide carboxylique est ajouté, de préférence l'acide acétique. Il est entendu que d'autres additifs tel que des modificateurs de pH (acides, bases), minéralisateurs peuvent également être ajoutés.

### Voie micro-ondes

- la température de réaction est de préférence entre 30°C et 300°C, plus particulièrement entre 30°C et 150°C, tout particulièrement entre 50°C et 120°C
- le temps de réaction se situe entre 1 minute et 3 heures, plus particulièrement entre 10 et 50 minutes, tout particulièrement entre 1 et 30 minutes
- la concentration en ligand L' et en précurseur inorganique métallique se situe entre 200 mol/l et 10⁻² mol/l, plus particulièrement entre 100 et 10⁻² mol/l, tout particulièrement entre 10 et 10⁻² mol/l
- un modificateurs de pH est ajouté, de préférence l'acide chlorhydrique. Il est entendu que d'autres additifs tel que des modificateurs de pH (acides, bases), minéralisateurs ou agents favorisant l'arrêt de la croissante crystalline (monoacide carboxylique) peuvent également être ajoutés.

Les autres phases « MIL » peuvent être obtenues sous forme de nanoparticules dans des conditions opératoires similaires, en utilisant les gammes de température, temps de réaction et concentration précitées, et avec l'ajout éventuel d'additifs tels que ceux précités.

Le procédé de préparation de l'invention a l'avantage de permettre l'obtention des matériaux de structure souhaitées, de grande pureté, et homogènes, en un nombre réduit d'étapes et avec des rendements élevés. Ceci réduit la durée de synthèse et les coûts de fabrication.

En outre, ce procédé permet d'accéder à des matériaux de structure déterminée et de contrôler la taille des particules en modulant l'un ou plusieurs des paramètres suivants : le temps de synthèse, le pH, l'ajout d'additifs, l'agitation, la nature du solvant, l'utilisation de la voie micro-ondes, etc.

Par ailleurs, les inventeurs ont également mis en évidence que les caractéristiques structurales particulières du solide de la présente invention, notamment en terme de flexibilité ou de taille des pores, qui lui confères des propriétés particulièrement intéressantes, notamment en terme de capacité d'adsorption, d'adsorption sélective et de pureté. Ces matériaux rendent donc possible l'adsorption sélective de molécules, comme par exemple des molécules pharmaceutiques, avec un coût énergétique favorable et un temps de relarguage plus élevé. Ainsi, les travaux de recherche menés par les inventeurs leur ont permis de mettre en évidence l'intérêt des matériaux MOF pour l'adsorption et le transport de principe actifs.

Ainsi, l'invention concerne également l'utilisation du solide MOF selon l'invention, lequel comprenant dans ses pores ou à sa surface au moins une molécule choisie dans le groupe comprenant un principe pharmaceutiquement actif, une substance active entrant dans la formulation d'une préparation cosmétique ou un marqueur.

En particulier, l'invention concerne également l'utilisation du solide MOF selon l'invention chargé en principe pharmaceutiquement actif comme médicament. Le principe pharmaceutiquement actif peut être contenu soit dans les pores, soit à la surface du solide selon l'invention. C'est ce que l'on entend dans la suite de ce document par « solide MOF chargé en principe pharmaceutiquement actif ».

Plus généralement, on entend par « solide MOF chargé en composant X » un solide MOF selon l'invention contenant dans ses pores ou à sa surface le composant X. Le composant X peut être adsorbé ou lié par liaison covalente, par liaison hydrogène, par liaison de Van der Waals, par interaction électrostatique à la surface ou dans les pores du solide MOF. Ce composant X peut être, comme indiqué ci-dessus, un principe pharmaceutiquement actif. Ou le composant X peut être toute molécule ayant une activité biologique, une substance active entrant dans la formulation d'une préparation cosmétique ou un marqueur.

En effet, le solide MOF selon l'invention a l'avantage de présenter de grandes capacités d'adsorption. En outre, il permet d'adsorber efficacement des molécules pharmaceutiques qui présentent des difficultés particulières d'encapsulation, par exemple en raison de leur instabilité, leur grande réactivité, leur faible solubilité, leur forte tendance à cristalliser, leur caractère hydrophile, amphiphile, etc...

Par exemple, le solide selon l'invention peut être chargé avec au moins un principe pharmaceutiquement actif qui présente l'une ou plusieurs des caractéristiques suivantes : hydrophile, amphiphile, lipophile, instable, toxique, à forte tendance à cristalliser ou sensiblement insoluble.

On entend par « toxique » un principe pharmaceutiquement actif ayant des effets toxiques susceptibles d'entraver son utilisation dans des applications médicales ou vétérinaires. Il peut s'agir par exemple d'agents alkylants tels que le busulfan, le cisplatine, les nitroso-urées comme la lomustine. Les agents alkylants forment après métabolisation, des liaisons covalentes avec les acides nucléiques. La formation de ces liaisons peut entraîner :
- Des troubles de la transcription et de la réplication de l'ADN
- Des substitutions de bases dans l'ADN
- Des excisions de bases et des ruptures caténaires de l'ADN.

Leur activité pharmacologique principale se manifeste durant la phase de synthèse de l'ADN. Leurs effets toxiques incluent : la myelosuppression, la stérilité et la leucémie non-lymphocytaire.

le Cisplatine provoque des ponts ADN intra-caténaire , possède une faible myelotoxicité, mais il est sévèrement émétisant et peut être néphrotoxique.

On entend par «à forte tendance à cristalliser» un principe pharmaceutiquement actif qui a tendance à s'associer à lui-même dans un réseau cristallin au lieu de s'inclure dans d'autres structures. Ainsi, un tel composé tend à former des cristaux lors du procédé d'encapsulation utilisé, au lieu de s'inclure dans les particules. Il résulte donc en fin de procédé un mélange de particules peu chargées en principe pharmaceutiquement actif et des cristaux de celui-ci. Il peut s'agir par exemple du Busulfan. A forte dose, il présente un effet secondaire grave qui est la maladie veino-occlusive du foie. Celle-ci résulte probablement de la très forte tendance à cristalliser de cette molécule. L'empilement cristallin est régi par de fortes interactions dipôle-dipôle entre les groupements méthylsulfonate de ce principe actif.

On entend par « sensiblement insoluble» un principe pharmaceutiquement actif dont la solubilité est inférieure à 0.1 mg/mL dans l'eau. Il peut s'agir par exemple du Busulfan.

On entend par « instable» un principe pharmaceutiquement actif qui peut se décomposer, cristalliser et/ou reagir en perdant sa structure et son activité. Il peut s'agir par exemple du Busulfan.

En outre, le principe pharmaceutiquement actif peut être toute molécule ayant une activité biologique, comme par exemple, un médicament, notamment un anticancéreux, un agent antiviral, un analogue nucléosidique, modifié ou non, un acide nucléique, un anticorps, une protéine, une vitamine, etc...

Parmi les principes actifs hydrophiles, on peut citer par exemple, l'AZT TP, CDV (cidofovir), le 5-fluoroacil , la citarabine.

Parmi les principes actifs amphiphiles, on peut citer par exemple, le busulfan, le chlorure de doxorubicine, le chlorure d'imipramine.

Parmi les principes actifs lipophiles, on peut citer par exemple, le tamoxifene, le docétaxel, le paclitaxel, l'ibuprofene, la lidocaine, les vitamines liposolubles telles que les vitamines A (rétinol), D (calciférol), E (tocophérol), K1 (phylloquinone), K2 (ménaquinone).

En particulier, le solide selon l'invention peut être chargé avec au moins un principe pharmaceutiquement actif choisi par exemple dans le groupe comprenant le taxotère, le busulfan, l'azidothymidine (AZT), l'azidothymidine phosphatée (AZTP), le cidofovir, la gemcitabine, le tamoxifène.

Dans un mode de réalisation, le principe actif peut être une molécule fluorescente. Par exemple, il peut s'agir des rhodamines, de la fluoresceine, la luciférase, du pyrène et ses dérivés, de l'aminopyrrolidino-7-nitrobenzofurazan.

Dans un mode de réalisation, le principe actif peut être une molécule fluorée; c'est-à-dire comprenant au moins un substituant F. Il peut s'agir par exemple de l'une des molécules fluorées citées précédemment. Ces molécules fluorées sont adaptées aux utilisations en imagerie, particulièrement l'imagerie fluorescence telle que la technique TEP précitée.

Ainsi, l'invention concerne également l'utilisation de nanoparticules de MOF encapsulant une ou plusieurs molécules fluorées selon l'invention comme marqueur utilisable en imagerie médicale, telle que l'imagerie TEP.

En outre, le solide selon l'invention peut être chargé avec au moins un composé d'intérêt en cosmétique, c'est-à-dire une substance active entrant dans la formulation d'une préparation cosmétique.

On entend par « composé d'intérêt cosmétique » toute substance active entrant dans la formulation d'une préparation cosmétique, c'est-à-dire une préparation destinée à être mise en contact avec diverses parties superficielles du corps humain, notamment l'épiderme, les systèmes pileux et capillaires, les organes externes, les dents et les muqueuses, en vue, exclusivement ou principalement, de les nettoyer, protéger, parfumer, maintenir en bon état le corps humain, de modifier son aspect ou d'en corriger l'odeur. Par « substance active », on entend une substance qui assure l'efficacité de la préparation cosmétique.

Le composé d'intérêt cosmétique est une substance active entrant dans la préparation de toute préparation cosmétique connue de l'homme du métier, par exemple, les produits d'hygiène (e.g., démaquillant, dentifrice, déodorant, gel douche, savon, shampoing), les produits de soin (e.g., crème antirides, crème de jour, crème de nuit, crème hydratante, eau florale, gommage, lait, masque de beauté, baume pour les lèvres, tonique), les produits capillaires (e.g., après-shampoing, défrisant, gel, huile, laque, masque, teinture), les produits de maquillage (e.g., anti-cerne, autobronzant, eyeliner, fard, fond de teint, khôl, mascara, poudre, produit pour blanchir la peau, rouge à lèvres, vernis à ongles), les parfums (e.g., eau de Cologne, eau de toilette, parfum), les produits solaires (e.g., crèmes, huiles ou lotions après-soleil et solaires), les produits pour le rasage et les produits dépilatoires (e.g., après-rasage, crème dépilatoire, mousse à raser), ou les préparations pour bains et douches (e.g., bain moussant, huile de bain, sels de bain).

Selon l'invention, le composé d'intérêt en cosmétique peut être choisi par exemple dans le groupe comprenant :
- un antioxidant (par exemple, l'acide citrique, le beta-carotene, la vitamine E, l'acide glycolique, le glutathion, la vitamine C, les polyphenols, le lycopene, les flavonoides, les tanins, les anthocyanes, la N-acetylcysteine (antiradicaux libre))
- une vitamine (par exemple, la vitamine A, B3, B5, B6, B2, B1, B9, B8, B12, C, E, D, K, K1, K2)
- un liporégulateur (par exemple, la caféine, la theophylline)
- un agent photoprotecteur (par exemple, la benzophénone 3 (2-Hydroxy-4-Methoxy Benzophenone), la benzophénone 4 (acide 2-Hydroxy-4-methoxybenzophenone-5-sulphonique), le 2-phenylbenzimidazole-5-sulfonique))
- un agent hydratant (par exemple, l'urée, l'acide hyaluronique, le sorbitol).

Par exemple, le solide selon l'invention peut être chargé avec au moins une substance active entrant dans la formulation d'une préparation cosmétique choisi dans le groupe comprenant la benzophénone, la visnadine, l'acide salicylique, l'acide ascorbique, la benzophénone et ses dérivés, la caféine, l'urée, l'acide hyaluronique, etc.

En particulier, le solide selon l'invention peut être chargé en principe pharmaceutiquement actif avec une capacité de charge de 1 à 200% en poids de solide sec, par exemple de 1 à 70% en poids de solide sec, soit près de 10 à 700 mg par gramme de solide sec.

Dans le cadre de la présente invention, la capacité de charge signifie la capacité de stockage de molécules ou la quantité de molécules adsorbé dans le matériau. La capacité de charge peut être exprimée en capacité massique (gramme/gramme) ou en capacité molaire (mol/mol) ou en d'autres (mol/gramme, gramme/mol, etc.)

Ainsi, le solide selon l'invention a l'avantage de présenter une capacité de charge inattendue, encore jamais atteinte dans l'art antérieur, notamment dans le cas du Busulfan. En effet, le solide de l'invention présente un micro-environnement interne hydrophobe/hydrophile favorable, notamment pour l'incorporation de molécules amphiphiles comme le busulfan.

En outre, une autre problématique de l'art antérieur concerne la libération rapide et non contrôlée des molécules transportées en l'absence d'affinité. Le solide MOF selon l'invention présente l'avantage de permettre des temps de relargage plus longs, notamment grâce au micro-environnement interne mais également grâce à la structure des composés. En effet, les phases rigides et flexibles des structures MOF ont une influence sur la cinétique de relargage des molécules. Notamment, les phases flexibles peuvent permettre une libération des composés plus longue dans le temps, par exemple avec l'Ibuprofène et le composé MIL-53.

Le solide selon l'invention peut également comporter, par exemple sur les ligands espaceurs, des groupements fonctionnels qui peuvent modifier les interactions entre le solide MOF selon l'invention et la molécule d'intérêt. Ceci peut permettre de contrôler l'encapsulation et la libération des molécules d'intérêt. Les matériaux MOF de l'invention peuvent ainsi être adaptés et formulés (« designés ») en fonction des molécules d'intérêt à transporter afin de moduler le taux d'encapsulation, le relarguage des molécules et/ou la dégradabilité du solide.

De plus, le solide MOF selon l'invention a fait l'objet d'études de toxicité très positives, décrites dans la partie « Exemples » ci-dessous. Il paraît également biodégradable et les études de dégradabilité sont encore en cours.

Ainsi, le solide MOF de la présente invention utilisé pour le transport des principes actifs permet de pallier les problèmes de l'art antérieur cités précédemment, notamment les problèmes liés à la toxicité, l'instabilité, la forte tendance à cristalliser des principes actifs, à leur libération contrôlée, etc.

En outre, le solide MOF selon l'invention rend possible l'incorporation de marqueurs dans ce matériau ce qui présente également un intérêt.

Ainsi, selon un mode particulier de réalisation, le solide selon l'invention, peut être chargé avec au moins une molécule d'intérêt qui peut être un principe pharmaceutiquement actif et/ou un composé d'intérêt en cosmétique et/ou un marqueur. La molécule d'intérêt peut être contenue soit dans les pores, soit à la surface du solide selon l'invention.

Ainsi, les solides MOF selon l'invention peuvent être utilisés pour la fabrication de médicaments, de compositions cosmétiques et/ou de marqueurs utilisables en imagerie médicale.

Ainsi, il est fourni un procédé de traitement d'un sujet affecté par une maladie, ledit procédé comprenant l'administration audit sujet d'un solide MOF selon l'invention comprenant dans ses pores ou à sa surface au moins un principe actif connu pour traiter ladite maladie.

En particulier, le solide MOF selon l'invention peut être chargé avec au moins un marqueur choisi dans le groupe comprenant un marqueur d'imagerie médicale, un agent de contraste, un traceur, un marqueur radioactif, un marqueur fluorescent, un marqueur phosphorescent.

Par exemple, les inventeurs décrivent dans la partie « Exemples » ci-dessous une modification de surface avec un composé fluorescent, en particulier le dextrane marqué à la fluorescéine. Cette modification permet la détection des particules à l'aide d'un microscope confocal. Le microscope confocal à balayage laser - MCBL (en anglais CLSM pour « *confocal laser scanning microscope* ») est un microscope optique qui a la propriété de réaliser des images de très faible profondeur de champ (environ 600 nm) appelées « sections optiques ». En positionnant le plan focal l'objectif à différents niveaux de profondeur dans l'échantillon, il est possible de réaliser des séries d'images à partir desquelles on peut obtenir une représentation tridimensionnelle de l'objet. Quelques applications possibles sont :
i) l'étude des interactions avec des lignées cellulaires ;
ii) si les particules de solide présentent des relaxivités compatibles à leur observation en imagerie médicale, elles peuvent être utilisées en imagerie multimodale comme suggéré dans la publication Mulder WJ, et al. *Nanomed.* 2007 Jun, 2(3), 307-324 [21].

En particulier, le solide selon l'invention peut être chargé avec au moins un marqueur choisi dans le groupe comprenant : un composé fluorescent, un oxyde de fer, un complexe de gadolinium, des ions gadolinium directement présents dans la structure, par exemple sous forme de complexe avec le ligand organique, etc. Les protocoles de charge en marqueur sont ceux connus de l'homme du métier. Des exemples non limitatifs utilisables sont décrits par exemple dans A.K. Gupta, et al., Nanomed. 2007 2(1), 23-39 [22] ; dans P Caravan, Chem. Soc. Rev., 2006, 35, 512-523 [23] ; ou dans Yan-Ping Ren, et al., Angew. Chem. Int. Ed. 2003, 42, No. 5, 532 [24].

Ainsi, le solide MOF selon l'invention peut être utilisé pour la fabrication, le transport et/ou la vectorisation de marqueurs.

En outre, le solide de l'invention peut être utilisé pour la vectorisation de médicaments lorsqu'il est chargé en principe pharmaceutiquement actif et/ou pour la détection et le suivi de maladies faisant intervenir des cibles biologiques (telles que le cancer) lorsqu'il est utilisé comme marqueur.

En outre, en cumulant ces deux utilisations, le solide de la présente invention permet avantageusement de visualiser la biodistribution d'un médicament. Ceci présente un grand intérêt, notamment pour le suivi d'un traitement thérapeutique et l'étude de la biodistribution d'un médicament.

Selon un mode particulier de réalisation de l'invention, le procédé de préparation du solide selon l'invention, peut comprendre en outre une étape (ii) d'introduction dans les pores ou à la surface du solide MOF, d'au moins une molécule d'intérêt, qui peut être un principe pharmaceutiquement actif et/ou un composé d'intérêt en cosmétique et/ou marqueur.

Ladite étape d'introduction peut être réalisée au cours de l'étape réactionnelle (i) ou après celle-ci de façon à obtenir un solide chargé en molécule d'intérêt.

Toute méthode connue de l'homme du métier peut être utilisée au cours de l'étape d'introduction (ii). La molécule d'intérêt peut être par exemple introduite dans le matériau MOF de la présente invention :
- par imprégnation, en immergeant le matériau dans une solution de la molécule d'intérêt ;
- par sublimation de la molécule d'intérêt, puis le gaz est adsorbé par le matériau ; ou
- par broyage par cylindre rotatif consistant à mélanger mécaniquement le matériau et la molécule d'intérêt.

D'autres avantages pourront encore apparaître à l'homme du métier à la lecture des exemples ci-dessous, en référence aux figures annexées, donnés à titre illustratif, et non limitatif.

### Brève description des figures

- La figure 1 représente les clichés de MEB (Microscopie Electronique à Balayage) du matériau MIL-53nano synthétisé selon l'Exemple 2.
- La figure 2 représente les clichés de MEB (Microscopie Electronique à Balayage) du matériau MIL-89nano synthétisé selon l'Exemple 2.
- La figure 3 représente les clichés de MEB du matériau MIL-88Anano synthétisé selon l'Exemple 2.
- La figure 4 représente les clichés de MEB du matériau MIL-100nano synthétisé selon l'Exemple 2.
- La figure 5 représente les clichés de MEB du matériau MIL-88Btnano synthétisé selon l'Exemple 2.
- La figure 6 représente les clichés de MEB du matériau MIL-88Bnano synthétisé selon l'Exemple 2.
- La figure 7 représente, en haut, le phénomène de respiration du composé MIL-53(Fe), et en bas, les diffractogrammes RX du solide MIL-53(Fe) en présence de différents solvants.
- La figure 8 représente la respiration des solides MIL-88A, MIL-88B, MIL-88C, MIL-88D et MIL-89. L'amplitude de gonflement entre formes sèches (en haut) et formes ouvertes (en bas) est représentée en % en bas de la figure.
- La figure 9 représente, en haut, l'étude de la réversibilité du gonflement du solide MIL-88A par diffraction des RX (λ-1.79Å), en bas, les diffractogrammes RX du solide MIL-88A en présence de solvants.
- La figure 10 représente le schéma explicatif de la flexibilité dans les phases hybrides MIL-53 (a) et MIL-88 (b et c).
- La figure 11 représente les clichés de MEB du matériau MIL-88A synthétisé selon l'Exemple 6, sans agitation (figure 11a) ou avec agitation (figure 11b).
- La figure 12 représente le cliché de microscopie électronique du solide MIL-101(Cr) obtenu par synthèse (10 minutes à 220°C).
- La figure 13 représente les coupes histologiques de foie de rat mises en évidence par une coloration hematoxiline-eosine et une coloration de Proust. La figure 13a concerne le test de contrôle, la figure 13b est obtenue 7 jours après l'injection de 200 mg/kg du matériau MIL-88A et la figure 13c est obtenue 7 jours après l'injection de 200 mg/kg du matériau MIL-88Bt.
- La figure 14 représente les diffractogrammes de poudre de rayon X des solides MIL-53 encapsulant différentes espèces : Diméthylformamide (DMF), H₂O, et Busulfan (adsorbé à partir de solutions de chloroforme ou d'acétonitrile). Le diffractogramme RX de la forme sèche est également représenté (« empty »).
- La figure 15 représente les diagrammes de DRX du matériau MIL-88A non modifié avant (MIL88A) et après l'adition d'une goutte d'eau (MIL88A + H₂O) ; MIL-88A modifie avec le chitosane 7% avant (MIL88AQ100) et après l'adition d'une goutte d'eau (MIL88A Q100 + H₂O) ; MIL-88A modifie avec le chitosane 2% avant (MIL88AQ25) et après l'adition d'une goutte d'eau (MIL88A Q125 + H₂O).
- La figure 16 représente l'analyse thermogravimétrique du matériau MIL-88A non modifie (MIL88A ; vert), modifie avec le chitosane 2% (MIL-88A-Q25, noir) et modifié avec le chitosane 7% (MIL-88A-Q100, rouge).
- La figure 17 représente les images de microscopie confocale du matériau MIL-100(Fe) modifié superficiellement avec du Dextrane-Fluoresceine-Biotine.
- La figure 18 représente les diffractogrammes RX des différentes formes de solide MIL-53 (de bas en haut : formes sèches, hydratée, brut de synthèse, MIL-53Bu1 et MIL-53Bu2).
- La figure 19 représente l'analyse thermogravimétrique (sous air) du composé MIL-53(Fe) hydraté (vitesse de chauffe de 5°C/minute).
- La figure 20 représente les diffractogrammes RX des solides MIL-88A sec (bas) et hydraté (haut).
- La figure 21 représente l'analyse thermogravimétrique (sous air) du composé MIL-88A hydraté (vitesse de chauffe de 5°C/minute)
- La figure 22 représente le diffractogramme RX du solide MIL-100(Fe).
- La figure 23 représente l'isotherme d'adsorption d'azote à 77 K du solide MIL-100 (Po=1 atm.).
- La figure 24 représente l'analyse thermogravimétrique (sous air) du composé MIL-100(Fe) brut de synthèse (vitesse de chauffe de 5°C/minute)
- La figure 25 représente diffractogramme RX du solide MIL-101(Fe) (λ_{Cu}=1.5406 Å).
- La figure 26 représente l'analyse thermogravimétrique (sous air) du composé MIL-101(Fe) hydraté (vitesse de chauffe de 5°C/minute).
- La figure 27 représente les phases rigides MIL-68 (à gauche), MIL-100 (en haut à droite) et MIL-101 (en bas à droite).
- La figure 28 représente l'évolution de la taille de particules (P en nm) en fonction du temps de synthèse (t en min) par voie ultrasons (0°C en présence ou non d'acide acétique) (Exemple 8).
- La figure 29 représente l'évolution de la taille de particules (P en nm) en fonction du temps (t en min) de la synthèse n°1 (0°C en présence ou non de PEG, ajouté 15 min après le début de la synthèse) par voie ultrasons (Exemple 25).
- La figure 30 représente l'évolution de la taille de particules (P en nm) en fonction du temps (t en min) de la synthèse n°2 (0°C en présence ou non de PEG, ajouté au temps t=0) par voie ultrasons (Exemple 25).
- La figure 31 représente le montage expérimental d'encapsulation par sublimation (Exemple 21).
- La figure 32 représente les molécules de rhodamine 116 perchlorate (A) et de fluorescéine (B).
- La figure 33 représente les molécules d'acide 8-hydroxypyrene-1,3,6-trisulfonique (C) et de (R)-(-)-4-(3-aminopyrrolidino)-7-nitrobenzofurazan (D).
- La figure 34 représente la libération de l'acide fumarique du solide MIL-88A en pourcentage (%) en fonction du temps t (en jours).
- La figure 35 représente la structure du solide MIL-100(Fe). (a) : trimère d'octaèdre et ligand trimésique ; (b) : supertétraèdre ; (c) : structure 3d schématique ; (d) : les deux types de cages mésoporeuses.
- La figure 36 représente les fenêtres pentagonales et hexagonales du solide MIL-100(Fe) après activation sous vide.
- Figure 37 : Haut : construction du solide MIL-101 à partir de trimères d'octaèdres de fer, d'acide 1,4 Benzenedicarboxylique pour former un supertétraèdre hybride et finalement une structure zéolithique hybride à grande taille de pores. Bas : vue schématique de la charpente poreuse et représentation des deux types de cages mésoporeuses, avec leur dimensions libres. Les octaèdres de fer, les atomes de carbone sont en vert et noir, respectivement.
- La figure 38 représente la structure du solide MOF MIL-102(Fe). Gauche : vue selon l'axe des tunnels (axe c) ; droite : vue selon l'axe perpendiculaire aux tunnels (axe b, vue similaire selon l'axe a). Les atomes de fer, de carbone et les molécules d'eau sont en vert, noir et rouge, respectivement.
- Figure 39 : Structure du solide MOF MIL-88B_4CH₃(Fe). Gauche : vue selon l'axe des tunnels (axe c) ; droite : vue selon l'axe des cages (axes a et b équivalent). Les octaèdres de fer et les atomes de carbone sont en orange et noir, respectivement.
- Figure 40 : Structure du carboxylate de fer MIL-88A (hydraté). Gauche : vue selon l'axe des tunnels (axe c) ; droite : vue selon l'axe perpendiculaire aux tunnels (axe b, vue similaire selon l'axe a). Les octaèdres de fer, les atomes de carbone et les molécules d'eau sont en vert, noir et rouge, respectivement.
- Figure 41 : Structure du carboxylate de fer MIL-89(Fe). Gauche : vue selon l'axe des tunnels (axe c) ; droite : vue selon l'axe perpendiculaire aux tunnels (axe b, vue similaire selon l'axe a). Les atomes de fer, de carbone et les molécules d'eau sont en gris, noir et blanc, respectivement.

### EXEMPLES

### I. SYNTHESES PRELIMINAIRES

### Exemple 1 Synthèse de l'acétate de fer(III), précurseur utilisé, et synthèses de ligands

### a) Synthèse A : acétate de fer(III)

La synthèse A de l'acétate de fer(III), utilisé dans les synthèses de matériaux MOF selon l'invention décrits dans les exemples suivants, peut se référer à la publication C.T. Dziobkowski, et al. Inorg. Chem., 1982, 21, 671 [25].

La synthèse A est la suivante : 6,72 g de poudre de fer métallique (Riedel-de Haën, 99%), 64 mL d'eau déionisée et 33,6 mL d'acide perchlorique à 70 % dans l'eau (Riedel-de Haën) sont mélangés sous agitation magnétique et chauffés à 50°C pendant 3 heures. Après arrêt du chauffage, la solution est agitée pendant 12 h (heures). Le fer métallique résiduel est éliminé par décantation suivie d'un changement de récipient. 20,6 mL de solution de peroxyde d'hydrogène dans l'eau (Alfa Aesar, 35%) sont ajoutés goutte à goutte sous agitation, le tout étant maintenu dans un bain de glace à 0°C. On ajoute 19,7 g d'acétate de sodium (Aldrich, 99%) à la solution bleue sous agitation en maintenant la solution à 0-5°C. On laisse la solution s'évaporer pendant 3 jours sous la hotte dans un cristallisoir (Volume=0,5 1) en verre. Finalement, les cristaux rouges d'acétate de fer sont récupérés par filtration et lavés très rapidement avec de l'eau déionisée glacée. Les cristaux sont ensuite séchés sous air.

### b) Synthèse B : acide 2,5-diperfluoro-1,4 benzènedicarboxylique

La synthèse est réalisée selon le protocole opératoire décrit par Kim et al., Chem. Commun., 2005, 372-374.

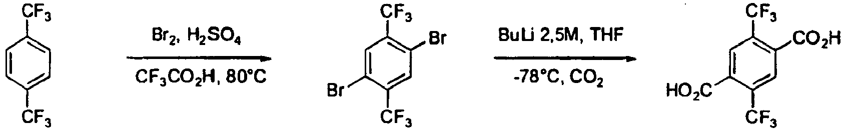

### Synthèse du 2,5-dibromo-1,4-bis(trifluorométhyl) benzene :

Dans un ballon d'un litre équipé d'un réfrigérant et d'un barreau aimanté, sont ajoutés successivement du 1,4-bis(trifluorométhyl)benzène (19 g, 88,7 mmol, ABCR), de l'acide trifluoroacétique (250 mL, SDS) et de l'acide sulfurique à 99% (60 mL, Acros). De la N-bromosuccinimide (47,4 g, 267 mmol, Aldrich) est ajoutée par petites portions à 60°C sur une période de 5 h. L'agitation est prolongée 48 h à cette température, puis le milieu est jeté dans de la glace (500 mL). Le précipité ainsi formé est filtré, séché sous vide (1 mmHg) pendant 24 h puis purifié par sublimation pour fournir 30 g (91%) d'un solide blanc.

Pt fusion : 65 ± 0.2°C ; RMN 1H (200 MHz, CDCl3) : 8,01 (2H, s) ; RMN 19F (188 MHz, CDCl3) : - 64,1 (2 X CF3, s).

### Synthèse de l'acide 2,5-bis(trifluorométhyl) téréphthalique:

Dans un bicol d'un litre équipé d'une ampoule de coulée et d'un barreau aimanté, une solution de, 2,5-dibromo-1,4-bis(trifluoromethyl)benzene (16 g, 43 mmol) dans le THF (100 mL, Acros) est additionnée goutte à goutte à une solution à -78°C, de butyl litio BuLi (2,5 M dans l'hexane, 38,4 mL, 67,2 mmol, Aldrich) dans le THF (75 mL). Après 30 min (minutes) d'agitation à cette température, de la carboglace (200 g) finement broyée est introduite dans le milieu réactionnel. La température est laissée remonter à l'ambiante et le milieu est extrait par une solution de soude (2M, 3 X 100). Les phases aqueuses sont rassemblées, acidifiées par une solution d'acide chlorhydrique 2M. Le précipité ainsi formé est filtré, séché sous vide (1 mmHg) pendant 24 h pour fournir 11 g (84%) d'un solide blanc.

Pt fusion : décomposition à 230°C ; RMN 1H (200 MHz, Acétone D6) : 8,31 (2H, s aromatique) ; RMN 19F (188 MHz, Acétone D6) : -55,9 (2 X CF3, s).

### c) Synthèse C : acide 2-méthyltéréphthalique

L'acide 2-méthyltéréphthalique est obtenu selon le mode de synthèse décrit par L. Anzalone, J. A. Hirsch, J. Org. Chem., 1985, 50, 2128-213 : 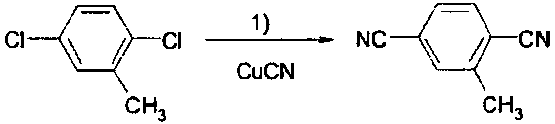 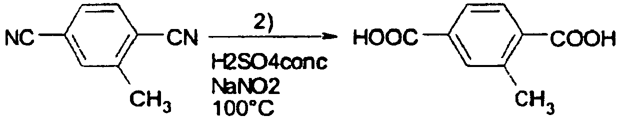
1) Dans un ballon sont introduits 10 g de CuCN (111,6 mmol), 4,2 mL de 2,5-dichlorotoluène (30,5 mmol) dans 26 mL de N-méthylpyrolidinone. Le tout est chauffés à reflux (200°C) pendant 24 h afin de substituer les atomes de Cl par des groupements nitriles.
   Après arrêt du chauffage, sont ajoutés au milieu réactionnel 50mL d'une solution aqueuse à 20% de NH₄OH, et 35 mL de toluène. Le mélange est agité, puis une fois refroidi à température ambiante, lui sont ajoutés 100 mL de d'éther et 50 mL d'une solution de NH₄OH à 20%. Les deux phases ainsi obtenues sont séparées à l'aide d'ajouts successifs d'éther (250mL), et enfin centrifugées (séparation difficile). La phase organique est ensuite lavée successivement par une solution de NH₄OH à 10% (4 x 50mL, jusqu'à ce que la phase aqueuse, basique, n'ait plus de coloration bleue), puis par H₂O, et enfin par une solution de HCl à 10%, et par une solution de NaCl saturée. Après séchage sur MgSO₄, filtration sur papier, et évaporation du solvant, 2,9 g de produit de coloration jaune sont obtenus. (Rendement de 67%).
2) Le 2-methyltéréphthalonitrile ainsi obtenu est ensuite ajouté à 70 mL de H₂SO₄ à 95%, le mélange est porté à 100 °C pendant une nuit, après arrêt du chauffage, lui sont ajoutés 35 mL d'H₂O, et une fois à température ambiante, 6,6 g de NaNO₂ dissous dans 30 mL d'H₂O. Le tout est porté à 110°C pendant une nuit. Enfin, après ajout de 200 mL d'H₂O sous agitation, filtration sur büchner, lavage à l'eau, et séchage sous vide à 50°C pendant une nuit, 2,13 g d'acide 2-methyl téréphthalique sont obtenus sous forme d'une poudre blanchâtre. (rendement de 58 %)

### d) Synthèse D : acide 3,5,3',5'-tétraméthylbiphényl-4,4'-dicarboxylique

Le schéma réactionnel de cette synthèse est représenté ci dessous :

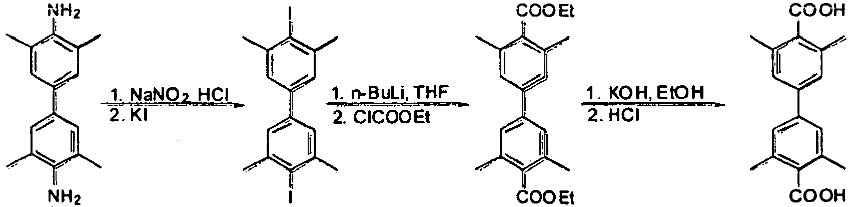

### 1^{ère} étape :

10,2 g de tétraméthylbenzidine (98%, Alfa Aesar) sont mis en suspension dans 39 mL d'acide chlorhydrique concentré (37%, commercialisé par la société Aldrich) à 0°C. La diazotation a été effectué par addition d'une solution du nitrite de sodium (6 g dans 50 mL d'eau). Après 15 min d'agitation à 0°C, une solution d'iodure de potassium (70 g dans 200 mL d'eau) a été ajoutée lentement à la solution violette résultante. Une fois l'addition terminée, le mélange est agité pendant 2 h à température ambiante. La suspension noire résultante est filtrée pour récupérer un précipité noir, lavé à l'eau. Le solide est mis en suspension dans le dichlorométhane (DCM, 98%, commercialisé par la société SDS) et une solution saturée de thiosulfate de sodium est ajoutée, causant la décoloration. Après 1 h d'agitation la phase organique est décantée et la phase aqueuse est extraite au DCM. La phase organique est séchée sur sulfate de sodium, puis évaporée pour donner l'intermédiaire diiodé sous forme d'un solide grisâtre. L'élution avec du pentane pur sur colonne de silice (commercialisé par la société SDS) permet d'obtenir le mélangé des composés monoiodé et diiodé. Le mélange de ces derniers a été directement employé dans l'étape suivante.

### 2^{e} étape :

7,2 g du composé iodé brut est solubilisé dans 100 mL de tétrahydrofurane (THF_{;} distillé sur sodium). Après refroidissement à -78°C, 35 mL de n-butyllithium dans le cyclohexane (2,5 M, commercialisé par la société Aldrich) sont ajoutés. La solution est laissée revenir à température ambiante, une suspension blanche apparaît après 2 h. Elle est à nouveau refroidie à -78°C et 12 mL d'éthylchloroformate sont ajoutés. Le mélange est laissé à température ambiante, une solution jaune limpide est obtenu après 1 h. Une partition entre l'eau et le dichlorométhane suivi d'une extraction au dichlorométhane donne le diester brut. Celui-ci est purifié par chromatographie sur gel de silice, avec pour éluant un mélange Et20/Pentane : 1/9, (rapport frontal : Rf = 0,3). 6,3 g de diester sont obtenus sous la forme d'un solide incolore (rendement de 42 % à partir de la benzidine).

Caractérisation du diester obtenu : RMN 1H (300 MHz, CDCl3): δ (ppm) : 1,29 (t, J = 7,2 Hz, 6H), 2,29 (s, 13H); 4,31 (q, J = 7,2 Hz, 4H); 7,12 (s, 4H). RMN 13C (75 MHz, CDCl3): δ (ppm) : 14,3 (CH3), 19,9 (CH3), 61,0 (CH2), 126,5 (CH), 133,2 (Cq), 135,5 (Cq), 141,4 (Cq), 169,8 (Cq).

### 3^{e} étape :

Finalement, le diester est saponifié avec 9,7 g d'hydroxyde de potassium (commercialisé par la société VWR) dans 100 mL d'éthanol 95% (commercialisé par la société SDS), au reflux pendant 5 jours. La solution est concentrée sous vide et le produit est solubilisé dans l'eau. De l'acide chlorhydrique concentré est ajouté jusqu'à pH 1, et un précipité blanc est formé. Il est récupéré par filtration, lavé à l'eau et séché. 5,3 g de diacide sont ainsi obtenus sous forme de solide blanc (rendement quantitatif).

### e) Synthèse E : acide 3,3'-dimethylbiphenyl 4,4'-dicarboxylique

Le schéma réactionnel de cette synthèse est représenté ci-dessous :

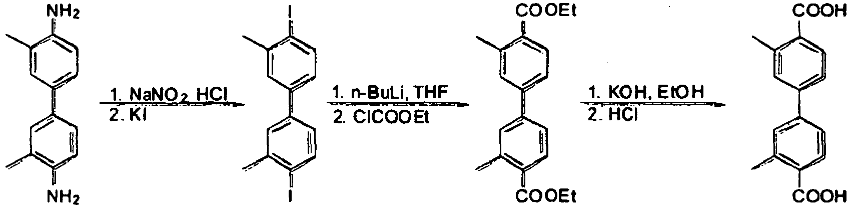

La même procédure que celle décrite pour la synthèse D a été utilisée en partant de 12,1 g de diméthylbenzidine. A l'issu de la 1ère étape, 18,4 g de 3,3'-diméthyl-4,4'-diiodo-biphényle sont obtenus (rendement: 74%).

Caractérisation du composé diiodé obtenu : RMN 1H (300 MHz, CDCl3): δ (ppm) : 2,54 (s, 6H), 7,10 (dd, J = 2,2 et 8,1 Hz, 2H), 7,46 (d, J = 2,2 Hz, 2H), 7,90 (d, J = 8,1 Hz, 2H). RMN 13C (75 MHz, CDCl3): δ (ppm) : 28,3 (CH3), 100,3 (Cq), 126,0 (CH), 128,3 (CH), 139,4 (CH), 140,4 (Cq), 141,9 (Cq).

A l'issu des 2e et 3e étapes, 6,9 g d'acide 3,3'-dimethyl-biphenyl-4,4'-dicarboxylique sont obtenus à partir des 18,4 g de composé diiodé.

Caractérisation des composés obtenus : Le diester obtenu à l'issu de la 2e étape et le diacide obtenu à l'issu de la 3e étape ont des signatures spectroscopiques identiques à celles décrites dans la littérature (Shiotani Akinori, Z. Naturforsch. 1994, 49, 12, 1731-1736).

### f) Synthèse F : acide 3,3'-dichloro4,4'-azobenzenedicarboxylique

15g d'acide o-chlorobenzoïque (Aldrich, 98%) et 50g de soude sont placés dans 225 mL d'eau distillée, et chauffés à 50°C sous agitation. Une solution de 100g de glucose (Aldrich, 96%) dissous dans 150 mL d'eau est ajoutée. Le mélange est agité 15 minutes, puis un bullage d'air est effectué pendant 3 h à température ambiante. Le sel de disodium est récupéré par filtration, lavé à l'éthanol, puis dissout à nouveau dans 120 mL d'eau. De l'acide chlorhydrique (Aldrich VWR, 37%) est ajouté jusqu'à obtenir un pH égal à 1. Le solide est récupéré par filtration et séché sous vide à 90°C.

### g) Synthèse G : acide 3,5,3',5'-azobenzenetetracarboxylique

19 g d'acide 5-nitroisophthalique (Aldrich, 98%) et 50 g de soude sont placés dans 250 mL d'eau distillée, et chauffés à 50°C sous agitation. Une solution de 100g de glucose (Aldrich, 96%) dissous dans 150 mL d'eau est ajoutée. Le mélange est agité 15 minutes, puis un bullage d'air est effectué pendant 3 h à température ambiante. Le sel de disodium résultant est récupéré par filtration, dissout dans 300 mL d'eau à température ambiante. De l'acide chlorhydrique (VWR, 37%) est ajouté jusqu'à obtenir un pH égal à 1. Le solide est récupéré par filtration et séché sous vide à 90°C.

### h) Synthèse H : acide chlorotéréphthalique

6 g (0,043 mol) de chloroxylene (commercialisé par la société Aldrich, > 99%), 16 mL d'acide nitrique (commercialisé par la société VWR, 70%) et 60 mL d'eau distillée sont introduits dans un corps en téflon de 120 mL. Celui-ci est placé dans une bombe métallique PAAR, chauffé à 170°C pendant 12h. Le produit est récupéré par filtration, puis lavé abondamment à l'eau distillée. Un rendement de 75% est obtenu.

RMN 1H (300 MHz, d6-DMSO) : δ (ppm) : 7,86 (d, J = 7,8 Hz), 7,93 (dd, J = 7,8; 1,2 Hz), 7,96 (d, J = 1,2 Hz)

### II. NANOPARTICULES SELON L'INVENTION ET PROCEDES DE PREPARATION DES NANOPARTICULES SELON L'INVENTION

### Exemple 2 : Synthèse de nanoparticules carboxylate de fer

### a) Synthèse de nanoparticules MIL-53nano

Le solide MIL-53nano a été obtenu dans sa forme de nanoparticules à partir de 270 mg de FeCl₃.6H₂O (1 mmol; Alfa Aesar, 98%), 166 mg d'acide téréphthalique (1 mmol; 1,4-BDC; Aldrich, 98%) dans 5 mL de diméthylformamide (DMF; Fluka, 98%), le tout introduit dans un corps en Téflon mis dans un corps métallique (autoclave) de marque Paar. Le tout est chauffé à 150°C pendant 2 ou 4 heures. Après retour à température ambiante, le solide est récupéré par centrifugation à 5000 rpm (ou rotation par minute) pendant 10 minutes.

200 mg du solide sont ensuite suspendus dans 100 mL d'eau distillée sous agitation pendant 15heures pour éliminer le solvant résiduel présent dans les pores. Après, le solide est récupéré par centrifugation à 5000 rpm pendant 10 minutes. La taille des particules mesurée par diffusion de lumière est d'environ 350 nm.

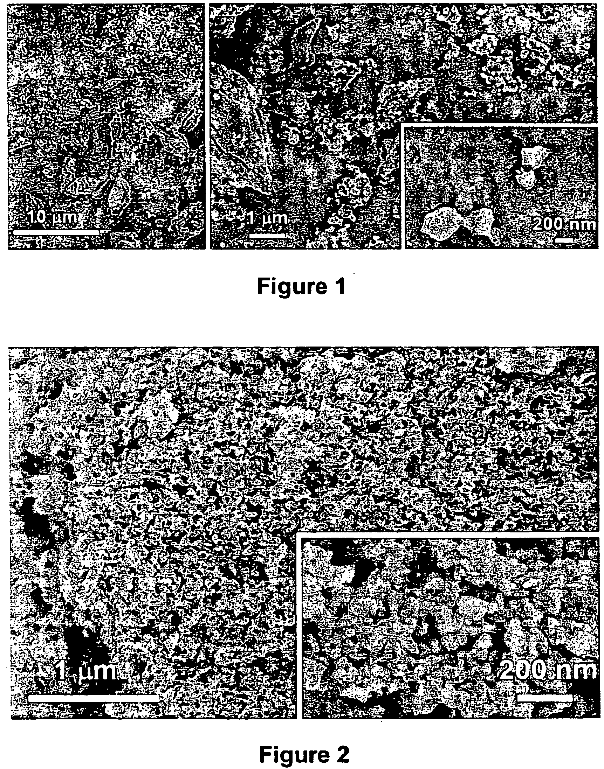

Les images de la microscopie électronique a balayage (MEB) du matériau MIL-53 de la présente invention sont présentées en figure 1 et montrent la présence de deux population de particules, une de grande taille (environ 5 µm) et des autres petites (environ 350 nm). Les grandes particules sont plutôt rhomboédriques, sans doute de l'acide carboxylique recristallisé ; par contre, la morphologie des petites particules est plutôt sphérique, et se présente sous forme d'agrégats.

### b) Synthèse de nanoparticules MIL-89nano

La synthèse du MIL-89nano se fait à partir de 210 mg d'acétate de fer (0,33 mmol; synthétisé au laboratoire selon la synthèse A décrite ci-dessus), de 142 mg d'acide muconique (1 mmol; Fluka, 97%) en présence de 5 mL d'éthanol (Riedel-de Haën, 99,8%) avec l'adition de 0,25mL d'hydroxyde de sodium 2M (Alfa Aesar, 98%) le tout introduit dans un corps en Téflon mis dans un corps métallique (autoclave) de marque Paar. Le tout est chauffé à 100°C pendant 12 heures.

Après retour à température ambiante, le produit est récupéré par centrifugation à 5000rpm pendant 10 minutes. 200 mg du solide sont alors suspendus dans 100mL d'eau distillée sous agitation pendant 15h pour éliminer le solvant résiduel présent dans les pores. Le solide est ensuite récupéré par centrifugation à 5000rpm pendant 10 minutes.

La taille des particules mesurée par diffusion de lumière est 400 nm. Les nanoparticules montrent une morphologie arrondie et légèrement allongée, avec une taille de particules, mesurée par microscopie électronique à balayage, très homogène de 50-100 nm (figure 2). Il est donc clair que les objets de 400 nm mesurés par diffusion de lumière correspondent à des agrégats de particules de MIL-89nano

### c) Synthèse de nanoparticules MIL-88Anano

Pour obtenir le matériau MIL-88Anano, 270 mg de FeCl₃.6H₂O (1 mmol; Alfa Aesar, 98%), 112 mg d'acide fumarique (1 mmol; Acros, 99%) sont mélangés dans 15 mL d'éthanol (Riedel-de Haën, 99,8%) et 1 mL d'acide acétique (Aldrich, 99,7%). La solution est placée dans un flacon en verre et chauffée à 65°C pendant 2 heures. Le solide est récupéré par centrifugation à 5000 rpm pendant 10 minutes.

200 mg du solide sont suspendus dans 100mL d'eau distillée sous agitation pendant 15h pour éliminer résiduel présent dans les pores. Le solide est ensuite récupéré par centrifugation à 5000rpm pendant 10 minutes.

La taille de particule mesurée par diffusion de lumière est de 250 nm.

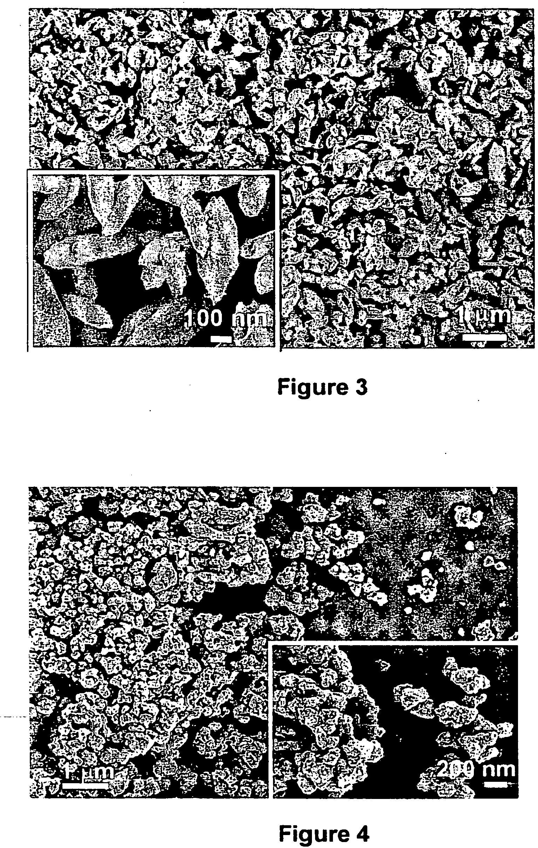

La microscopie électronique à balayage (figure 3) montrent des particules allongées avec des arêtes. Ils existent deux tailles de particules, environ 500 nm et 150 nm. La taille mesurée par diffusion de lumière correspond donc à une taille moyenne de MIL-88Anano.

### d) Synthèse de nanoparticules MIL-100nano

La synthèse du MIL-100nano est effectuée à partir de 270 mg de FeCl₃.6H₂O (1 mmol; Alfa Aesar, 98%), 210 mg d'acide 1,3,5-benzenetricarboxylique (1,3,5-BTC ; 1 mmol; Aldrich, 95%) dans 3 mL d'eau distillée. Le tout est introduit dans un corps en Téflon mis dans un corps métallique (autoclave) de marque Paar. Le tout est chauffé pendant 12 heures à 100°C. Le produit est récupéré par centrifugation à 5000 rpm pendant 10 minutes.

200 mg de solide sont suspendus dans 100mL d'eau distillée sous agitation et reflux pendant 3h pour éliminer l'acide résiduel présent dans les pores. Le solide est ensuite récupéré par centrifugation é 5000rpm pendant 10 minutes.

La taille de particule mesurée par diffusion de lumière est de 535 nm.

La microscopie électronique à balayage (figure 4) montre une forte agrégation des particules. Celles-ci sont plutôt sphériques, avec une taille approximative de 40 à 60 nm.

### e) Synthèse de nanoparticules MIL-101nano

Pour l'obtention du solide MIL-101nano, 270 mg de FeCl₃.6H₂O (1 mmol; Alfa Aesar, 98%), 250 mg d'acide 1,4-benzenedicarboxylique (1,5 mmol; 1,4-BDC Aldrich, 98%) sont mélangés dans 10 mL de diméthylformamide (Fluka, 98%), le tout placé dans une bombe Paar et chauffé à 100°C pendant 15 heures. Le solide est récupéré par centrifugation à 5000 rpm pendant 10 minutes.

Pour éliminer l'acide résiduel présent dans les pores, le produit est chauffe à 200°C sous vide pendant 1 jour. Noter qu'il faut alors conserver sous vide ou atmosphère inerte car le produit n'est pas stable à l'air ou en présence d'eau. La taille de particule mesurée par diffusion de lumière est 310 nm.

### f) Synthèse de nanoparticules MIL-88Btnano

Le solide MIL-88Btnano est synthétisé à partir de 270 mg de FeCl₃.6H₂O (1 mmol; Alfa Aesar, 98%), 222 mg d'acide 1,4-benzènetetramethyldicarboxylique (1 mmol; Chem Service) et de 10 mL de diméthylformamide (Fluka, 98%) en présence de 0,4 mL de solution aqueuse de NaOH 2M. Le tout est introduit dans un corps en Téflon mis dans un corps métallique (autoclave) de marque Paar, puis chauffé à 100°C pendant 2 heures. Après retour à température ambiante, (la bombe métallique est refroidie dans de l'eau froide), le produit est récupéré par centrifugation à 5000rpm pendant 10 minutes.

200 mg du solide sont ensuite suspendus dans 100mL d'eau distillée sous agitation pendant 15h pour éliminer le solvant résiduel présent dans les pores. Le solide est récupéré par centrifugation à 5000rpm pendant 10 minutes.

La mesure de taille de particule par diffusion de lumière montre deux populations de nanoparticules de 50 et 140 nm.

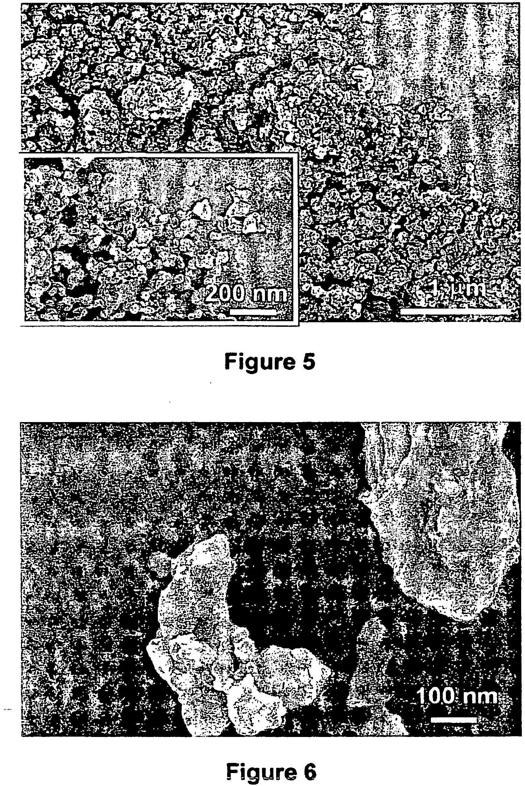

La microscopie électronique à balayage (figure 5) montre que les particules possèdent une morphologie sphérique avec une taille d'environ. 50 nm Seulement une fraction minoritaire possède une taille d'environ 200 nm. On peut y observer aussi des agglomérats de petites particules.

### g) Synthèse de nanoparticules MIL-88Bnano

Le solide MIL-88Bnano est synthétisé à partir de 240 mg d'acétate de fer (0,33 mmol, synthétisé au laboratoire selon la synthèse A décrite ci-dessus), 166 mg d'acide 1,4-benzenedicarboxylique (1 mmol ; 1,4-BDC Aldrich, 98%) introduit dans 5 mL de méthanol (Alcrich, 99%). Le tout est introduit dans un corps en Téflon mis dans un corps métallique (autoclave) de marque Paar, et chauffé à 100°C pendant 2 heures. Après retour à température ambiante, (la bombe métallique est refroidie dans de l'eau froide), le produit est récupéré par centrifugation à 5000rpm pendant 10 minutes.

200 mg du solide sont suspendus dans 100mL d'eau distillée sous agitation pendant 15h pour éliminer le solvant résiduel. Le solide est ensuite récupéré par centrifugation à 5000rpm pendant 10 minutes.

La mesure de la taille de particule par diffusion de lumière montre une distribution bimodale de nanoparticules de 156 et 498 nm.

La morphologie des particules observée par microscopie est très irrégulière, avec une taille moyenne proche de 300 nm (figure 6).

La détermination de la taille de particule par diffusion de lumière a été effectuée avec un appareil Malvern Zetasizer Nano série - Nano-ZS; modèle Zen 3600 ; serial N° 500180 ; UK.

La microscopie électronique à balayage a été réalisée en utilisant un microscope Topcon (Akashi) EM 002B ultra-haut résolution 200kV.

Les différences entre les valeurs obtenues à partir de ces deux techniques s'expliquent d'une part par la coloration orange de particules de carboxylates de fer, ce qui n'est pas idéal compte tenu de la couleur rouge du faisceau laser de l'appareil de diffusion de lumière, et d'autre part à une tendance plus ou moins prononcée des particules à s'agglomérer.

### h) MIL-102(Fe) ou Fe₆O₂X₂[C₁₀H₂-(CO₂)₄]₃·nH₂O (X=F, Cl...)

### Synthèse du solide MIL-102(Fe) non fluoré :

270 mg (1 mmol) de FeCl₃.6H₂O (Alfa Aesar, 98%) et 268 mg (1 mmol) d'acide 1,4,5,8-naphthalenetétracarboxylique sont dispersés dans 5 ml d'eau distillée. Le mélange est laissé dans un corps en Téflon de 23 ml mis dans une Bombe métallique PAAR pendant 15 heures à 100°C. Le solide est récupéré par filtration.

### Données caractéristiques du solide MIL-102(Fe)

Ce composé présente une surface spécifique faible (surface de Langmuir ; 101 m²/g) à l'azote à 77 K.

### Obtention du solide MIL-102(Fe) fluoré:

0,2 g de solide MIL-102(Fe) non fluoré de formule Fe₆O₂Cl₂[C₁₀H₄(CO₂)₄]₃.nH₂O obtenu selon le mode opératoire décrit précédemment est mis en présence de 1 g de fluorure de sodium NaF dans 100 ml d'eau distillée. Le mélange est laissé sous agitation dans un corps en Téflon de 125 ml pendant 15 heures à température ambiante. Le solide est récupéré par filtration et lavé à cinq reprises dans de l'eau distillée pour évacuer les traces de NaF. L'analyse semi-quantitative par EDX indique une teneur en fluor de 0.17 fluor par fer. Le solide ainsi traité possède une formule approximative de type Fe₆O₂F(OH) [C₁₀H₄(CO₂)₄]₃.nH₂O.

### i) Données analytiques des nanoparticules carboxylate de fer

La taille de particules est mesurée avec une équipe de diffusion de lumière Coulter N4MD, coulter Electronics, Margency, France) en utilisant des suspensions aqueuses du matériau à 0,5 mg/mL.

Le potentiel Z est mesuré à partir des suspensions aqueuses à 0,5 mg/mL dans un milieu NaCl 0,1 M sur un appareil Malvern Zetasizer Nano serie - Nano-ZS equipment ; model Zen 3600.

La taille de particule est mesurée sur l'appareil de potentiel Z en utilisant des solutions aqueuses de matériau 0,5 mg/mL.

Le Tableau 1 ci-dessous présente les caractéristiques des différents matériaux MOF obtenus, notamment la taille des nanoparticules, estimées par diffusion quasi élastique de lumière ou par microscopie électronique, la taille des pores et le potentiel zeta.

**Tableau 1. Structures « MIL » de quelques carboxylates de fer(III) selon l'invention.**

| **Nanosolide MIL-n** | **Fraction organique** | **Formule** |
|---|---|---|
| **MIL-53** | Acide 1,4-Benzene dicarboxylique (Acide téréphthalique ou Acide 1,4-BDC) | Fe(OH)[O₂C-C₆H₄-CO₂].H₂O |
| **MIL-88A** | Acide fumarique | Fe₃OX[O₂C-C₂H₂-CO₂]₃.nH₂O |
| **MIL-88B** | Acide téréphthalique | Fe₃OX[O₂C-C₆H₄-CO₂]₃.nH₂O |
| **MIL-88BT** | Acide tetraméthyl téréphtalique | Fe₃OX[O₂C-C₆(CH₃)₄-CO₂]₃.nH₂O |
| **MIL-89** | Acide muconique | Fe₃OCl[O₂C-C₄H₄-CO₂]₃.nH₂O |
| **MIL-100** | Acide 1,3,5-Benzene tricarboxylique (Acide 1,4-BTC) | Fe₃OX[C₆H₃-[CO₂]₃].nH₂O |
| **MIL-101** | Acide téréphthalique | Fe₃OX[O₂C-C₆H₄-CO₂]₃.nH₂O |

**Tableau 2. Caractéristiques des structures « MIL » de carboxylates de fer(III).**

| **MIL-n** | **% de Fer*** | **Diamètre des pores (Å)**** | **Taille particules (diffusion) (nm)** | **Taille particules (microscopie) (nm)** | **Pot. Z *** (mV)** | **Flex. ****** | **Base métallique** |
|---|---|---|---|---|---|---|---|
| **MIL-53** | 23,6 % | 8,6 | 350 | 350 et 5000 | 6 | oui | Chaîne d'octaèdre |
| **MIL-88A** | 30,8 % | 6 | 250 | 150 et 500 | 13-34 | oui | Trimère d'octaèdre |
| **MIL-88B** | 24,2 % | 9 | 150 et 500 | 300 | 14 | oui | Trimère d'octaèdre |
| **MIL-88BT** | 19,4 % | 8 | 50 et 140 | 50 et 200 | 19 | oui | Trimère d'octaèdre |
| **MIL-89** | 26,2% | 11 | 400 | 50-100 | 7 | oui | Trimère d'octaèdre |
| **MIL-100** | 27,3 % | 25-29 | 530 | 40-60 | -25 | non | Trimère d'octaèdre |
| **MIL-101** | 24,2 % | 29-34 | 310 | - | 29 | non | Trimère d'octaèdre |

| | | | | | | | |
|---|---|---|---|---|---|---|---|
| * % théorique de fer en phase sèche ** taille de pore calculée à partir des structures cristallographiques *** Potentiel Z **** Flexibilité | | | | | | | |

### Exemple 3 : Synthèse de matériaux MOF à base de ligands fonctionnalisés

### a) MIL-101-Cl (Fe) ou Fe₃O[Cl-C₆H₃-(CO₂)₂]₃.X.nH₂O (X=F, Cl, OH)

Les conditions de synthèse sont les suivantes : 0,27 g (1 mmol) de FeCl₃.6H₂O et 210 mg d'acide chloro-1,4-benzenedicarboxylique (1,0 mmol, Cl-1,4-BDC, synthétisé selon la synthèse H décrite dans l'exemple 1) sont dispersés dans 10 ml de DMF (diméthylformamide, Fluka, 98%). Le tout est laissé 12 h (heures) à 100°C dans un corps en Téflon de 23 ml mis dans une Bombe métallique PAAR. Le solide est ensuite filtré et lavé avec de l'acétone.

L'optimisation des conditions de vidage des pores est en cours.

Paramètres de maille du solide MIL-101(Fe) à 298 K : a=89,0 Å et V=707000 Å³, groupe d'espace Fd-3m (n°227).

La taille de particule monodisperse (Indice de polydispersité, PDI=0,225) mesurée par diffusion de lumière est 400 nm.

### b) MIL-101-NH₂(Fe) ou Fe₃O[NH₂-C₆H₃-(CO₂)₂]₃.X.nH₂O (X=F, Cl, OH)

2,25 g (0,92 mmol) de FeCl₃.6H₂O et 0,75 mg d'acide amino-1,4benzènedicarboxylique (0,41 mmol, NH₂-1,4-BDC, Aldrich 99%) sont dispersés dans 50 ml de DMF (diméthylformamide, Fluka, 98%). Le tout est laissé 24 h à 110°C dans un corps en Téflon de 23 ml mis dans une Bombe métallique PAAR. Le solide est ensuite filtré et lavé avec de l'acétone.

Le solide est chauffe a 120°C sous vide pendant 16 h pour éliminer l'acide qui reste dans les pores. Par contre, l'optimisation de ces conditions de vidage des pores est encore en cours.

Paramètres de maille du solide MIL-101(Fe) à 298 K : a=89,0 Å et V=707000 Å³, groupe d'espace Fd-3m (n°227).

La taille de particule monodisperse (PDI=0.086) mesurée par diffusion de lumière est de 391 nm.

### c) MIL-101-2CF₃ (Fe) ou Fe₃O[(CF₃)₂-C₆H₂-(CO₂)₂]₃-X-n3₂O (X=F, Cl, OH)

135 mg (0,5 mmol) de FeCl₃.6H₂O et 151 mg d'acide 2,5-diperfluoro-1,4 benzenedicarboxylique (0,5 mmol, 2CF,-1,4-BDC, synthétisé selon la synthèse B décrite dans l'exemple 1) sont dispersés dans 5 ml de DMF (Fluka, 98%). Le tout est laissé 12 h à 90°C dans un corps en Téflon de 23 ml mis dans une Bombe métallique PAAR. Le solide est ensuite récupéré par centrifugation à 10000 rpm pendant 10 min.

L'optimisation des conditions de vidage des pores est en cours.

Paramètres de maille du solide MIL-101(Fe) à 298 K : a=89,0 Å et V=707000 Å³, groupe d'espace Fd-3m (n°227)

La taille de particule monodisperse (PDI=0.145) mesuré par diffusion de lumière est 340 nm.

### d) MIL-88B-NO₂ (Fe) ou Fe₃O[C₆H₃NO₂-(CO₂)₂]₃.X.nH₂O (X=F, Cl, OH)

0,27 g de FeCl₃.6H₂**O** (1 mmol, Alfa Aesar, 98%) et 211 mg (1 mmol) d'acide 1,4-nitrotéréphthalique (Acros, 99%) sont dispersés dans 5 ml de l'eau distillée. Le tout est laissé dans un corps en Téflon de 23 ml mis dans une Bombe métallique PAAR pendant 12 h à 100°C. Le solide est récupéré par filtration.

200 mg du solide sont suspendus dans 10mL de l'éthanol absolu dans un corps en Téflon de 23 ml mis dans une Bombe métallique PAAR pendant 12 h à 100°C pour éliminer l'acide restant dans les pores. Puis, le solide est récupéré par filtration et séchée a 100°C.

**Tableau 3. Analyse élémentaire (CNRS, Vernaison).**

| Elément/ % massique | % Fer | % Carbone | % Azote |
|---|---|---|---|
| MIL-88B NO₂ | 20,6 | 39,3 | 4,6 |

La taille de particule mesurée monodisperse (PDI=0.005) par diffusion de lumière est 345 nm.

### e) MIL-88B-20H (Fe) ou Fe₃O[C₆H₂(OH)₂-(CO₂)₂]₃-X-nH₂O (X=F, Cl, OH)

354 mg de Fe(ClO₄)₃.xH₂O (1 mmol, Aldrich, 99%) et 198 mg (1 mmol) d'acide 1,4-dihydroxoterephthalique (obtenu par hydrolyse de l'ester diéthylique correspondant, Aldrich 97%) sont dispersés dans 5 ml de DMF (Fluka, 98%). Le tout est laissé dans un corps en Téflon de 23 ml mis dans une Bombe métallique PAAR pendant 12 h à 85°C. Le solide est récupéré par filtration, puis calciné à 150°C sous vide pendant 15 h pour éliminer l'acide restant dans les pores.

**Tableau 4. Analyse élémentaire (CNRS, Vernaison).**

| élément/ % massique | % Fer | % Carbone |
|---|---|---|
| MIL-88B 20H | 15,4 | 36,5 |

La taille de particule légèrement polydisperse (PDI=0.305) mesurée par diffusion de lumière est de 213 nm.

### f) MIL-88B-NH₂ (Fe) ou Fe₃O[C₆H₃NH₂-(CO₂)₂]_{3·}X.nH₂O (X=F, Cl, OH)

0,27 g de FeCl₃.6H₂O (1 mmol, Alfa Aesar, 98%) et 180 mg (1 mmol) d'acide 1,4-aminoterephthalique (Fluka, 98%) sont dispersés dans 5 ml de l'éthanol absolu. Le tout est laissé dans un corps en Téflon de 23 ml mis dans une Bombe métallique PAAR pendant 3 jours à 100°C. Le solide est récupéré par filtration puis calciné à 200°C pendant 2 jours pour éliminer l'acide restant dans les pores.

La taille de particule monodisperse (PDI=0.268) mesurée par diffusion de lumière est de 102 nm.

### g) MIL-88B-CH₃ (Fe) ou Fe₃O[C₆H₃CH₃-(CO₂)₂]₃.X.nH₂O (X=F, Cl, OH)

354 mg de Fe(ClO₄)₃.xH₂O (1 mmol, Aldrich, 99%) et 180 mg (1 mmol) d'acide 1,4-methylterephthalique (préparé suivant la synthèse C) sont dispersés dans 5 ml de méthanol (Fluka, 99%). Le tout est laissé dans un corps en Téflon de 23 ml mis dans une Bombe métallique PAAR pendant 3 jours à 100°C. Le solide est récupéré par filtration.

200 mg du solide sont suspendus dans 10mL de DMF sous agitation à température ambiante pour échanger l'acide restant par le DMF, puis le DMF est éliminé DMF par évaporation à 150°C sous vide pendant 12 h.

La taille de particule mesurée monodisperse (PDI=0.231) par diffusion de lumière est de 430 nm.

### h) MIL-88B-Cl (Fe) ou Fe₃O[C₆H₃Cl-(CO₂)₂]₃.X.nH₂O (X=F, Cl, OH)

354 mg de Fe(ClO₄)₃.xH₂O (1 mmol, Aldrich, 99%) et 200 mg (1 mmol) d'acide 1,4-chloroterephthalique (préparé selon la synthèse décrite dans l'exemple 1) sont dispersés dans 10 ml de DMF avec 0,1 mL de HF 5M (acide fluorhydrique, SDS, 50%) et 0,1 mL de HCl 1M (acide chlorhydrique, Aldrich, 37%). Le tout est laissé dans un corps en Téflon de 23 ml mis dans une Bombe métallique PAAR pendant 5 jours à 100°C. Le solide est récupéré par filtration puis calciné à 150°C sous vide.

La taille de particule mesurée par diffusion de lumière est 255 nm, avec une deuxième population de plus de 1 micron.

### i) MIL-88B-4CH₃ (Fe) ou Fe₃O[C₆(CH₃)₄-(CO₂)₂]₃-X-nH₂O (X=F, Cl, OH)

0,27 g de FeCl₃.6H₂O (1 mmol, Alfa Aesar, 98%) et 222 mg (1 mmol) d'acide 1,4-tétraméthyltéréphthalique (Chem Service, 95%) sont dispersés dans 10 ml de DMF (Fluka, 98%) avec 0,4 mL de NaOH 2M (Alfa Aesar, 98%). Le tout est laissé dans un corps en Téflon de 23 ml mis dans une Bombe métallique PAAR pendant 12 h à 100°C. Le solide est récupéré par filtration.

200 mg du solide sont suspendus dans 100mL. d'eau sous agitation à température ambiante pendant 12 h pour éliminer l'acide restant dans les pores. Puis, le solide est récupéré par filtration.

La taille de particule monodisperse (PDI=0,005) mesurée par diffusion de lumière est de 549 nm.

### j) MIL-88B-4F (Fe) ou Fe₃O[C₆F₄-(CO)₂]₃.X.nH₂O (X=F, Cl, OH)

270 mg de FeCl₃.6H₂O (1 mmol, Alfa Aesar, 98%) et 230 mg (1 mmol) d'acide 1,4-tétrafluorotéréphthalique (Aldrich, 98%) sont dispersés dans 10 ml de l'eau distillée. Le tout est laissé dans un corps en Téflon de 23 ml mis dans une Bombe métallique PAAR pendant 12 h à 85°C. Le solide est récupéré par filtration.

200 mg du solide sont suspendus dans 20mL d'eau sous agitation à température ambiante pendant 2 heures pour éliminer l'acide que reste dans les pores. Après, le solide est récupéré par filtration.

La taille de particule mesurée légèrement polydisperse (PDI=0.289) par diffusion de lumière est 399 nm.

### k) MIL-88B-Br (Fe) ou Fe₃O[C₆H₃Br-(CO₂)₂]₃.X.nH₂O (X=F, Cl, OH)

270 mg de FeCl₃.6H₂O (1 mmol, Alfa Aesar, 98%), 250 mg (1 mmol) d'acide 1,4bromoterephthalique (Fluka, 95%) dispersés dans 10 ml de DMF (Fluka, 98%)avec 0,2 mL de HF 5M (SDS, 50%), le tout laissé dans un corps en Téflon de 23 ml mis dans une Bombe métallique PAAR pendant 12 h à 150°C. Le solide est récupéré par filtration puis calciné à 150°C sous vide pendant 15 h pour éliminer l'acide restant dans les pores.

La taille de particule mesurée monodisperse (PDI=0.005) par diffusion de lumière est de 1127 nm.

### 1) MIL-88B-2CF₃(Fe) ou Fe₃O[(CF₃)₂-C₆H₂-(CO₂)₂]₃.X.nH₂O (X=F, Cl, OH)

135 mg de FeCl₃.6H₂O (0,5 mmol, Alfa Aesar, 98%) et 151 mg (0,5 mmol) d'acide 2,5-diperfluoro-1,4-téréphthalique (synthétisé selon la synthèse B décrite dans l'exemple 1) sont dispersés dans 5 ml de DMF (Fluka, 98%)avec 0,2 mL de NaOH 2M (Alfa Aesar, 98%). Le tout est laissé dans un corps en Téflon de 23 ml mis dans une Bombe métallique PAAR pendant 16 h à 100°C. Le solide est récupéré par filtration.

La taille de particule mesurée par diffusion de lumière est > 1micron.

### m) MIL-88D 4CH₃ (Fe) ou Fe₃O[C₁₂H₄(CH₃)₄-(CO₂₎₂]₃.X.nH₂O (X=F, Cl, OH)

354 mg de Fe(ClO₄)₃.xH₂O (1 mmol, Aldrich, 99%) et 298 mg (1 mmol) d'acide tetramethylbiphenyl 4,4'-dicarboxylique (préparé selon la synthèse D) sont dispersés dans 5 ml de DMF (Fluka, 98%)avec 0,2 mL de NaOH 2M (Alfa Aesar, 98%). Le mélange est laissé dans un corps en Téflon de 23 ml mis dans une Bombe métallique PAAR pendant 12 h à 100°C. Le solide est récupéré par filtration.

200 mg du solide sont suspendus en 10mL de DMF sous agitation a température ambiante pendant 2 h pour échanger l'acide restant dans les pores. Puis, le solide est récupéré par filtration puis calciné à 150°C sous vide pendant 15 h pour éliminer le DMF restant dans les pores.

Ce composé ne présente pas de surface accessible (supérieure à 20 m²/g) à l'azote à 77 K, puisque la structure sèche possède une taille de pores trop faible pour incorporer de l'azote N₂.

La taille de particule mesurée par diffusion de lumière est > 1 micron (2032, PDI=0.005).

### n) MIL-88D 2CH₃ (Fe) ou Fe₃O[C₁₂H₆(CH₃)₂-(CO₂)₂]₃.X.nH₂O (X=F, Cl, OH)

270 mg de FeCl₃.6H₂O (1 mmol, Alfa Aesar, 98%) et 268 mg (1 mmol) d'acide diméthylbiphényl 4,4'-dicarboxylique (préparé selon la synthèse E) sont dispersés dans 5 ml de DMF (Fluka, 98%) avec 0,25 mL de HF 5M (SDS, 50%). Le tout est laissé dans un corps en Téflon de 23 ml mis dans une Bombe métallique PAAR pendant 12 h à 150°C. Le solide est récupéré par filtration puis calciné à 150°C sous vide pendant 15 heures pour éliminer l'acide restant dans les pores.

La taille de particule monodisperse (PDI=0.005) mesurée par diffusion de lumière est 458 nm.

### o) MIL-88E(Pyr) (Fe) ou Fe₃O[C₄H₃N₂-(CO₂)₂]₃.X.nH₂O (X=F, Cl, OH)

270 mg de FeCl3.6H₂O (1 mmol, Alfa Aesar, 98%) et 204 mg (1 mmol) d'acide 2,5-pyrazynedicarboxylique (Aldrich, 98%) sont dispersés dans 5 ml de DMF (Fluka, 98%) avec 0,05 mL de HF 5M (SDS, 50%). Le tout est laissé dans un corps en Téflon de 23 ml mis dans une Bombe métallique PAAR pendant 3 jours à 100°C. Le solide est récupéré par filtration.

La taille de particule mesurée par diffusion de lumière est >1 micron (2 *µ*m).

### p) MIL-88F (Thio) (Fe) ou Fe₃O[C₄H₂S-(CO₂)₂]₃.X.nH₂O (X=F, Cl, OH)

354 mg de Fe(ClO₄)₃.xH₂O (1 mmol, Aldrich, 99%) et 258 mg (1 mmol) d'acide thiofène dicarboxylique (Aldrich, 99%) sont dispersés dans 2,5 ml de DMF (Fluka, 98%)avec 0,1 mL de HF 5M (SDS, 50%). Le tout laissé dans un corps en Téflon de 23 ml mis dans une Bombe métallique PAAR pendant 3 jours à 100°C. Le solide est récupéré par filtration.

200 mg du solide sont suspendus dans 100mL d'eau sous agitation à température ambiante pendant 12 h pour éliminer l'acide restant dans les pores. Puis, le solide est récupéré par filtration.

La taille de particule mesurée par diffusion de lumière est 449 nm, avec une deuxième population minoritaire de plus de 1 micron.

### q) MIL-53-20H(Fe) ou FeO(OH) [C₆H₂(OH)₂-(CO₂)₂].X.nH₂O (X=F, Cl, OH)

354 mg de Fe(ClO₄)₃.xH₂O (1 mmol, Aldrich, 99%) et 297 mg (1,5 mmol) d'acide 1,4-dihydroxotéréphthalique (préparé selon la synthèse C décrite dans l'exemple 1) sont dispersés dans 5 ml de DMF (Fluka, 98%) avec 0,2 mL de HF 5M (SDS, 50%) et 1 mL de HClO₄ 5M (Aldrich, 70%). Le mélange est laissé dans un corps en Téflon de 23 ml mis dans une Bombe métallique PAAR pendant 3 jours à 150°C. Le solide est récupéré par filtration puis calciné à 150°C pendant 15 h pour éliminer l'acide restant dans les pores.

**Tableau 5. Analyse élémentaire (CNRS, Vernaison).**

| élément/ % massique | % Fer | % Carbone |
|---|---|---|
| MIL-88B 2OH | 15,4 | 36,5 |

La taille de particule mesurée par diffusion de lumière est > 1 microns.

### r) MIL-53-NH₂(Fe) ou FeO(OH)[C₆H₂-NH₂-(CO₂)₂].X.nH₂O (X=F, Cl, OH)

270 mg de FeCl₃.6H₂O (1 mmol, Alfa Aesar, 98%) et 180 mg (1 mmol) d'acide 1,4-aminoterephthalique (Fluka, 98%) sont dispersés dans 10 ml d'eau. Le mélange est laissé dans un corps en Téflon de 23 ml mis dans une Bombe métallique PAAR pendant 3 jours à 150°C. Le solide est récupéré par filtration.

Pour éliminer l'acide libre dans les pores, 200 mg du solide sont suspendus dans 15 mL d'éthanol absolu dans un corps en téflon de 23mL mis dans une bombe métallique PAAR pendant 2 jours. Le solide est récupéré par filtration lavé une deuxième fois. Enfin, le solide est récupéré par filtration et séché à 150°C.

La taille de particule mesurée par diffusion de lumière présente deux populations (PDI=0.296), une majoritaire à 172 nm et une autre minoritaire à 728 nm.

### s) MIL-53-Cl (Fe) ou FeO(OH)[C₆H₂ Cl-(CO₂)₂].X.nH₂O (X=F, Cl, OH)

354 mg de Fe(ClO₄)₃.xH₂O (1 mmol, Aldrich, 99%) et 200 mg (1 mmol) d'acide 1,4-chlorotéréphthalique (préparé selon la synthèse décrite dans l'exemple 1) sont dispersés dans 5 ml de DMF. Le mélange est laissé dans un corps en Téflon de 23 ml mis dans une Bombe métallique PAAR pendant 2 jours à 150°C avec une rampe de chauffage de 12 h. Le solide est récupéré par filtration puis calciné à 150°C pendant 3 jours.

La taille de particule mesurée par diffusion de lumière supérieure à 1 micron.

### t) MIL-53-Br(Fe) ou FeO(OH)[C₆H₂Br-(CO₂)₂].X.nH₂O (X=F, Cl, OH)

270 mg de FeCl₃.6H₂O (1 mmol, Alfa Aesar, 98%) et 250 mg (1 mmol) d'acide 1,4-bromotéréphthalique (Fluka, 95%) sont dispersés dans 10 ml de DMF (Fluka, 98%) avec 0,4 mL de HF 5M (SDS, 50%). Le mélange est laissé dans un corps en Téflon de 23 ml mis dans une Bombe métallique PAAR pendant 12 h à 150°C. Le solide est récupéré par filtration puis calciné à 150°C sous vide pendant 15 h pour éliminer l'acide restant dans les pores.

La taille de particule mesurée par diffusion de lumière est 196 nm, avec la présence très minoritaire de particules > 1 micron.

### u) MIL-53-2CF₃ (Fe) ou FeO(OH)[C₆H₂(CF₃)₂-(CO₂)₂].X.nH₂O (X=F, Cl, OH)

135 mg de FeCl₃.6H₂O (0,5 mmol, Alfa Aesar, 98%), 151 mg (0,5 mmol) d'acide 2,5-diperfluoro-1,4-terephthalique (synthétisé selon la synthèse B décrite dans l'exemple 1) sont dispersés dans 5 ml d'eau. Le tout est laissé dans un corps en Téflon de 23 ml mis dans une Bombe métallique PAAR pendant 16 h à 100°C. Le solide est récupéré par filtration.

La taille de particule mesurée par diffusion de lumière est > 1 micron.

### v) MIL-53-CH, (Fe) ou FeO(OH)[C₆H₃-CH₃-(CO₂)₂].X.nH₂O (X=F, Cl, OH)

177 mg (0,5 mmol, Aldrich, 99%) de perchlorate de fer, 90 mg (0,5 mmol) d'acide 2-méthyltéréphthalique (préparé selon la synthèse C décrite dans l'exemple 1) et 0,05 mL de HF (5M) (0,25 mmol) ont été introduits dans 2,5 mL de DMF (Fluka, 98%). Le tout est laissé dans un corps en Téflon de 23 ml mis dans une Bombe métallique PAAR pendant 16 h à 150°C. Le solide est récupéré par filtration et calciné à 200°C pendant 72 heures pour éliminer le DMF restant dans les pores.

La taille de particule mesurée par diffusion de lumière est > 1 micron.

### w) MIL-53-2COOH(Fe) ou FeO(OH)[C₆H₃-(CO₂)₄].X.nH₂O (X=F, Cl, OH)

354 mg (1 mmol, Aldrich, 99%) de perchlorate de fer et 254 mg (1 mmol) d'acide 1,2,4,5-benzène tétracarboxylique (Aldrich, 99%) ont été introduits dans 5 mL d'eau distillée. Le tout est laissé dans un corps en Téflon de 23 ml mis dans une Bombe métallique PAAR pendant 16 h à 150°C. Le solide est récupéré par filtration.

Pour éliminer l'acide restant dans les pores, 200 mg de solide sont suspendus dans 100 mL d'eau distillée pendant une nuit. Le solide est récupéré par filtration.

La taille de particule mesurée par diffusion de lumière est > 1 micron.

### Exemple 4 Synthèse de matériaux MOF à base de ligands fluorés

### a) MIL-53(HF)

Le solide MIL-53(HF) a été obtenu dans sa forme de nanoparticules à partir de FeCl₃.6H₂O (1 mmol; Alfa Aesar, 98%) d'acide terephthalique (1 mmol; 1,4-BDC; Aldrich, 98%) dans 5 mL dimethylformamide (DMF; Fluka, 98%) avec 0,1 mL de acide fluorhydrique 5M (Prolabo, 50%) le tout introduit dans un autoclave de type "Paar bomb" à 150°C pendant 15 heures. Le solide est récupéré par centrifugation à 5000rpm pendant 10 minutes.

200 mg du solide sont ensuite suspendus dans 100mL d'eau distillée sous agitation pendant 15h pour éliminer le solvant résiduel. Puis, le solide est récupéré par centrifugation à 5000rpm pendant 10 minutes.

La taille de particule est finalement mesurée par diffusion de lumière et est de 625 nm.

### b) MIL-100(HF)

Le solide MIL-100(HF) a été obtenu à partir de FeCl₃.6H₂O (1 mmol; Alfa Aesar, 98%)de trimesate d'éthyle (0,66 mmol; 1,3,5-BTC; Aldrich, 98%) dans 5 mL de l'eau et 0,1 mL d'acide fluorhydrique 5M (Prolabo, 50%) le tout introduit dans un autoclave de type "Paar bomb à 130°C pendant 15 heures. Le solide est récupéré par centrifugation à 5000rpm pendant 10 minutes.

200 mg du solide sont ensuite suspendus dans 100mL d'eau distillée à reflux sous agitation pendant 3h pour éliminer l'acide résiduel. Puis, le solide est récupéré par centrifugation à 5000rpm pendant 10 minutes.

La taille de particule est finalement mesurée par diffusion de lumière et est de 1260 nm.

### c) MIL-88Bx4F

Le solide MIL-88Bx4F a été obtenu à partir de FeCl₃.6H₂O (1 mmol; Alfa Aesar, 98%) d'acide tétrafluorotéréphthalique (1 mmol; 4xF-BDC; Aldrich, 98%) dans 10 mL d'eau le tout introduit dans un autoclave de type "Paar bomb" à 85°C pendant 15 heures. Le solide est récupéré par centrifugation à 5000rpm pendant 10 minutes.

200 mg du solide sont finalement suspendus dans 100mL d'eau distillée sous agitation pendant 2h pour éliminer l'acide résiduel. Puis, le solide est récupéré par centrifugation à 5000rpm pendant 10 minutes.

La taille de particule a été mesurée par diffusion de lumière et est de 850 nm.

On pourra se référer par exemple aux synthèses décrites dans l'Exemple 3 (Exemple 3c : MIL101-2CF3, Exemple 3F=MIL88B-4F, Exemple 3L=MIL88B-2CF3, Exemple 3u=MIL53-2CF3) et dans l'Exemple 7 (Exemple 7d=MIL88B-2CF3, Exemple 7f=MIL53-2CF3).

Les solides hybrides peuvent également être synthétisés par ces procédés et en utilisant le radioisotope F18 pour l'imagerie TEP (tomographie par émission de positrons.

Ces synthèses sont à base de ligands modifiés avec des groupes perfluoro. Pour la modification avec le 18F, on va utiliser de préférence la méthode d'échange ionique avec 18F compte tenu:
- de la courte durée de vie moyenne du 18F
- de la difficulté de synthétiser des ligands fluorés à base de 18F
- l'imagerie TEP ne nécessite pas beaucoup de fluor, donc avec la quantité liée comme contre-ion sera suffisante.

### d) Matériaux MOF avec du Fluor 18

Des MOFs comportant des ions fluorure comme contre anions pourraient également servir en imagerie.

Par exemple, pour l'imagerie TEP (tomographie par émission de positrons), le fluor-18 est certainement le radio-isotope de choix à cause de ses caractéristiques radiophysiques favorables. La technique TEP permet d'obtenir des images très détaillées des tissus vivants. Le radio-isotope fluor-18 (t1/2 = 110 minutes) est un émetteur de positrons ; les positrons émis sont instantanément annihilés par les électrons de la matière environnante et c'est les rayons gamma résultants qui sont détectés.

Le fluor 18 est produit dans une line synchrotron. Aussi, pour la synthèse de matériaux hybrides poreux avec le radio isotope F18, on doit se placer proche d'une line synchrotron car son temps de vie moyen est très court (110 minutes).

Il y a deux méthodes possibles pour l'obtention de solides hybrides poreux avec du F18 :

### Méthode 1 :

Les solides hybrides sont obtenus en présence de HF (F18) ou de ligands fluorés F18 par voie microondes pour réduire le temps de synthèse à quelque minutes (3-30 min). Les nanoparticules sont récupérées par centrifugation à 10000 rpm pendant 5 min.

Pour l'obtention d'une petite taille de particule des solides hybrides poreux, il est préférable d'utiliser des temps de synthèse par voie hydro-solvothermale très courts. Ainsi, quelques carboxylates de fer peuvent être synthétisés également par voie solvothermale quand le temps de synthèse ne dépassent pas les 30 min.

### Méthode 2 :

0,1 mmol de nanoparticules de solides hybrides poreux déjà synthétisés par voie solvothermale ou microondes et activés sont placés en suspension dans 1 mL d'une solution 0,01 et 0,001 M de HF (F18) pour procéder à l'échange de l'anion OH par de fluor sous agitation pendant 15 min. Le solide fluoré est récupéré par centrifugation à 10000 rpm pendant 5 min.

### Exemple 5 : Synthèse de matériaux MOF à base de ligands bioactifs

L'utilisation des ligands avec une activité biologique présente un intérêt pour :
- la libération de composé actif par dégradation du matériau MOF
- l'encapsulation d'autres molécules actives pour des thérapies combinées

Des tests de l'activité antimicrobienne, ainsi que la dégradation dans des milieux physiologiques et l'activité sur des cellules seront réalisés sur des carboxylates de fer poreux a structure flexible de type MIL-88 utilisant les acides 4,4'azobenzene dicarboxylique et 3,3'-dichlore 4,4'-azobenzene dicarboxylique, entre autres.

Dans les synthèses qui suivent, divers molécules bioactives sont utilisées pour préparer les matériaux MOF de la présente invention, et notamment : l'azobenzène, l'acide azélaïque et l'acide nicotinique.

L'azobenzene (AzBz), de formule C₆H₅-N=N-C₆H₅, peut être incorporé aux matrices de polymère comme stabilisateur. De plus, la structure rigide des molécules azoïques leur permet de se comporter en tant que mésogènes à cristal liquide dans de nombreux matériaux. Par ailleurs, l'azobenzène peut être photoisomérisé (isomère *cis* ou *trans)* d'où son utilisation pour photomoduler l'affinité d'un ligand (par exemple un médicament) pour une protéine. En effet, l'azobenzène peut jouer le rôle de photo-interrupteur entre un ligand et une protéine en permettant ou en empêchant la liaison protéine- médicament suivant l'isomère cis ou trans de l'azobenzène (une extrémité de l'azobenzene peut par exemple être substituée par un groupement se liant à la protéine cible tandis que l'autre extrémité est reliée à un ligand (médicament) de la protéine).

L'acide Azelaïque (HO₂C-(CH₂)7-CO₂H) est un acide dicarboxylique saturé qui a des propriétés antribactérienne, kératolytique et comédolytique. Il est utilisé notamment dans le traitement de l'acné et la rosacea.

L'acide nicotinique (C₅H₄N-CO₂H) est l'une des deux forme de la vitamine B3 avec la nicotinamide. La vitamine B3 est notamment nécessaire au métabolisme des glucides, lipides et protéines.

### e) MIL-88G(AzBz)(Fe) ou Fe₃O[Cl₁₂H₈N₂-(CO₂)₂]₃.X-nH₂O (X=F, Cl, OH)

118 mg de Fe(ClO₄)₃.xH₂O (0.33 mmol, Aldrich, 99%) et 90 mg (0,33 mmol) d'acide 4,4'-azobenzenedicarboxylique (synthétisé selon la méthode décrite par Ameerunisha et al., J. Chem. Soc. Perkin Trans. 2, 1679, 1995) sont dispersés dans 15 ml de DMF (Fluka, 98%). Le tout est laissé dans un corps en Téflon de 23 ml mis dans une Bombe métallique PAAR pendant 3 jours à 150°C. Le solide est récupéré par filtration.

200 mg du solide sont suspendus dans 10mL de DMF sous agitation à température ambiante pendant 2 h pour échanger l'acide restant dans les pores. Le solide est ensuite récupéré par filtration puis calciné à 150°C sous vide pendant 15 heures pour éliminer le DMF restant dans les pores.

La taille de particule mesurée par diffusion de lumière est > 1 micron.

### f) MIL-88G-2Cl(AzBz-2Cl)(Fe) ou Fe₃O[C₁₂H₆N₂Cl₂-(CO₂)₂]₃.X.nH₂O (X=F, Cl, OH)

177 mg de Fe(ClO₄)₃.xH₂O (0,5 mmol, Aldrich, 99%) et 169 mg (0,5 mmol) d'acide dichloro-4,4'-azobenzenedicarboxylique (préparé selon la synthèse F décrite dans l'exemple 1) sont dispersés dans 15 ml de DMF (Fluka, 98%). Le tout est laissé dans un corps en Téflon de 23 ml mis dans une Bombe métallique PAAR pendant 12 h à 150°C. Le solide est récupéré par filtration.

200 mg du solide sont suspendus dans 10mL de DMF sous agitation à température ambiante pendant 2 h pour échanger l'acide restant dans les pores. Le solide est ensuite récupéré par filtration puis calciné à 150°C sous vide pendant 15 h pour éliminer le DMF restant dans les pores.

La taille de particule mesurée par diffusion de lumière est > 1 micron.

### g) Azobenzene 3,3',5,5'-Tetracarboxylate de fer 1

118 mg de Fe(ClO₄)₃.xH₂O (0,3 mmol, Aldrich, 99%) et 119 mg (0,3 mmol) d'acide 3,3',5,5'-azobenzènetétracarboxylique (préparé selon la synthèse G décrite dans l'exemple 1) sont dispersés dans 15 ml de DMF (Fluka, 98%) avec 0,1 mL de HF 5M (SDS, 50%). Le tout est laissé dans un corps en Téflon de 23 ml mis dans une Bombe métallique PAAR pendant 3 jours à 150°C. Le solide est récupéré par filtration et lavé avec de l'acétone.

Le solide obtenu a une structure cubique rigide.

La taille de particule mesurée par diffusion de lumière est > 1 micron.

### h) Azobenzene 3,3',5,5'-Tetracarboxylate de fer 2

118 mg de Fe(ClO₄)₃.xH₂O (0,3 mmol, Aldrich, 99%) et 119 mg (0,3 mmol) d'acide 3,3',5,5'-azobenzènetétracarboxylique (préparé selon la synthèse G décrite dans l'exemple 1) sont dispersés dans 15 ml de l'eau distillée avec 0,1 mL de HF 5M (SDS, 50%). Le tout est laissé dans un corps en Téflon de 23 ml mis dans une Bombe métallique PAAR pendant 3 jours à 150°C. Le solide est récupéré par filtration et lavé avec l'acétone.

La taille de particule mesurée par diffusion de lumière est 498 nm, avec une deuxième population minoritaire de 1100 nm.

### i) Azélate de fer 1

270 mg de FeCl₃.6H₂O (1 mmol, Aldrich, 99%) et 188 mg (1 mmol) d'acide azelaique (Aldrich, 99%) sont dispersés dans 5 ml d'eau distillée. Le tout est laissé dans un corps en Téflon de 23 ml mis dans une Bombe métallique PAAR pendant 3 jours à 100°C. Le solide est récupéré par filtration et lavé à l'acétone.

200 mg de solide sont suspendus dans 50 mL d'éthanol absolu sous agitation pendant 5 h pour l'activer. Le solide est récupéré par filtration.

La taille de particule mesurée par diffusion de lumière est > 1 micron (1500 nm).

### j) Nicotinate de fer 1

Les conditions de synthèse dans l'eau sont les suivantes :
135 mg de FeCl₃.6H₂O (1 mmol, Aldrich, 99%) et 62 mg (1 mmol) d'acide nicotinique (Aldrich, 99%) sont dispersés dans 5 ml d'eau distillée avec 0,1 mL de NaOH 2M. Le tout est laissé dans un corps en Téflon de 23 ml mis dans une Bombe métallique PAAR pendant 16 heures à 100°C. Le solide est récupéré par filtration et lavée avec l'acétone.

Les conditions de synthèse dans le DMF sont les suivantes :
135 mg de FeCl₃.6H₂O (1 mmol, Aldrich, 99%) et 62 mg (1 mmol) d'acide nicotinique (Aldrich, 99%) sont dispersés dans 5 ml de DMF (Fluka, 98%). Le tout est laissé dans un corps en Téflon de 23 ml mis dans une Bombe métallique PAAR pendant 16 h à 100°C. Le solide est récupéré par filtration et lavée avec l'acétone.

La taille de particule monodisperse (PDI=0.241) mesurée par diffusion de lumière est 662 nm.

### III. PROCEDE MODULABLE DE PREPARATION DES NANOPARTICULES

### Exemple 6 : Contrôle de la taille des particules, influence des différents paramètres

Dans cet exemple, le contrôle de la taille des particules peut être obtenu en changeant un ou plusieurs des paramètres suivants durant la synthèse
- Le temps de synthèse
- Le pH
- L'ajout d'un additif (monoacide de type acide acétique...)
- L'agitation
- La nature du solvant
- La synthèse micro-ondes
- La synthèse sous ultra-sons

Après la synthèse, les nanoparticules sont lavées avec des solvants, récupérées par centrifugation, séchées sous vide, sous air ou sous atmosphère contrôlée éventuellement avec chauffage.

Les nanoparticules sont ensuite analysées par une combinaison de techniques permettant de déterminer les structures et la composition des phases : Rayons X, spectroscopie IR, thermodiffraction RX, analyse thermogravimétrique, analyse élémentaire, microscopie électronique, mesure de potentiel Zeta et mesure de taille de particules.
- Les diagrammes de poudre RX sont collectés sur un diffractomètre RX conventionnel haute résolution (θ-2θ) D5000 Siemens X Pert MDP (λ_{cu}, Kα₁, Kα₂) typiquement entre 5 et 30º (2θ) utilisant un pas de 0,02° et 4 secondes de temps de comptage en mode continu.
- La thermodiffractometrie RX est réalisée dans le four d'un diffractomètre RX Siemens D-5000 en mode θ-θ mode sous air.
- Les mesures de surface spécifique sont obtenues avec un appareil d'adsorption Microméritics ASAP 2010 en utilisant comme gaz N₂ (calcul de porosimétrie de type BJH).
- Les spectres IR sont réalisés avec un spectromètre Nicolet-Magma IR550.
- Les analyses thermogravimétriques sont faites avec un appareil de marque TA 2050 entre 25 et 600°C, avec une vitesse de chauffe de 2°C.min⁻¹
- Les mesures de taille de particules et de potentiel Zeta sont faites avec un instrument Malvern Zetasizer Nano serie - Nano-ZS equipment; model Zen 3600 ; serial N° 500180 ; UK.
- La microscopie électronique à balayage a été faite avec un Topcon (Akashi) EM 002B ultrahaute résolution 200kV.

### a) Influence du temps de synthèse, application à la synthèse de nanoparticules de MIL-53(Fe)

Le mélange d'une solution de FeCl₃.6H₂O (1 mmol; Alfa Aesar, 98%), d'acide téréphthalique (1 mmol; 1,4-BDC; Aldrich, 98%) dans 5 mL de diméthylformamide (DMF; Fluka, 98%), est placé dans un insert en Téflon mis dans un autoclave à une température de 150°C pendant 72 h avec 12 h de rampe de chauffe et 24 heures de refroidissement jusqu'à la température ambiante. Après réaction, le précipité est filtré et lavé avec de l'eau déionisée. Le solide MIL-53(Fe) est obtenu sous la forme de cristaux de plusieurs centaines de microns.

La diminution du temps de synthèse (sans rampe de chauffe ni de refroidissement) conduit à des tailles de particules plus petites (Tableau 6), avec par exemple une taille de 335 nm pour 4 heures de synthèse à 150°C. Ensuite, pour retirer le solvant des pores du solide, typiquement 200mg de solide est dispersé dans 100 mL d'eau déionisée pendant une nuit sous agitation suivi d'une filtration ou d'une centrifugation (selon la taille des particules).

Le Tableau 6 ci dessous répertorie les tailles des nanoparticules obtenues. Il montre que des temps de synthèse courts favorisent la présence de petites particules.

**Tableau 6. Tailles des particules de MIL-53(Fe) en fonction du temps de synthèse**

| Temps de synthèse (en heures) | Diamètre des particules Dp (en nm) |
|---|---|
| 24 | 6220 |
| 12 | 2460 |
| 6 | 720 |
| 5 | 800 |
| 4 | 335 |
| 2 | 380 |

### b) Influence du pH, application à la synthèse de nanoparticules de MIL-89(Fe)

L'acétate de fer(III) (1 mmol; synthétisé selon la synthèse A décrite ci-dessus) est mélangé sous agitation avec de l'acide muconique (1 mmol; Fluka, 97%) en milieu méthanol (5mL; Aldrich, 99,9%) ou éthanol (5 mL; Riedel-de Haën, 99,8%). Le tout est placé sans agitation à 100°C pendant 12h en présence de 0,25mL de solution aqueuse d'hydroxyde de sodium 2 Mol/1 (Alfa Aesar, 98%) donne de plus petites tailles de particule.

Le Tableau 7 ci-dessous répertorie les tailles de nanoparticules obtenues en fonction de l'ajout de base ou non et montre que l'ajout de base favorise la présence de petites particules.

**Tableau 7. Tailles des particules de MIL-89(Fe) en fonction de l'ajout de base.**

| **Solvant** | **Volume de solution aqueuse 2M de NaOH ajouté (mL)** | **Dp (nm)** |
|---|---|---|
| méthanol | 0 | 585 |
| méthanol | 0,25 | 471 |
| éthanol | 0 | 493 |
| éthanol | 0,25 | 398 |

Ainsi, une augmentation de pH provoque une déprotonation plus aisée du ligand carboxylique, ce qui accélère la vitesse de réaction.

### c) Influence de l'ajout d'un additif, application à la synthèse de nanoparticules de MIL-88A(Fe)

Le fumarate de fer MIL-88A, est obtenu à partir de 270 mg de FeCl₃.6H₂O (1 mmol; Alfa Aesar, 98%), 112 mg d'acide fumarique (1 mmol; Acros, 99%) introduits dans 15 mL d'éthanol (Riedel-de Haën, 99,8%), des quantités variables d'acide acétique (Aldrich, 99,7%) sont ajoutées. La solution est ensuite chauffée pendant 2 ou 4 heures à 65°C.

**Tableau 8. Tailles des particules de MIL-88A(Fe) obtenu en présence de quantités variables d'acide acétique à deux temps de synthèse différents (2 ou 4 heures).**

| **Volume d'acide acétique introduit (mL)** | **Dp (nm)** | **Dp (nm)** |
|---|---|---|
| | **t = 2h** | **t = 4h** |
| 0 | 417 | 532 |
| 0,25 | 381 | 592 |
| 0,5 | 433 | 587 |
| 1 | 256 | 301 |

Ces résultats montrent clairement que l'ajout d'un monoacide carboxylique ralentit la croissance cristalline et permet ainsi d'obtenir des nanoparticules de plus petite taille. L'acide acétique peut être ajouté à n'importe quelle moment de la réaction.

### d) Influence de l'agitation

L'influence de l'agitation a été étudiée lors de la synthèse du composé MIL-88A.

La méthode utilisée pour préparer les nanoparticules de composé MIL-88A consiste à introduire 1 mmol de chlorure de fer(III) hexahydraté (270 mg) et 1 mmol d'acide fumarique (112 mg) dans 4,8 mL de DMF, et d'ajouter 0,4 mL de solution NaOH 2M. Le tout est chauffé à 150°C pendant 2h, avec ou sans agitation.

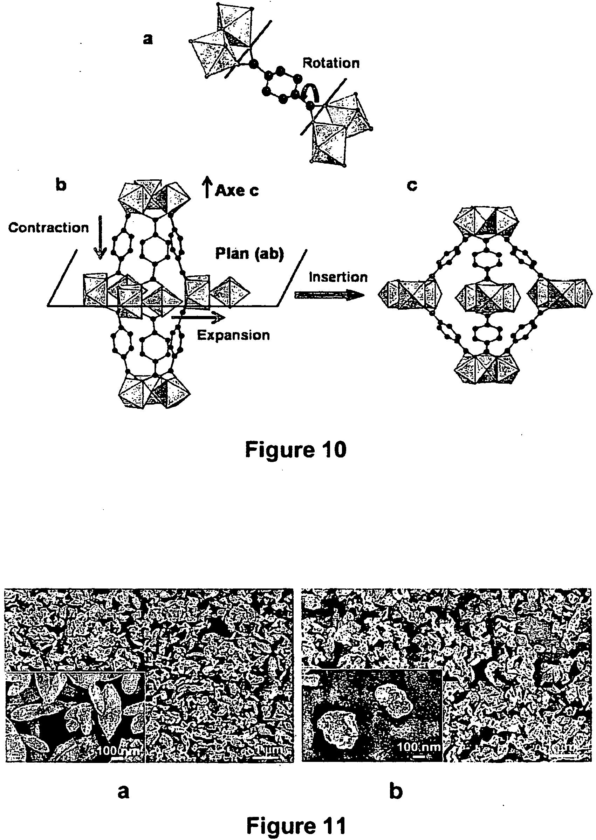

La microscopie électronique montre que les particules obtenues en l'absence d'agitation possèdent une morphologie différente de celles obtenues avec agitation comme le montre la figure 11. L'agitation provoque donc une diminution de la taille des particules mais aussi change la morphologie ce qui pourrait avoir un effet sur la toxicité des solides.

### e) Influence du solvant, application à la synthèse de nanoparticules MIL-88A(Fe)

La synthèse du solide MIL-88A a été effectuée d'une part dans l'eau, d'autre part dans le méthanol. Un mélange contenant le chlorure de fer (1 mmol), l'acide fumarique (1 mmol) dans 15 mL de solvant (méthanol ou eau déionisée) est mis en présence de quantités variables d'acide acétique, utilisé comme cosolvant, sans agitation à 65°C pendant 2 ou 4 heures. Les tailles de particules obtenues sont répertoriées dans le Tableau 9.

**Tableau 9. Tailles des particules de MIL-88A(Fe) en fonction du solvant, de la quantité d'acide acétique pour deux temps de synthèse.**

| **Solvant** | **Volume d'acide acétique introduit (mL)** | **Dp (nm)** | **Dp (nm)** |
|---|---|---|---|
| | | **t** = **2h** | **t = 4h** |
| méthanol | 0 | 417 | 532 |
| méthanol | 0,25 | 381 | 592 |
| méthanol | 0,5 | 433 | 587 |
| méthanol | 1 | 256 | 301 |
| eau | 0 | 328 | 451 |
| eau | 0,25 | 265 | 364 |
| eau | 0,5 | 336 | 535 |
| eau | 1 | 198 | 238 |

Les particules de MIL-88A obtenues dans l'eau sont plus petites que celles obtenues dans le méthanol. Ainsi, la nature du solvant utilisé lors de la synthèse a une forte influence sur la taille des particules.

### Exemple 6B : Contrôle de la taille de particules de MIL-88A, influence de 4 paramètres : température, temps de réaction, concentration des réactifs et ajout d'un composé monocarboxylé

### a) Synthèse de fumarate de fer sans additif monocarboxylé

5 ml d'une solution aqueuse de fumarate de fer hexahydrate (FeCl₃.6H₂O, 1 mmol; Alfa Aesar, 98%) et d'acide fumarique (1 mmol; Acros, 99%) sont placés dans un corps en téflon de 23 ml placé dans une bombe métallique PAAR. Le tout est placé en autoclave à une température de 65, 100 ou 150°C, pendant des temps allant de 30 min à 3 jours. Ensuite, le précipité obtenu est récupéré par centrifugation à 5000 rpm pendant 10 min. Il est séché à 100°C en étuve et pesé pour déterminer le rendement de synthèse. Le diamètre des particules est déterminé par diffusion quasi-élastique de la lumière. Les diffractogrammes RX sont obtenus comme décrit précédemment.

Nous avons cherché à trouver des conditions opératoires (température, des temps de réaction) optimales pour obtenir simultanément : i) une bonne cristallisation (+++); ii) un diamètre inférieur à 1000 nm (nanoparticules) ; iii) un rendement satisfaisant (>25% pds) et iv) l'absence d'oxydes de fer.

Le tableau suivant regroupe les résultats obtenus lors des diverses synthèses. Les rendements sont considérés insatisfaisants lorsqu'ils sont inférieurs à 25% pds. L'absence de cristallisation est indiqué par -, une bonne cristallisation par +++, et la cristallisation insuffisante par + ou ++.

| Température | Temps (h) | Diamètre (nm) | cristallinité | rendement (% mass) |
|---|---|---|---|---|
| 65°C | 2 | 300-400 | - | < 1% |
| | 6 | 300-600 | ++ | < 5% |
| | 16 | 300-600 | +++ | > 25% |
| | 72 | 300-600 | +++ | > 50% |
| 100°C | 0.5 | 400-500 | + | < 10% |
| | 2 | 500-600 | + | < 10% |
| | 6 | 500-800 | +++ | > 50% |
| | 16 | 600-1000 | +++ | > 75% |
| | 72 | 600-2000 | +++ | > 75% |
| 150°C | 0,5 | 500-700 | - | < 10 % |
| | 2 | 400-500* | + | < 10 % |
| | 6 | 600-1000* | +++ | > 50 % |
| | 16 | 800-2000* | +++ | > 75 % |
| | 72 | 800-2000* | +++ | > 75 % |

| | | | | |
|---|---|---|---|---|
| * Présence d'oxydes de fer | | | | |

A 65°C, la réaction doit être poursuivie pendant au moins 16 heures pour obtenir les conditions i-iv, tandis qu'à 100°C, 6 heures de réaction suffisent, mais les diamètres sont plus gros (500-800 nm). A 150°C, il a été impossible de réunir toutes les conditions i à iv nécessaires, car dès 2 heures de réaction, il y a formation d'oxydes de fer au détriment de la formation d'un réseau cristallin.

Les meilleurs résultats ont été obtenus à 65°C, avec un temps de réaction de 16 heures, permettant l'obtention des particules les plus fines (300-600 nm).

### b) SYNTHESE DU fumarate de fer (MIL 88A) PAR ULTRASONS

Des nanoparticules de MIL-88A ont été synthétisées dans l'eau, par voie ultrason à 0°C en modifiant le temps de réaction (entre 30 et 120 min) à partir d'acide fumarique (C₄H₄O₄, ACROS, 99%) et de chlorure de fer III hexahydraté (FeCl₃, 6H₂O, ACROS, 97%).

Les deux réactifs solides sont pesés séparément sur une balance de précision puis le solvant (l'eau) est ajouté pour chacun des solides : 5,4g de FeCl₃ + eau distillée dans une fiole de 200 ml et 2,32g d'acide fumarique + eau distillée dans une fiole de 200 ml.

On obtient ainsi deux solutions de concentrations 27 mg/mL et 11,6 mg/mL en Chlorure de fer III et en Acide Fumarique, respectivement. La solution d'acide fumarique est portée à 70°C sous agitation pendant 120 min environ pour solubiliser le produit. Le chlorure de fer est agité à l'aide d'un agitateur magnétique pendant 30min.

5 ml de chaque solution sont mélangés dans des flacons en verre de 20ml (vials). Les vials sont placés en même temps dans un bain de sonication à 0°C (Labo-moderne TK 52H n° de série : 164046192 SONOCLEAN), pendant des durées de 30 à 120 min.

La taille des particules obtenues (mesurée par diffusion de la lumière (Nanosizer)) dépassait le micron, quel que soit le temps de sonication.

Un deuxième essai a été fait, en rajoutant cette fois-ci 30 *µ*l d'acide acétique 15min avant la fin de la synthèse (correspondant à la sortie du bain de sonication). Le diamètre des particules était d'environ 500 nm après 30 min de synthèse, 800 nm après 60 min, puis dépassait le micron après 90 min de synthèse.

L'addition d'acide acétique (un monoacide) produit l'arrêt de la croissance cristalline puisqu'il coordonne le fer ; le fer ne pourra pas se lier à un autre atome de fer (faute du 2nd COOH). De cette façon, l'acide acétique permet l'obtention de nanoparticules de plus petite taille.

Dans le but d'obtenir des particules de taille encore plus fine, un troisième essai a été réalisé en diminuant la concentration des solutions d'acide fumarique et de Chlorure de fer, les autres conditions étant les mêmes.

En diminuant les concentrations d'un facteur deux, en absence d'additifs (acide acétique) les diamètres restaient supérieurs au micron. Cependant, une diminution des concentrations d'un facteur dix a permis d'obtenir des nanoparticules d'environ 200 nm avec un temps de synthèse de 30 min.

Il est à noter qu'une augmentation de la température de réaction entraine une augmentation de la taille des particules. Par exemple, pour une diminution des concentrations d'un facteur dix, en absence d'additifs, le diamètre augment de 200 nm à 240 nm lorsque la synthèse a lieu pendant 30 min, à 20°C au lieu de 0°C. Dans tous les cas, une augmentation du temps de synthèse conduit à une augmentation de la taille des particules.

Il existe donc des conditions opératoires optimales (concentrations, température et temps de réaction, présence d'additifs) permettant de réduire la taille des particules. Ces dernières peuvent avoir une taille inférieure au micron ou même de l'ordre de 200 nm (adapté pour une administration intraveineuse).

Ces conditions opératoires optimales peuvent être déterminées empiriquement pour tous les solides MOF selon la présente invention.

Ces conditions optimales de synthèse (0°C, à partir de solutions très diluées de réactifs) ont été utilisée pour greffer le PEG à la surface des nanoparticules.

### C) Préparation de nanoparticules MIL-88A PEGylées

Ainsi, des nanoparticules PEGylées (à surface modifiée par le PEG) de MIL-88A ont été synthétisés dans l'eau, par voie ultrason à 0°C, à partir d'acide fumarique (C₄H₄O₄, ACROS, 99%) et de chlorure de fer III hexahydraté (FeCl₃, 6H₂O, ACROS, 97%).

Les deux réactifs solides sont pesés séparément sur une balance de précision puis le solvant (l'eau) est ajouté pour chacun des solides : 0,54 g de FeCl₃. + eau distillée dans une fiole de 200 ml et 0,232g d'acide fumarique + eau distillée dans une fiole de 200 ml. On obtient ainsi deux solutions de concentrations 2,7 mg/mL et 1,16 mg/mL en Chlorure de fer III et en Acide Fumarique, respectivement. La solution d'acide fumarique est portée à 70°C sous agitation pendant 120 min environ pour solubiliser le produit. Le chlorure de fer est agité à l'aide d'un agitateur magnétique pendant 30min.

5 ml de chaque solution sont mélangés dans des flacons en verre de 20ml (vials). Les vials sont placés en même temps dans un bain de sonication (Labo-moderne TK 52H n° de série : 164046192 SONOCLEAN). Après 30 min, on rajoute 5 mg de monométhoxy poly(éthylène glycol) monoacide (MeO-PEG-COOH, Sigma, masse molaire 5000 g/mole). On laisse la réaction se poursuivre sous ultrasons pendant encore 90 min.

Le diamètre des nanoparticules (mesurée par diffusion de la lumière) a été de 230 nm et le rendement de fabrication de 50% (pds). Dans cet exemple, l'addition du monoacide MeO-PEG-COOH entraine également l'arrêt de la croissance cristalline puisqu'il coordonne le fer ; le fer ne pourra pas se lier à un autre atome de fer (faute du 2nd COOH). De cette façon, MeO-PEG-COOH permet l'obtention de nanoparticules de plus petite taille.

### Exemple 7 :Procédé de synthèse de matériaux MOF par voie microondes

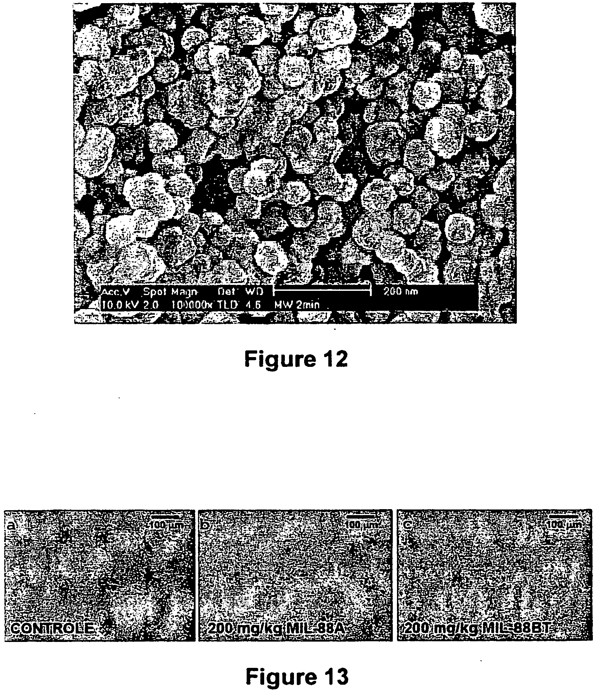

Une autre voie de synthèse possible de nanoparticules de solides hybrides poreux utilise l'énergie micro-ondes. Cela permet de contrôler la taille des nanoparticules et d'obtenir des tailles monodisperses. Ainsi, récemment, les inventeurs ont élaboré en collaboration avec un groupe coréen, la synthèse micro-ondes du carboxylate de chrome MIL-101(Cr), comme décrit dans Sung Hwa Jhung, et al., Adv. Mater (2006), 19(1), 121-124 [32]. Cette synthèse a permi l'obtention de nanoparticles de taille entre 40 et 90 nm sur un temps de synthèse très court (1-60 minutes) (figure 12).

La méthode de synthèse utilisée est la suivante : 400 mg de nitrate de chrome hydraté (Aldrich, 99 %), 166 mg d'acide téréphtalique (Alfa Aesar, 98 %), 0,2 ml de solution de HF 5mol/l dans l'eau, et 4,8 ml d'eau déionisée sont mélangés et introduits dans un autoclave en Téflon. Le tout est placé dans un four micro-ondes (Mars-5) et monté à 210°C en 2 minutes puis maintenu à cette température pendant 1 à 60 minutes. La mixture résultante est ensuite filtrée une première fois avec un filtre en papier de porosité 100 µm pour retirer l'acide téréphthalique recristallisé. L'acide reste sur le papier filtre et le solide MIL-101 passe au travers du filtre. On récupère le filtrat et le solide MIL-101 est récupéré par filtration sur un filtre 40 µm. Dans un second temps, un traitement solvothermal dans l'éthanol à 95 % à 100°C pendant 20 heures. Le solide final est refroidi, filtré et lavé avec de l'eau déionisée puis séché à 150°C sous air.

### a) MIL-100(Fe) ou Fe₃O[C₆H₃-(CO₂)₃]₂.X.nH₂O (X=F, Cl, OH)

Les conditions de synthèse microondes sans fluor sont les suivantes :
9,7 g de Fe(NO₃)₃.9H₂O (24 mmol, Aldrich, 98%), 3,38 mg (16 mmol) d'acide 1,3,5 benzènetricarboxylique (1,3,5-BTC ; Aldrich, 99%) sont dispersés dans 40 ml de l'eau distillée. Le tout est laissé dans un corps en Téflon à 180°C pendant 30 minutes (puissance 600W). Le solide est récupéré par centrifugation à 10000 rpm pendant 10 minutes.
200 mg du solide sont suspendus dans 100mL d'eau distillée à reflux sous agitation pendant 3h pour éliminer l'acide restant dans les pores. Le solide est ensuite récupéré par centrifugation à 10000 rpm pendant 10 minutes.

La taille de particule monodisperse mesurée par diffusion de lumière est de 400 nm.

### b) MIL-101(Fe)-NH₂ ou Fe₃O[NH₂-C₆H₃-(CO₂)₂]₃.X.nH₂O (X=F, Cl, OH)

Les conditions de synthèse microondes sont les suivantes :
135 mg (0,5 mmol) de FeCl₃.6H₂O et 90 mg d'acide amino-1,4 benzenedicarboxylique (0,5 mmol, NH₂-1,4-BDC, Aldrich 99%) sont dispersés dans 25 ml d'eau distillée avec 0,25 mL de HCl 1M (Aldrich, 35% ; ajoutés goutte à goutte). Le tout est laissé dans un corps en Téflon 5 min à 60°C avec un rampe de chauffage de 40 secondes (puissante 400W). Le solide marron foncée est récupéré par centrifugation à 10000 rpm pendant 10min. Le composé est lavé avec de l'éthanol absolu afin d'éliminer l'acide non consommé puis centrifugé à nouveau.

Afin d'éviter la dégradation, le composé est gardé humide.

La taille de particule monodisperse (PDI=0.005) mesurée par diffusion de lumière est 271 nm.

Surface Langmuir = 2042,7091 m²/g.

### c) MIL-88B-NH₂(Fe) ou Fe₃O[NH₂-C₆H₃-(CO₂)₂]₃.X.nH₂O (X=F, Cl, OH)

Les conditions de synthèse microondes sont les suivantes :
405 mg (1,5 mmol) de FeCl₃.6H₂O et 534 mg d'acide amino-1,4 benzenedicarboxylique (3 mmol, NH₂-1,4-BDC, Aldrich 99%) sont dispersés dans 25 ml d'éthanol absolu (Aldrich). Le tout est laissé dans un corps en Téflon 5 min à 100°C avec une rampe de chauffage de 40 secondes (puissance 800W). Le solide marron foncée est récupéré par centrifugation à 10000rpm pendant 10min. Le composé est lavé à l'éthanol absolu afin d'éliminer l'acide non consommé, puis centrifugé à nouveau.

La taille de particule monodisperse (PDI=0.069) mesurée par diffusion de lumière est 106 nm.

### d) MIL-88B-2CF₃(Fe) ou Fe₃O[(CF₃)₂-C₆H₂-(CO₂)₂]₃.X.nH₂O (X=F, Cl, OH)

Les conditions de synthèse microondes sont les suivantes : 675 mg (2,5 mmol) de FeCl₃.6H₂O, 755 mg d'acide 2,5-bis(trifluoromethyl)terephthalique (2,5 mmol, synthèse B de l'exemple 1) sont dispersés dans 25 ml d'éthanol absolu (Aldrich). Le mélange est laissé dans un corps en Téflon 5 min à 100°C avec un rampe de chauffage de 30 secondes (puissance 400W). Le solide est récupéré par centrifugation à 10000rpm pendant 10min.

Le solide est ensuite calciné sous vide à 200°C pendant 15 heures.

La taille de particule monodisperse (PDI=0.209) mesurée par diffusion de lumière est de 78 nm.

### e) MIL-88B-NO₂(Fe) ou Fe₃O[NO₂-C₆H₃-(CO₂)₂]₃.X.nH₂O (X=F, Cl, OH)

Les conditions de synthèse microondes sont les suivantes : 1,35 mg (10 mmol) de FeCl₃.6H₂O et 1,055 mg d'acide nitrotéréphthalique (10 mmol, Aldrich, 98%) sont dispersés dans 25 ml d'eau distillée. Le tout est laissé dans un corps en Téflon 5 min à 100°C avec un rampe de chauffage de 90 secondes (puissance 400W). Le solide est récupéré par centrifugation à 10000rpm pendant 10min.

La taille de particule monodisperse (PDI=0.005) mesurée par diffusion de lumière est de 408 nm.

### f) MIL-53-2CF₃ (Fe) ou FeO(OH) [C₆H₂-(CF₃)₂-(CO₂)₂].X.nH₂O (X=F, Cl, OH)

Les conditions de synthèse microondes sont les suivantes : 675 mg (2,5 mmol) de FeCl₃.6H₂O et 755 mg d'acide 2,5-bis(trifluoromethyl)terephthalique (2,5 mmol, synthèse B) sont dispersés dans 25 ml d'eau distillée. Le tout est laissé dans un corps en Téflon 20 min à 100°C avec un rampe de chauffage de 90 secondes (puissance 400W). Le solide jaune claire est récupéré par centrifugation à 10000rpm pendant 10min. Le composé est ensuite calciné sous vide à 250°C pendant 15 heures.

La taille de particule (PDI=0.245) mesurée par diffusion de lumière est de 330 nm.

### g) MIL-88A (Fe) ou Fe₃O[(C₄H₂-(CO₂)₂]₃.X.nH₂O (X=F, Cl, OH)

Les conditions de synthèse microondes sont les suivantes : 270 mg (1 mmol) de FeCl₃.6H₂O, 116 mg d'acide fumarique (1,0 mmol, Acros 99%) sont dispersés dans 30 mL de l'eau distillée. Le tout est laissé dans un corps en téflon 2 min à 100°C avec une rampe de chauffage de 1 min (puissance 1600W).

Le solide est récupéré par centrifugation à 10000rpm pendant 10min.

200 mg du produit sont suspendus dans 100 mL d'eau distillée pour échanger l'acide fumarique restant. Le solide hydraté est récupéré par centrifugation à 10000rpm pendant 10min.

La taille de particule monodisperse mesurée par diffusion de lumière est de 120 nm.

### Exemple 8 : Procédé de synthèse de matériaux MOF par voie ultrasons

Le solide MIL-88A est synthétisé par voie ultrason à 0°C avec plusieurs temps de réaction différents (30, 60, 90 et 120 minutes).

La synthèse est réalisée à partir d'acide fumarique et de chlorure de fer III hexahydraté dans l'eau. Les deux réactifs solides sont pesés et dissous séparément dans l'eau dans les proportions données dans le tableau ci-dessous. La solution d'acide fumarique est portée à 70°C sous agitation pendant 120 min pour solubiliser le produit. Le chlorure de fer est agité à l'aide d'un agitateur magnétique pendant 30 min.

**Tableau 10. Solutions d'acide fumarique et de chlorure de fer III.**

| **Réactif** | **Formule** | **Fournisseur** | **Quantité** |
|---|---|---|---|
| Chlorure de fer III | FeCl₃, 6H₂O | ACROS, 97% | 0,54g |
| | | | dans 200ml d'eau (soit 2,7mg/ml) |
| Acide Fumarique | C₄H₄O₄ | ACROS, 99% | 0,232g |
| | | | dans 200ml d'eau (soit 1,16mg/ml) |

5 ml de chacune des 2 solutions ci-dessus sont ajoutées dans une fiole de 20 mL. Au total, 8 fioles sont préparées :
- 4 fioles où les réactions sont réalisées pour les 4 temps de synthèse : 30, 60, 90 et 120 min,
- 4 fioles où de l'acide acétique (30 µl) est ajouté 15 min avant la fin de chacune des synthèse de durée 30, 60, 90 et 120 min (la fin de la synthèse correspondant à la sortie du bain à ultrasons). L'addition du monoacide (l'acide acétique) produit l'arrêt de la croissance cristalline puisqu'il ne permet pas de relier deux atomes de fer (contrairement à l'acide fumarique qui est un acide dicarboxylique). De cette façon, l'acide acétique permet l'obtention de nanoparticules de plus petite taille et des suspensions de ces particules plus stables (évite la sédimentation de grandes particules et l'agrégation particulaire).

Les 8 fioles sont placés en même temps dans un bain de sonication à 0°C, pendant les durées t correspondantes (30, 60, 90 et 120 min).

Après la synthèse, un volume de 0,1 ml de solution est prélevé dans chaque fiole afin de déterminer la taille des particules par diffusion de lumière grâce à un appareil de Dynamic Light Scattering (DLS, Nanosizer). Le reste de solution est ensuite passé à la centrifugeuse à 10 000 rpm à 0°C pendant 15 min afin de séparer le surnageant du solide formé. Le surnageant est éliminé à l'aide d'une pipette pasteur et le culot récupéré est placé sous la hotte à température ambiante.

Appareillage utilisé :
- Bain de sonication : Labo-moderne TK 52H n° de série : 164046192 SONOCLEAN
- Centrifugeuse : JOUAN MR 1812
- Nanosizer : Coulter N4 PLUS USA ; Malvern

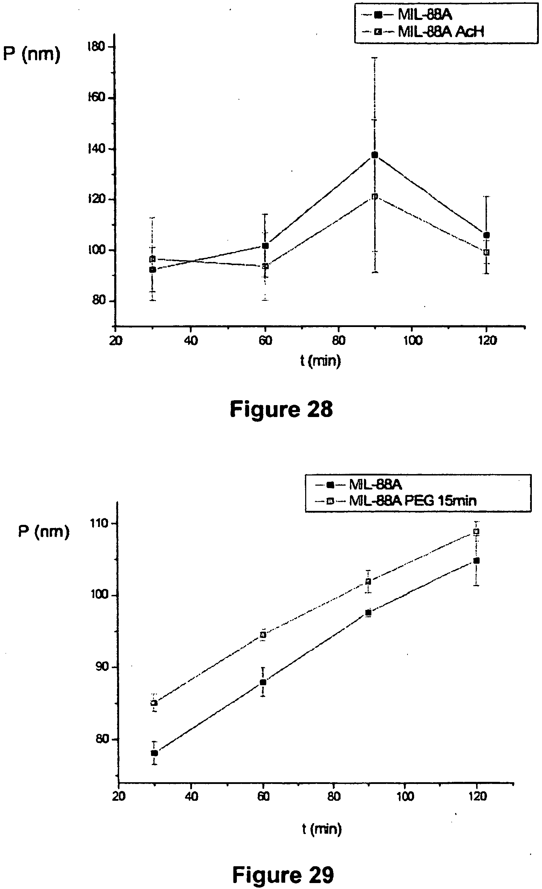

L'évolution de la taille de particule (P en nm) en fonction du temps (t en min) est représenté dans la figure 28. Il est possible observer une diminution de la taille de particule en présence de l'acide fumarique.

L'ajout d'acide acétique produit une diminution de la cristallinité, plus marqué à temps plus courts. Toutefois, il est possible de distinguir des réflexions caractéristique de la phase MIL-88A. Ces réflexions sont très élargis comme conséquence de la taille nanométrique du cristallite.

A des temps de synthèse plus longs, il n'y a pas de différence significative de la cristallinite entre entre la synthèse sans acide acétique et celle effectuée en présence de ce monoacide.

### IV. CARACTERISTIQUES ET PROPRIETES DES NANOPARTICULES OBTENUES

### Exemple 9 :Données analytiques et cristallographiques sur les carboxylates de fer

### a) Phase MIL-53(Fe) ou Fe(OH)[O₂C-C₆H₄-CO₂].H₂O

Les conditions de synthèse sont les suivantes : 0,27 g (1mmol) de FeCl₃.6H₂O, 166 mg (1mmol) d'acide 1,4 benzènedicarboxylique (1,4-BDC) dispersés dans 5 ml de diméthylformamide (DMF), le tout laissé 12 heures à 150°C dans un corps en Téflon de 23 ml mis dans une Bombe métallique PAAR. Le solide est ensuite filtré et lavé avec de l'acétone.

Les paramètres de maille de différentes formes de la phase flexible MIL-53(Fe) sous sa forme sèche (sec ; pores vides), hydratée (H₂O ; l'eau dans le pores), brut de synthèse (brut ; DMF dans les pores) et contenant du Busulfan (formes Bu1 et Bu2):

**Tableau 11. Paramètres de maille de différentes formes de la phase flexible MIL-53(Fe)**

| **Phase** | **a (Å)** | **b (Å)** | **c (Å)** | **Beta (°)** | **Volume (Å³)** | **Groupe d'espace** |
|---|---|---|---|---|---|---|
| MIL-53sec | 21,312 | 6,633 | 6,871 | 115,22 | 878,77 | C2/c |
| MIL-53(H₂O ) | 21,12 | 7,66 | 6,83 | 114,87 | 1003,0 | C2/c |
| MIL-53brut | 19,07 | 11,29 | 6,87 | 108,92 | 1398,3 | C2/c |
| MIL-53Bu1 | 17,61 | 6,75 | 10,25 | 112,97 | 1122,2 | C2/m |
| MIL-53Bu2 | 18,10 | 9,46 | 7,17 | 119,70 | 1202,5 | C2/c |

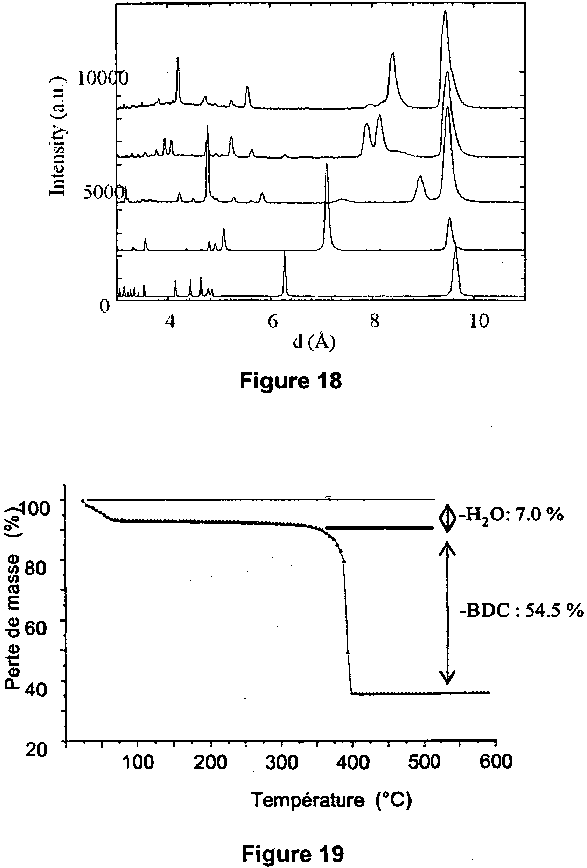

La figure 18 et la figure 19 représentent respectivement les diffractogrammes RX des différentes formes de solide MIL-53 et l'analyse thermogravimétrique du composé MIL-53(Fe) hydraté.

Le solide MIL-53 au fer ne présente pas de surface spécifique supérieure à 20 m²/g, puisque la structure poreuse est ferme dans la forme sec (Adsorption d'azote pars du vide)

**Tableau 12. Analyse élémentaire**

| Elément/ % massique | % Fer | % Carbone | % Hydrogène |
|---|---|---|---|
| MIL-53(Fe) | 21,11 | 37,60 | 2,75 |

### b) Phase MIL-88A ou Fe₃O[O₂C-C₂H₂-CO₂]₃.X.nH₂O (X=F, Cl, OH)

Les conditions de synthèse sont les suivantes : 0,27 g (1 mmol) de FeCl₃.5H₂O et 116 mg (1 mmol) d'acide fumarique sont dispersés dans 5 ml d'eau. Le tout est laissé 12 heures à 100°C dans un corps en Téflon de 23 ml mis dans une Bombe métallique PAAR. Le solide est ensuite filtré et lavé avec de l'acétone.

**Tableau 13. Paramètres de maille du solide MIL-88A, sec et hydraté**

| **Phase** | **a(Å)** | **c(Å)** | **Volume (Å³)** | **Groupe d'espace** |
|---|---|---|---|---|
| MIL-88A sec | 9,25 | 15,30 | 1135 | P-62c |
| MIL-88A hydraté | 13,9 | 12,66 | 2110 | P-62c |

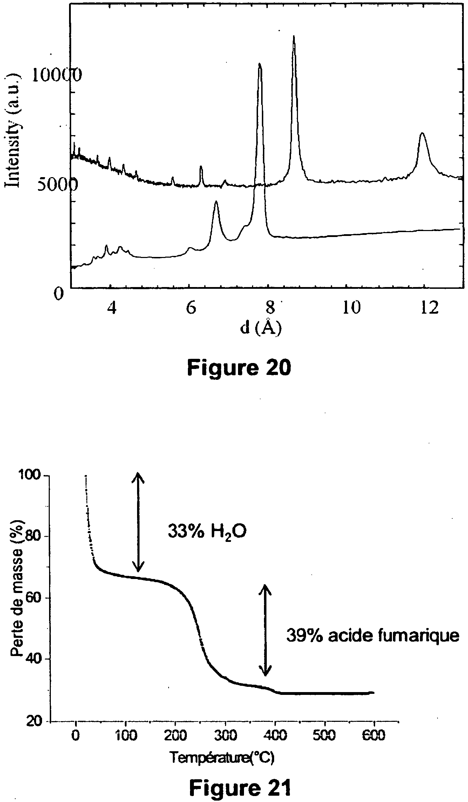

La figure 20 représente les diffractogrammes RX des solides MIL-88A sec et hydraté.

La figure 21 représente l'analyse thermogravimétrique du composé MIL-88A hydraté

Ce composé ne présente pas de surface accessible (supérieure à 20 m²/g) à l'azote à 77 K, puisque la structure sèche n'est pas poreuse.

**Tableau 14. Analyse élémentaire**

| Elément/ % massique | % Fer | % Carbone |
|---|---|---|
| MIL-88A (brut) | 21,8 | 24,0 |

### c) MIL-100(Fe) ou Fe₃O[C₆H₃-(CO₂)₃]₂.X.nH₂O (X=F, Cl, OH)

Les paramètres de maille sont a=73,1 Å et V=393000Å3, groupe d'espace Fd-3m (n°227).

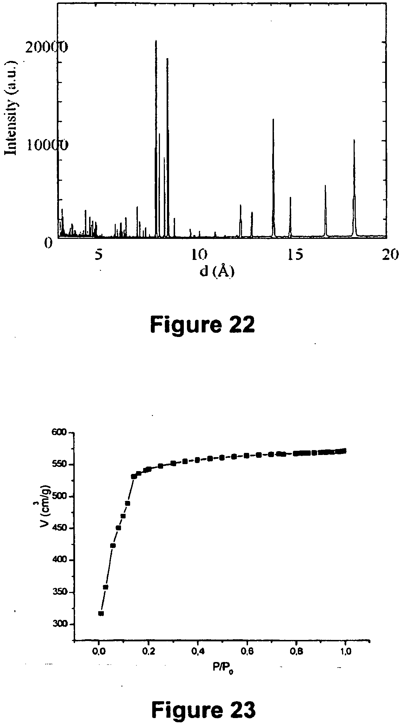

La figure 22 représente le diffractogramme RX du solide MIL-100(Fe).

La surface (Langmuir) spécifique de ce solide est proche de 2900 m².g⁻¹. La figure 23 représente l'isoterme d'adsorption d'azote à 77 K du solide MIL-100 (Po=1 atm.).

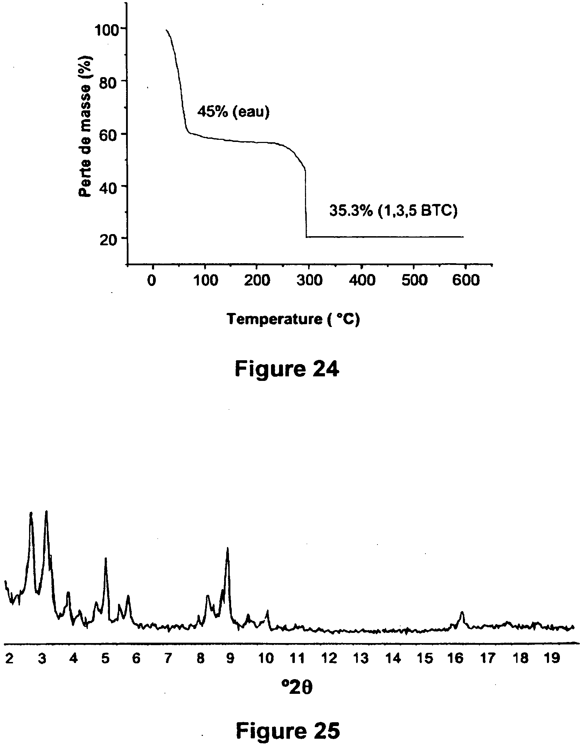

La figure 24 représente l'analyse thermogravimétrique (sous air) du composé MIL-100(Fe) hydrate (vitesse de chauffe de 5°C/minute).

**Tableau 15. Analyse élémentaire (CNRS, Vernaison) du solide MIL-100(Fe) avec X=F.**

| Elément/ % massique | % Fer | % Carbone | % Fluor |
|---|---|---|---|
| MIL-100(Fe) | 13,8 | 23,5 | 1,3 % |

### d) Phase MIL-101(Fe) ou Fe₃O[C₆H₄-(CO₂₎₂]₃.X.nH₂O (X=F, Cl, OH)

Les conditions de synthèse sont les suivantes : 0,27 g (1 mmol) de FeCl₃.5H₂O, 249 mg (1,5 mmol) d'acide 1,4 BDC dispersés dans 10 ml de DMF, le tout laissé 12 heures à 100°C dans un corps en Téflon de 23 ml mis dans une Bombe métallique PAAR. Le solide est ensuite filtré et lavé avec de l'acétone.

Les paramètres de maille du solide MIL-101(Fe) à 298 K sont : a=89,0 Å et V=707000Å3, groupe d'espace Fd-3m (n°227).

La figure 25 représente le diffractogramme RX du solide MIL-101(Fe) (λ_{Cu}=1,5406 Å).

L'optimisation des conditions de vidage des pores est en cours et donc aucune mesure de surface spécifique n'est disponible.

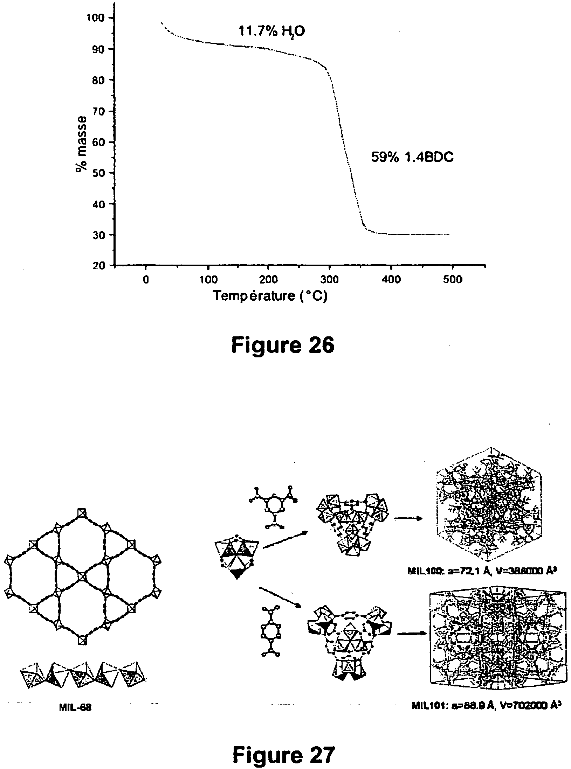

La figure 26 représente l'analyse thermogravimétrique (sous air) du composé MIL-101(Fe) hydraté (vitesse de chauffe de 5°C/minute).

La composition théorique du solide sec (X=F) est la suivante : Fe 24,2 % ; C 41,4 % ; F 2,7 % ; H 1,7 %.

### Exemple 10 : Mise en évidence de la flexibilité des solides

Deux types de solides flexibles sont concernés ici. Tout d'abord les carboxylates métalliques poreux nommés MIL-53 et MIL-69, de formule Fe(OH)[O₂C-C₆H₄-CO₂] et Fe(OH)[O₂C-C₁₀H₆-CO₂], respectivement, sont constitués de chaînes d'octaèdres reliées par des fonctions dicarboxylates, conduisant à un réseau poreux monodimensionnel, comme décrit dans C. Serre et al. J. Am. Chem. Soc., 2002, 124, 13519 [26] et dans T. Loiseau et al. C.R. Acad. Sci., 2005, 8, 765 [27]. A température ambiante, les solide sont hydratés et les pores sont fermés ; lorsque l'on imprègne ces matériaux avec des solvants organiques, les pores s'ouvrent et une porosité importante (environ 8-12 Å) devient accessible. La variation de volume de maille entre les formes hydratées et les formes gonflées varie entre 40 et 110%.

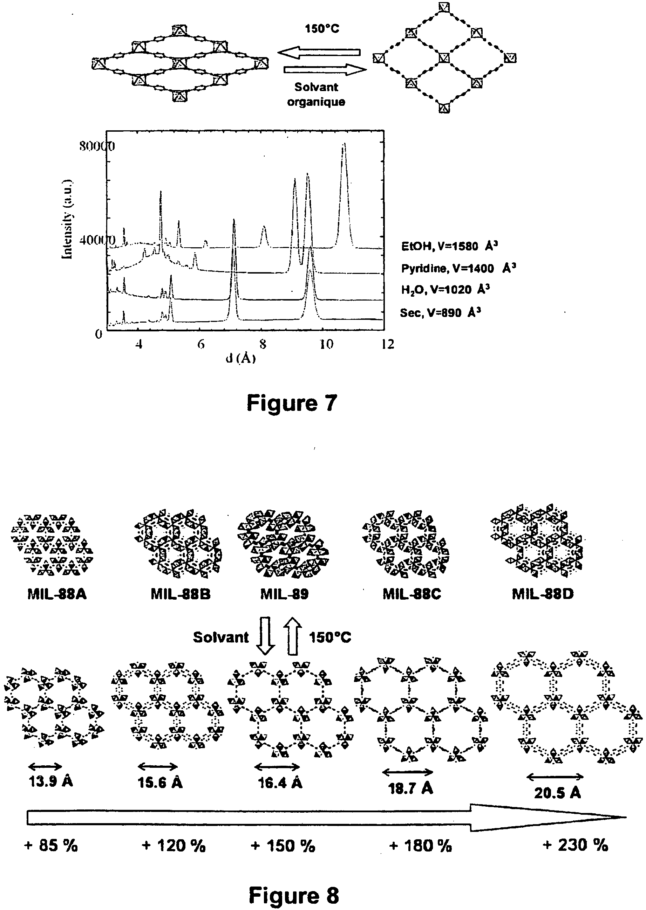

Ce phénomène est totalement réversible comme représenté sur la figure 7 annexée. L'ouverture des pores est aussi fonction de la nature du solvant (figure 7). Cela traduit une adaptation géométrique de la structure à la taille de l'adsorbat mais également une optimisation des interactions entre les molécules adsorbées et la charpente.

La seconde catégorie de solides hybrides flexibles est dénommée MIL-88. Ces composés sont construits à partir de trimères d'octaèdres de fer, c'est-à-dire trois atomes de fer connectés par un oxygène central et par six fonctions carboxylates connectant deux à deux les atomes de fer ; une molécule d'eau terminale, coordinée à chaque atome de fer vient ensuite compléter la coordinence octaédrique du métal. Ces trimères sont ensuite reliés entre eux par des acides dicarboxyliques aliphatiques ou aromatiques pour former les solides MIL-88A, B, C, D et MIL-89 à partir (-A pour acide fumarique, -B pour acide téréphthalique, -C pour acide 2,6-naphtalenedicarboxylique, -D pour acide 4,4'-biphényldicarboxylique et MIL-89 pour l'acide trans, trans muconique), comme décrit dans C. Serre et al., Angew. Chem. Int. Ed. 2004, 43, 6286 [28] et dans C. Serre et al., Chem. Comm. 2006, 284-286 [29].

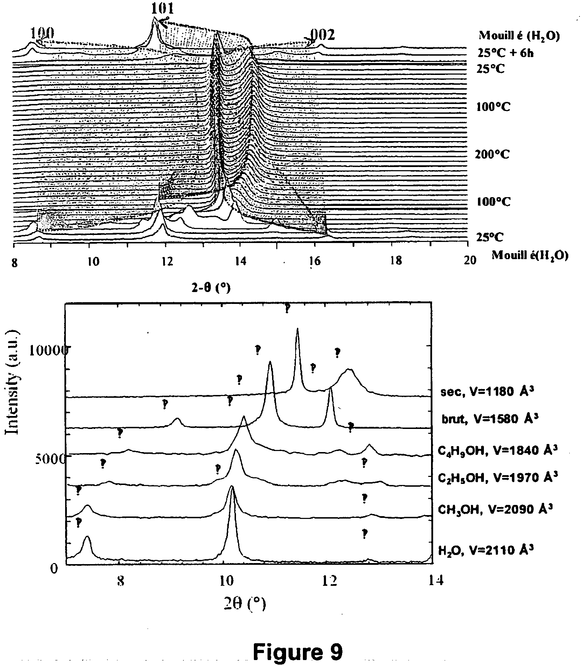

L'étude du comportement de ces solides par diffraction des RX, a permis d'établir que ces composés sont flexibles avec des amplitudes de « respiration » considérables entre leur forme sèche et leur forme solvatée. Il en résulte des variations de volume de maille entre 85 et 230 % selon la nature de l'espaceur organique (figure 8), comme décrit dans C. Serre et al., Science, 2007, 315, 1828 [30]. Les inventeurs ont noté que les formes sèches ne sont pas poreuses avec une taille de pores (tunnels) à peu près identique quelque soit le ligand carboxylique utilisé. Par contre, le gonflement du solide hybride en phase liquide est fonction de la longueur de l'espaceur organique. Ainsi, la distance entre trimères dans la forme gonflée passe de 13.8 Å avec l'acide fumarique (MIL-88A) à 20.5 Å avec le ligand biphényl (MIL-88D). La taille des pores des formes gonflées varie ainsi entre 7 Å (MIL-88A) à 16 Å (MIL-88D). Le gonflement est réversible, comme le montre l'exemple du solide MIL-88A en présence d'eau dans la figure 9 et dépend également de la nature du solvant utilisé comme décrit dans C. Serre et al. J. Am. Chem. Soc., 2005, 127, 16273-16278 [31]. La respiration s'effectue de manière continue, sans rupture apparente de liaisons durant la respiration. Par ailleurs, de retour à température ambiante, le solide gonfle à nouveau par resolvatation, confirmant le caractère réversible de la respiration.

Comment expliquer cette flexibilité ? Tout d'abord, dans le cas des solides MIL-53 et MIL-69, ils sont constitués de chaînes d'octaèdres reliées par des ponts dicarboxylates (figure 10a) ; l'examen des angles entre les fonctions carboxylates connectées aux centres métalliques et l'axe des chaînes inorganiques est très révélateur. Si le ligand carboxylate reste plan au cours de la respiration, ce dernier tourne de plusieurs degrés autour de l'axe des chaînes permettant ainsi à la structure de changer l'ouverture de ses pores. Tout se passe comme si l'axe O-O des fonctions carboxylates jouait le rôle d'une rotule avec une rotation de la liaison C-C du carboxylate autour du cycle aromatique pour relaxer les contraintes dues à la contraction. En ce qui concerne les composés MIL-88 et MIL-89, la situation est différente (figure 10a et c). En effet, si l'on regarde de près l'arrangement entre les trimères constitutifs de la structure, chaque trimère est relié à six autres trimères, trois en dessous et trois au dessus, par les dicarboxylates ce qui conduit à la formation de cages bipyramidales de trimères. Au sein de ces cages, la connexion entre trimères s'effectue uniquement selon l'axe c et l'absence de tout lien dans le plan (ab) est à l'origine de la flexibilité. En effet, lorsque l'on insère un solvant dans le matériau, la cage se déforme avec un rapprochement des trimères selon l'axe c et un éloignement dans les directions a et b, ce qui provoque une augmentation globale du volume de la cage (figure 10). Finalement, la flexibilité de ces solides hybrides est remarquable mais cependant comparable à celle de certains polymères. La principale différence concerne la cristallinité des solides hybrides, les polymères étant amorphes. Enfin, contrairement aux polymères, le gonflement se produit dans les solides hybrides de manière anisotrope.

### Exemple 11 : Mesures de relaxivité d'un carboxylate de fer(III) selon l'invention

Dans cet exemple, la relaxivité est mesurée sur le fumarate de fer (III) FeTCF MIL-88A.

### a) Préparation du fumarate de fer (III)

Une solution de chlorure de fer (1 mmol) et d'acide fumarique (1 mmol) dans 4,8 mL de dimethylformamide (DMF) et 0,4 mL NaOH 2M est placée dans un récipient en téflon avec un corps métallique et chauffée à 150°C pendant 2 heures. Puis, la bombe métallique est tout de suite refroidie sous l'eau. Le solide résultant est récupéré par centrifugation à 10000 rpm pendant 10 min. Pour éliminer le solvant qui reste dans les pores, 200 mg de solide sont suspendu dans 100 mL de l'eau sous agitation pendant une nuit puis récupéré par centrifugation (à 10000 rpm pendant 10 min). Des nanoparticules de 210 nm sont obtenues.

### b) Mesure de la relaxivité des nanoparticules

Les particules de fumarate de fer(III) FeTCF MIL-88A ayant une taille de 210 nm sont dispersées dans l'eau contenant 5% en poids de glucose, afin d'obtenir une concentration en nanoparticules de 59 mg/ml.

Les relaxivités r1 et r2 ont été évaluées à 9,4T à l'aide d'un spectromètre à aimant vertical (Bruker) par la mesure du temps de relaxation Ti (i=1,2) du milieu sans particules et des temps de relaxation Tic en fonction de la concentration en fer (en mM).

A 20°C :
- la relaxivité transversale r2 est de 8,6 mMs⁻¹.
- la relaxivité longitudinale r1 est de 0,12 mMs⁻¹.

### c) Imagerie in vivo des nanoparticules MIL-88A

Les expériences d'imagerie par résonance magnétique (IRM) ont été conduites à 300 MHz à l'aide d'un aimant horizontal de bore de 7 T (Oxford, UK) guidé par Paravision (marque enregistrée : Bruker, Allemagne) et équipé d'un système à gradient (360 mT/m).

Les rats Wistar, femelles, ont été injectés avec des doses de 200 mg/kg de nanoparticules de MIL88A(Fe) dont la synthèse est décrite précédemment.

Chaque rat a été sacrifié 30 minutes après injection par une overdose d'isoflurane, puis introduit dans une sonde, équipée d'une bobine *loop-gap* de 60 mm de diamètre. Après un rapide repérage spatial, une série d'images pondérées en densité de proton est réalisée sur chaque animal : séquence RARE [ TR/TE=1781,21/8,8ms; Facteur Rare = 4 ; 4 moyennes; 39 tranches contigües de 1mm; FOV 70*70mm; matrice d'acquisition 384*384; matrice de reconstruction 512*512; résolution dans le plan 136m*136m; temps d'expérience 8min 32s] et séquence FLASH [ TR/TE=564.4/6.7ms; 2 moyennes; 39 tranches contigües de 1mm; FOV 70*70mm; matrice d'acquisition 384*384; matrice de reconstruction 512*512; résolution dans le plan 136m*136m; temps d'expérience 7min 13s].

Quatre tranches ont été analysées dans chaque zone d'intérêt (le foie et la rate). De plus, pour chaque organe, les valeurs moyennes des RI ont été calculées et normalisées par rapport à la zone correspondant au muscle dorsal. Les résultats obtenus en IRM montrent clairement qu'une injection de nanoparticules entraine des différences importantes de contraste dans les deux organes étudiés, le foie et la rate. Par ailleurs, les deux séquences, FLASH et RARE montrent que les organes des rats injectés avec les nanoparticules apparaissent plus foncés que ceux des rats non traités. La bonne détection *in vivo* des nanoparticules de carboxylate de fer les positionne comme candidats d'intérêt pour l'imagerie par résonance magnétique.

### d) Imagerie in vitro des nanoparticules MIL-88A

Pour chaque type de nanoparticules, 6 échantillons contenant différentes concentrations sont préparé en les mettant en suspension dans une solution eau-glucose 5% (C: 1mg/ml, 0,5mg/ml, 0,2 mg/ml, 0,1mg/ml, 0,05mg/ml, 0 mg/ml).

Les expériences IRM sont réalisées à l'aide d'un aimant horizontal de bore de 9,4T (Oxford, UK) guidé par Paravision (Bruker, Allemagne). Les exériences d'imagerie « Spin-echo » sont utilisées pour la determination des temps de relaxation T1 et T2 (FOV 15x15mm; épaisseur des tranches : 1mm; matrice d'acquisition 32x32). T1 est determiné selon une méthode de rétablissement par saturation (séquence Spin-echo; TE=10 ms TR=4000, 2000, 1000, 500, 200, 100 ms) et T2 selon la méthode de Carr-Purcell-Meiboom-Gill (séquence RARE; images-écho de facteur rare 8 et 8; TR/TE=15000/8 ms). Les échantillons d'agent de contraste sont introduits dans le tube. Les mesures de T1 et T2 sont effectuées, induisant un temps d'acquisition total de moins de 6 min. La relaxivité de chaque type de nanoparticules à un champ magnétique donné est donné par la pente de la droite représentant le taux de relaxation en fonction de la concentration de produit.

La bonne détection des nanoparticules de MOF en font de bons candidats comme agents de contraste. L'efficacité des agents de contraste est directement liée à leur relaxivité ou leur capacité à modifier les temps de relaxation des protons de l'eau dans le milieu environnant quand un champ magnétique est appliqué. Plus la quantité et la mobilité des molécules d'eau dans la 1^{ère} et 2^{e} sphère de coordination du métal est grande, plus la relaxivité est grande. Aussi, les nanoparticules MOF ne possèdent pas seulement des atomes de fer paramagnétique, mais une structure poreuse interconnectée avec de nombreuses molécules d'eau. Le Tableau 16 répertorie les valeurs de relaxivités des nanoparticules de fumarate de fer obtenues avec un champ magnétique de 9.4T. Les valeurs de relaxivités r1 and r2 des nanoparticules MIL-88A sont de l'ordre de 1 s⁻¹ M⁻¹ et de 100 s⁻¹ mM⁻¹ respectivement, ce qui est satisfaisant pour une utilisation *in vivo* (ref. Roch et al, J Chem Phys 110, 5403-5411, 1999). Les valeurs de relaxivité ne sont pas seulement liées à la teneur en fer mais également à la taille des nanoparticules. Les nanoparticules PEGylées (dont la surface est modifiées par le PEG ou polyéthylèneglycol) ont des relaxivités r1 inférieures, mais des r2 égaux ou légèrement supérieurs à ceux des matériaux non non-PEGylés. Le revêtement PEG peut modifier les relaxivités selon deux effets opposés : d'une part il accroit la taille des particules, d'autre part, il diminue leur capacité à s'aggréger.

**Tableau 16. Relaxivités des nanoparticules nanoMIL88A et nanoMIL100, PEGylées ou non, measurées à 9.4T.**

| | **Fe(mmol/l** | **PEG (wt%)** | **r1 (s⁻¹mM⁻¹)** | **r2 (s⁻¹mM⁻¹)** |
|---|---|---|---|---|
| **MIL88a** | 3,75 | 0 | **1,3** | **80** |
| **MIL88a+PEGPO4** | 2,91 | 13,6 | **0,86** | **117** |
| **Mil 100** | 3,00 | 0 | **0,47** | **60** |
| **MIL100+PEGPO4** | 2,70 | 13,3 | **Non mesuré** | **53** |

### Exemple 12 : Essais in vivo de toxicité des carboxylates de fer (III)

### a) Carboxylates de fer testés

Les deux solides carboxylates de fer suivants (synthétisés selon les modes opératoires de l'exemple 1) sont respectivement testés :
- MIL-88A(Fe) de composition Fe₃O(O₂C-C₂H₂-CO₂]₃·OH·*n*H₂O
- MIL-88Bt(Fe) de composition Fe₃O[O₂C-C₆(CH₃)₄-CO₂]₃·OH·*n*H₂O

### b) Tests de toxicité

L'étude de toxicité aiguë *in vivo* est effectuée sur des rats Wistar femelles de 4 semaines (-125 g) en injectant par voie intraveineuse aux rats des doses croissantes (50, 100 et 200 mg/kg) de nanoparticules MIL-88A (de 210 nm) et MIL-88Bt (de 100 nm) suspendus dans 0,5 mL d'une solution de glucose 5%.

Les nanoparticules sont stables dans ce milieu.

Le temps de stabilité de ces suspensions est réduit à quelques minutes quand la concentration de particules est maximale (200 mg/kg, -25 mg/0,5mL). Pour cette raison, les prélèvements sont réalisés sous agitation douce des suspensions de nanoparticules. Il n'a pas été possible d'administrer des doses supérieures à 200 mg/kg, puisque le volume maximal injectable à des rats est 0,5 ml.

Les résultats sont prometteurs étant donné qu'aucun signe de toxicité majeur n'est observé après 7 jours d'essai. Les valeurs sériques de l'albumine, cholestérol et transaminases (ASAT/ALAT) ne présentent pas de variation significative après 7 jours d'essai et le poids des organes par rapport au poids corporel ne varie pas significativement (Tableau 7).

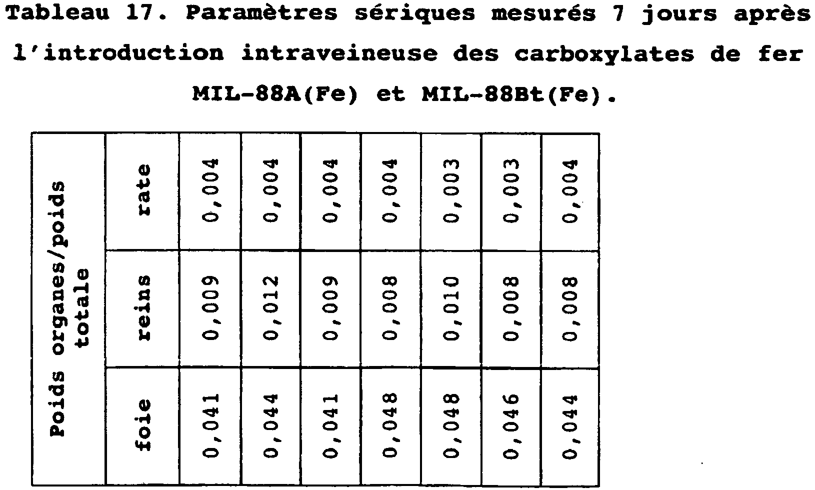

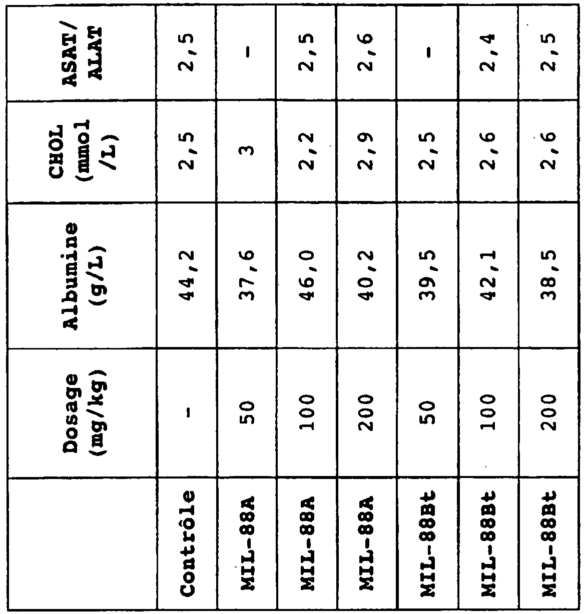

Les coupes histologiques du foie sont observées par coloration de Proust (fer en bleu), et présentées en figure 13. Elles montrent une accumulation de fer dans le foie. Bien qu'il soit nécessaire d'effectuer une étude plus approfondie sur les effets de ces solides dans l'organisme à long terme, ces résultats sont très prometteurs, et permettent d'envisager des applications biomédicales pour ces matériaux.

Des études de toxicité aiguë et subaiguë plus approfondies ont été réalisées.

Les animaux utilisés pour l'expérimentation sont des rats Wistar femelles de quatre semaines avec un poids de 161,36 ± 16,1 g.

Tous les essais ont été réalisés dans l'animalerie de l'Université de Pharmacie dans des conditions de température et humidité, et après 3 jours d'adaptation des animaux a l'animalerie (3 jours).

Pour les tests de toxicité aiguë, une injection intrajugulaire unique des matériaux MIL-88A (150 et 500 nm), MIL-88Bt (50 et 140 nm) ou de glucose 5% (groupe témoin) est effectuée à 4 groupes (à respectivement 1 jour, 1 semaine, 1 mois et 3 mois) de 8 rats choisies au hasard et anesthésiés sous isoflurane.

L'évolution du poids des animaux ainsi que leur comportement ont été suivis.

Des prélèvements de sang dans la jugulaire sous anesthésie par isoflurane ont également été réalisés à différents temps : 1 et 3 jours, 1 et 2 semaines, 1, 2 et 3 mois. Le sérum a été isolé pour mesurer des paramètres sériques tels que IL-6 (interleukine 6), albumine, Fe sérique, PAS, GGT, bilirubine, cholestérol et transaminases.

Par ailleurs, chaque groupe d'animaux a été sacrifié après respectivement 1 jour, 1 semaine, 1 et 3 mois. Les animaux ont été anesthésiés à l'isoflurane puis la rate, les reins, le foie et le coeur ont été prélevés et conservés pour des études histologiques. Quatre fois ont également été utilisés pour réaliser une extraction microsomale afin de mesurer l'activation du cytochrome P450.

Pour les tests de toxicité subaiguë, une injection intrajugulaire par jour est effectuée pendant 4 jours consécutifs sur 26 rats répartis au hasard dans différents groupes où les animaux sont sacrifiés au bout de 5 ou 10 jours.

L'évolution du poids des animaux isolés ainsi que leur comportement alimentaire (mesure des quantités d'eau et de nourriture consommées) ont été suivis. Les urines et déjections ont également été récupérés.

Des prélèvements de sang dans la jugulaire ont également été effectués sur différents groupes de rat à 3 et 5 jours, 8 et 10 jours. Le sang subit le même traitement que pour l'essai de toxicité aigue et le sérum obtenu est destiné aux mêmes analyses.

Les jours des sacrifices, aux 5 et 10 jours, les animaux sont anesthésiés à l'isoflurane puis la rate, les reins, le foie, le coeur et les poumons sont prélevés et traités de même que pour l'essai de toxicité aiguë.

### c) Résultats

### Evolution du poids des animaux :

Les animaux ont été pesés tous les jours dans le but de comparer l'évolution du poids des différents groupes. Une moyenne a été effectuée pour chaque jour et dans chacun de ces groupes.

Pour les tests de toxicité subaiguë, l'augmentation du poids observée avec le groupe du glucose est légèrement diminuée quand on administre le matériau. Cette variation est plus évidente quand la dose administrée est plus élevée

Les études en toxicité aiguë montrent que l'administration des matériaux MIL-88A et MIL-88Bt ne produit pas de variation significative du poids au cours du temps.

### Evolution de la consommation d'eau et de nourriture :

En toxicité subaiguë, l'évolution est globalement similaire pour le groupe témoin et le groupe ayant reçu une injection de 25 mg/kg. Une différence plus marquée est observée dans le groupe ayant reçu la plus forte dose et se caractérise par une plus faible consommation de nourriture pendant l'étude. Cette observation est confirmée et en totale concordance avec les résultats obtenus par l'évolution du poids.

### Comparaison du poids des organes prélevés :

Résultats de toxicité subaiguë : il n'apparait pas de différence significative entre le poids de rate, reins et coeur des différents groupes. Le poids des poumons parait être légèrement augmenté à 5 jours comme à 10 jours.

Toxicité aiguë : on observe une augmentation du poids de la rate jusqu'à une semaine après l'administration, et un retour a la normale à 1 et 3 mois pour le MIL-88A et MIL-88Bt, respectivement. Le poids du foie augmente de façon importante quand les matériaux sont injectés, ce qui traduit peut être l'accumulation du fer dans le foie. On observe que la situation revient à la normale pour le MIL-88A après 3 mois, mais pas pour le MIL-88Bt ou le poids reste élevé.

### Dosage du cytochrome P450 dans les suspensions microsomales :

Le cytochrome P450 est un enzyme associé à la face interne du réticulum endoplasmique lisse qui est fortement impliqué dans la dégradation des molécules exogènes. Cet enzyme possède une très faible spécificité de substrat et est capable de catalyser la transformation de composés nouvellement synthétisés tels que les médicaments. La plupart des cytochromes P450 peuvent être induits ou réprimés, au niveau transcriptionnel, par différents xénobiotiques ; ceci est souvent la cause d'effets secondaires des médicaments. Le dosage de cet enzyme permet de déterminer si le matériau MOF utilisé est métabolisé par le cytochrome P450 auquel cas il activerait ou inhiberait son activité.

La quantité de cytochrome n'est interprétable qu'à condition d'avoir été rapportée à la quantité totale de protéines contenue dans chaque échantillon. Le dosage de protéines contenues dans l'échantillon a été effectué grâce à l'utilisation d'un kit BCA fourni par PIERCE (lot #HI106096). Cette méthode combine la réduction du Cu²⁺ en Cu⁺ par les protéines en milieu alcalin avec la détection colorimétrique, très sensible et sélective, du cation Cu⁺ grâce à un réactif contenant l'acide Bicinchoninique (BCA).

Le rapport entre la concentration de cytochrome et la quantité de protéines totale donne l'activité du cytochrome exprimée en mol.g⁻¹. Les résultats en toxicité aiguë montrent qu'il n'y a pas de grosse différence d'activité entre le groupe témoin négatif (ayant reçu le glucose) et le groupe « MIL-88A » dont le matériau n'est pas métabolisé par le Cyp450. Le matériau MIL-88Bt ne semble pas non plus être métabolisé par le Cyp450.

### Dosage de l'interleukine 6 dans le sérum :

L'interleukine 6 (IL-6) est une cytokine multifonctionnelle qui joue un rôle important dans la défense de l'hôte, les réponses immunitaires, les fonctions des cellules nerveuses et l'hématopoïèse. Un niveau élevé d'IL-6 dans le sérum a par exemple été observé lors d'infections virales et bactériologiques, de trauma, de maladies auto-immunes, d'inflammation, ou de cancer.

Cette étude a pour de déterminer s'il existe une réaction inflammatoire après l'administration des nanoparticules de carboxylates de fer. Ainsi, il est possible de voir si le niveau d'IL-6 est augmenté par rapport aux groupes témoins (injection de glucose, donc réaction inflammatoire locale par la piqûre).

Le dosage a été effectué par l'utilisation d'un kit « Quantikine, Rat IL-6 » fourni par les laboratoires R&D Systems.

Résultats de toxicité subaiguë : les variations ne sont pas significatives. Une augmentation du taux plasmatique observé (activation de la production d'IL-6) semble être dû phénomène de piqûre lors des injections qui produisent une inflammation locale, si on compare de façon isolée les différents groupes avec le groupe témoin (glucose).

Résultats de toxicité aiguë : les variations ne sont pas significatives et conduisent aux mêmes conclusions que dans le cas de la toxicité subaiguë.

### Dosage des paramètres sériques :

Tous les dosages ont été réalisés en utilisant des dispositifs automatiques. Quelques paramètres clés ont été déterminés pour évaluer les conséquences des injections de nanoparticules au niveau du foie, des taux de transaminases (Alanine amino transférase ou ALAT et aspartate amino transférase ou ASAT), phosphatases alcalines (PAS), γ-glutamate transférase (GGT), bilirubine, cholestérol, albumine et fer sérique.

Les résultats montrent que les niveaux sériques de l'ALAT sont tout à fait normaux, tout comme les niveaux de bilirubine (< 2 µmol/L) et de γ-glutamique transférase (<2 UI/L).

Les niveaux d'albumine sérique ont légèrement diminués après le premier jour d'injection pour les deux matériaux, en accord avec un processus inflammatoire local du à la piqûre et avec l'augmentation de la IL-6 observée précédemment. Après 3 jours, les niveaux reviennent à la normale.

Les niveaux sériques de l'ASAT sont augmentés un jour après l'injection, ce qui peut indiquer un processus de cytolyse. Toutefois, 3 jours après l'administration des nanoparticules, les valeurs retournent à la normale. De la même façon, la phosphatase alcaline se trouve augmentés après 1 jour, indiquant un processus de cytolyse, mais la situation revient à la normale après 3 jours. Le retour à la normale après 3 jours indique qu'il s'agit d'un processus de cytolyse transitoire et non permanent. Il n'y a donc pas de perte de fonction cellulaire.

Les niveaux de cholestérol sont normaux.

Les niveaux de fer sériques sont diminués en comparaison avec le groupe témoin, et cela est plus prononcé dans le groupe MIL-88A. Cela pourrait s'expliquer par une complexation du fer sérique par les nanoparticules. La situation revient à la normale 3 jours après l'administration.

Les paramètres sériques ont aussi été dosés à 1 semaine et d'après ces résultats, il n'y a plus de différence entre les 3 groupes au niveau du fer sérique ; les rats traités au MIL-88A et MIL-88Bt ont récupéré une concentration en fer sérique comparable à celle du groupe témoin. Par ailleurs, concernant les niveaux des autres paramètres sériques, il n'y a pas une différence significative en comparaison avec le groupe témoin.

### Coupes histologiques :

Les coupes histologiques, d'une épaisseur de 5 *µ*m, sont effectuées dans un cryostat, déshydratées et colorées (coloration hématoxyline/éosine puis coloration au bleu Proust : coloration du fer en bleu).

Par l'observation des coupes histologiques, il est possible de déterminer la voie d'élimination des composés du matériau ou leur stockage dans certains organes : foie, reins, rate et poumons, le coeur étant utilisé comme témoin.

Résultats de toxicité aiguë : les coupes histologiques de foie montrent une accumulation du fer dans le foie après l'injection des matériaux qui est plus importante pour le solide MIL-88A. Le matériau semble être sous sa forme de nanoparticules non degradées. L'accumulation est moins importante pour le matériau MIL-88Bt, ce qui peut signifier une plus faible captation pour le foie ou la réutilisation plus rapide du fer stocké. Apres 1 et 3 mois le contenu en fer dans la rate et le foie revient à la normale.

### Dosage du fer dans les suspensions injectées et dans les organes :

Le dosage du fer contenu dans les suspensions de MIL-88A et MIL-88Bt injectées aux animaux est effectué par spectrophotométrie UV-Visible à la longueur d'onde de 520 nm, par colorimétrie spécifique des ions ferreux avec la bipyridine (formation d'un complexe rouge), après solubilisation de l'oxyde de fer dans de l'acide sulfurique concentré, et réduction des ions ferriques en ions ferreux par l'acide ascorbique.

Le dosage du fer dans les organes est réalisé de même que pour le dosage en fer des suspensions expliqué précédemment, après broyage de l'organe à tester. Ce dosage permet de déterminer la voie d'élimination des composés du matériau ou leur stockage dans certains organes : foie, reins, rate et poumons, le coeur étant utilisé comme témoin.

### d) Conclusion

Lors des essais de toxicité, l'observation minutieuse des animaux n'a révélé aucun signe apparent de nocivité du matériau injecté. En effet, les animaux ont gardé un comportement tout à fait normal. Pendant les études, les animaux ont bien grossi en comparaison avec le groupe témoin, même si pour l'étude de toxicité subaiguë l'augmentation du poids est moins importante que pour le groupe témoin, probablement lié a l'administration consécutive des doses importantes. La consommation d'eau reste elle globalement normale dans l'essai de toxicité subaiguë.

Un dosage du cytochrome P-450 a permis d'observer l'état de l'activité du cytochrome P-450 sur une longue période. Ce cytochrome est connu pour sa capacité à métaboliser certains xénobiotiques. L'étude montre que le taux d'activité, bien que fluctueux, reste en dessous des valeurs observées sur les rats témoins qui ont reçu une injection de phénobarbital, activateur de cytochrome P-450, ce qui indique que les matériaux ne sont pas métabolisés par voie Cyp450, en concordance avec la polarité importante des ligands dicarboxyliques.

Les résultats sont très prometteurs et indiquent déjà que les matériaux MIL-88A et MIL-88Bt n'induisent aucun signe de toxicité sévère, même si des études de toxicité complémentaires doivent être effectuées. Le devenir et les effets des nanoparticules dans l'organisme sont en cours d'étude afin de mettre en relation le bénéfice apporté par ces matériaux par la vectorisation de médicaments délicats à encapsuler et présentant un grand potentiel thérapeutique. Des études similaires sont également en cours de réalisation avec d'autres nanovecteurs de structure et/ou de composition différentes.

### Exemple 13 : Dégradation in vitro des nanoparticules MIL-88A

### a) Etude N°1

La dégradation des nanoparticules de MIL 88A a été étudiée lors de leur incubation à 37°C dans une solution tampon phosphate (PBS) à pH 7,4, sous agitation bidimensionnelle. La concentration des nanoparticules était de 50 µg/mL. Après différents temps d'incubation, les suspensions de nanoparticules ont été centrifugées (10000rpm, 15 min, 0°C). L'acide fumarique libéré par dégradation des nanoparticules a été quantifié dans le surnageant par HPLC en phase inverse, grâce à une détection spectrophotométrique (λ= 210 nm). Nous avons utilisé une colonne Symmetry (marque enregistrée) C18 phase inverse (5µm, 3,9X150 mm, Part. NO. WAT046980, Waters). La phase mobile était un mélange de méthanol (25% en volume) (Aldrich, HPLC grade) et d'acide phosphorique 10mM (75% en volume) (Aldrich, HPLC grade). Le débit de la phase mobile était de 0,5 mL.min⁻¹ et la température de la colonne de 25°C. Le volume d'injection était de 10 µL. Le système a été calibré avec des solutions standard d'acide fumarique. Le temps de rétention de ce produit était d'environ 2 min.

Nous avons observé qu'après deux jours d'incubation, environ 88% de la quantité totale d'acide fumarique entrant dans la composition des nanoparticules avait été libérée : la figure 34 représente la libération de l'acide fumarique du solide MIL-88A en pourcentage (%) en fonction du temps t (en jours). Une dégradation totale (100% d'acide fumarique libéré) a été observée au bout de trois semaines d'incubation.

### b) Etude N°2

La dégradation du matériau MIL-88Anano (150 et 500nm) a été étudié à l'aide d'une suspension des nanoparticules (50 mg/mL) dans une solution de PBS pH 7,4 à 37°C sous agitation bidimensionnelle. A différents temps le surnageant est récupéré par centrifugation (10000rpm/10min à 0°C) et la quantité d'acide fumarique libéré est déterminée par HPLC (phase reverse, pompe Waters 501 HPLC, autoéchantillon Waters™ 717 plus, détecteur Waters™ 486 et spectrophotomètre UV-vis à λ= 210nm). La colonne de phase inverse Symmetry® C18 (5µm, 3,9X150 mm, Part.NO. WAT046980, Waters) est utilisée. La phase mobile est un mélange de méthanol (25 vol%) (Aldrich, HPLC grade) et d'acide phosphorique 10mM (75 vol%) (Aldrich, HPLC grade). Le débit est de 0,5 mL.min⁻¹ et la température de la colonne est de 25ºC. Le volume d'injection est de 10 µL.

La rétention de l'acide fumarique est de 2 min. La concentration de l'acide fumarique est déterminée à l'aide d'une courbe de calibration avec des standards.

88% de la quantité total d'acide fumarique des nanoparticules est libéré après 2 jours d'incubation, et environ 96% après 9 jours. La dégradation totale se produit après 3 semaines d'essai.

### V. NANOPARTICULES CHARGEES EN MOLECULE D'INTERET

### Exemple 14 : Formulation de nanoparticules de carboxylate de fer (III) chargées en principe actif : le busulfan

Le Busulfan (1,4-butanediol-dimethylsulfonate) est un agent alkylant de la classe des alkylsulfonates qui présente un intérêt thérapeutique certain pour le traitement du cancer. Prescrit dans des protocoles de chimiothérapie « haute dose » avant autogreffe ou allogreffe de cellules souches hématopoïétiques, il constitue une excellente alternative à l'irradiation corporelle totale et par conséquent intéresse particulièrement la pédiatrie. Cependant, majoritairement capté par le foie, il présente une toxicité importante d'où l'intérêt de développer des systèmes de transport.

Les systèmes de libération de Busulfan testés jusqu'à présent posent un véritable défi quant à son encapsulation. Les charges maximales obtenues avec des liposomes ne dépassent pas les 0,5 % (en poids). L'utilisation de polymères biodégradables s'est révélée mieux adaptée mais le taux d'encapsulation de Busulfan ne dépasse pas les 5% (en poids) de Busulfan dans des nanoparticules à base de poly(cyanoacrylate d'alkyle).

Dans cet exemple, le busulfan est incorporé dans divers carboxylates de fer(III) selon l'invention, et notamment les matériaux MIL-53, MIL-88A, MIL-89 et MIL-100.

### a) Préparation du carboxylate de fer MIL-53

Le solide MIL-53 est synthétisé à partir de 270 mg de FeCl₃.6H₂O (1 mmol; Alfa Aesar, 98%), 166 mg d'acide 1,4-dicarboxylique (1 mmol; Aldrich, 98%) et de 5 mL de diméthylformamide (Fluka, 98%). le tout introduit dans un corps en Téflon mis dans un corps métallique (autoclave) de marque Paar, puis chauffé à 150°C pendant 24 heures. Après retour à température ambiante, le produit est récupéré par filtration et lavé avec de l'eau et l'acétone.

200 mg du solide sont ensuite suspendus dans 100mL d'eau distillée sous agitation pendant 15h pour éliminer le solvant résiduel présent dans les pores. Le solide est récupéré par filtration.

La taille de particule mesurée par diffusion de lumière est de 6,2 microns.

### b) Préparation du carboxylate de fer MIL-88A

Pour obtenir le matériau MIL-88A, on mélange 270 mg de FeCl₃.6H₂O (1 mmol; Alfa Aesar, 98%), 112 mg d'acide fumarique (1 mmol; Acros, 99%) dans 5 mL de dimthylformamide (Fluka, 98%) et on ajoute 0,4 mL de NaOH 2M. le tout introduit dans un corps en Téflon mis dans un corps métallique (autoclave) de marque Paar, puis chauffé à 100°C pendant 15 heures. Après retour à température ambiante, le produit est récupéré par filtration et lavé avec de l'eau et l'acétone.

200 mg du solide sont suspendus dans 100mL d'eau distillée sous agitation pendant 15h pour éliminer résiduel présent dans les pores. Le solide est ensuite récupéré par filtration.

La taille de particule mesurée par diffusion de lumière est de 2,6 microns.

### c) Préparation du carboxylate de fer MIL-89

A 240 mg d'acétate de fer (0,33 mmol) et 140 mg d'acide muconique (1 mmol) sont ajoutés dans 9 mL de méthanol. Ensuite, nous ajoutons lentement une solution de 0,35 mL NaOH 2M dans 1 mL de méthanol. Le tout est placé dans un récipient en téflon avec un corps métallique et chauffé à 150°C pendant 6h. Puis, la bombe métallique est tout de suite refroidie sous l'eau. Le solide résultant est récupéré par centrifugation à 5000 rpm 10 min. Pour éliminer le solvant qui reste dans les pores, 200 mg de solide est suspendu dans 100 mL de l'eau sous agitation pendant une nuit puis récupéré par centrifugation (5000 rpm 10 min).

La mesure de taille de particule par diffusion de lumière montre deux populations de nanoparticules de 1,1 microns.

### d) Préparation du carboxylate de fer MIL-100

La synthèse du MIL-100 se fait a partir de 56 mg de fer métallique (1 mmol; Aldrich, 99%) et 210 mg d'acide 1,3,5-benzenetricarboxylique (1,3,5-BTC ; 1 mmol; Aldrich, 95%) dans 3 mL d'eau distillée auquel nous ajoutons 0,4 mL de acide fluorhydrique (HF; 5M) et 0,6 mL d'acide nitrique 2N. Le tout est introduit dans un corps en Téflon mis dans un corps métallique (autoclave) de marque Paar. Le tout est chauffé avec une rampe de chauffage de 12h (25 à 150°C) pendant 6 jours à 150°C et avec une rampe de refroidissement de 24 h. Le produit est récupéré par filtration et lavé avec de l'eau et l'acétone.

200 mg de solide sont suspendus dans 100mL d'eau distillée sous agitation et reflux pendant 3h pour éliminer l'acide résiduel présent dans les pores. Le solide est ensuite récupéré par filtration à chaud.

La taille de particule mesurée par diffusion de lumière est de 1,7 microns.

### e) Introduction du busulfan dans les carboxylates de fer

L'insertion de Busulfan dans les pores des matériaux est effectuée par adsorption, en suspendant 25 mg de solide déshydraté dans 2,5 mL d'une solution contenant le médicament avec une concentration égale à 100% ou 80% de sa saturation dans le solvant, le tout sous agitation pendant 16 heures à température ambiante. Les particules sont ensuite récupérées par centrifugation (20ºC, 5000 rpm, 15 min). Le culot est séché (évaporation sous vide primaire mm Hg, 72 heures) jusqu'à obtention d'un poids constant. La quantification du Busulfan présent dans le solide poreux est effectué par comptage radioactif (³H-Busulfan), analyse thermogravimétrique (ATG) et analyse élémentaire.

Les solvants choisis pour l'imprégnation sont ceux dans lesquels le Busulfan possède une solubilité non négligeable (acétone, acetonitrile, chloroforme, dichlorométhane, diméthylcarbonate) (Tableau 18).

**Tableau 18. Solubilité du busulfan dans différents solvants**

| **Solvant** | **Solubilité Busulfan (mg/mL)** |
|---|---|
| Acetonitrile | 30 |
| Acétone | 20 |
| Dichloromethane | 10 |
| Chloroforme | 8 |
| Dimethylcarbonate | 13 |
| Ethylacetate | 6,6 |
| Tetrahydrofurane | -5 |
| Toluène | -4 |
| Eau | 0,1 |
| Ethanol | <<<4 |
| Méthanol | <<<4 |
| Hexane | <<<4 |
| Ether | <<<4 |

Les premiers essais d'introduction de busulfan dans les matériaux MIL-53, MIL-88A, MIL-89 et MIL-100 sont répertoriés dans le Tableau 19.

**Tableau 19. Solides hybrides utilisés pour les tests d'encapsulation du Busulfan**

| **MIL-n** | **Espaceur organique** | **Symétrie** | **Flexibilité** | **Taille des pores (nm)** | **Dp (nm)** |
|---|---|---|---|---|---|
| MIL-53 | Acide 1,4-BDC | *C2*/*c* | Oui | 0,86 | 6200 |
| MIL-88A | Acide fumarique | P-*62c* | Oui | 0,6 | 2600 |
| MIL-89 | Acide muconique | *Pbnn* | Oui | 0,9 | 1100 |
| MIL-100* | Acide 1,3,5-BTC | *Fd3m* | Non | 2,5-2,9 | 1700 |

| | | | | | |
|---|---|---|---|---|---|
| (*) Remarque : le solide MIL-100 comprend deux types de cages (25 et 29 Å) mais celles-ci sont accessibles par des fenetres microporeuses pentagonales ou hexagonales de dimensions 4,8*5,8 et 8,6 Å. | | | | | |

Les capacités de charge en Busulfan obtenues sont importantes, jusqu'à 122 et 153 mg/g solide hydraté contenant le médicament dans MIL-53 et MIL-100, respectivement (Tableau 10). Les solides de départ contiennent respectivement 7,3 et 44,5 % pds d'eau.

En considérant la teneur relative à celle du solide sec, la capacité de stockage dépasse le 25% en poids de principe actif, ce qui surpasse d'un facteur 60 celles obtenues avec des liposomes et d'un facteur 4 celles obtenues avec les meilleurs systèmes à base de polymères.

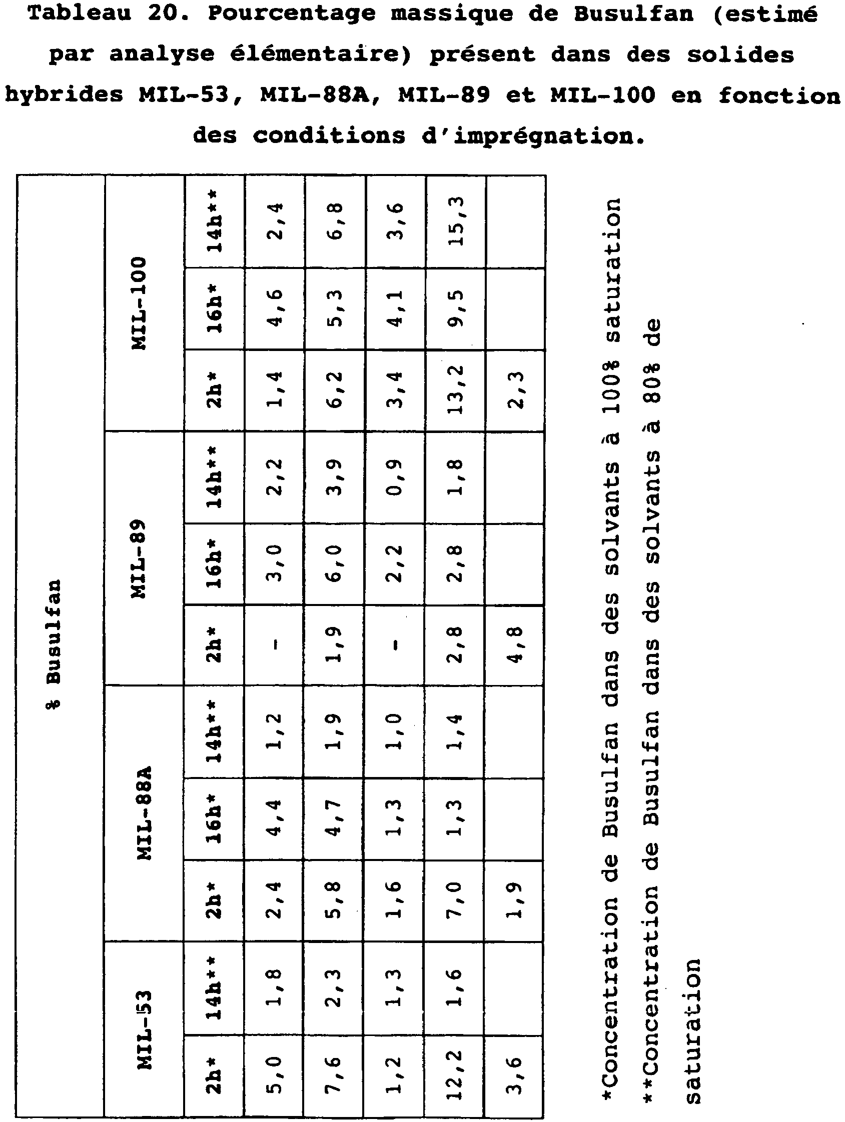

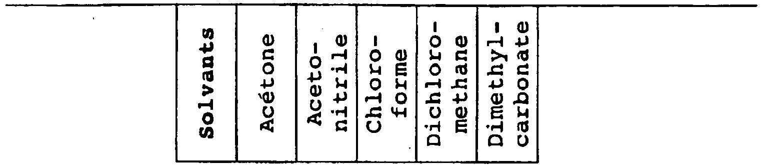

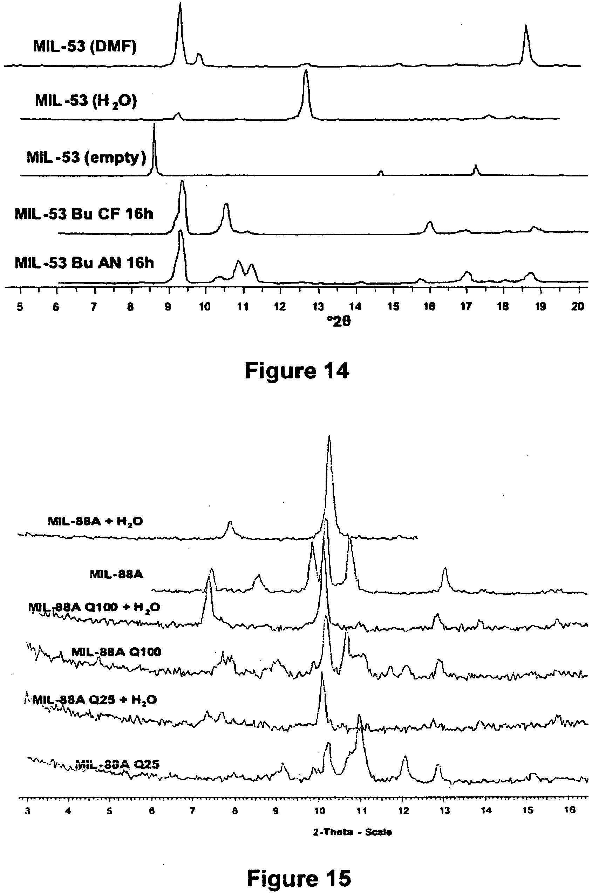

Ces résultats sont très encourageants et une optimisation des conditions d'imprégnation est en cours afin d'augmenter encore le taux d'encapsulation. Signalons également que la quantité adsorbée dans les solides hybrides flexibles type MIL-53 ou MIL-88, peut être suivie qualitativement par diffraction de rayon X (figure 14) puisque l'ouverture des pores du solide hybride dépend la teneur en principe actif dans les pores.

L'encapsulation du Busulfan peut être optimisée en testant :
- Plusieurs cycles d'imprégnation
- Différents temps d'imprégnation
- Encapsulation par sublimation
- Utilisation de plusieurs solides avec un plus grand volume poreux (MIL-101 biphenyl, MIL88D modifie avec différents groupes organiques) et de solides hybrides avec des ligands modifiés (NH2, Cl, NO2, COOH, CH3, etc) pour optimiser les interactions médicament-solide. A ce stade, la simulation numérique sera utilisée pour prédire la meilleure fonction pour retenir le Busulfan dans les pores.
- En fonction des résultats d'encapsulation, nous utiliserons des nanoparticules de solides modifiés en surface avec du PEG pour des tests d'encapsulation.

### Exemple 15 : Formulation de nanoparticules de carboxylate de fer (III) MIL-100 et MIL-101 chargées en principes pharmaceutiquement actifs : l'AZTP

Des tests d'encapsulation ont été réalisés avec d'autres médicaments, tels que le Cidofovir (CDV ; antiviral) ou l'Azidothymidine triphosphaté (AZTP ; rétroviral). Compte tenu des dimensions de ces molécules, les carboxylates de fer poreux à structure rigide MIL-100 et MIL-101 ont été choisis car ils possèdent des cages de 25-34 Å.

### a) Encapsulation et libération de l'AZTP

Etude préliminaire de l'encapsulation de l'AZTTP dans les nanoparticules de MIL-100 et MIL-101 : l'encapsulation de l'agent rétroviral Azidothymidine triphosphaté (AZTP) a été réalisé dans les carboxylates de fer poreux à structure rigide MIL-100 (nanoparticules 500 nm) et MIL-101 nanoparticules 500nm), qui possèdent des cages de dimensions libres entre 25 à 34 Å.

L'insertion du médicament a été réalisée par immersion de 2,5 mg des solides déshydratés (100°C/12h) dans des solutions - aqueuses contenant respectivement 50, 100, 250 et 500 µg AZTP en 500 µL et 50 µg/50 µL sous agitation pendant 1h. Après l'adsorption du médicament, le solide chargé de médicament est récupéré par centrifugation à 5000 rpm pendant 15 min et séché sous vide pendant 3 jours. La quantification du contenu de médicament adsorbé a été effectuée par comptage radioactif (³H-AZTP).

On observe que l'utilisation de solutions plus concentrées en médicament conduit à une augmentation de la charge en PA du matériau, jusqu'à un maximum de 9% en poids (un record !!) de médicament dans les nanoparticules de MIL-100. En considérant que le solide MIL-101 possède un volume poreux presque double (2 cm³/g vs 1.2 cm³/g) on s'attend avec ce dernier à des capacités beaucoup plus importantes. A concentration égales mais avec un rapport introduit initialement en poids AZTP/matériau plus élevé, la charge en principe actif augmente. En outre, l'efficacité de l'encapsulation est excellente.

L'encapsulation de l'AZTP peut être optimisée de la manière suivante :
- Augmentation de la concentration de la solution de départ en AZTP
- Plusieurs cycles d'imprégnation
- Différents temps d'imprégnation
- Utilisation de solides modifiés en surface avec PEG
- Utilisation de différents solides avec une plus grande capacité (MIL101 biphenyl, MIL88D modifie avec différents groupes organiques) et de solides hybrides avec des ligands modifiés (NH₂, Cl, NO₂, COOH, etc) pour optimiser les interactions médicament-solide

### b) Encapsulation de l'AZTTP dans le nanoparticules de MIL-100 PEGylées ou non

Les nanoparticules du MIL-100 ont été synthétisées par voie microondes (Microondes CEM) en partent d'une solution de 9,7 g de nitrate de fer hexahydrate (Aldrich, 97%), 3,38 g d'acide 1,3,5-benzenetricarboxylique (1,3,5-BTC, Aldrich, 99%) et 40 g d'eau distillée à 180°C pendant 30min (puissance 600W). La taille de particule mesurée par diffusion de lumière est de 400nm.

Les nanoparticules pegylées du MIL-100 ont été obtenus par la modification de surface des particules citées dans l'Exemple 24. 30 mg du MIL-100 ont été suspendus dans 3mL d'une solution aqueuse de 10mg du polyethyleneglycol aminoterminal (PEG-NH₂ 5000 g/mole, Aldrich, 97%) à 30°C pendant 3 heures sous agitation. Ces nanoparticules ont été récupèrées par centrifugation (10000 rpm/10 min) et lavées avec de l'eau distillée.

La quantité du PEG en surface a été déterminée par la méthode de Baleux et Champertier, basée dans la formation d'un complexe coloré par l'iodine-iodide sur le PEG, lequel est sélectivement mesuré par spectrophotométrie à 500nm). La quantité du PEG est de 19% en masse et la taille de particules après la PEGylation augmentée à 800nm). Par contre, l'observation de nanoparticules PEGylées et non PEGylées par microscopie électronique a balayage (SEM) montre des nanoparticules de 150nm dans les deux cas. Cette différence peut être dûe à des phénomènes d'agrégation particulaire.

L'adsorption de l'AZT-TP (Azidothymidinetriphosphate; 1-[(2R,4S,5S)-4-azido-5-(hydroxymethyl) tetrahydrofuran-2-yl]-5-méthylpyrimidine-2,4(1H,3H)-dione, Moravek) est étudiée avec les nanoparticules du MIL-100 PEGylées ou non, en utilisant l'AZT-TP marqué au tricium (3H-AZTP; moravek; 3,8Ci/mmol, 1mCi/ml, 133,4*µ*g/ml, 250*µ*l)

Les expériences sont faites en quadruple, en suspendant 2,5 mg du solide MIL-100 préalablement déshydraté (150°C/nuit) dans 500 µL d'une solution aqueuse de 1mg/mL AZT-TP (50*µ*l de 3H AZT-TP+ 3ml d'AZT-TP) à température ambiante sous agitation pendant 16h.

Les nanoparticules encapsulant l'AZT-TP sont récupèrées par centrifugation (10000 rpm/10 min, à température ambiante) et sèchées sous vide pendant 3 jours. La radioactivité dans le surnageant est déterminée par contage de la radioactivité (Beckman Coulter LS 6500 multi purpose scintillation counter) et l'AZT-TP adsorbée dans les matériaux est quantifiée par la différence avec la radioactivité de la solution mère.

Les nanoparticules sont dégradées dans des conditions acides (2,5 mg de nanoparticules sont dégradées dans 1ml de HCl 5M à 50°C pendant la nuit) et la radioactivité est déterminé.

La quantité d'AZT-TP adsorbé dans les matériaux est de 8% dans le MIL-100 et 5% en masse dans le MIL-100 PEGylé.

### c) Libération contrôlée de l'AZTTP depuis les nanoparticules du MIL-100 et MIL-100 PEGylées

La libération de l'AZT-TP est faite en suspendant 2,5mg de nanoparticules (2,5 mg de nanoparticules + 8wt% d'AZT-TP pour le MIL-100 et 5wt% pour le MIL-100 recouvert du PEG) à 37°C dans 1 mL de tampon phosphate pH 7,4 (Aldrich). Les suspensions sont maintenues sous agitation bidimensionnelle pendant les différents temps d'incubation (30min, 2,5h, 5h, 7,5h, 24h, 48h, 72h, 96h, 168h, 240h). Ensuite, les suspensions ont été centrifugées (10000 rpm, 10 min) et 0,5 mL de surnageant ont été prélevés et remplacés avec du PBS (Phosphate Buffered Saline) frais. La quantité d'AZT-TP libéré est déterminée par mesure de radioactivité dans les surnageants (milieu de libération).

Les nanoparticules non recouvertes de PEG libèrent leur contenu en principe actif de manière progressive pendant deux jours, plus lentement que celles recouvertes de PEG. Cela pourrait s'expliquer par une localisation différente de l'AZT-TP au sein des nanoparticules. Probablement, la « brosse » de PEG à la surface empêche de manière stérique le principe actif de pénétrer plus profondément dans les nanoparticules. Celui-ci, localisé dans les couches supérieures du matériau, est libéré plus rapidement.

### Exemple 16 : Formulation de nanoparticules de carboxylate de fer (III) chargées en principes pharmaceutiquement actifs : Cidofovir

### a) Encapsulation et libération du Cidofovir (CDV)

L'adsorption de Cidofovir (L (S)-1-(3-hydroxy-2-phosphonyl-methoxypropyl)cytosine, CDV, Moravek) a été etudiée dans les nanoparticules du MIL-88A, MIL-89, MIL-100 et MIL-101, en utilisant le CDV marqué au ¹⁴C (14C-CDV)

Les expériences sont faites en triple, en suspendant 2 mg du matériau préalablement déshydraté (150°C/nuit pour MIL_100 et pour le reste 100°C/nuit) dans 1 mL d'une solution aqueuse de 400*µ*g/ml CDV (50*µ*l de 14C-CDV+ 3ml de CDV) à température ambiante sous agitation pendant 16h.

Les nanoparticules encapsulant le CDV sont récupèrées par centrifugation (10000 rpm/10 min, température ambiante) et sèchées sous vide pendant 3 jours. La radioactivite dans le surnageant est déterminé par contage de la radioactivité (Beckman Coulter LS 6500 multi purpose scintillation counter) et le CDV adsorbée dans les matériaux est quantifié par la différence avec la radioactivité de la solution mère.

Les nanoparticules sont dégradés dans des conditions acides (2mg de nanoparticules sont dégradés dans 1ml de HCl 5M à 50°C pendant la nuit) et la radioactivité est déterminée.

La quantité d'AZT-TP adsorbé dans les matériaux est de 8% dans le MIL100 et 5% en masse dans le MIL-100 PEGylé.

**Tableau 21. Encapsulation de CDV dans les different nanoparticules de carboxylates de fer.**

| **Solide** | **% CDV** | **% efficacité** |
|---|---|---|
| MIL-88A | 2,6 | 12,2 |
| MIL-100 | 17,6 | 83,8 |
| MIL-101 | 2,8 | 14,0 |
| MIL-89 | 14,1 | 81,1 |

Les capacités, très importantes, varient entre 3 et 18%. Aussi les efficacités d'encapsulation sont très élevées (plus du 80% pour les solides MIL-100 et MIL-89).

### b) Libération de CDV par les nanoparticules de MIL-88A, MIL-100, MIL-101 et MIL-89

La libération du CDV a été faite en suspendant 2 mg de nanoparticules (2 g de nanoparticules + wt% du CDV) à 37°C dans 1 mL de tampon phosphate pH 7,4 (Aldrich). Les suspensions ont été maintenues sous agitation bidimensionnelle pendant les différents temps d'incubation. Ensuite, les suspensions ont été centrifugées (10000 rpm, 10 min) et 0,5 mL de surnageant ont été prélevés et remplacés avec du PBS frais. La quantité d'AZT-TP libéré a été déterminée par mesure de radioactivité dans les surnageants (milieu de libération).

L'encapsulation du Cidofovir peut être optimisée en jouant sur les paramètres suivants :
- Augmentation de la concentration de solution de départ
- Plusieurs cycles d'imprégnation
- Différents temps d'imprégnation
- Utilisation de différents solides avec une plus grande capacité (MIL101 biphenyl, MIL88D modifie avec différents groupes organiques) et de solides hybrides avec des ligands modifies (NH2, Cl, NO2, CH3, COOH, etc) pour optimiser les interactions médicament-solide
- En fonction des résultats d'encapsulation, nous utiliserons des nanoparticules de solides modifiés en surface avec du PEG pour des tests d'encapsulation.
- Test de libération seront réalisées dans un milieu physiologique (tampon phosphate, NaCl, etc)

### Exemple 17 : Encapsulation d'autres principes pharmaceutiquement actifs

### a) Formulation de carboxylates de fer (III) chargés en paclitaxel

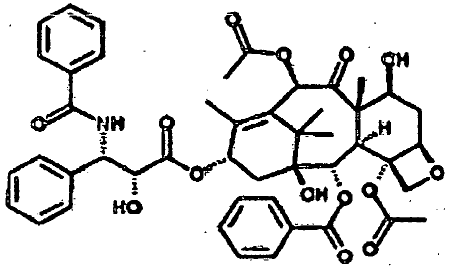

Le paclitaxel, commercialisé sous le nom de taxol, est soluble dans l'éthanol et dans le DMSO (environ 50 g/L).

Il peut être encapsulé par imprégnation des nanoparticules dans des solutions de DMSO de concentrations de 20 à 50 g/L, selon un protocole similaire à celui utilisé pour encapsuler le busulfan. La seule différence est que des solutions de paclitaxel dans du DMSO ou de l'éthanol sont utilisées.

### b) Formulation de carboxylates de fer (III) chargés en docétaxel

Le docétaxel est un analogue du paclitaxel, de structure et d'activité voisines, mais il en diffère surtout par sa toxicité et son efficacité antitumorale. Commercialisé sous le nom de taxotere (il s'agit d'une perfusion), ce principe actif présente des effets secondaires (risque de rétention hydrique, ascite, épanchements pleuraux ou péricardiques, réactions cutanées, alopécie...). Ainsi, son encapsulation dans des nanoparticules et sa libération au niveau d'une tumeur serait une avancée majeure.

L'encapsulation de docetaxel peut être réalisée en utilisant :
- Solutions aqueuses ou EtOH concentrées en docetaxel
- Différents temps d'imprégnation
- Plusieurs cycles d'imprégnation
- Utilisation de différents solides avec une plus grand capacité (MIL101 biphenyl, MIL88D modifie avec différents groupes organiques) et de solides hybrides avec des ligands modifies (CH3, COOH, etc) pour optimiser les interactions médicament-solide
- En fonction des résultats d'encapsulation, nous utiliserons des nanoparticules de solides modifiés en surface avec du PEG pour des tests d'encapsulation.

Le docetaxel peut être encapsulé également par imprégnation à partir de solutions de DMSO.

### c) Formulation de carboxylates de fer (III) chargés en gemcitabine

La gemcitabine (dFdC) est un analogue de la dé-oxy-cytidine. C'est un antimétabolite spécifique de la phase S du cycle cellulaire (phase de synthèse de l'ADN. La gemcitabine est métabolisée dans les cellules par des nucléosides kinases en nucléosides diphosphate (dFdCDP) et triphosphate (dFdCTP). Ceux-ci sont les métabolites actifs.

La gemcitabine est soluble dans l'eau. Ainsi, son encapsulation peut être réalisée selon un protocole identique à l'encapsulation de l'AZT triphosphate (imprégnation dans des solutions aqueuses de principe actif).

L'encapsulation de la Gemcitabine peut être optimisée en jouant sur les paramètres suivants :
- Augmentation de la concentration de solution de départ
- Plusieurs cycles d'imprégnation
- Différents temps d'imprégnation
- Utilisation de différents solides avec une plus grande capacité (MIL101 biphenyl, MIL88D modifie avec différents groupes organiques) et de solides hybrides avec des ligands modifies (NH2, Cl, NO2, CH3, COOH, etc) pour optimiser les interactions médicament-solide
- En fonction des résultats d'encapsulation, nous utiliserons des nanoparticules de solides modifiés en surface avec du PEG pour des tests d'encapsulation.
- Test de libération seront réalisées dans un milieu physiologique (tampon phosphate, NaCl, etc)

### Exemple 18 : Formulation de carboxylases de fer (III) chargés en composé d'intérêt en cosmétique

L'encapsulation de différentes molécules d'intérêt cosmétique est réalisé dans les carboxylates de fer poreux à structure rigide MIL-100 et à structure flexible MIL-53. Les molécules choisies pour les exemples sont l'acide ascorbique pour ses caractères antiradicaux libres, la caféine pour son activité liporégulateur, l'urée comme agent hydratant. Le procédé décrit pour l'encapsulation de la benzophénone est également applicable pour ces composés. Enfin, tout comme la benzophenone 3 (filtre UV), de caractère hydrophobe, la benzophenone 4 de caractère hydrophile peut également être encapsulée.

Pour l'insertion de cosmétiques, les solides déshydratés (100 ou 150°C/12h) ou non sont placés dans des suspensions aqueuses ou dans de l'alcool en présence de quantités variables de cosmétiques le tout sous agitation pendant des temps différents. Après adsorption, le solide chargé en cosmétiques est récupéré par centrifugation à 5000 rpm pendant 15 min et séché sous air. La quantification de la teneur en cosmétique adsorbé est effectuée par analyse élémentaire et ATG.

A des fins cosmétiques, les nanoparticules recouvertes d'acide hyaluronique et encapsulant des principes actifs sont particulièrement intéressantes. En effet, l'acide hyaluronique est un constituant naturel du derme et joue un rôle important dans l'hydratation et l'élasticité de la peau. Cette substance diminuant avec l'âge, notre peau se dessèche et se ride. Environ 56 % de l'acide hyaluronique contenu dans le corps se trouve dans la peau.

Recouvrir les MOFs par ce polymère pourrait leur conférer des propriétés bioadhésives pour la peau.

### a) Encapsulation de la benzophénone 3

La benzophénone 3 (2-Hydroxy-4-Methoxy Benzophenone) (BZ3) est un solide très peu soluble dans l'eau (0,0037 g/l (20°C)) :

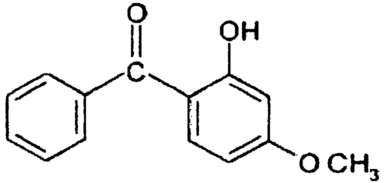

Il s'agit d'un filtre solaire anti-UV. La benzophénone-3 est utilisée dans les crèmes solaires et les produits cosmétiques comme substance « anti-vieillissement ». Elle est aussi utilisée comme agent protecteur des substances actives contenues dans les cosmétiques : elle permet d'éviter la dégradation par les UV de ces substances, comme les parfums, les colorants. Cette substance est un allergène reconnu, à très fort pouvoir allergisant. Elle peut être photosensibilisante. Son encapsulation serait utile afin d'éviter le contact direct avec la peau.

Le protocole expérimental utilisé est le suivant : les nanoparticules séchées (MIL 53(Fe), diamètre moyen 1,1 microns) ont été dispersées dans 10 mL de solution contenant 10 microgrammes de BZ3 par mL, afin d'obtenir des concentrations finales en particules égales à 1 et 0,5 mg/mL. La faible concentration en BZ3 choisie ici s'explique par sa faible solubilité dans l'eau. Le composé MIL-53(Fe) possède en outre une très bonne affinité pour les molécules aromatiques, ce qui justifie le choix de ce matériau pour encapsuler la Benzophénone.

Cette solution de BZ3 a été obtenue à partir d'une solution de BZ3 dans du DMSO (1 g/L). Un mL de cette solution a été prélevé puis introduit dans 100 mL eau MilliQ.

Les nanoparticules ont été incubées pendant 12h à température ambiante, puis récupérées par ultracentrifugation (30000 rpm, 30 min). Le surnageant a été prélevé puis dosé (spectrophotométrie UV, longueur d'onde 298 nm), permettant ainsi de déterminer la quantité non encapsulée. La quantité encapsulée a été déterminée par différence avec la quantité de BZ3 introduite initialement. Les expériences ont été réalisées en triplicate.

**Tableau 22. Taux de charge obtenus**

| **Concentration en particules (mg/mL)** | **Rendement d'encapsulation (%mass)** | **Charge (%mass)** |
|---|---|---|
| 1 | 76 ± 3 | 0,76 |
| 0,5 | 74 ± 3 | 1,49 |

Les rendements d'encapsulation (% encapsulé par rapport à la quantité de BZ3 introduite) sont satisfaisants (74-76%) En revanche, les charges faibles s'expliquent par la faible quantité de BZ3 introduite comparativement à la quantité de particules. Nous constatons néanmoins que la charge augmente lorsque la concentration introduite en particules (par rapport au BZ3) diminue. Par conséquent, sachant que la solution aqueuse de BZ3 utilisée était peu concentrée, il est tout à fait envisageable d'améliorer considérablement la charge des particules en BZ3 en imprégnant celles-ci dans un solvant organique approprié ou la solubilité du BZ3 sera nettement supérieure.

### b) Encapsulation de la benzophénone 4

La benzophénone 4 (acide 2-Hydroxy-4-methoxybenzophenone-5-sulphonique) (BZ4) est un solide très soluble dans l'eau (100 mg/mL (20°C)) :

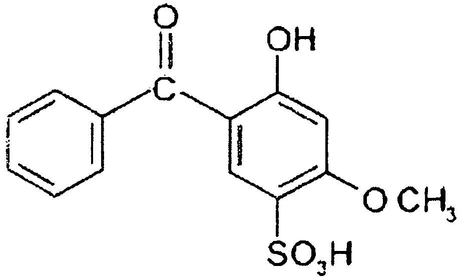

Il s'agit d'un filtre solaire anti-UV A et B, particulièrement employé quand une formulation hydrosoluble est exigée. La benzophénone-4 est utilisée dans les crèmes solaires et les produits cosmétiques comme substance « anti-vieillissement ». Elle est aussi utilisée comme agent protecteur des substances actives contenues dans les cosmétiques : elle permet d'éviter la dégradation par les UV de ces substances, comme les parfums, les colorants. Toutefois, elle peut entraîner des réactions immunitaires, sous la forme de démangeaisons, sensation de brulure, desquamation, urticaire, et des boursouflures de la peau, ou une réaction respiratoire sévère. Son encapsulation permettrait de maintenir son activité tout en évitant un contact direct avec la peau.

### c) Encapsulation de l'urée

L'urée est une substance active d'origine naturelle, qui se retrouve dans tous les organes, tissus et liquides du corps humain. Elle possède une très importante solubilité dans l'eau (1,08 g/mL (20°C)).

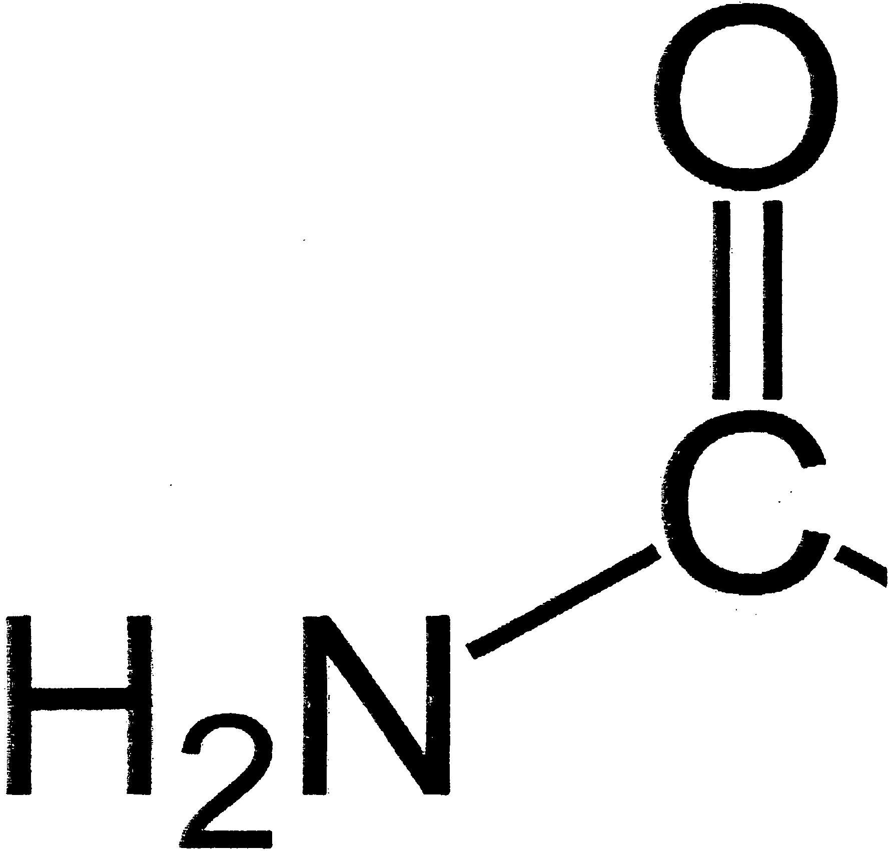

C'est un agent hydratant important de la couche cornée. Elle possède différents effets :
- Elle apaise les démangeaisons, avantage majeur dans le cas de la neurodermite infantile.
- Elle hydrate la couche cornée.
- Elle a un effet desquamant et elle normalise la division cellulaire.
- Elle possède des propriétés anti-bactériennes, empêchant les surinfections, en particulier dans l'eczéma chronique.
- Elle favorise la pénétration d'autres substances médicamenteuses appliquées simultanément sur la peau, comme par exemple les glucocorticoïdes. Ainsi, on peut réduire le dosage des médicaments sans diminuer leur efficacité et en limiter ainsi les effets secondaires.

### d) Encapsulation de la caféine

La caféine agent lipolytique reconnu pour ses propriétés amincissantes. Son action lipo-réductrice est puissante et dose-dépendante. La caféine est la forme la plus active car elle est directement assimilable par la cellule. Mais des sels de caféine sont les plus utilisés en pratique car elles sont plus facilement intégrables dans une crème.

Elle possède une bonne solubilité dans l'eau (22 mg/mL (20°C)).

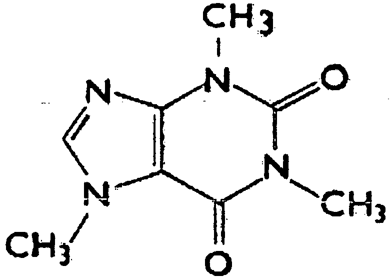

### e) Protocoles expérimentaux d'encapsulation

150 mg de nanoparticules séchées de MIL-53(Fe) (diamètre moyen 1,1 microns ; déshydraté à 150°C/8 heures) et de MIL 100(Fe) (diamètre moyen 0,5 microns; déshydraté à 100°C/8 heures) ont été dispersées dans 10 mL de solutions aqueuses contenant différents agents cosmétiques (concentrations proches de la saturation ; voir tableau ci-dessous), le tout sous agitation pendant 2 heures ou 3 jours à température ambiante. Les particules sont ensuite récupérées par centrifugation (20ºC, 5000 rpm, 15 min).

Les solides chargés en cosmétiques sont d'abord caractérisés par Diffraction des Rayons X sur poudre (DRX). La teneur massique en cosmétique encapsulé dans le solide poreux est estimée par analyse thermogravimétrique (ATG) et analyse élémentaire.

**Tableau 23. Quantification du cosmétique dans les solides MIL-53 et MIL-100 par ATG (Figure 4 et 5) et analyse élémentaire**

| Matériau | Cosmétique | Concentration (mg/mL) | Temps imprégnation (h) | |
|---|---|---|---|---|
| | | | | ATG |
| MIL-100 | C | 22 | 72 | 24,2 |
| | U | 500 | 72 | 69,2 |
| | BZ4 | 10 | 72 | 13,7 |
| | BZ4 | 10 | 2 | 15,2 |
| MIL-53 | C | 22 | 72 | 23,1 |
| | U | 500 | 72 | 63,5 |
| | BZ4 | 10 | 72 | 5,0 |
| | BZ4 | 10 | 2 | -------- |
| | BZ4 (EtOH) | 10 | 2 | 1,0 |
| MIL-88 | C | 22 | 72 | 43,6 |
| MIL-53 2COOH | C | 22 | 72 | 9,0 |
| MIL-53 20H | C | 22 | 72 | 30,2 |
| MIL-53 NH2 | C | 22 | 72 | 25,4 |

| | | | | |
|---|---|---|---|---|
| (C : Caféine ; U : urée ; BZ4 : Benzophénone n°4) | | | | |

La concentration des solutions de départ est très proche de la saturation pour forcer l'insertion des cosmétiques dans les pores et éviter déplacer l'équilibre vers une libération en phase liquide (Tableau ci-dessus).

La capacité d'encapsulation de cosmétiques est dans tous les cas très importante, jusqu'à 60-70% pour l'urée, une petite molécule très polaire. Pour la caféine, molécule de plus grande taille, nous observons une insertion de cosmétique un peu moins élevée, autour de 25-40% en masse. Cette molécule peut interagir avec les parties polaires (métal) et apolaires (ligand) des solides hybrides.

La benzophenone 4 est finalement bien encapsulée dans le MIL-100 (jusqu'à 15%) mais elle montre une d'insertion beaucoup plus faible dans le solide MIL-53 (<5%) en accord avec des dimensions proches de la taille maximale des pores de ce composé.

La conservation de la structure cristalline ordonnée est vérifié dans touts les solides de structure rigide après l'encapsulation par DRX.

La libération de la caféine depuis les solides avec différent structure (MIL53, MIL88 et MIL100), et dans les solides à base de ligands modifiés MIL53_2COOH, MIL53_20H et MIL53_NH2 a été réalisé par la suspension de 50mg de matériaux contenant la caféine dans 5mL d'une solution de tampon phosphate saline PBS (Aldrich) pH=7.4 a 37°C sous agitation.

Ces suspension sont centrifuges (10000 rpm 10 min a 20°C) et 1mL du surnageant prélevé et remplace par de PBS frais dans le différents temps de libération, La concentration de caféine libérée au milieu a été déterminée par spectroscopie UV-Vis à 254 nm.

On peut observer que c'est possible module la dose administre pendant le temps en fonction de la structure et la composition du matériau.

### Exemple 19 : Encapsulation de molécules fluorescentes

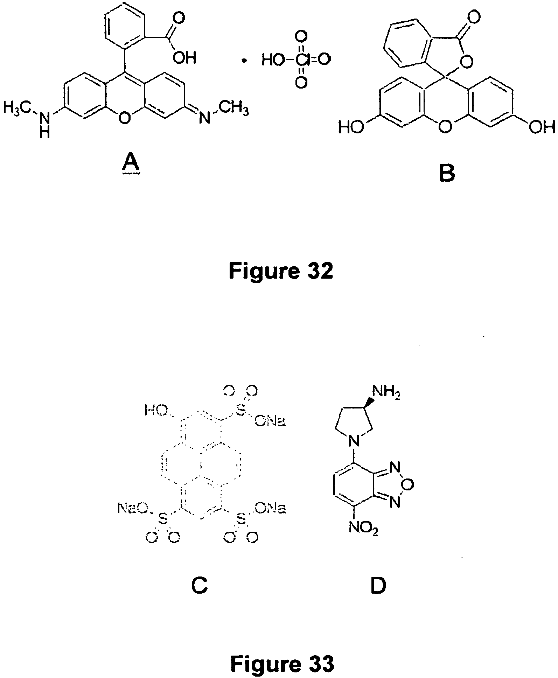

Les molécules fluorescentes telles que le perchlorate de Rhodamine (A), la fluorescéine (B), le sel de sodium de l'acide 8-hydroxypyrene-1,3,6-trisulfonique (C) ou le (R)-(-)-4-(3-aminopyrrolidino)-7-nitrobenzofurazan (D) ont pû être encapsulées dans le solide MIL-101-NH₂ suivante le protocole décrit ci-dessous. Ces molécules sont représentées en figure 32 et 33.

### Protocole opératoire :

200 mg de solide aminotéréphthalate de fer rigide MIL-101-NH₂ (synthétisé selon la méthode microondes décrite dans l'Exemple 7) sont suspendus dans 10 mL d'une solution de 2 mg/mL de molécule fluorescente dans l'éthanol :
- A : rhodamine 116 perchlorate (R116, Aldrich),
- B : fluorescéine (Aldrich),
- C : sel de trisodium de l'acide 8-hydroxypyrene-1,3,6-trisulfonique (PSO3, Aldrich, 98%), ou
- D : (*R*)-(-)-4-(3-aminopyrroldino)-7-nitrobenzofurazan (APNF, Aldrich, 98%).

Le mélange de solide en solution de molécule fluorescente est agité à température ambiante sous agitation pendant 15 heures. Le solide chargé en molécule fluorescente est récupéré par centrifugation à 10000 rpm/10 min.

La quantification des molécules fluorescentes encapsulées est réalisée par ATG et/ou analyse élémentaire. Les matériaux encapsulant les molécules fluorescentes sont caractérisés par DRX pour vérifier la conservation de la structure cristalline, par FTIR pour étudier les interactions matrice-molécule et par microscopie confocale de fluorescence pour déterminer la présence de fluorescence dans les pores ou en surface (les propriétés de fluorescence de chacune de ces molécules sont présentée dans le tableau suivant).

**Tableau 24. Propriétés de fluorescence**

| **Molécule fluorescente** | **λ excitation (nm)** | **λ émission (nm)** | **solvant** |
|---|---|---|---|
| A | 516 | 540 | éthanol |
| (Rhodamine 116) | | | |
| B | 490 | 514 | 0,1M Tris pH 8,0 |
| Fluoresceine | | | |
| C | 460 | 510 | 0,1M Tris pH 8,0 |
| (PS03) | | | |
| D | 490 | 535 | acetonitrile |
| (APNF) | | | |

Les propriétés en imagerie *in vivo* (bioluminescence, fluorescence) peuvent être étudiées selon des protocoles connus de l'homme du métier. On pourra se référer par exemple à la publication de Kathryn E. Luker et al., Antiviral Research, Volume 78, Issue 3, June 2008, Pages 179-187.

En outre, le même protocole est applicable pour l'encapsulation des molécules A, B, C et D dans le cas du solide MOF diméthyl 4,4'-biphényl dicarboxylate de fer (synthétisé selon la méthode solvothermale décrite dans l'Exemple 3).

### Exemple 20 : Encapsulation de molécules fluorées :

### 1-(Pentafluoropropionyl)imidazole :

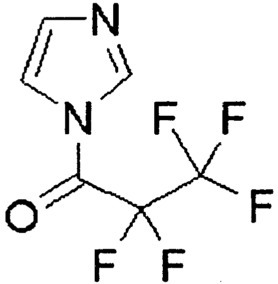

200 mg du trimesate de fer rigide MIL-100 (synthétisé par la méthode solvothermale décrite précédemment) sont préalablement déshydratés à 150°C pendant une nuit et sont suspendus dans 10mL d'une solution de 2 mg/mL d'acide perfluoropentanoique (Aldrich, 97%) ou de 1-(Pentafluoropropionyl)imidazole (Aldrich, 98%) dans l'éthanol sous agitation à température ambiante pendant 15 heures. Le solide est récupéré par centrifugation à 10000 rpm/10 min.

La quantification des molécules fluorées encapsulées est réalisée par ATG et/ou analyse élémentaire. Les matériaux encapsulant les molécules fluorescentes sont caractérisés par DRX pour vérifier la conservation de la structure cristalline, par FTIR et 19F-RMN pour étudier les interactions matrice-molécule.

### Exemple 21 : Encapsulation de l'urée par sublimation

Ce protocole est notamment applicable pour des molécules à encapsuler de faible température d'évaporation, comme l'urée, permettant une plus simple sublimation.

Ici, la dimension réduite de la molécule urée permet son encapsulation dans les pores des solides flexible MIL-53 et rigide MIL-100 (synthèses décrits antérieurement).

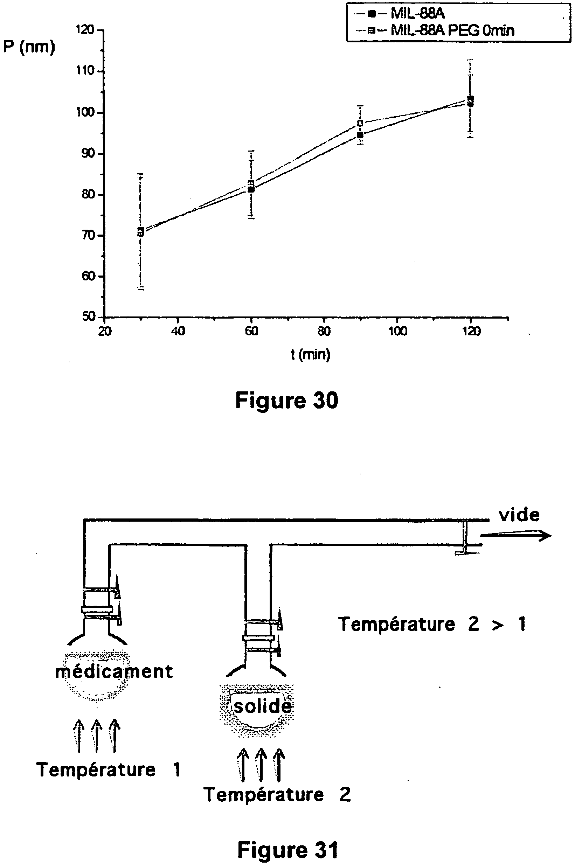

Le montage expérimental utilisé pour insérer par sublimation le PA, est présenté en figure 31.

Le protocole d'encapsulation par sublimation est décrit dans les étapes suivantes :
1. Le solide poreux est préalablement déshydraté à 150°C sous vide pendant 12h (la valve du ballon contenant le médicament est fermée et la valve du ballon contenant le solide est ouverte vers le vide).
2. L'urée est chauffée sous vide pour la sublimer à la température T1 de sublimation sous vide de l'urée. Le solide MOF est quant à lui chauffé à la température T2, de 5°C supérieure à T1. Tout le circuit est chauffé également pour éviter la recristallisation de l'urée (la valve du ballon « medicament » est ouverte vers le vide et la valve du « solide » est fermée).
3. La valve du « solide » est ensuite ouverte pour mettre en contact le médicament sublimé et le solide déshydraté afin de réaliser l'encapsulation du médicament dans le matériau.
5. La valve du ballon contenant le « solide » est ensuite fermée.
6. De l'azote gazeux est introduit dans le ballon du solide.
7. Le solide encapsulant l'urée est ensuite récupéré.

### VI. NANOPARTICULES A SURFACE MODIFIEE

### Exemple 22 : Formulation de carboxylates de fer (III) à surface modifiée par le chitosane

La modification de surface des nanoparticules avec le chitosane permet d'envisager différentes voies d'administration des nanoparticules grâce aux propriétés de bio-adhésion spécifiques à ce polymère.

Dans cet exemple la modification superficielle est réalisée pendant la synthèse du matériau MIL-88A.

### a) Préparation des nanoparticules à surface modifiée

A une solution de FeCl₃.6H₂O (1 mmol, 270 mg; Alfa Aesar, 98%) et d'acide fumarique (1 mmol, 116 mg; Acros, 99%) dans 5 mL d'eau distillée, dans un bombe en téflon de 23 mL, ont été ajoutés 7 mg de l'agent modifiant de surface, le chitosane modifié. Deux types de chitosane modifiés avec des chaînes alkyle (C12, lauryle) ont été utilisés ; l'un avec un modification de 2% de chaînes alkyles (Q25) et l'autre modifie avec 7% (Q100).

Pour la dissolution complète du chitosane, la solution est placée sous agitation pendant 45 minutes.

La bombe en téflon est placée dans un corps métallique fermé hermétiquement et chauffé dans un étuve à 80ºC pendant 12 heures.

Le solide obtenu est récupéré par centrifugation à 5000 rpm pendant 10 minutes et lavé avec de l'eau distillée et l'acétone.

### b) Analyse et caractérisation

La taille de particules obtenues est mesurée avec un appareil potentiel Z Malvern Zetasizer Nano serie - Nano-ZS; model Zen 3600 ; serial N° 500180 ; UK, en observant une taille de 2,64 et 0,91 microns pour MIL88A-Q 25 et MIL88A-Q 100, respectivement.

Les diagrammes de diffraction de rayon X (DRX) sont collectés avec un diffractomètre Siemens D5000 X'Pert MDP (λ_{Cu} Kα₁, Kα₂) de 3 à 20º (2θ) avec un taille de pas de 0,04º et 2 s par pas.

Les diagrammes de DRX présentés en figure 15 ont permi de vérifier que la phase obtenue est bien le MIL-88A. La flexibilité du matériau est également vérifiée par DRX en ajoutant une goutte d'eau sur le solide.

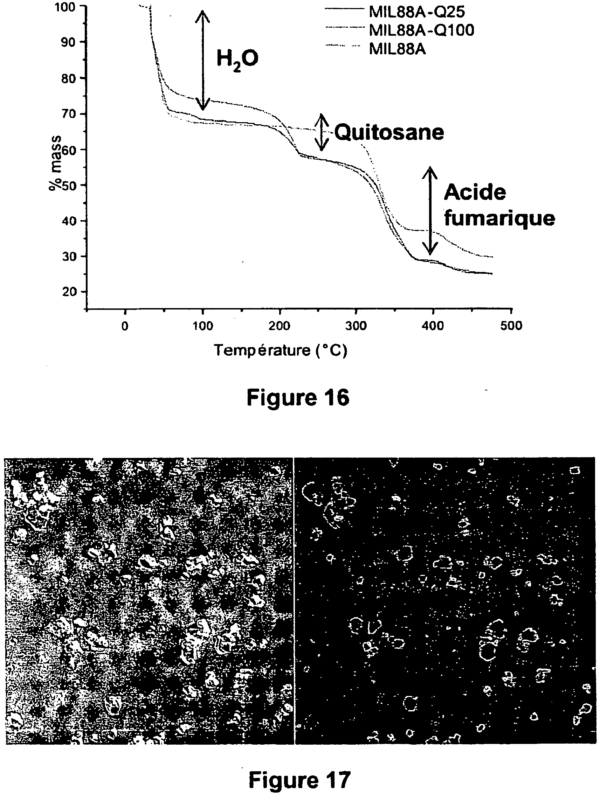

La quantité de chitosane incorporée au matériau est estimée par analyse thermogravimétrique (ATG) présentée en figure 16. L'appareil utilisé est un appareil TGA2050 TA de 25 à 500°C avec un rampe de chauffage de 2°C/min sous flux d'oxygène (100mL/min). Dans les matériaux, la quantité d'acide fumarique est bien autour du 45% (par rapport au produit déshydraté). Les matériaux MIL88A-Q25 et MIL-88A-Q100 contiennent une quantité de chitosane de l'ordre de 16% et de 22% (pds) par rapport au produit déshydraté, respectivement.

### Exemple 23 : Formulation de carboxylates de fer (III) à surface modifiée par le dextrane fluoresceine biotin

Dans cet exemple, le dextrane utilisé est greffé d'une part avec la fluorescéine et d'autre part avec de la biotine (Dex B FITC 10000 g/mol, anionique, lysine fixable, Molecular Probes, catalog D7178).

Les caractéristiques du dextrane sont les suivantes : Dextrane fluorescein and biotin, poids moléculaire 10 000 g/mole, anionique, capable de fixer la lysine (« mini-emerald ») lot 36031A, D7178, « Molecular probes », Technologie de détection in vitro, 1 mole fluorescéine/mole, 2 moles biotine/mole.

### a) Préparation des nanoparticules à surface modifiée

Les particules de 1,3,5-benzenetricarboxylate de fer MIL-100 (diamètre de particule 1,79 microns) ont été lavées avec de l'eau MilliQ.

Cinq milligrammes de particules ont été dispersées dans 0,5 mL eau MilliQ. Nous avons rajouté à cette suspension 0,5 mL de solution aqueuse de Dex B FITC (5 mg/mL). Elles ont été incubées a température ambiante pendant 24 h, puis récupérées par centrifugation (3800 rpm, 10 minutes). Le surnageant a été prélevé, puis le culot (particules) a été resuspendu dans 0,5 ml d'eau MilliQ. Après une nouvelle centrifugation, les particules ainsi lavées du Dex B FITC en excès ont été placées sur une lamelle afin d'être observées dans le microscope confocal (excitation 488 nm, émission 515 nm).

### b) Analyse et caractérisation

La fluorescéine permet la détection des particules à l'aide d'un microscope confocal à balayage laser, alors que la biotine, hydrophobe, permet :
- l'ancrage dans le coeur des particules
- la fonctionnalisation avec des ligands biotinylés.

La figure 17 présente les coupes optiques ainsi obtenues. On distingue une auréole autour des particules, indiquant la présence de dextrane (seul composé fluorescent) uniquement à la surface. En effet, les longues chaînes de polymère n'ont pas pu pénétrer au coeur des particules.

Cette méthode de modification de surface présente l'avantage de ne pas perturber le coeur des particules (contenant des principes actifs) et de se faire post-synthèse, donc d'offrir une variété de recouvrements possibles.

### Exemple 24 : Formulation de carboxylates de fer (III) à surface modifiée par le polyéthylène glycol (PEG)

Pour réduire au minimum la toxicité du Busulfan dans le foie, il faut empêcher les nanoparticules d'être dirigées vers le foie ; la meilleure méthode consiste à greffer en surface des nanoparticules hybrides des chaînes hydrophiles de type poly(ethylène glycol) (PEG), afin de diminuer leur accumulation dans cet organe. Nous envisageons une étude complète de la dégradation *in vitro* des particules recouvertes ou non de PEG, dans des milieux différents.

Les chaînes du PEG pourront avoir différent groupes terminaux pour se greffer à la surface des matériaux. Ainsi, l'interaction du PEG avec la surface de particule peut être modifié en utilisant differents types de PEG.
- PEG-NH₂ (alpha-t-Butyloxycarbonylamino-omega-amino poly(ethylenglycol) (PEG; Boc-NH-PEG-NH2, 5000 MW, Iris Biotech)
- PEG-COOH (poly(ethylenglycol) carboxylic acid, Iris Biotech)
- PEG-PO₄ synthétisé au laboratoire selon le procédé suivant :

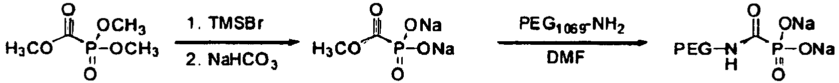

Le groupe phosphonate est attaché a le PEG-NH₂ voie une condensation amide avec un ester lie a un groupe phosphonate. Le sel de sodium du phosphonate a été utilisé. Ensuite, le couplage a ete réalisé a partir du trimethyl-phosphonoformate[CAS 31142-23-1] selon la procédure Robert A. Moss, Hugo Morales-Rojas, Saketh Vijayaraghavan and Jingzhi Tian, *J. Am*. *Chem. Soc.,* **2004,** *126*, (35), 10923 - 10936.

Une dissolution du PEG-NH2 (87.6 mg, M = 5400, Iris Biotech GmbH, PEG1069) dans 2 mL de DMF (Fluka, 97%) avec un excès de disodiummethylphosphonoformate (50 mg, M = 183,99) a été chauffé a 100°C pendant 15h sous agitation. Apres, le solvant est éliminé sous vide et le résidu suspendu dans éthanol absolu. L'excès de phosphonoformate est insoluble, donc il peut être éliminé par filtration. Le filtre es concentre pour obtenir le produit (85 mg). NMR ³¹P, (D₂O), d = 1,3 ppm.

### La PEGylation pourra se réalisé pendant la synthèse ou postsynthese:

### a) Modification superficielle avec du PEG-COOH pendant la synthèse

Les synthèses des MOFs se font directement en présence de monométhoxy PEG monoacide (MeO-PEG-COOH) (Sigma, masse molaire 5000 g/mole) : CH₃-O-(CH₂-CH₂-O)ₙ-CH₂-CH₂-COOH.

Le monométhoxy PEG monoacide est introduit à 3 ; 8,5 ou 13 % par rapport au poids total de solide utilisé en synthèse.

### Procédé de préparation :

L'acétate de fer (1 mmol; synthétisé selon la synthèse A décrite dans l'exemple 1) et l'acide muconique (1 mmol; Fluka 97%) sont mélangés dans 10 mL de méthanol (Aldrich, 99,9%). Le tout est introduit dans un corps en téflon de 23 mL. Le monoacide PEG est ensuite introduit à hauteur de 3 ; 8,5 ou 13 % en masse par rapport au poids total de solide. 0,35 mL de soude 2 M est éventuellement ajoutée. La solution est placée sous agitation pendant 20 minutes.

La bombe en téflon est placée dans un corps métallique fermé hermétiquement et chauffé dans une étuve à 100°C pendant 12 heures.

Le solide obtenu est récupéré par centrifugation à 5000 rpm pendant 10 minutes et lavé avec de l'eau distillée et l'acétone.

Le dosage du PEG dans les carboxylates de fer est effectué comme suit : les particules sont dégradées totalement en milieu acide (HCl 5M) afin de libérer le PEG associé. Après neutralisation des solutions obtenues (à pH=7) et destruction des nanoparticules avec de la soude, le dosage du PEG a été effectué par spectrophotométrie UV (à la longueur d'onde de 500 nm), d'après la méthode décrite dans B. Baleux et al. C.R. Acad. Sciences Paris, série C, 274 (1972) pages 1617-1620 [33]. Les principaux résultats sont répertoriés dans le tableau suivant.

**Tableau 25. Modification du matériau MIL88A avec le PEG 5000 g/mole.**

| **Ajout de solution aqueuse de NaOH** | **% massique de PEG introduit en début de synthèse** | **% massique de PEG dans la composition des nanoparticules** | **Diamètre des nanoparticules (nm) (mesuré par diffusion de lumière)** |
|---|---|---|---|
| - | 3 | 3,8 | 570 |
| oui | 3 | 4,8 | 230 |
| - | 8,5 | 13,4 | 590 |
| oui | 8,5 | 13 | 230 |
| - | 13 | 18,5 | 565 |
| oui | 13 | 18 | 310 |

Nous pouvons constater que :
- l'ajout de soude permet de diminuer la taille des nanoparticules
- le % massique de PEG dans les nanoparticules est supérieur au % de PEG introduit en début de synthèse
- de manière remarquable, il est possible d'obtenir des particules d'environ 230 nm contenant 13 % en poids de PEG, ce qui est intéressant pour des applications médicales (« furtivité »)

En effet, les nanoparticules « furtives » décrites dans la littérature contiennent généralement moins de 10 % mass de PEG, comme décrit dans R. Gref et al. Colloids and Surfaces B: Biointerfaces, Volume 18, Issues 3-4, October 2000, pages 301-313 [34].

### b) Modification superficielle des nanoparticules MIL-100 avec du PEG Par la méthode post-synthèse

Les nanoparticules du MIL-100 sont synthétisées par voie microondes (Microondes CEM) en partant d'une solution de 9,7 g nitrate de fer hexahydrate (Aldrich, 97%), 3,38g d'acide 1,3,5-benzenetricarboxylique (1,3,5-BTC, Aldrich, 99%) et 40 g d'eau distillée à 180°C pendant 30min (puissance 600W). La taille de particule mesurée par diffusion de lumière est de 400nm.

Les nanoparticules pegylées du MIL-100 sont obtenues par la modification de surface des particules citées antérieurement. 30 mg du MIL-100 sont suspendus dans 3mL d'une solution aqueuse de 10mg du polyethyleneglycol aminoteminal (PEG-NH2 5000 g/mole, Aldrich, 97%) à 30°C pendant 3 heures sous agitation. Ces nanoparticules sont récupèrées par centrifugation (10000 rpm/10 min) et lavées avec de l'eau distillée.

La quantité du PEG en surface est déterminée par la méthode de Baleux et Champertier, basée dans la formation d'un complexe coloré par l'iodine-iodide sur le PEG, lequel est sélectivement mesuré par spectrophotométrie à 500nm). La quantité du PEG est de 19% en masse et la taille de particules après la PEGylation augmente à 800nm). Par contre, l'observation de nanoparticules PEGylées et non PEGylées par microscopie électronique à balayage (SEM) montre de nanoparticules de 150nm dans les deux cas. Cette différence est peut être dûe a des phénomènes d'agrégation particulaires.

### Exemple 25 : Synthèse par voie ultrasons de carboxylates de fer (III) à surface modifiée par le polyéthylène glycol (PEG)

La synthèse par voie ultrasons de nanoparticules de solide MIL-88A à surface modifiée par le PEG est basée sur le protocole opératoire de l'Exemple 8 et a été réalisée à différents temps de reaction (30, 60, 90 et 120 minutes).

Dans les exemples qui suivent deux modes opératoire ont été effectués :
a) dans le 1^{er} mode opératoire, le PEG est ajouté 15 minutes avant la fin de la synthèse
b) dans le second mode opératoire, le PEG est ajouté dès le début de la synthèse (t = 0 min).

Pour chacune des synthèses ci-dessous, des solutions aqueuses de chlorure de fer III (2,7 mg/ml) et d'acide fumarique (1,16 mg/ml) sont préparées comme décrit dans l'Exemple 8 (Tableau 10).

### a) Synthèse n° 1 :

Dans chacune des 8 fioles de 20 mL sont ajoutés 5 mL de solution de chlorure de fer III (2,7 mg/ml) et 5 mL de solution d'acide fumarique (1,16 mg/ml) :
- 4 fioles servent de témoin où les réactions sont réalisées pour les 4 temps de synthèse : 30, 60, 90 et 120 min,
- dans les 4 autres fioles, 5 mg de PEG sont ajoutés 15 min avant la fin de chacune des synthèses de durée 30, 60, 90 et 120 min (la fin d'une synthèse correspond à la sortie du bain à ultrasons).

Les 8 fioles sont placés en même temps dans un bain de sonication à 0°C, pendant les durées t correspondantes (30, 60, 90 et 120 min).

Après la synthèse, un volume de 0,1 ml de solution est prélevé dans chaque fiole afin de déterminer la taille des particules par diffusion de lumière grâce à un appareil de Dynamic Light Scattering (DLS, Nanosizer). Le reste de solution est ensuite passé à la centrifugeuse à 10 000 rpm à 0°C pendant 15 min afin de séparer le surnageant du solide formé. Le surnageant est éliminé à l'aide d'une pipette pasteur et le culot récupéré est placé sous la hotte à température ambiante.

L'évolution de la taille de particule (P en nm) en fonction du temps (t en min) est représenté dans la figure 29. La présence du PEG 15 min avant la fin de la synthèse produit une augmentation de la taille de particule d'environ 5 nm, ce qui peut être dû à l'épaisseur de la couche de PEG (5000 Da).

### b) Synthèse n° 2 :

Dans chacune des 8 fioles de 20 mL sont ajoutés 5 mL de solution de chlorure de fer III (2,7 mg/ml) et 5 mL de solution d'acide fumarique (1,16 mg/ml) :
- 4 fioles servent de témoin où les réactions sont réalisées pour les 4 temps de synthèse : 30, 60, 90 et 120 min,
- dans les 4 autres fioles, 5 mg de PEG sont ajoutés dès le début de chacune des synthèses de durée 30, 60, 90 et 120 min.

Les 8 fioles sont placés en même temps dans un bain de sonication à 0°C, pendant les durées t correspondantes (30, 60, 90 et 120 min).

Après la synthèse, un volume de 0,1 ml de solution est prélevé dans chaque fiole afin de déterminer la taille des particules par diffusion de lumière grâce à un appareil de Dynamic Light Scattering (DLS, Nanosizer). Le reste de solution est ensuite passé à la centrifugeuse à 10 000 rpm à 0°C pendant 15 min afin de séparer le surnageant du solide formé. Le surnageant est éliminé à l'aide d'une pipette pasteur et le culot récupéré est placé sous la hotte à température ambiante.

L'évolution de la taille de particule (P en nm) en fonction du temps (t en min) est représenté dans la figure 30. Celle-ci montre qu'il n'y a pas de variation significative après l'ajoute du PEG au temps initial de synthèse.

Que ce soit en presence ou non de PEG au temps initial de la synthese, il est possible d'observer en DRX un epaulement à 11°, caracteristique de la phase MIL-88A qui semble augmenter en intensité avec le temps de synthese.

### c) Conclusion de l'étude:

Le but de cette étude était d'optimiser la taille des particules qui doivent être inférieures à 200 nm afin de pouvoir les rendre compatible avec une administration par voie intraveineuse. Les résultats obtenus sont satisfaisants puisque les diamètres de particules obtenus sont inférieurs à 200 nm (avec vérification des structures cristallines type MIL-88A dans la plupart de solides). De plus, même si les rendements sont inférieurs à ceux obtenus par voie solvothermale ou voie microondes, on peut les considérer comme acceptables (tableau ci-dessous).

**Tableau 26. Rendements de synthèse par voie ultrasons**

| **Temps (min)** | **Rendement (%)** | | | |
|---|---|---|---|---|
| | **Témoin** | **AcH** | **PEG t=0** | **PEG tf-15min** |
| **30** | 24 | 13,4 | 31,4 | 20,1 |
| **60** | 27,2 | 15 | 29,4 | Non mesuré |
| **90** | 35,6 | 14 | 24 | 28,3 |
| **120** | 35,1 | 19,1 | 32 | 41,2 |

Il est possible d'observer que la taille des particules augmente en fonction du temps de synthèse.

De même la pegylation à t=0 min abouti à des diamètres de particules plus petits que la pegylation à t=f-15min, due probablement au fait que la croissance cristalline soit arrêtée plus tôt.

### Exemple 26 : Formulation de solides MOF à surface modifiée par le polyéthylène glycol (PEG) et l'acide folique (FA)

Acide folique :

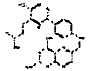

### a) Synthèse n° 1 : modification de surface après formulation des nanoparticules

### Modification de surface avec du PEG :

100 mg de nanoparticules de MIL100, MIL88, MIL53 ou MIL101 (préalablement deshydraté à 100°C/nuit) sont dispersées sous sonication dans 100 mL de solution à 17,9 mM de 2-(méthoxy(polyéthylèneoxy)-propyl)triméthoxysilane dans le toluène anhydre. Le mélange est soumis à des ultrasons à 60°C pendant 4 h, sous débit de gaz inerte (azote). La suspension colloïdale résultante, contenant les nanoparticules à surface modifiée avec du PEG, est lavée deux fois avec de l'éthanol et deux fois dans une solution de citrate de sodium 20 m*M* (pH 8,0) et resuspendue finalement dans l'eau.

### Modification de surface avec du PEG et du FA :

Le FA a été attaché aux nanoparticules grâce à un espaceur bifonctionnel, le silane-PEG-trifluoroethylester (TFEE) synthétisé selon une méthode décrite dans la littérature par Kohler N. et al., J Am Chem Soc 2004; 126: 7206-7211.

100 mg de nanoparticules sont recouvertes par du PEG-TFEE selon la même méthode que décrit ci-dessus, en utilisant le silane-PEG-TFEE à la place du 2-méthoxy (polyéthylèneoxy)-propyltrimethoxysilane.

Les nanoparticules résultantes, recouvertes de PEG-TFEE, sont lavées deux fois puis resuspendues dans 100 mL de toluène sec. Une amine primaire a été greffée aux groupements terminaux des chaines de PEG en rajoutant 1 mL de éthylènediamine (Sigma) aux nanoparticules maintenues sous débit d'azote. Le mélange a été soumis aux ultrasons (4h, 60°C). Les nanoparticules résultantes, recouvertes par l'amine, ont été lavées trois fois avec de l'éthanol, et trois fois avec du diméthyl sulfoxide (DMSO). Les nanoparticules ont été finalement resuspendues dans 50 mL de DMSO anhydre. Le FA a été couplé aux groupements amine terminaux des chaines de PEG en rajoutant 50 mL de solution de FA (23 m*M* FA dans du DMSO) avec des quantités équimolaires de dicyclohexylcarbodiimide (DCC) (Sigma) et 10 µL de pyridine. Le mélange a été protégé de la lumière et laissé réagir pendant une nuit sous agitation bidimensionnelle (180 rpm). Les nanoparticules conjuguées avec du PEGH et du FA (NP-PEG-FA) ont été lavées deux fois avec de l'éthanol, et deux fois avec une solution de citrate de sodium 20 m*M* (pH 8.0) et resuspendues finalement dans cette même solution de citrate de sodium.

### b) Synthèse n°2 : modification de surface pendant la synthèse des nanoparticules

La modification de la surface des solides MOF peut également se faire pendant la synthèse.

Dans l'exemple qui suit, la modification de surface est réalisée avec du chitosane préalablement greffé avec de l'acide folique (FA).

Un exemple de synthèse de chitosane greffé avec de l'acide folique via un espaceur PEG est décrite dans la publication de Peggy Chan et al., Biomaterials, Volume 28, Issue 3, 2007, pp 540-549.

Les réactifs suivants ont été utilisés pour la réalisation de cet exemple :
- chitosane (masse molaire Mn de 255 kDa, viscosité: 200-800 cps dans 1% d'acide acetique, commercialisé par la société Sigma-Aldrich)
- le N-hydroxylsuccinimide-PEG-Maleimide (NHS-PEG-MAL, Mn 3400 Da, commercialisé par la société Nektar, NOF Corporation, Tokyo, Japan)
- l'ester succinimidyl de monométhoxy-PEG (mPEG-SPA, Mn 5000 Da, commercialisé par la société Nektar, NOF Corporation, Tokyo, Japan).

Le chitosane est préalablement déacétylé pour obtenir un degré d'acétylation de 82% (determiné par ¹H-NMR) selon le procédé décrit par Wang LS (Thesis, National University of Singapore, Singapore, 2001).

100 mg de chitosane ont été dissous dans 50 mL de solution d'acide acétique (20%). Le pH de la solution a été ajusté à 6 par addition d'hydroxyde de sodium et le mPEG-SPA a été introduit dans le mélange réactionnel. Le mélange est laissé réagir pendant 24 h à température ambiante sous agitation. Le produit obtenu est dialysé pendant 24 h contre de l'eau de-ionisée en utilisant une membrane avec un seuil de coupure de 12,000 Da (Spectrum Laboratories, USA) et finalement, lyophilisé.

Pour synthétiser le chitosane greffé avec du PEG et du FA, l'ester de N-hydroxysuccinimide de FA a été préparé selon la méthode décrite par J.H. Van Steenis et al., J Control Release 87 (2003), pp. 167-176.

Brièvement, 1 g de FA a été ajouté à un mélange de DMSO anhydre (40 mL) et triéthylamine (TEA, 0,5 mL). Le mélange a été agité à l'abri de la lumière pendant la nuit, dans des conditions anhydres. Les autres réactifs, dicyclohexylcarbodiimide (DCC, 0.5 g) et N-hydroxysuccinimide (NHS, 0.52 g) ont été ajoutés et le mélange est laissé réagir pendant 18h à l'abri de la lumière et dans des conditions anhydres. Le produit secondaire, la dicyclohexylurée (DCU) ayant précipité, a été enlevé par filtration. Le DMSO et le TFA ont été évaporés sous vide. Le produit de la réaction a été séché sous vide, puis dissout dans 1,5 mL de mélange 2 :1 (v :v) de DMSO et TEA. Une quantité équimolaire de 2-aminoethanethiol (Wako) a été rajoutée et la reaction a été laissée se poursuivre pendant la nuit dans des conditions anhydres. Ainsi, un groupement thiol a pu être introduit à l'acide folique et le produit résultats est dénommé FA-SH.

100 mg de chitosane sont dissous dans 50 mL de solution d'acide acétique (20%). Le pH de la solution est ajusté à 6 par addition d'hydroxyde de sodium et 100 mg de NHS-PEG-Mal sont introduits dans le mélange réactionnel. La mélange est laissé réagir pendant 3 h à température ambiante sous agitation, puis le pH est ajusté à 7. Le mélange est laissé réagir pendant la nuit, dans des conditions anhydres. Le FA-SH synthétisé comme précédemment a été rajouté de manière graduée sous agitation et le pH a été ajusté à 6,5-7,5 avec de la soude.

Le conjugué obtenu dénommé FA-PEG chi porte du FA couplé au chitosane via un bras espaceur de PEG, ce qui est un avantage pour atteindre le récepteur de l'acide folique (comme il a été décrit dana la littérature, voir par exemple : A. Gabizon, H. Shmeeda, A.T. Horowitz and S. Zalipsky, Tumor cell targeting of liposome-entrapped drugs with phospholipids-anchored folic acid-PEG conjugates, Adv Drug Deliv Rev 56 (2004), pp. 1177-1192.

Le degré de substitution peut être ajusté en variant le rapport massique PEG : chitosane utilisé dans la réaction. Ce polymère a été dialysé pendant 48 h contre de l'eau de-ionisée en utilisant une membrane avec un seuil de coupure de 12,000 Da (Spectrum Laboratories, USA) et finalement, lyophilisé.

### c) Synthèse n°3 :

Les solides hybrides peuvent être modifiés en surface par adsorption de polysaccharides tels le dextrane greffé avec la biotine.

Nous pouvons donc envisager d'adsorber, à la place du dextrane greffé avec la biotine, du chitosane greffé avec l'acide folique (synthétisé comme décrit dans la publication citée plus haut), et, éventuellement, si nécessaire, greffé aussi avec d'autres composés hydrophobes comme le cholestérol ou des chainons aliphatiques, afin d'assurer une meilleure adhésion au niveau de la surface des nanoparticules.

Une fonctionnalisation de surface pourrait également se faire *via* l'adsorption d'autres polysaccharides greffés avec de le FA.

### d) Synthèse n°4 :

Les solides hybrides peuvent être modifiés en surface avec du PEG lors de leur synthèse. Nous proposons de substituer le monométhoxy PEG monoacide utilisé lors de cette synthèse par du PEG monoacide comportant une fonction réactive bloquée en bout de chaine comme le produit commercial :
Boc-PEG-carbonateNHS, MW 5000, Boc = tert-butoxycarbonyl
Reference SUNBRIGHT^{®} BO-050TS, NOF Corporation

Après réaction, comme indiqué dans l'exemple, sauf que nous utiliserons des mélanges Me0-PEG-COOH et Boc-PEG-carbonateNHS (rapports massiques 1 :0.05 à 1 :0,5) à la place de Me0-PEG-COOH, la deprotection se fera par rajout d'acide trifluoroacétique (TFA)

### Protocole possible :

On rajoute 0.6 mL de TFA à une suspension de 300 mg de nanoparticules dans 2 mL d'eau. On laisse réagir pendant 1h à T ambiante sous agitation magnétique. On isole les particules par centrifugation et on les lave trois fois avec de l'eau bidistillée.

On fonctionnalise les groupements réactifs en surface avec des ligands tels le FA, par exemple comme dans la synthèse A.

### e) Caractérisation des nanoparticules :

La quantité d'acide folique effectivement couplée aux nanoparticules pourra être déterminée après dégradation de celles-ci dans un milieu acide, neutralisation à pH7, puis redissolution dans un solvant approprié, tel le dichlorométhane, le DMSO, ou un mélange de ces deux solvants. L'acide folique pourra alors être quantifié par une mesure de l'absorbance UV (à 358 nm, le coefficient d'extinction molaire E de l'acide folique est 15,76 M⁻¹ cm⁻¹).

Pour vérifier que l'acide folique se trouve bien en surface des nanoparticules, nous pourrions utiliser une technique telle la résonance de surface du plasmon (BIAcore). La protéine qui se lie à l'acide folique (« folate binding protein ») sera immobilisée au niveau de la surface du détecteur, sur un mince film de dextrane activé (procédure classique recommandée par le constructeur BIAcore). La quantité de nanoparticules effectivement attachée à ce support sera évaluée par rapport à celle de nanoparticules non recouvertes d'acide folique.

### Exemple 27 : Formulation de solides MOF mixtes : à base de Gadolinium et de fer)

Deux conditions de synthèse sont possibles :
*Synthèse N° 1* :
   0,028 g (0,5 mmol) de poudre de Fer métallique Fe° (Riedel de Haën, 99 %), 0,225 g de nitrate de gadolinium(III) hexahydrate (0,5 mmol, Aldrich, 99,9 %), 0,140 g d'acide 1,3,5 benzenetricarboxylique (0,666 mmol, Aldrich 95 %) dispersés dans 10 ml d'eau déionisée, le tout laissé 24 heures à 180°C dans un corps en Téflon de 23 ml mis dans une Bombe métallique PAAR. Le solide est ensuite filtré et lavé avec de l'eau puis de l'éthanol.
*Synthèse N° 2 :*
   0,065 g (-0,5 mmol pour 1 fer par trimère) d'acétate de fer(III) (préparé selon la synthèse décrite dans l'exemple 1), 0,225 g de nitrate de gadolinium(III) hexahydrate (0,5 mmol, Aldrich, 99,9 %), 0,140 g d'acide 1,3,5 benzenetricarboxylique (0,666 mmol, Aldrich 95 %) dispersés dans 10 ml d'eau déionisée (ou de méthanol ou d'éthanol ou de diméthylformamide), le tout laissé 12 heures à 150°C dans un corps en Téflon de 23 ml mis dans une Bombe métallique PAAR. Le solide est ensuite filtré et lavé avec de l'eau puis de l'éthanol.

### Exemple 28 : Autres solides MOF : encapsulation de l'urée dans un carboxylate de Zirconium et dans un phosphonate de titane.

Il n'y a pas d'évidence qui montre que le titane (Ti) ou le zirconium (Zr) constituent un risque pour la sante, c'est pourquoi ils ont été choisis pour la réalisation de cet exemple. En effet, le titane est actuellement utilisé dans des implants comme dioxyde de titane, où il n'y a aucune réaction adverse dans le tissus, suggèrent qu'il est biologiquement compatible. De plus, l'administration intraveineuse de 250 mg/kg sur des rats montre le dioxyde de titane comme une substance inerte (C.B. Huggings, J.P. Froehlich, J. exp. Med., 124, 1099, 1966). Plus concrètement, des études sur des titanates ont montré une dose létale 50 (DL50) orale sur des rats de plus de 12 g/kg (J.R. Brown, E. Mastromatteo, Ind. Med. Surg., 31, 302,1962).

Zr est un élément trace dans l'organisme et même si sa présence dans l'organisme est relativement élevée, aucune fonction métabolique spécifique en rapport avec l'élément Zr n'a encore été démontrée. Apparemment, ce métal n'est ni toxique ni essentiel (S. Ghosh, A. Sharma, G. Talukder, Biological Trace Element Resarch, 35 (3), 247, 1992). Par contre, depuis son utilisation dans de nouveaux matériaux ou comme agent de contraste par rayon X, des études plus approfondis sur son toxicité sont nécessaires (oral DL50 sur le rat du ZrCl₄ 1.7 g/kg).

De la même façon, il est possible d'utiliser les ligands organiques avec des groupes complexant le métal autres que les carboxylates, comme des imidazolates, catecolates ou phosphonates. Dans les exemples qui suivent, le ligand organique N,N'-piperazinebis(methylenephosphonate) a été choisi comme ligand phosphonate complexant. Les phosphonates sont généralement plus complexantes que les carboxylates, en donnant des structures plus stables. En effet, on pourra modifier le métal, le ligand et le groupe complexant pour modifier les propriétés des solides (biodégradation, hydrophile, interactions molécule active-matrice...).

### a) Synthèse du diphosphonate de titane MIL-91(Ti) : (TiO(H₂L) • nH₂O (n - 4.5, L = O₃P-CH₂-NC₄H₈N-CH₂-PO₃)

Le solide poreux N,N'-piperazinebis(methylenephosphonate de titane (IV) MIL-91(Ti) a été préparé par synthèse hydrothermale à partir d'un mélange de 96 mg de TiO₂.H₂O, 270 mg d'acide N,N'-piperazinebismethylenephosphonique (élaboré selon le procédé décrit par Moedritzer, K.; Irani, R. R. J. Org. Chem. 31, 1603, 1966), 0,04 ml d'acide fluorhydrique HF (Prolabo Normapur 40%), et 4,95 ml d'eau déionisée le tout introduit sans agitation dans un corps en téflon de 23 ml ; ce dernier est ensuite placé dans un corps métallique PAAR et le tout est porté ensuite à 210°C pendant 96 heures.

Le dioxide de titane hydraté TiO₂.H₂O est préparé à partir de 500 ml de TiCl₄ (Aldrich, 99%) introduit lentement dans 500 ml d'une solution d'acide hydrochlorique HCl (Prolabo, 36%) sous agitation. La solution jaune est ensuite neutralisée lentement par ajout d'ammoniaque (Prolabo, 20%) à température ambiante sous agitation. L'introduction de la base est stoppée lorsque le pH de la suspension atteint 7-8. Le précipité résultant blanc est d'abord filtré puis lavé à trois reprises avec un excès d'eau distillée. Le solide est ensuite séché à 100°C pendant 24 heures puis redispersé dans un grand volume d'eau (1 litre) pour retirer les traces de sels d'ammonium puis filtré et rincé plusieurs fois avec de l'eau distillée. Le solide final est séché sous air.

La taille de particule monodisperse mesurée par diffusion de lumière est 265

### b) Synthèse du carboxylate de zirconium ZrBDC

Un mélange préparé à partir de 235 mg de ZrCl₄ (Alfa Aesar, 99,5 %), 230 mg d'acide téréphtalique (Aldrich, 98 %) et 5 ml de diméthylformamide (Acros Organics, extra-dry) est introduit dans un corps en téflon sous agitation pendant 10 minutes à température ambiante.

Le corps en Téflon est ensuite introduit dans un corps métallique PAAR puis mis à l'étuve à 150°C pendant 15 heures. Après retour à température ambiante, le solide est récupéré par filtration et séché à l'air. Le solvant contenu dans les pores est retiré par calcination du solide à 200°C sous air pendant 15 heures.

La taille de particule mesurée par diffusion de lumière présente deux populations, une majoritaire à 150 nm et une très minoritaire à 1 *µ*m.

### c) Encapsulation de l'urée

Pour l'insertion de cosmétiques dans les matériaux MOF, 150 mg de solide MIL-91(Ti) ou de solide ZrCO, préalablement déshydratés (150°C/12h), sont suspendus dans 10 mL d'une solution d'urée dans l'eau distillée (500mg/mL) ou dans de l'éthanol (30mg/mL) sous agitation à température ambiante pendant 5 jours. Après adsorption, le solide chargé en cosmétiques est récupéré par centrifugation à 10000 rpm pendant 15 min et séché sous air. Les solides sont caractérisés par DRX et FTIR. La quantification de la teneur en cosmétique adsorbé est effectuée par ATG.

La DRX permet de vérifier que la structure cristalline des deux matériaux est maintenue après l'encapsulation de l'urée. L'intensité relative de premiers pics de diffraction varie dans le phosphonate de titane MIL-91 après l'adsorption du cosmétique dû à la présence de l'urée dans les pores.

Dans le téréphtalate de zirconium contenant de l'urée, encapsulée à partir d'une solution dans l'éthanol, un élargissement des raies (pics de diffraction) est observé. Il peut s'expliquer par une petite dégradation après 5 jours dans l'éthanol.

La Spectroscopie infrarouge par la transformée de Fourier (FTIR) permet de vérifier l'apparition de bandes correspondent à l'urée à 1680 (amide ν(C=O)) et 3400 cm⁻¹ (ν(N-H)), ce qui indique que l'urée est bien présente dans les solides.

Les quantités d'urée encapsulées par les matériaux MIL-91 et ZrCO sont très importantes, jusqu'à le 41 et 66% référées au solide sec, respectivement (comme décrit dans le tableau ci-dessous).

**Tableau 27. Quantification de l'urée encapsulée dans le phosphonate de Ti et carboxylate de Zr par ATG**

| | **H₂O (%)** | **Urée (%, referee au solide sec)** | **Ligand (%, referee au solide sec)** |
|---|---|---|---|
| **MIL-91** | 17,8 | - | 26,8 |
| **MIL-91 U H₂O** | 13,0 | 41,1 | 29,9 |
| **ZrCO** | 23,8 | - | 52,7 |
| **ZrCO U H₂O** | 5,3 | 65,9 | 51,5 |
| **ZrCO U EtOH** | 16,2 | 17,5 | 54,8 |

### LISTE DES REFERENCES

[1] Demande de brevet US 10/039,733
[2] Demande de brevet US 10/061,147
[3] Demande de brevet US 10/137,043
[4] Brevet US 5,648,508
[5] Brevet US 6,638,494
[6] Bone Marrow Transplant. 2002, 30 (12), 833-841
[7] Brevet US 4,329,332
[8] J. Bouligand, P. Couvreur, A. Layre, A. Deroussent, A. Paci, E. Delain, G. Vassal, R. Gref « Busulfan-loaded long-circulating nanospheres, a very attractive challenge for both galenists and pharmacologists », Int. J. Pharm., 2004
[9] K. Byrapsa, M. Yoshimura, « Handbook of hydrothermal technology », Noyes Publications, Parkridge, New Jersey USA, William Andrew Publishing, LLC, Norwich NY USA, 2001
[10] G. Tompsett, W. C. Conner, K. S. Yngvesson, ChemPhysChem. 2006, 7, 296
[11] S.-E. Park, J.-S. Chang, Y. K. Hwang, D. S. Kim, S. H. Jhung, J. -S. Hwang, Catal. Survey Asia 2004, 8, 91
[12] C. S. Cundy, Collect. Czech. Chem. Commum. 1998, 63, 1699
[13] S. H. Jhung, J.-H. Lee, J.-S. Chang, Bull. Kor. Chem. Soc. 2005, 26, 880
[14] A. Pichon, Cryst. Eng. Comm. 8, 2006, 211-214
[15] D. Braga, Angew. Chem. Int. Ed. 45, 2006, 142-246
[16] D. Braga, Dalton Trans., 2006, 1249-1263.
[17] Sabine Balthasar, Kerstin Michaelis, Norbert Dinauer, Hagen von Briesen, Jörg Kreuter and Klaus Langer, "Preparation and characterisation of antibody modified gelatin nanoparticles as drug carrier system for uptake in lymphocytes", Biomaterials, 2005, 26, 15, 2723-2732.
[18] Ruxandra Gref, Patrick Couvreur, Gillian Barratt and Evgueni Mysiakine, "Surface-engineered nanoparticles for multiple ligand coupling", Biomaterials, 2003, 24, 24, 4529-4537
[19] Stella B, Marsaud V, Arpicco S, Geraud G, Cattel L, Couvreur P, Renoir JM., "Biological characterization of folic acid-conjugated poly(H2NPEGCA-co-HDCA) nanoparticles in cellular models", J Drug Target. 2007, 15(2), 146-53
[20] Hattori Y, Maitani Y., "Folate-linked lipid-based nanoparticle for targeted gene delivery", Curr Drug Deliv. 2005, 2(3), 243-52
[21] Mulder WJ, Griffioen AW, Strijkers GJ, Cormode DP, Nicolay K, Fayad ZA, « Magnetic and fluorescent nanoparticles for multimodality imaging », Nanomed. 2007, 2(3), 307-324
[22] A.K. Gupta, R.R. Naregalkar, V.D. Daidya, M. Gupta, « Recent advances on surface engineering or magnetic iron oxide nanoparticles and their biomedical applications », Nanomed. 2007, 2(1), 23-39
[23] P Caravan, « Strategies for increasing the sensitivity of gadolinium based MRI contrast agents », Chem. Soc. Rev., 2006, 35, 512-523
[24] Yan-Ping Ren, La-Sheng Long, Bing-Wei Mao, You-Zhu Yuan, Rong-Bin Huang, and Lan-Sun Zheng, Angew. Chem. Int. Ed. 2003, 42, No. 5, 532
[25] C.T. Dziobkowski, T.J. Wrobleski, D.B. Brown, Inorg. Chem., 1982, 21, 671
[26] C. Serre, F. Millange, C. Thouvenot, M. Nogues, G. Marsolier, D. Loüer, G. Férey J. Am. Chem. Soc., 2002, 124, 13519
[27] T. Loiseau, C. Mellot-Draznieks, H. Muggera, G. Férey, M. Haouas, F. Taulelle, C.R.Acad.Sci, 2005, 8, 765
[28] C. Serre, F. Millange, S. Surblé, G. Férey Angew. Chem. Int. Ed. 2004, 43, 6286
[29] Suzy Surblé, Christian Serre, Caroline Mellot-Draznieks, Franck Millange, and Gérard Férey: Chem. Comm. 2006 284-286
[30] C. Serre, C. Mellot-Draznieks, S. Surblé, N. Audebrand, Y. Fillinchuk and G. Férey: Science, 2007, 315, 1828
[31] C. Mellot-Draznieks, C. Serre, S. Surblé, N. Audebrand and G. Férey: J. Am. Chem. Soc., 2005, 127, 16273-16278
[32] Sung Hwa Jhung, Jin-Ho Lee, Ji Woong Yoon, Christian Serre, Gérard Férey and Jong-San Chang « Facile Synthesis of the Chromium Terephthalate MIL-101 with Giant Pores and Its Sorption Ability for Benzene », Adv. Mater, 2006, 19(1), 121-124.
[33] B. Baleux, G. Champetier « Chimie analytique-dosage colorimétrique d'agents de surface non ioniques polyoxyéthylènes à l'aide d'une solution iode-iodurée », C.R. Acad. Sciences Paris, 1972, série C, 274, 1617-1620
[34] R. Gref, M. Lück, P. Quellec, M. Marchand, E. Dellacherie, S. Harnisch, T. Blunk and R. H. Müller, "Stealth" corona-core nanoparticles surface modified by polyethylene glycol (PEG): influences of the corona (PEG chain length and surface density) and of the core composition on phagocytic uptake and plasma protein adsorption", Colloids and Surfaces B: Biointerfaces, 2000, 18, 3-4, 301-313
[35] G. Oros, T. Cserhati, E. Forgacs, Chemosphere 52, 2003, 185
[36] A.M. Badawi, E.M.S. Azzam, S.M.I. Morsy, Bioorg. Med. Chem., 14, 2006, 8661
[37] W-J. Tsai, Y-J Shiao, S-J Lin, W-F Chiou, L-C Lin, T-H Yang, C-M teng, T-S Wu, L-M Yang, Bioorg. Med. Chem. Letters 16, 2006, 4440
[38] Kim, Y. ; Swager, T. M. Chem. Commun., 2005, 372-374
[39] L. Anzalone, J. A. Hirsch, J. Org. Chem., 1985, 50, 2128-213
[40] Shiotani Akinori, Z. Naturforsch. 1994, 49, 12, 1731-1736
[41] Ameerunisha et al., J. Chem. Soc. Perkin Trans. 2, 1679, 1995
[42] Kohler N, Fryxell GE, Zhang MQ. A bifunctional poly(ethylene glycol) silane immobilized on metallic oxide-based nanoparticles for conjugation with cell targeting agents. J Am Chem Soc 2004; 126: 7206-7211
[43] Peggy Chan, Motoichi Kurisawa, Joo Eun Chung, Yi-Yan Yang, Synthesis and characterization of chitosan-g-poly(ethylene glycol)-folate as a non-viral carrier for tumor-targeted gene delivery, Biomaterials, Volume 28, Issue 3, 2007, pp 540-549
[44] Wang LS. Polyelectrolyte complex (PEC) membrane composed of chitosan and alginate for wound dressing application. Thesis, National University of Singapore, Singapore, 2001.
[45] J.H. Van Steenis, E.M. van Maarseveen, F.J. Verbaan, R. Verrijk, D.J.A. Crommelin and G. Storm et al., Preparation and characterization of folate-targeted PEG-coated pDMAEMA-based polyplexes, J Control Release 87 (2003), pp. 167-176.
[46] Roch et al, J Chem Phys 110, 5403-5411, 1999
[47] Kathryn E. Luker et Gary D. Luker, Applications of bioluminescence imaging to antiviral research and therapy: Multiple luciferase enzymes and quantitation, Antiviral Research, Volume 78, Issue 3, June 2008, Pages 179-187
[48] C.B. Huggings, J.P. Froehlich, J. exp. Med., 124, 1099, 1966)
[49] J.R. Brown, E. Mastromatteo, Ind. Med. Surg., 31, 302,1962
[50] S. Ghosh, A. Sharma, G. Talukder, Biological Trace Element Resarch, 35 (3), 247, 1992
**[51]** Moedritzer, K.; Irani, R. R. J. Org. Chem. 31, 1603, 1966

## Revendications

1. Solide MOF cristallin poreux isoréticulaire comprenant une succession tridimensionnelle de motifs répondant à la formule (I) suivante :
MₘOₖXₗLₚ Formule (I)
dans laquelle :
chaque occurrence de M représente indépendamment un ion métallique Fe²⁺ ou Fe³⁺;
m, k, 1 et p sont des nombres ≥ 0 choisis de façon à respecter la neutralité des charges du motif ; de préférence, m, k, l et p sont indépendamment 0 à 4, par exemple m et p sont indépendamment 1, 2 ou 3 et/ou k et 1 sont indépendamment 0 ou 1 ;
X est un ligand choisi dans le groupe comprenant OH⁻, Cl⁻, F⁻, I⁻, Br⁻, SO₄²⁻, NO₃⁻, ClO₄⁻, R¹-(COO)ₙ⁻, R¹-(SO₃)ₙ⁻, R¹-(PO₃)ₙ⁻, où R¹ est un hydrogène, un alkyle en C₁ à C₈, linéaire ou ramifié, n = 1 à 6 ;
L est un ligand espaceur di-, tri-, tétra- ou hexa-carboxylate choisi dans le groupe constitué de :
| | |
|---|---|
| 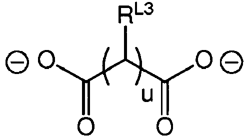 | 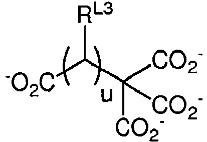 |
| 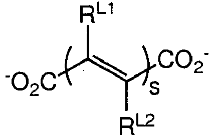 | 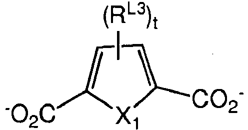 |
| 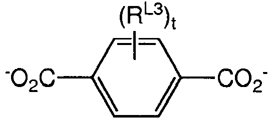 | 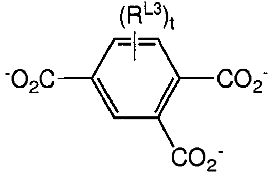 |
| 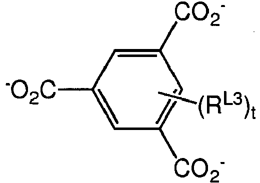 | 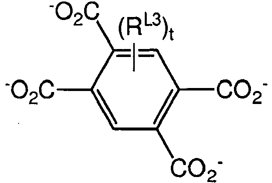 |
| 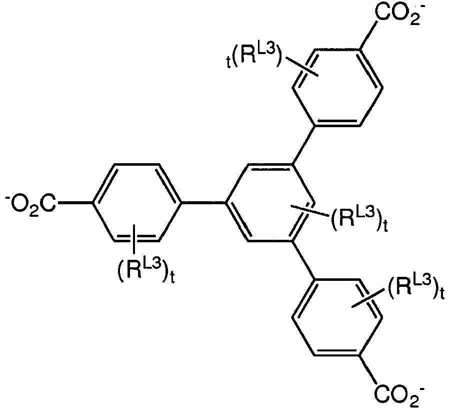 | 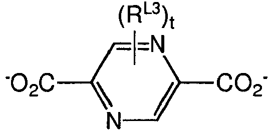 |
| 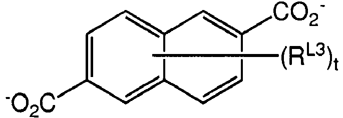 | 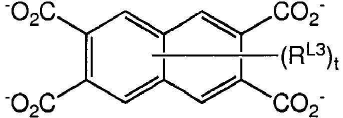 |
| 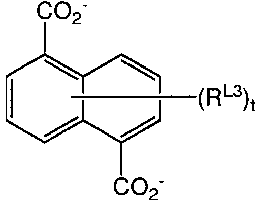 | 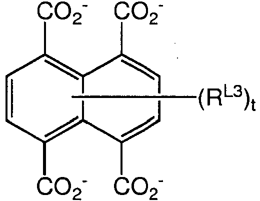 |
| 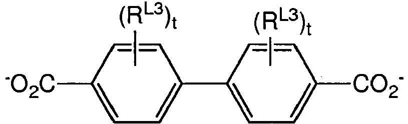 | |
| 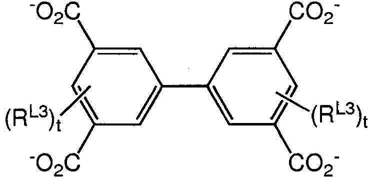 | |
| 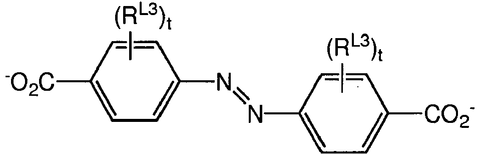 | |
| 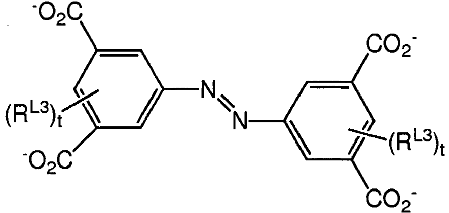 | |
| 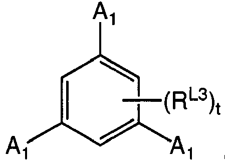 | où A₁, A₂ et A₃ représentent |
| indépendamment | 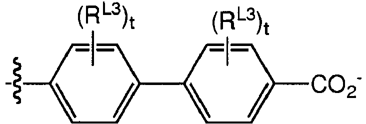 |
dans lesquels :
X₁ représente O ou S,
s représente un entier de 1 à 4,
chaque occurrence de t représente indépendamment un entier de 1 à 4,
u représente un entier de 1 à 7,
R^{L1} et R^{L2} représentent indépendamment H, un halogène ou un alkyle en C₁ à C₆, et
chaque occurrence de R^{L3} représente indépendamment H, un halogène, OH, NH₂, NO₂ ou un alkyle en C₁ à C₆ ;
dans lequel la surface est modifiée en ce qu'elle comprend au moins un agent de surface organique choisi dans le groupe comprenant un oligosaccharide, un polysaccharide, un glycosaminoglycanne, un polymère, un tensioactif, une vitamine, un coenzyme, un anticorps ou fragment d'anticorps, un aminoacide ou un peptide.

2. Solide selon la revendication 1, dans lequel le ligand L est un ligand di-, tri- ou tétra- carboxylate choisi dans le groupe constitué de : C₂H₂(CO₂⁻)₂ (fumarate), C₂H₄(CO₂⁻)₂ (succinate), C₃H₆(CO₂⁻)₂ (glutamate), C₄H₄(CO₂⁻)₂ (muconate), C₄H₈(CO₂⁻)₂ (adipate), C₇H₁₄(CO₂⁻)₂ (azelate), C₅H₃S(CO₂⁻)₂ (2,5-thiophènedicarboxylate), C₆H₄(CO₂⁻)₂ (téréphtalate), C₆H₂N₂(CO₂⁻)₂ (2,5-pyrazine dicarboxylate), C₁₀H₆(CO₂⁻)₂ (naphtalène-2,6-dicarboxylate), C₁₂H₈(CO₂⁻)₂ (biphényle-4,4'-dicarboxylate), C₁₂H₈N₂(CO₂⁻)₂ (azobenzènedicarboxylate), C₆H₃(CO₂⁻)₃ (benzène-1,2,4-tricarboxylate), C₆H₃(CO₂⁻)₃ (benzène-1,3,5-tricarboxylate), C₂₄H₁₅(CO₂⁻)₃ (benzène-1,3,5-tribenzoate), C₆H₂(CO₂⁻)₄ (benzène-1,2,4,5-tétracarboxylate, C₁₀H₄(CO₂⁻)₄ (naphtalène-2,3,6,7-tétracarboxylate), C₁₀H₄(CO₂⁻)₄ (naphtalène-1,4,5,8-tétracarboxylate), C₁₂H₆(CO₂⁻)₄ (biphényl-3,5,3',5'-tétracarboxylate), et les analogues modifiés choisis dans le groupe comprenant le 2-aminotéréphtalate, le 2-nitrotéréphtalate, le 2-méthyltéréphtalate, le 2-chlorotéréphtalate, le 2-bromotéréphtalate, le 2,5-dihydroxotéréphtalate, le tétrafluorotéréphtalate, le tétraméthyltéréphtalate, le diméthyl-4,4'-biphényldicarboxylate, le tétraméthyl-4,4'-biphényldicarboxylate, le dicarboxy-4,4'-biphényldicarboxylate, le 2,5-pyrazyne dicarboxylate, le 2,5 diperfluoroterephthalate, azobenzene 4,4'-dicarboxylate, 3,3'-dichloro azobenzene 4,4'-dicarboxylate, 3,3'-dihydroxo azobenzene 4,4'-dicarboxylate, 3,3'-diperfluoro azobenzene 4,4'-dicarboxylate, 3,5,3',5'-azobenzene tetracarboxylate, 2,5-dimethyl terephthalate, perfluorosuccinate, perfluoromuconate, perfluoro glutarate, 3,5,3',5' perfluoro-4,4'-azobenzene dicarboxylate, 3,3'-diperfluoro azobenzene 4,4'-dicarboxylate.

3. Solide selon l'une quelconque des revendications 1 à 2, dans lequel le ligand L est un ligand fluoré choisi dans le groupe constitué de tétrafluorotéréphtalate, perfluorosuccinate, perfluoromuconate, perfluoro glutarate, 2,5 diperfluoroterephtalate, 3,6 perfluoro 1,2,4,5 benzenetetracarboxylate, 3,5,3',5' perfluoro-4,4'-azobenzene dicarboxylate, 3,3'-diperfluoro azobenzene 4,4'-dicarboxylate.

4. Solide selon l'une quelconque des revendications 1 à 2, dans lequel le ligand L est un ligand biologiquement actif choisi dans le groupe constitué de C₇H₁₄(CO₂⁻)₂ ; aminosalicylate ; chlodronate, pamidrontate, alendronate, etidronate ; meprobamate ; des porphyrines comprenant des groupes carboxylates, phosphonates et/ou amino ; des aminoacides ; des azobenzènes comprenant des groupes carboxylates, phosphonates, et/ou amino ; le dibenzofuran-4,6-dicarboxylate, le dipicolinate; le glutamate, le fumarate, le succinate, le suberate, l'adipate, le nicotinate, nicotinamide, purines, pyrimidines.

5. Solide selon l'une quelconque des revendications 1 à 4, dans lequel le ligand X est choisi dans le groupe constitué de OH⁻, Cl⁻, F⁻, CH₃-COO⁻, PF₆⁻, ClO₄⁻.

6. Solide selon l'une quelconque des revendications 1 à 4, dans laquelle au moins une occurrence du ligand X est ¹⁸F⁻.

7. Solide selon l'une quelconque des revendications 1 à 6, ledit solide étant sous la forme de nanoparticule.

8. Solide selon l'une quelconque des revendications 1 à 7, dans lequel l'agent de surface organique est choisi dans le groupe constitué d'un oligosaccharide, un polysaccharide, le chitosan, le dextran, l'acide hyaluronique, l'héparine, le fucoïdane, l'alginate, la pectine, l'amilose, les cyclodextrines, l'amidon, la cellulose, le xylane, le polyéthylène glycol (PEG), le pluronic, l'alcool polyvinylique, le polyéthylène imine.

9. Solide selon l'une quelconque des revendications 1 à 7, dans lequel l'agent de surface organique est une molécule de ciblage choisie dans le groupe constitué de : la biotine, l'acide folique, l'acide lipoique, l'acide ascorbique, un anticorps ou fragment d'anticorps, un peptide.

10. Solide selon l'une quelconque des revendications 1 à 9, ledit solide comprenant dans ses pores ou à sa surface au moins un principe pharmaceutiquement actif et/ou une substance active entrant dans la formulation d'une préparation cosmétique et/ou un marqueur.

11. Solide selon l'une quelconque des revendications 1 à 10, dans lequel le principe pharmaceutiquement actif est choisi dans le groupe constitué de : le taxotère, le busulfan, l'azidothymidine (AZT), l'azidothymidine phosphatée (AZTP), le cidofovir, la gemcitabine, le tamoxifène.

12. Solide selon l'une quelconque des revendications 1 à 11, ledit solide comprenant dans ses pores ou à sa surface au moins une molécule fluorescente choisie dans le groupe constitué de : les rhodamines, la fluoresceine, la luciférase, le pyrène et dérivés, et l'aminopyrrolidino-7-nitrobenzofurazan.

13. Solide selon l'une quelconque des revendications 1 à 11, ledit solide comprenant dans ses pores ou à sa surface au moins un principe pharmaceutiquement actif avec une capacité avec une capacité de charge de 1 à 200% en poids de solide sec.

14. Solide selon l'une quelconque des revendications 1 à 13, ledit solide comprenant dans ses pores ou à sa surface au moins une substance active entrant dans la formulation d'une préparation cosmétique choisie dans le groupe comprenant la benzophénone, la visnadine et l'acide salicylique.

15. Solide selon l'une quelconque des revendications 1 à 14, ledit solide comprenant dans ses pores ou à sa surface au moins un marqueur, celui-ci étant choisi dans le groupe comprenant un marqueur d'imagerie médicale, un agent de contraste, un traceur, un marqueur radioactif, un marqueur fluorescent, un marqueur phosphorescent.

16. Solide selon la revendication 15, dans lequel le marqueur est choisi dans le groupe constitué de : un composé fluorescent, un oxyde de fer, un complexe de gadolinium, des ions gadolinium directement présents dans la structure.

17. Procédé de préparation d'un solide tel que défini dans l'une quelconque des revendications 1 à 16, comprenant :
a) au moins une étape réactionnelle (i) consistant à mélanger dans un solvant polaire :
- au moins une solution comprenant au moins un précurseur inorganique métallique se présentant sous la forme de métal M, d'un sel de métal M ou d'un complexe de coordination comprenant l'ion métallique M dans laquelle M est tel que défini dans la revendication 1 ;
- au moins un ligand L' di-, tri-, tétra- ou hexadentate choisi dans le groupe constitué de :
| | |
|---|---|
| 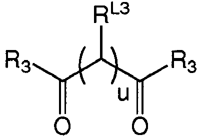 | 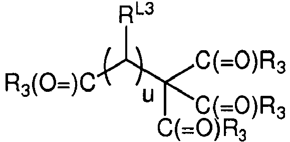 |
| 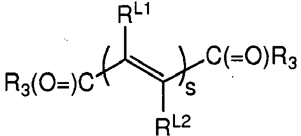 | 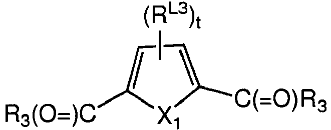 |
| 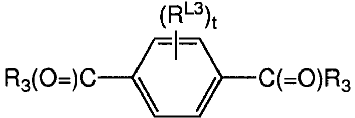 | 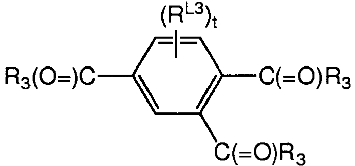 |
| 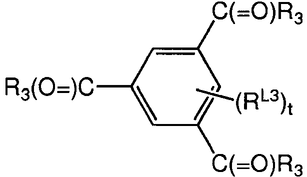 | 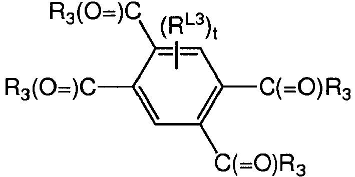 |
| 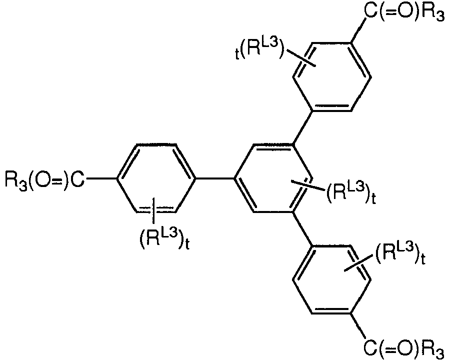 | 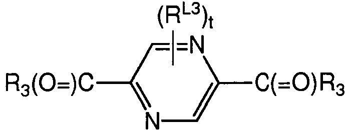 |
| 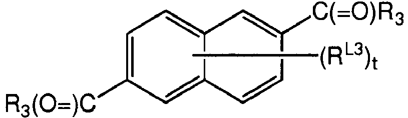 | 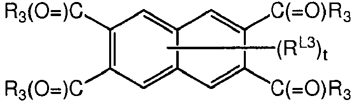 |
| 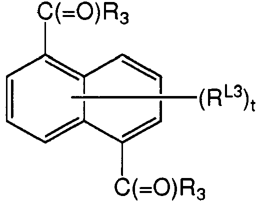 | 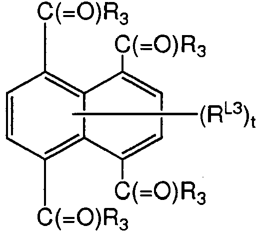 |
| 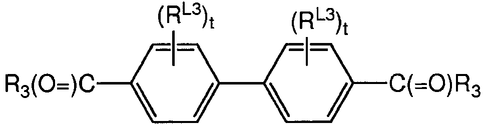 | |
| 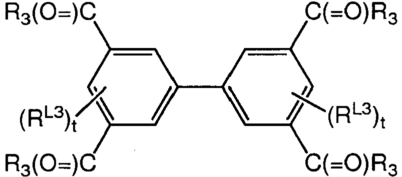 | |
| 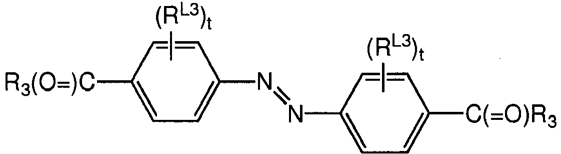 | |
| 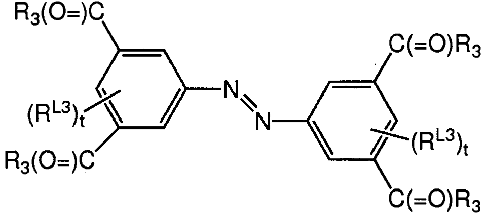 | |
| 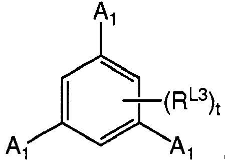 | où A₁, A₂ et A₃ représentent |
| indépendamment | 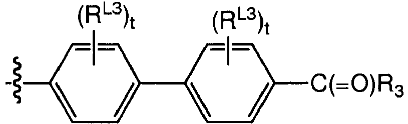 |
dans lesquels :
R₃ est choisi dans le groupe constitué d'un radical - OH, un radical -OY où Y représente un cation de métal alcalin, un halogène, ou un radical -OR⁴, -O-C(=O)R⁴ ou -NR⁴R⁴', où R⁴ et R⁴' sont des radicaux alkyles en C₁₋₁₂,
X₁ représente O ou S,
s représente un entier de 1 à 4,
chaque occurrence de t représente indépendamment un entier de 1 à 4,
u représente un entier de 1 à 7,
R^{L1} et R^{L2} représentent indépendamment H, un halogène ou un alkyle en C₁ à C₆, et
chaque occurrence de R^{L3} représente indépendamment H, un halogène, OH, NH₂, NO₂ ou un alkyle en C₁ à C₆;
b) une étape (iii) de fixation sur ledit solide d'au moins un agent de surface organique choisi dans le groupe comprenant un oligosaccharide, un polysaccharide, un glycosaminoglycanne, un polymère, un tensioactif, les vitamines, les coenzymes, les anticorps ou fragment d'anticorps, les aminoacides ou peptides ;
de façon à obtenir ledit solide.

18. Procédé selon la revendication 17, dans lequel le ligand L' utilisé est un acide di-, tri- ou tétra- carboxylique choisi dans le groupe constitué de: C₂H₂(CO₂H)₂ (acide fumarique), C₂H₄(CO₂H)₂ (acide succinique), C₃H₆(CO₂H)₂ (acide glutarique), C₄H₄(CO₂H)₂ (acide muconique), C₄H₈(CO₂H)₂ (acide adipique), C₇H₁₄(CO₂H)₂ (acide azelaique), C₅H₃S(CO₂H)₂ (acide 2,5-thiophènedicarboxylique), C₆H₄(CO₂H)₂ (acide téréphtalique), C₆H₂N₂(CO₂H)₂ (acide 2,5-pyrazine dicarboxylique), C₁₀H₆(CO₂H)₂ (acide naphtalène-2,6-dicarboxylique), C₁₂H₈(CO₂H)₂ (acide biphényle-4,4'-dicarboxylique), C₁₂H₈N₂(CO₂H)₂ (acide azobenzènedicarboxylique), C₆H₃(CO₂H)₃ (acide benzène-1,2,4-tricarboxylique), C₆H₃(CO₂H)₃ (acide benzène-1,3,5-tricarboxylate), C₂₄H₁₅(CO₂H)₃ (acide benzène-1,3,5-tribenzoïque), C₆H₂(CO₂H)₄ (acide benzène-1,2,4,5-tétracarboxylique, C₁₀H₄(CO₂H)₄ (acide naphtalène-2,3,6,7-tétracarboxylique), C₁₀H₄(CO₂H)₄ (acide naphtalène-1,4,5,8-tétracarboxylique), C₁₂H₆(CO₂H)₄ (acide biphényl-3,5,3',5'-tétracarboxylique), et les analogues modifiés choisis dans le groupe comprenant l'acide 2-aminotéréphtalique, l'acide 2-nitrotéréphtalique, l'acide 2-méthyltéréphtalique, l'acide 2-chlorotéréphtalique, l'acide 2-bromotéréphtalique, l'acide 2,5-dihydroxotéréphtalique, l'acide tétrafluorotéréphtalique, l'acide 2,5-dicarboytéréphtalique, l'acide diméthyl-4,4'-biphénydicarboxylique, l'acide tétraméthyl-4,4'-biphénydicarboxylique, l'acide dicarboxy-4,4'-biphénydicarboxylique, l'acide 2,5-pyrazyne dicarboxylique, l'acide 2,5 diperfluoroterephthalique, l'acide azobenzene 4,4'-dicarboxylique, l'acide 3,3'-dichloro azobenzene 4,4'-dicarboxylique, l'acide 3,3'-dihydroxo azobenzene 4,4'-dicarboxylique, l'acide 3,3'-diperfluoro azobenzene 4,4'-dicarboxylique, l'acide 3,5,3',5'-azobenzene tetracarboxylique, l'acide 2,5-dimethyl terephthalique, l'acide perfluoro glutarique.

19. Procédé de préparation d'un solide selon l'une quelconque des revendications 17 à 18, dans lequel l'étape réactionnelle (i) est réalisée avec au moins une des conditions réactionnelles ci-dessous :
(i) une température de réaction de 0°C à 220°C ;
(ii) une vitesse d'agitation de 0 à 1000 rpm ;
(iii) un temps de réaction de 1 minute à 96 heures ;
(iv) un pH de 0 à 7 ;
(v) l'addition d'au moins un co-solvant au solvant, au précurseur, au ligand ou au mélange de ceux-ci, ledit co-solvant étant choisi dans le groupe comprenant l'acide acétique, l'acide formique, l'acide benzoïque ;
(vi) le solvant est choisi dans le groupe comprenant l'eau, les alcools R^{s}-OH où R^{s} est un radical alkyle en C₁ à C₆ linéaire ou ramifié, le diméthylformamide, diméthylsulfoxide, l'acétonitrile, le tétrahydrofurane, le diéthylformamide, le chloroforme, le cyclohexane, l'acétone, le cyanobenzène, le dichlorométhane, le nitrobenzène, l'éthylèneglycol, le diméthylacétamide ou des mélanges de ces solvants, miscibles ou non ;
(vii) dans un milieu supercritique ;
(viii) sous micro-ondes et/ou sous ultra-sons ;
(ix) dans des conditions d'électrolyse électrochimique ;
(x) dans des conditions utilisant un broyeur à cylindre ;
(xi) dans un flux gazeux.

20. Procédé de préparation d'un solide selon l'une quelconque des revendications 17 à 19, comprenant en outre une étape (ii) d'introduction dans ledit solide d'au moins un principe pharmaceutiquement actif et/ou une substance active entrant dans la formulation d'une préparation cosmétique et/ou marqueur.

21. Solide susceptible d'être obtenu par un procédé selon l'une quelconque des revendications 17 à 20.

22. Utilisation d'un solide selon l'une des revendications 1 à 16 pour la fabrication d'un marqueur utilisable en imagerie médicale.

23. Utilisation d'un solide selon l'une des revendications 10 à 16 comprenant dans ses pores ou à sa surface au moins un principe pharmaceutiquement actif, pour la fabrication d'un médicament.

24. Utilisation d'un solide selon l'une quelconque des revendications 3, 6 et 10 pour la fabrication d'un marqueur utilisable en imagerie à tomograhie par émission de positrons.

## Patentansprüche

1. Isoretikulärer poröser kristalliner MOF-Feststoff mit einer dreidimensionalen Abfolge von Einheiten der folgenden Formel (I):
MₘOₖXₗLₚ Formel (I)
worin:
M jeweils unabhängig für ein Fe²⁺- oder Fe³⁺-Metallion steht;
m, k, l und p Zahlen ≥ 0 sind, die so gewählt sind, dass die Ladungsneutralität der Einheit gewahrt bleibt; m, k, l und p vorzugsweise unabhängig für 0 bis 4 stehen, beispielsweise m und p unabhängig für 1, 2 oder 3 stehen und/oder k und l unabhängig für 0 oder 1 stehen;
X für einen Liganden aus der Gruppe umfassend OH⁻, Cl⁻, F⁻, I⁻, Br⁻, SO₄²⁻, NO₃⁻, ClO₄⁻, R¹-(COO)ₙ⁻, R¹-(SO₃)ₙ⁻, R¹- (PO₃)ₙ⁻ steht, wobei R¹ für Wasserstoff oder ein lineares oder verzweigtes C₁- bis C₈-Alkyl steht und n = 1 bis 6;
L für einen Di-, Tri-, Tetra- oder Hexacarboxylat-Spacerliganden aus der Gruppe bestehend aus:
| | | |
|---|---|---|
| 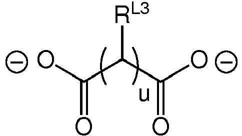 | | 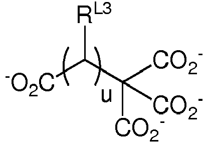 |
| 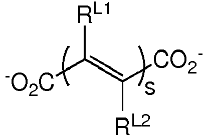 | | 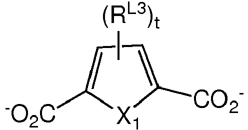 |
| 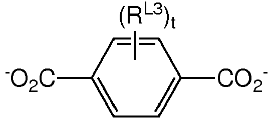 | | 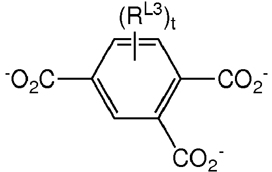 |
| 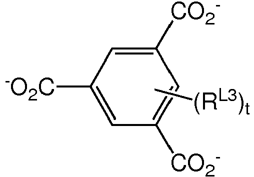 | | 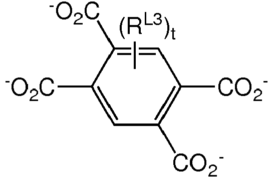 |
| 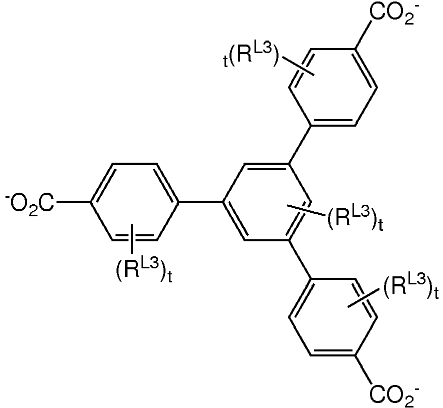 | | 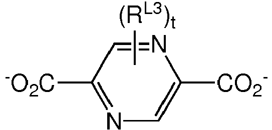 |
| 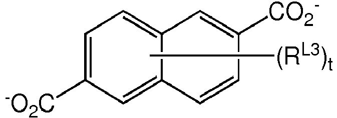 | | 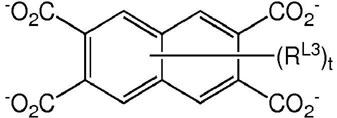 |
| 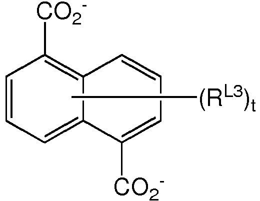 | | 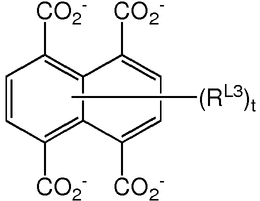 |
| 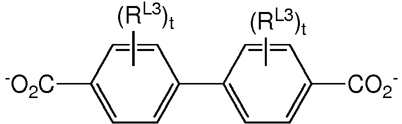 | | |
| 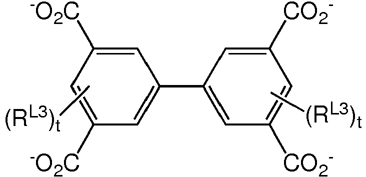 | | |
| 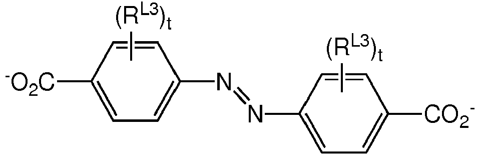 | | |
| 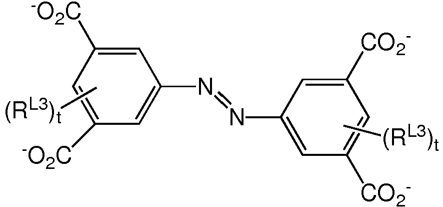 | | |
| 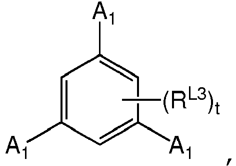 | wobei A₁, A₂ und A₃ unabhängig für | |
| 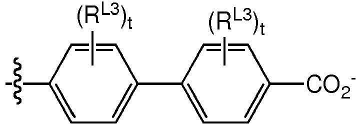 | | stehen, |
steht, wobei:
X₁ für 0 oder S steht,
s für eine ganze Zahl von 1 bis 4 steht,
t jeweils unabhängig für eine ganze Zahl von 1 bis 4 steht,
u für eine ganze Zahl von 1 bis 7 steht,
R^{L1} und R^{L2} unabhängig für H, ein Halogen oder ein C₁- bis C₆-Alkyl stehen und
R^{L3} jeweils unabhängig für H, ein Halogen, OH, NH₂,
NO₂ oder ein C₁- bis C₆-Alkyl steht;
wobei die Oberfläche so modifiziert ist, dass sie mindestens ein organisches Oberflächenmittel aus der Gruppe umfassend ein Oligosaccharid, ein Polysaccharid, ein Glykosaminoglykan, ein Polymer, ein Tensid, ein Vitamin, ein Coenzym, einen Antikörper oder ein Antikörperfragment, eine Aminosäure oder ein Peptid umfasst.

2. Feststoff nach Anspruch 1, wobei es sich bei dem Liganden L um einen Di-, Tri-, Tetra- oder Hexa-carboxylatliganden handelt, der ausgewählt ist aus der Gruppe bestehend aus : C₂H₂(CO₂⁻)₂ (Fumarat), C₂H₄(CO₂⁻)₂ (Succinat), C₃H₆(CO₂⁻)₂ (Glutarat), C₄H₄(CO₂⁻)₂ (Muconat), C₄H₈(CO₂⁻)₂ (Adipat), C₇H₁₄(CO₂⁻)₂ (Azelat), C₅H₃S(CO₂⁻)₂ (2, 5-Thiophendicarboxylat), C₆H₄(CO₂⁻)₂ (Terephthalat), C₆H₂N₂(CO₂⁻)₂ (2,5-Pyrazindicarboxylat), C₁₀H₆(C⁻₂⁻)₂ (Naphthalin-2,6-dicarboxylat), C₁₂H₈(CO₂⁻)₂ (Biphenyl-4,4'-dicarboxylat), C₁₂H₈N₂(CO₂⁻)₂ (Azobenzoldicarboxylat), C₆H₃(CO₂⁻)₃ (Benzol-1,2,4-tricarboxylat), C₆H₃(CO₂⁻)₃ (Benzol-1,3,5-tricarboxylat), C₂₄H₁₅(CO₂⁻)₃ (Benzol-1,3,5-tribenzoat), C₆H₂(CO₂⁻)₄ (Benzol-1,2,4,5-tetracarboxylat), C₁₀H₄(CO₂⁻ ) ₄ (Naphthalin-2,3,6,7-tetracarboxylat), C₁₀H₄(CO₂⁻)₄ (Naphthalin-1,4,5,8-tetracarboxylat), C₁₂H₆(CO₂⁻)₄ (Biphenyl-3,5,3',5'-tetracarboxylat) und modifizierten Analogen aus der Gruppe umfassend 2-Aminoterephthalat, 2-Nitroterephthalat, 2-Methylterephthalat, 2-Chlorterephthalat, 2-Bromoterephthalat, 2,5-Dihydroxoterephthalat, Tetrafluorterephthalat, Tetramethylterephthalat, Dimethyl-4,4'-biphenyldicarboxylat, Tetramethyl-4,4'-biphenyldicarboxylat, Dicarboxy-4,4'-biphenyldicarboxylat, 2,5-Pyrazindicarboxylat, 2,5-Diperfluorterephthalat, Azobenzol-4,4'-dicarboxylat, 3,3'-Dichlorazobenzol-4,4'-dicarboxylat, 3,3'-Dihydroxoazobenzol-4,4'-dicarboxylat, 3,3'-Diperfluorazobenzol-4,4'-dicarboxylat, 3,5,3',5'-Azobenzoltetracarboxylat, 2,5-Dimethylterephthalat, Perfluorsuccinat, Perfluormuconat, Perfluorglutarat, 3,5,3',5'-Perfluor-4,4'-azobenzoldicarboxylat, 3,3'-Diperfluorazobenzol-4,4'-dicarboxylat.

3. Feststoff nach einem der Ansprüche 1 bis 2, wobei es sich bei dem Liganden L um einen fluorhaltigen Liganden handelt, der ausgewählt ist aus der Gruppe bestehend aus Tetrafluorterephthalat, Perfluorsuccinat, Perfluormuconat, Perfluorglutarat, 2,5-Diperfluorterephthalat, 3,6-Perfluor-1,2,4,5-benzoltetracarboxylat, 3,5,3',5'-Perfluor-4,4'-azobenzoldicarboxylat, 3,3'-Diperfluorazobenzol-4,4'-dicarboxylat.

4. Feststoff nach einem der Ansprüche 1 bis 2, wobei es sich bei dem Liganden L um einen biologisch aktiven Liganden handelt, der ausgewählt ist aus der Gruppe bestehend aus C₇H₁₄(CO₂⁻)₂; Aminosalicylat; Chlodronat, Pamidrontat, Alendronat, Etidronat; Meprobamat; Porphyrinen mit Carboxylat-, Phosphonat- und/oder Aminogruppen; Aminosäuren; Azobenzolen mit Carboxylat-, Phosphonat- und/oder Aminogruppen; Dibenzofuran-4,6-dicarboxylat, Dipicolinat; Glutamat, Fumarat, Succinat, Suberat, Adipat, Nicotinat, Nicotinamid, Purinen, Pyrimidinen.

5. Feststoff nach einem der Ansprüche 1 bis 4, wobei der Ligand X ausgewählt ist aus der Gruppe bestehend aus OH⁻, Cl⁻, F⁻, CH₃-COO⁻, PF₆⁻, ClO₄⁻.

6. Feststoff nach einem der Ansprüche 1 bis 4, wobei mindestens einer der Liganden X für ¹⁸F⁻ steht.

7. Feststoff nach einem der Ansprüche 1 bis 6, wobei der Feststoff in Form von Nanopartikeln vorliegt.

8. Feststoff nach einem der Ansprüche 1 bis 7, wobei das organische Oberflächenmittel ausgewählt ist aus der Gruppe bestehend aus einem Oligosaccharid, einem Polysaccharid, Chitosan, Dextran, Hyaluronsäure, Heparin, Fucoidan, Alginat, Pektin, Amylose, Cyclodextrinen, Stärke, Cellulose, Xylan, Polyethylenglykol (PEG), Pluronic, Polyvinylalkohol, Polyethylenimin.

9. Feststoff nach einem der Ansprüche 1 bis 7, wobei es sich bei dem organischen Oberflächenmittel um ein Zielsteuerungsmolekül handelt, das ausgewählt ist aus der Gruppe bestehend aus: Biotin, Folsäure, Liponsäure, Ascorbinsäure, einem Antikörper oder Antikörperfragment, einem Peptid.

10. Feststoff nach einem der Ansprüche 1 bis 9, wobei der Feststoff in seinen Poren oder an seiner Oberfläche mindestens einen pharmazeutischen Wirkstoff und/oder eine Wirksubstanz, die in die Formulierung einer kosmetischen Zubereitung Eingang findet, und/oder einen Marker umfasst.

11. Feststoff nach einem der Ansprüche 1 bis 10, wobei der pharmazeutische Wirkstoff ausgewählt ist aus der Gruppe bestehend aus: Taxotere, Busulfan, Azidothymidin (AZT), Azidothymidinphosphat (AZTP), Cidofovir, Gemcitabin, Tamoxifen.

12. Feststoff nach einem der Ansprüche 1 bis 11, wobei der Feststoff in seinen Poren oder an seiner Oberfläche mindestens ein fluoreszierendes Molekül umfasst, das ausgewählt ist aus der Gruppe bestehend aus: Rhodaminen, Fluorescein, Luciferase, Pyren und Derivaten und Aminopyrrolidino-7-nitrobenzofurazan.

13. Feststoff nach einem der Ansprüche 1 bis 11, wobei der Feststoff in seinen Poren oder an seiner Oberfläche mindestens einen pharmazeutischen Wirkstoff mit einer Beladungskapazität von 1 bis 200 Gew.-%, bezogen auf den trockenen Feststoff, umfasst.

14. Feststoff nach einem der Ansprüche 1 bis 13, wobei der Feststoff in seinen Poren oder an seiner Oberfläche mindestens eine Wirksubstanz, die in die Formulierung einer kosmetischen Zubereitung Eingang findet, aus der Gruppe bestehend aus Benzophenon, Visnadin und Salicylsäure umfasst.

15. Feststoff nach einem der Ansprüche 1 bis 14, wobei der Feststoff in seinen Poren oder an seiner Oberfläche mindestens einen Marker umfasst, wobei dieser ausgewählt ist aus der Gruppe umfassend einen Marker für die medizinische Bildgebung, ein Kontrastmittel, einen Tracer, einen radioaktiven Marker, einen Fluoreszenzmarker, einen Phosphoreszenzmarker.

16. Feststoff nach Anspruch 15, wobei der Marker ausgewählt ist aus der Gruppe bestehend aus: einer fluoreszierenden Verbindung, einem Eisenoxid, einem Gadoliniumkomplex, direkt in der Struktur vorliegenden Gadoliniumionen.

17. Verfahren zur Herstellung eines Feststoffs gemäß einem der Ansprüche 1 bis 16, umfassend:
a) mindestens einen Reaktionsschritt (i), der daraus besteht, dass man in einem polaren Lösungsmittel Folgendes mischt:
- mindestens eine Lösung, die mindestens eine anorganische Metallvorstufe in Form eines Metalls M, eines Salzes eines Metalls M oder eines Koordinationskomplexes mit einem Metallion M umfasst, wobei M wie in Anspruch 1 definiert ist;
- mindestens einen zwei-, drei-, vier- oder sechszähnigen Liganden L' aus der Gruppe bestehend aus:
| | | |
|---|---|---|
| 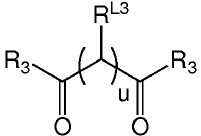 | | 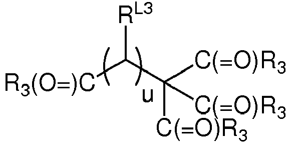 |
| 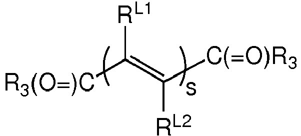 | | 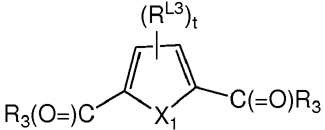 |
| 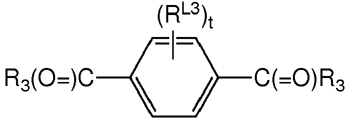 | | 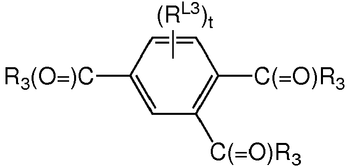 |
| 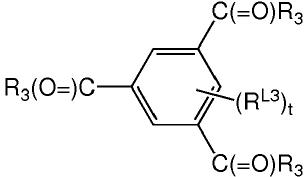 | | 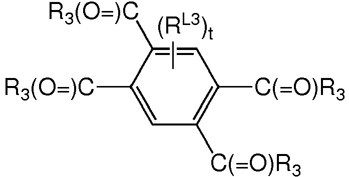 |
| 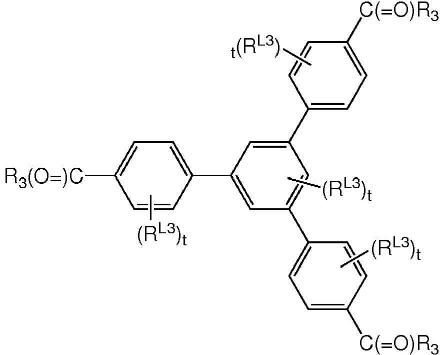 | 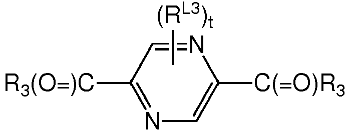 | |
| 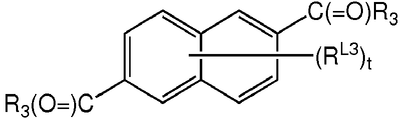 | 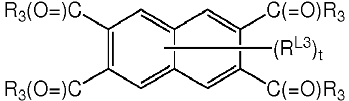 | |
| 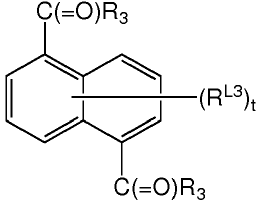 | 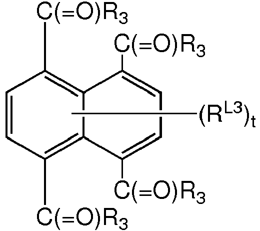 | |
| 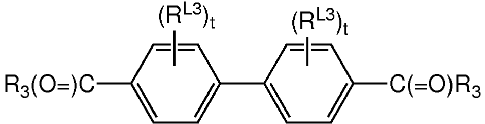 | | |
| 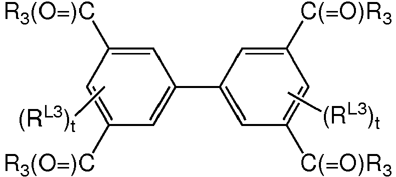 | | |
| 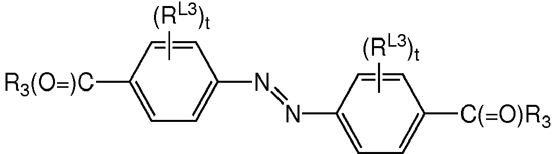 | | |
| 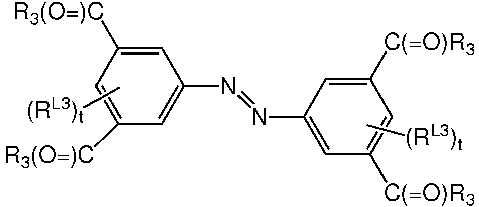 | | |
| 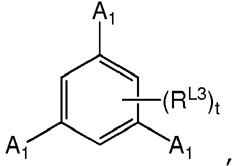 | wobei A₁, A₂ und A₃ unabhängig für | |
| 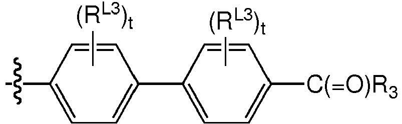 | | stehen; |
wobei:
R₃ ausgewählt ist aus der Gruppe bestehend aus einem-OH-Rest, einem -OY-Rest, worin Y für ein Alkalimetallkation, ein Halogen, oder einen -OR⁴-, -0-C(=O)R⁴- oder -NR⁴R⁴-Rest, worin R⁴ und R⁴' C₁₋₁₂-Alkylreste bedeuten, steht,
X₁ für 0 oder S steht,
s für eine ganze Zahl von 1 bis 4 steht,
t jeweils unabhängig für eine ganze Zahl von 1 bis 4 steht,
u für eine ganze Zahl von 1 bis 7 steht,
R^{L1} und R^{L2} unabhängig für H, ein Halogen oder ein C₁-bis C₆-Alkyl stehen und
R^{L3} jeweils unabhängig für H, ein Halogen, OH, NH₂, NO₂ oder ein C₁- bis C₆-Alkyl steht;
b) einen Schritt (iii) des Fixierens mindestens eines organischen Oberflächenmittels aus der Gruppe umfassend ein Oligosaccharid, ein Polysaccharid, ein Glykosaminoglykan, ein Polymer, ein Tensid, Vitamine, Coenzyme, Antikörper oder Antikörperfragmente, Aminosäuren oder Peptide auf dem Feststoff;
wodurch man den Feststoff erhält.

18. Verfahren nach Anspruch 17, bei dem es sich bei dem verwendeten Liganden L' um eine Di-, Tri- oder Tetracarbonsäure handelt, die ausgewählt ist aus der Gruppe bestehend aus: C₂H₂(CO₂H)₂ (Fumarsäure), C₂H₄(CO₂H)₂ (Bernsteinsäure), C₃H₆(CO₂H)₂ (Glutarsäure), C₄H₄(CO₂H)₂ (Muconsäure), C₄H₈(CO₂H)₂ (Adipinsäure), C₇H₁₄(CO₂H)₂ (Azelainsäure), C₅H₃S(CO₂H)₂ (2,5-Thiophendicarbonsäure), C₆H₄(CO₂H)₂ (Terephthalsäure), C₆H₂N₂(CO₂H)₂ (2, 5-Pyrazindicarbonsäure), C₁₀H₆(CO₂H)₂ (Naphthalin-2,6-dicarbonsäure), C₁₂H₈(CO₂H)₂ (Biphenyl-4,4'-dicarbonsäure), C₁₂H₈N₂(CO₂H)₂ (Azobenzoldicarbonsäure), C₆H₃(CO₂H)₃ (Benzol-1,2,4-tricarbonsäure), C₆H₃(CO₂H)₃ (Benzol-1, 3, 5-tricarbonsäure), C₂₄H₁₅(CO₂H)₃ (Benzol-1,3,5-tribenzoesäure), C₆H₂(CO₂H)₄ (Benzol-1,2,4,5-tetracarbonsäure), C₁₀H₄(CO₂H)₄ (Naphthalin-2,3,6,7-tetracarbonsäure), C₁₀H₄(CO₂H)₄ (Naphthalin-1, 4, 5, 8-tetracarbonsäure), C₁₂H₆(CO₂H)₄ (Biphenyl-3,5,3',5'-tetracarbonsäure) und modifizierten Analogen aus der Gruppe, umfassend 2-Aminoterephthalsäure, 2-Nitroterephthalsäure, 2-Methylterephthalsäure, 2-Chlorterephthalsäure, 2-Bromterephthalsäure, 2,5-Dihydroxoterephthalsäure, Tetrafluorterephthalsäure, 2,5-Dicarboxyterephthalsäure, Dimethyl-4,4'-biphenyldicarbonsäure, Tetramethyl-4,4'-biphenyldicarbonsäure, Dicarboxy-4,4'-biphenyldicarbonsäure, 2,5-Pyrazindicarbonsäure, 2,5-Diperfluorterephthalsäure, Azobenzol-4,4'-dicarbonsäure, 3,3'-Dichlorazobenzol-4,4'-dicarbonsäure, 3,3'-Dihydroxoazobenzol-4,4'-dicarbonsäure, 3,3'-Diperfluorazobenzol-4,4'-dicarbonsäure, 3,5,3',5'-Azobenzoltetracarbonsäure, 2,5-Dimethylterephthalsäure, Perfluorglutarsäure.

19. Verfahren zur Herstellung eines Feststoffs nach einem der Ansprüche 17 bis 18, bei dem man den Reaktionsschritt (i) mit mindestens einer der nachstehenden Rekationsbedingungen durchführt:
(i) einer Reaktionstemperatur von 0°C bis 220°C;
(ii) einer Rührgeschwindigkeit von 0 bis 1000 U/min;
(iii) einer Reaktionszeit von 1 Minute bis 96 Stunden;
(iv) einem pH-Wert von 0 bis 7;
(v) der Zugabe mindestens eines Cosolvens zu dem Lösungsmittel, der Vorstufe, dem Liganden oder der Mischung davon, wobei das Cosolvens aus der Gruppe umfassend Essigsäure, Ameisensäure und Benzoesäure ausgewählt wird;
(vi) Wahl des Lösungsmittels aus der Gruppe umfassend Wasser, Alkohole R^{S}-OH, wobei R^{S} für einen linearen oder verzweigten C₁- bis C₆-Alkylrest steht, Dimethylformamid, Dimethylsulfoxid, Acetonitril, Tetrahydrofuran, Diethylformamid, Chloroform, Cyclohexan, Aceton, Cyanobenzol, Dichlormethan, Nitrobenzol, Ethylenglykol, Dimethylacetamid oder Mischungen dieser Lösungsmittel, die mischbar oder nicht mischbar sein können;
(vii) in einem überkritischen Medium;
(viii) unter Mikrowellen und/oder unter Ultraschall;
(ix) unter den Bedingungen einer elektrochemischen Elektrolyse;
(x) unter Bedingungen unter Verwendung eines Walzwerks;
(xi) in einem Gasstrom.

20. Verfahren zur Herstellung eines Feststoffs nach einem der Ansprüche 17 bis 19, das außerdem einen Schritt (ii) umfasst, bei dem man in den Feststoff mindestens einen pharmazeutischen Wirkstoff und/oder eine Wirksubstanz, die in die Formulierung einer kosmetischen Zubereitung Eingang findet, und/oder einen Marker einträgt.

21. Feststoff, der nach einem Verfahren nach einem der Ansprüche 17 bis 20 erhältlich ist.

22. Verwendung eines Feststoffs nach einem der Ansprüche 1 bis 16 zur Herstellung eines Markers, der bei der medizinischen Bildgebung verwendet werden kann.

23. Verwendung eines Feststoffs nach einem der Ansprüche 10 bis 16, der in seinen Poren oder an seiner Oberfläche mindestens einen pharmazeutischen Wirkstoff umfasst, zur Herstellung eines Medikaments.

24. Verwendung eines Feststoffs nach einem der Ansprüche 3, 6 und 10 zur Herstellung eines Markers, der bei der positronenemissionstomographischen Bildgebung verwendet werden kann.

## Claims

1. Isoreticular porous crystalline MOF solid comprising a three-dimensional succession of units having formula (I) below:
MₘOₖXₗLₚ Formula (I)
in which:
- each occurrence of M independently represents a Fe²⁺ or Fe³⁺ metal ion;
- m, k, 1 and p are numbers ≥ 0 chosen so as to respect the charge neutrality of the unit; preferably, m, k, 1 and p are independently 0 to 4, for example m and p are independently 1, 2 or 3 and/or k and 1 are independently 0 or 1;
- X is a ligand chosen from the group comprising OH⁻, Cl⁻, F⁻, I⁻, Br⁻, SO₄²⁻, NO₃⁻, ClO₄⁻, R¹-(COO)ₙ⁻, R¹-(SO₃)ₙ⁻, R¹-(PO₃)ₙ⁻, in which R¹ is a hydrogen atom, a linear or branched C₁ to C₈ alkyl, n = 1 to 6;
- L is a di-, tri-, tetra- or hexacarboxylate spacer chosen from the group consisting of:
| | | |
|---|---|---|
| 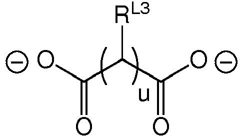 | | 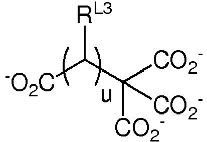 |
| 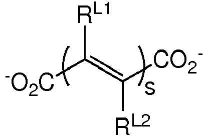 | | 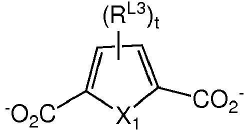 |
| 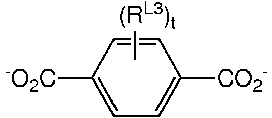 | | 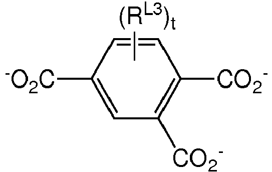 |
| 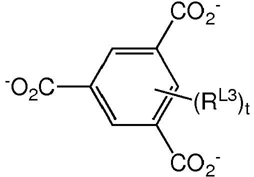 | | 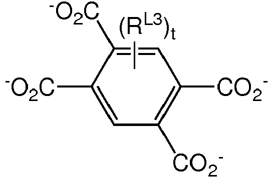 |
| 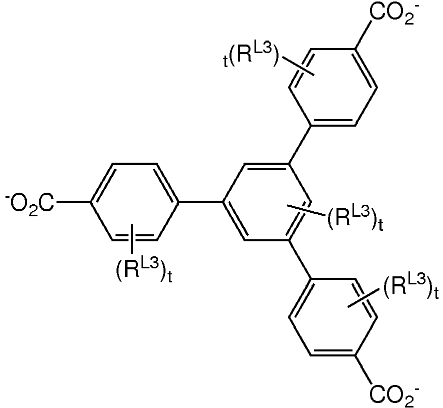 | | 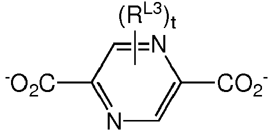 |
| 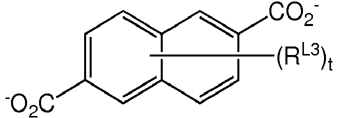 | | 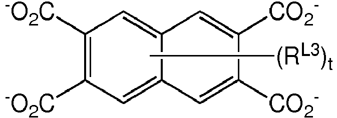 |
| 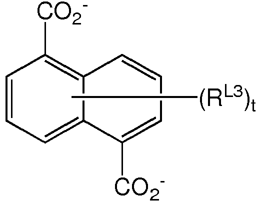 | | 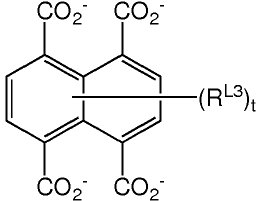 |
| 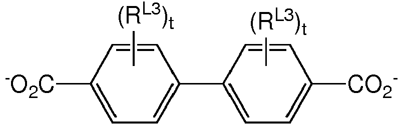 | | |
| 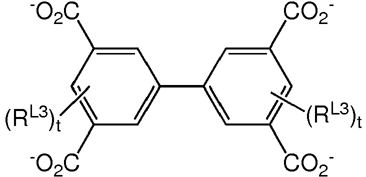 | | |
| 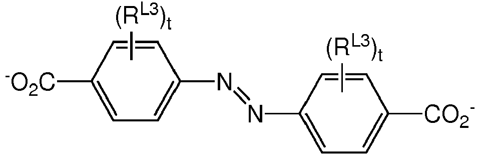 | | |
| 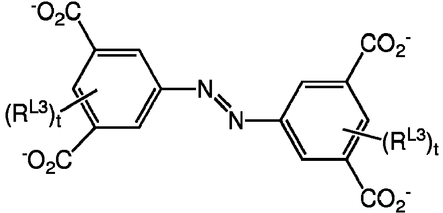 | | |
| 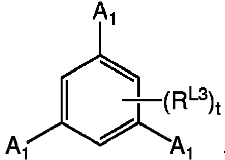 | | wherein A₁, A₂ and A₃ independently |
| represent | 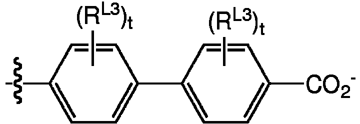 | |
in which:
X₁ represents O or S,
s represents an integer from 1 to 4,
each occurrence of t independently represents an integer from 1 to 4,
u represents an integer from 1 to 7,
R^{L1} and R^{L2} independently represent H, a halogen or a C₁ to C₆ alkyl, and
each occurrence of R^{L3} independently represents H, a halogen, OH, NH₂, NO₂ or a C₁ to C₆ alkyl;
in which the surface is modified so that it comprises at least one organic surface agent chosen from the group comprising an oligosaccharide, a polysaccharide, a glycosaminoglycan, a polymer, a surfactant, a vitamin, a coenzyme, an antibody or antibody fragment, an amino acid and a peptide.

2. Solid as claimed in claim 1, in which the ligand L is a di-, tri- or tetracarboxylate ligand chosen from the group consisting of: C₂H₂(CO₂⁻)₂ (fumarate), C₂H₄(CO₂⁻)₂ (succinate), C₃H₆(CO₂⁻)₂ (glutarate), C₄H₄(CO₂⁻)₂ (muconate), C₄H₈(CO₂⁻)₂ (adipate), C₇H₁₄(CO₂⁻)₂ (azelate), C₅H₃S(CO₂⁻)₂ (2,5-thiophenedicarboxylate), C₆H₄(CO₂⁻)₂ (terephthalate), C₆H₂N₂(CO₂⁻)₂ (2,5-pyrazinedicarboxylate), C₁₀H₆(CO₂⁻)₂ (naphthalene-2,6-dicarboxylate), C₁₂H₈(CO₂⁻)₂ (biphenyl-4,4'-dicarboxylate), C₁₂H₈N₂(CO₂⁻)₂ (azobenzenedicarboxylate), C₆H₃(CO₂⁻)₃ (benzene-1,2,4-tricarboxylate), C₆H₃(CO₂⁻)₃ (benzene-1,3,5-tricarboxylate), C₂₄H₁₅(CO₂⁻)₃ (benzene-1,3,5-tribenzoate), C₆H₂(CO₂⁻)₄ (benzene-1,2,4,5-tetracarboxylate), C₁₀H₄(CO₂⁻)₄ (naphthalene-2,3,6,7-tetra-carboxylate), C₁₀H₄(CO₂⁻)₄ (naphthalene-1,4,5,8-tetra-carboxylate), C₁₂H₆(CO₂⁻)₄ (biphenyl-3,5,3',5'-tetracarboxylate), and modified analogs chosen from the group comprising 2-aminoterephthalate, 2-nitroterephthalate, 2-methylterephthalate, 2-chloroterephthalate, 2-bromoterephthalate, 2,5-dihydroxoterephthalate, tetrafluoroterephthalate, tetramethylterephthalate, dimethyl-4,4'-biphenyldicarboxylate, tetramethyl-4,4'-biphenyldicarboxylate, dicarboxy-4,4'-biphenyl-dicarboxylate, 2,5-pyrazinedicarboxylate 2,5-diperfluoroterephthalate, azobenzene-4,4'-dicarboxylate, 3,3'-dichloroazobenzene-4,4'-dicarboxylate, 3,3'-dihydroxoazobenzene-4,4'-dicarboxylate, 3,3'-diperfluoroazobenzene-4,4'-dicarboxylate, 3,5,3',5'-azobenzenetetracarboxylate, 2,5-dimethylterephthalate, perfluorosuccinate, perfluoromuconate, perfluoroglutarate, 3,5,3',5'-perfluoro-4,4'-azobenzenedicarboxylate, 3,3'-diperfluoroazobenzene-4,4'-dicarboxylate.

3. Solid as claimed in any one of claims 1 to 2, in which the ligand L is a fluoro ligand chosen from the group consisting of tetrafluoroterephthalate, perfluorosuccinate, perfluoromuconate, perfluoroglutarate, 2,5-diperfluoroterephthalate, 3,6-perfluoro-1,2,4,5-benzenetetracarboxylate, 3,5,3',5'-perfluoro-4,4'-azobenzenedicarboxylate or 3,3'-diperfluoroazobenzene-4,4'-dicarboxylate.

4. Solid as claimed in any one of claims 1 to 2, in which the ligand L is a biologically active ligand chosen from the group consisting of C₇H₁₄(CO₂⁻)₂; aminosalicylate; chlodronate, pamidrontate, alendronate, etidronate; meprobamate; porphyrins comprising carboxylate, phosphonate and/or amino groups; amino acids; azobenzenes comprising carboxylate, phosphonate and/or amino groups; dibenzofuran-4,6-dicarboxylate, dipicolinate; glutamate, fumarate, succinate, suberate, adipate, nicotinate, nicotinamide, purines, pyrimidines.

5. Solid as claimed in any one of claims 1 to 4, in which the ligand X is chosen from the group consisting of OH⁻, Cl⁻, F⁻, CH₃-COO⁻, PF₆⁻ and ClO₄⁻.

6. Solid as claimed in any one of claims 1 to 4, in which at least one occurrence of the ligand X is ¹⁸F⁻.

7. Solid as claimed in any one of claims 1 to 6, said solid being in the form of nanoparticles.

8. Solid as claimed in any one of claims 1 to 7, in which the organic surface agent is chosen from the group consisting of an oligosaccharide, a polysaccharide, chitosan, dextran, hyaluronic acid, heparin, fucoidan, alginate, pectin, amylose, cyclodextrins, starch, cellulose, xylan, polyethylene glycol (PEG), Pluronic, polyvinyl alcohol and polyethyleneimine.

9. Solid as claimed in any one of claims 1 to 7, in which the organic surface agent is a targeting molecule chosen from the group consisting of: biotin, folic acid, lipoic acid, ascorbic acid, an antibody or antibody fragment, and a peptide.

10. Solid as claimed in any one of claims 1 to 9, said solid comprising in its pores or at its surface at least one pharmaceutically active principle and/or one compound of cosmetic interest and/or one marker.

11. Solid as claimed in any one of claims 1 to 10, in which the pharmaceutically active principle is chosen from the group consisting of: taxotere, busulfan, azidothymidine (AZT), azidothymidine phosphate (AZTP), cidofovir, gemcitabine, tamoxifen.

12. Solid as claimed in any one of claims 1 to 11, said solid comprising in its pores or at its surface at least one fluorescent molecule chosen from the group consisting of: rhodamines, fluorescein, luciferase, pyrene and derivatives, and aminopyrrolidino-7-nitrobenzofurazan.

13. Solid as claimed in any one of claims 1 to 11, said solid comprising in its pores or at its surface at least one pharmaceutically active principle with a capacity with a loading capacity of from 1% to 200% by weight of dry solid.

14. Solid as claimed in any one of claims 1 to 13, said solid comprising in its pores or at its surface at least one compound of cosmetic interest chosen from the group comprising benzophenone, visnadine and salicylic acid.

15. Solid as claimed in any one of claims 1 to 14, said solid comprising in its pores or at its surface at least one marker, said marker being chosen from the group comprising a medical imaging marker, a contrast agent, a tracer, a radioactive marker, a fluorescent marker and a phosphorescent marker.

16. Solid as claimed in claim 15, in which the marker is chosen from the group consisting of: a fluorescent compound, an iron oxide, a gadolinium complex, gadolinium ions directly present in the structure.

17. A process for preparing a solid as defined in any one of claims 1 to 16, comprising:
a) at least one reaction step (i) that consists in mixing in a polar solvent:
- at least one solution comprising at least one metallic inorganic precursor in the form of a metal M, a salt of a metal M or a coordination complex comprising the metal ion M in which M is as defined in claim 1,
- at least one di-, tri-, tetra- or hexadentate ligand L' selected from the group consisting of:
| | | | |
|---|---|---|---|
| 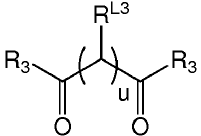 | | | 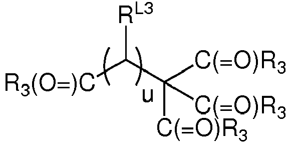 |
| 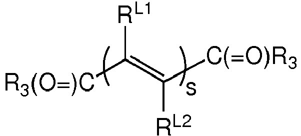 | | | 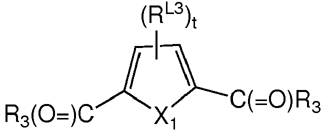 |
| 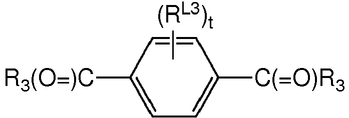 | | | 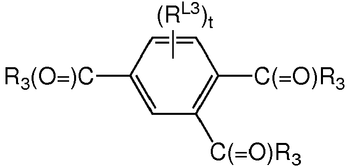 |
| 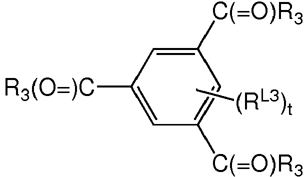 | | | 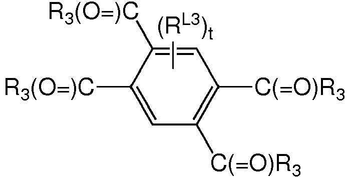 |
| 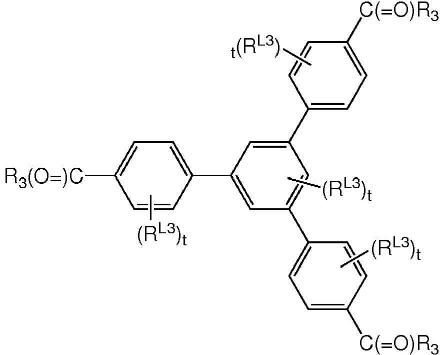 | | | 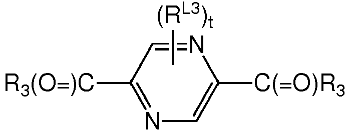 |
| 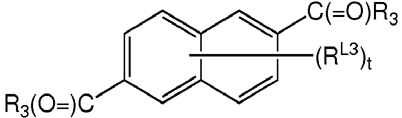 | | | 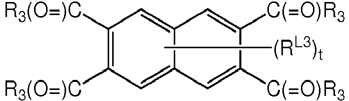 |
| 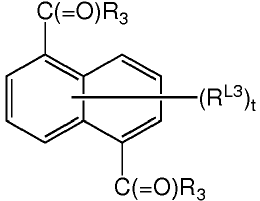 | | | 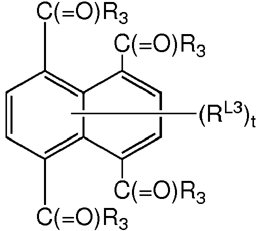 |
| 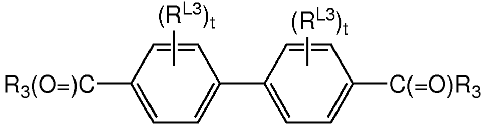 | | | |
| 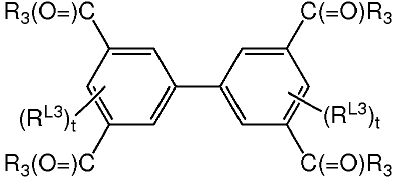 | | | |
| 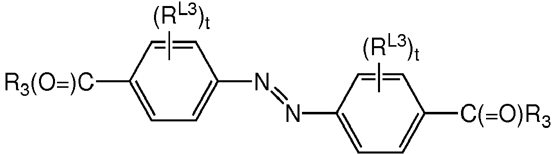 | | | |
| 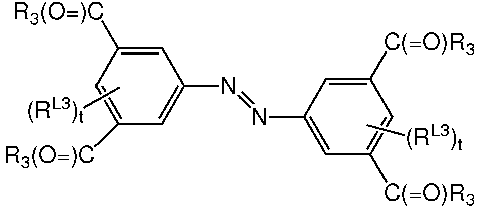 | | | |
| 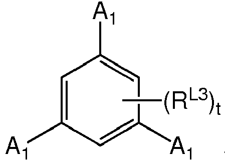 | | wherein A₁, A₂ et A₃ independently | |
| represent | 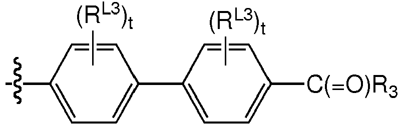 | | |
in which R₃ is selected from the group consisting of a radical -OH, a radical -OY in which Y represents an alkali metal cation, a halogen atom or a radical -OR⁴, -O-C(=O)R⁴ or -NR⁴R⁴', in which R⁴ and R⁴' are C₁₋₁₂ alkyl radicals,
X₁ represents O or S,
s represents an integer from 1 to 4,
each occurrence of t independently represents an integer from 1 to 4,
u represents an integer from 1 to 7,
R^{L1} and R^{L2} independently represent H, a halogen or a C₁ to C₆ alkyl, and
each occurrence of R^{L3} independently represents H, a halogen, OH, NH₂, NO₂ or a C₁ to C₆ alkyl;
b) a step (iii) of binding to said solid at least one organic surface agent chosen from the group comprising an oligosaccharide, a polysaccharide, a glycosaminoglycan, a polymer, a surfactant, vitamins, coenzymes, antibodies or antibody fragments, amino acids and peptides;
so as to obtain said solid.

18. Process as claimed in claim 17, in which the ligand L' used is a di-, tri- or tetracarboxylic acid chosen from the group consisting of: C₂H₂(CO₂H)₂ (fumaric acid), C₂H₄(CO₂H)₂ (succinic acid), C₃H₆(CO₂H)₂ (glutaric acid), C₄H₄(CO₂H)₂ (muconic acid), C₄H₈(CO₂H)₂ (adipic acid), C₇H₁₄(CO₂H)₂ (azelaic acid), C₅H₃S(CO₂H)₂ (2,5-thiophenedicarboxylic acid), C₆H₄(CO₂H)₂ (terephthalic acid), C₆H₂N₂(CO₂H)₂ (2,5-pyrazinedicarboxylic acid), C₁₀H₆(CO₂H)₂ (naphthalene-2,6-dicarboxylic acid), C₁₂H₈(CO₂H)₂ (biphenyl-4,4'-dicarboxylic acid), C₁₂H₈N₂(CO₂H)₂ (azobenzenedicarboxylic acid), C₆H₃(CO₂H)₃ (benzene-1,2,4-tricarboxylic acid), C₆H₃(CO₂H)₃ (benzene-1,3,5-tricarboxylic acid), C₂₄H₁₅(CO₂H)₃ (benzene-1,3,5-tribenzoic acid), C₆H₂(CO₂H)₄ (benzene-1, 2, 4, 5-tetracarboxylic acid), C₁₀H₄(CO₂H)₄ (naphthalene-2,3,6,7-tetra-carboxylic acid), C₁₀H₄(CO₂H)₄ (naphthalene-1,4,5,8-tetracarboxylic acid), C₁₂H₆(CO₂H)₄ (biphenyl-3,5,3',5'-tetracarboxylic acid), and modified analogs chosen from the group comprising 2-aminoterephthalic acid, 2-nitro-terephthalic acid, 2-methylterephthalic acid, 2-chloro-terephthalic acid, 2-bromoterephthalic acid, 2,5-dihydroxoterephthalic acid, tetrafluoroterephthalic acid, 2,5-dicarboxyterephthalic acid, dimethyl-4,4'-biphenyldicarboxylic acid, tetramethyl-4,4'-biphenyl-dicarboxylic acid, dicarboxy-4,4'-biphenyldicarboxylic acid, 2,5-pyrazinedicarboxylic acid, 2,5-diperfluoroterephthalic acid, azobenzene-4,4'-dicarboxylic acid, 3,3'-dichloroazobenzene-4,4'-dicarboxylic acid, 3,3'-dihydroxoazobenzene-4,4'-dicarboxylic acid, 3,3'-diperfluoroazobenzene-4,4'-dicarboxylic acid, 3,5,3',5'-azobenzenetetracarboxylic acid, 2,5-dimethylterephthalic acid, perfluoroglutaric acid.

19. Process for preparing a solid as claimed in any one of claims 17 to 18, in which the reaction step (i) is performed with at least one of the reaction conditions below:
(i) a reaction temperature from 0°C to 220°C;
(ii) a stirring speed from 0 to 1000 rpm;
(iii) a reaction time from 1 minute to 96 hours;
(iv) a pH from 0 to 7;
(v) the addition of at least one cosolvent to the solvent, to the precursor, to the ligand or to the mixture thereof, said cosolvent being chosen from the group comprising acetic acid, formic acid and benzoic acid;
(vi) the solvent is chosen from the group comprising water, alcohols R^{S}-OH in which R^{S} is a linear or branched C₁ to C₆ alkyl radical, dimethylformamide, dimethyl sulfoxide, acetonitrile, tetrahydrofuran, diethylformamide, chloroform, cyclohexane, acetone, cyanobenzene, dichloromethane, nitrobenzene, ethylene glycol, dimethylacetamide, or mixtures of these solvents, which may be miscible or immiscible;
(vii) in a supercritical medium;
(viii) under microwaves and/or under ultrasound;
(ix) under electrochemical electrolysis conditions;
(x) under conditions using a roll mill;
(xi) in a gas stream.

20. Process for preparing a solid as claimed in any one of claims 17 to 19, further comprising a step (ii) of introducing into said solid at least one pharmaceutically active principle and/or a compound of cosmetic interest and/or marker.

21. A solid obtainable via a process as claimed in any one of claims 17 to 20.

22. Use of a solid as claimed in one of claims 1 to 16, for the manufacture of a marker that may be used in medical imaging.

23. Use of a solid as claimed in one of claims 10 to 16, comprising in its pores or at its surface at least one pharmaceutically active principle, for the manufacture of a medicament.

24. Use of a solid as claimed in any one of claims 3, 6 and 10, for the manufacture of a marker that may be used in positron emission tomography imaging.
